# EUROPEAN PATENT SPECIFICATION

(11) **EP 4 259 627 B1**
(45) Date of publication and mention of the grant of the patent: **20.08.2025**
(21) Application number: 21816500.9
(22) Date of filing: 03.12.2021
(51) Int. Cl.: C07D 487/04, A01N 43/90

(54) **SULFOXIMINE PESTICIDES**
SULFOXIMINPESTIZIDE
PESTICIDES DE SULFOXIMINE

(30) Priority: 14.12.2020 IN 202021054384; 25.01.2021 EP 21153132
(43) Date of publication of application: 18.10.2023
(73) Proprietor: BASF SE, 67056 Ludwigshafen am Rhein (DE)
(72) Inventor: SHAIKH, Rizwan Shabbir, Mumbai 400705 (IN); SCHROEDER, Birte, 67056 Ludwigshafen (DE); MAITY, Pulakesh, Mumbai 400705 (IN); ADISECHAN, Ashokkumar, Mumbai 400705 (IN); CHAUDHURI, Rupsha, Mumbai 400705 (IN); SAMBASIVAN, Sunderraman, Mumbai 400705 (IN)
(74) Representative: BASF IP Association
(86) International application number: PCT/EP2021/084092
(87) International publication number: WO 2022/128524

(56) References cited:
- WO-A1-2017/167832
- WO-A1-2018/099812
- WO-A1-2018/202540
- WO-A1-2019/076778

## Description

The invention relates to compounds of formula (I) or an agrochemically or verterinarily acceptable salt, stereoisomer, tautomer, or N-oxide thereof 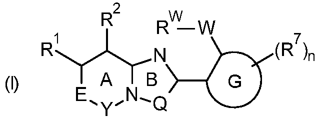 wherein the variables are as defined below. The invention also relates to the use of compounds of formula (I) as an agrochemical pesticide; to pesticidal mixtures comprising a compound of formula (I) and another pesticidal ingredient; to a method for combating or controlling invertebrate pests, which method comprises contacting said pest or its food supply, habitat or breeding grounds with a pesticidally effective amount of at least one compound of the formula (I) the pesticidal mixture; to a method for protecting growing plants from attack or infestation by invertebrate pests, which method comprises contacting a plant, or soil or water in which the plant is growing, with a pesticidally effective amount of at least one compound of the formula (I) or the pesticidal mixture; and to seeds comprising a compound of the formula (I) or the pesticidal composition in an amount of from 0.1 g to 10 kg per 100 kg of seeds; to a use of a compound of the formula (I) or of the pesticidal compositions, for protecting growing plants from attack or infestation by invertebrate pests; and to a compound of the formula (I) for use in a method for treating or protecting an animal from infestation or infection by invertebrate pests which comprises bringing the animal in contact with a pesticidally effective amount of a compound of the formula (I).

Invertebrate pests and in particular insects, arachnids and nematodes destroy growing and harvested crops and attack wooden dwelling and commercial structures, thereby causing large economic loss to the food supply and to property. Accordingly, there is an ongoing need for new agents for combating invertebrate pests.

WO2017/167832 discloses bicyclic pyrimidone compounds and their pesticidal activity. The invention differs from the disclosure of WO'32 in that at least one substituent R⁷ is a sulfoximine residue as further defined below. Similar compounds with pesticidal activity are disclosed in WO2018/099812, WO2019/076778 and WO2018/202540.

Due to the ability of target pests to develop resistance to pesticidally-active agents, there is an ongoing need to identify further compounds, which are suitable for combating invertebrate pests such as insects, arachnids and nematodes. Furthermore, there is a need for new compounds having a high pesticidal activity and showing a broad activity spectrum against a large number of different invertebrate pests, especially against difficult to control insects, arachnids and nematodes.

It is therefore an object of the invention to identify and provide compounds, which exhibit a high pesticidal activity and have a broad activity spectrum against invertebrate pests.

It has been found that these objects can be achieved by substituted bicyclic compounds of formula (I), as depicted and defined below, including their stereoisomers, their salts, in particular their agriculturally or veterinarily acceptable salts, their tautomers and their N-oxides.

In a first aspect, the invention relates to a compound of formula (I), 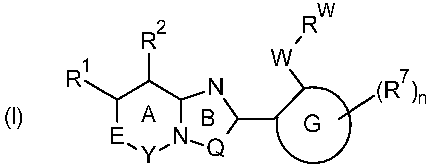 wherein
the rings A and B are fully unsaturated;
- Y: is C=X, wherein X is O or S;
- E: is N(R³) or C(R⁴);
- Q: is N, N(R⁵) or C(R⁶);
- G: is phenyl, or a 5- or 6-membered hetaryl;
- W: is S, S(O), S(O)₂; or S(O)(NR^{W});
R¹ is H, halogen, C₁-C₆-alkyl, C₂-C₆-alkenyl, C₂-C₆-alkynyl, C₁-C₆-alkoxy, C₁-C₆-alkoxy-C₁-C₆-alkyl, C₃-C₆-cycloalkyl, C₃-C₆-cycloalkoxy, C₁-C₆-alkylsulfenyl, C₁-C₆-alkylsulfinyl, or C₁-C₆-alkylsulfonyl, which groups are unsubstituted or halogenated;
R³, R⁵
are independently C₁-C₆-alkyl, C₁-C₆-alkoxy, C₂-C₆-alkenyl, C₂-C₆-alkynyl, C₁-C₆-alkoxy-C₁-C₄-alkyl, C₁-C₆-alkoxy-C₁-C₄-alkoxy, C₃-C₆-cycloalkyl, C₃-C₆-cycloalkyl-C₁-C₄-alkyl, C₃-C₆-cycloalkoxy-C₁-C₄-alkyl, which are unsubstituted or halogenated;
C(=O)OR^{A}, NR^{B}R^{C}, C₁-C₆-alkylen-NR^{B}R^{C}, O-C₁-C₆-alkylen-NR^{B}R^{C}, C₁-C₆-alkylen-CN, NH-C₁-C₆-alkylen-NR^{B}R^{C}, C(=O)NR^{B}R^{C}, C(=O)R^{D}, C(=S)R^{D}, SO₂NR^{B}R^{C}, S(=O)ₘR^{E};
phenyl or benzyl, wherein the phenyl ring is unsubstituted or substituted with one or more R^{F};

R², R⁴, R⁶
are independently H, halogen, N₃, CN, NO₂, SCN, SF₅, C₁-C₆-alkyl, C₁-C₆-alkoxy, C₂-C₆-alkenyl, tri-C₁-C₆-alkylsilyl, C₂-C₆-alkynyl, C₁-C₆-alkoxy-C₁-C₄-alkyl, C₁-C₆-alkoxy-C₁-C₄-alkoxy, C₃-C₆-cycloalkyl, C₃-C₆-cycloalkoxy, C₃-C₆-cycloalkyl-C₁-C₄-alkyl, C₃-C₆-cycloalkoxyx-C₁-C₄-alkyl, which groups are unsubstituted or substituted with halogen,
C(=O)OR^{A}, NR^{B}R^{C}, NOR^{A}, ONR^{B}R^{C}, C₁-C₆-alkylen-NR^{B}R^{C}, O-C₁-C₆-alkylen-NR^{B}R^{C}, C₁-C₆-alkylen-CN, NH-C₁-C₆-alkylen-NR^{B}R^{C}, C(=O)NR^{B}R^{C}, C(=O)R^{D}, C(=S)R^{D}, SO₂NR^{B}R^{C}, S(=O)ₘR^{E};
phenyl or benzyl, wherein the phenyl ring is unsubstituted or substituted with one or more R^{F};

each R⁷
is independently H, halogen, OH, CN, NC, NO₂, N₃, SCN, NCS, NCO, SF₃; C₁-C₆-alkyl, C₃-C₆-cycloalkyl, C₂-C₆-alkenyl, C₃-C₆-cycloalkenyl, C₂-C₆-alkynyl, C₃-C₆-cycloalkyl-C₁-C₃-alkyl, which groups are unsubstituted, or substituted with one or more R^{G};
a 3- to 12-membered saturated, partially unsaturated, or fully unsaturated heterocyclic ring or ring system, wherein said heterocyclic ring or ring system comprises one or more, same or different heteroatoms O, N, or S, and is unsubstituted, or substituted with one or more R^{H};
phenyl, which is unsubstituted, or substituted with one or more R^{J};
OR^{K}, SR^{K}, OC(=O)R^{K}, OC(=O)OR^{K}, OC(=O)NR^{L}R^{M}, OC(=O)SR^{K}, OC(=S)NR^{L}R^{M}, OC(=S)SR^{K}, OS(=O)ₘR^{K}, OS(=O)ₘNR^{L}R^{M}, ONR^{L}R^{M}, ON=CR^{N}R^{O}, NR^{L}R^{M}, NOR^{K}, ONR^{L}R^{M}, N=CR^{N}R^{O}, NNR^{L}, N(R^{L})C(=O)R^{K}, N(R^{L})C(=O)OR^{K}, S(=O)ₘR^{V}, SC(=O)SR^{K}, SC(=O)NR^{L}R^{M}, S(=O)ₘNR^{L}R^{M}, C(=O)R^{P}, C(=S)R^{P}, C(=O)NR^{L}R^{M}, C(=O)OR^{K}, C(=S)NR^{L}R^{M}, C(=S)OR^{K}, C(=S)SR^{K}, C(=NR^{L})R^{M}, C(=NR^{L})NR^{M}R^{R}, Si(R^{S})₂R^{T};
or two substituents R⁷ form, together with the ring members of ring G to which they are bound, a 5- or 6- membered saturated, partially unsaturated, or fully unsaturated carbo- or heterocycle, which carbo- or heterocycle is unsubstituted, or substituted with one or more R^{J}, and wherein said heterocycle comprises one or more, same or different heteroatoms O, N, or S; or
-C(CN)R^{X}R^{Y}, -C(R^{O})=N-NR^{M}R^{N}, -C(R^{O})=N-OR^{L}, or C₃-C₆-cycloalkyl, which is substituted with CN and which either does not have any further substituents, or which is further substituted with one or more R^{Z};
wherein at least one R⁷ is a group of formula (S) 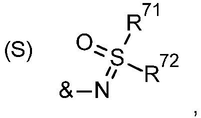
wherein each R⁷¹, R⁷² is independently selected from C₁-C₆-alkyl, C₃-C₆-cycloalkyl, C₂-C₆-alkenyl, C₃-C₆-cycloalkenyl, C₂-C₆-alkynyl, C₃-C₆-cycloalkyl-C₁-C₃-alkyl, which groups are unsubstituted, or substituted with one or more R^{G};
a 3- to 12-membered saturated, partially unsaturated, or fully unsaturated heterocyclic ring or ring system, wherein said heterocyclic ring or ring system comprises one or more, same or different heteroatoms O, N, or S, and is unsubstituted, or substituted with one or more R^{H};
phenyl, which is unsubstituted, or substituted with one or more R^{J};
or two substituents R⁷¹, R⁷² form, together with the sulfur atom to which they are bound, a 4-, 5-, or 6- membered saturated, partially unsaturated, or fully unsaturated heterocycle, which heterocycle is unsubstituted, or substituted with one or more R^{J}, and wherein said heterocycle comprises no, one or more, same or different heteroatoms O, N, or S in addition to the sulfur atom to which R⁷¹ and R⁷² are bound to;

each R^{A}
is independently H, C₁-C₆-alkyl, C₂-C₆-alkenyl, C₂-C₆-alkynyl, C₁-C₆-alkoxy-C₁-C₄-alkyl, C₃-C₆-cycloalkyl, C₃-C₆-cycloalkyl-C₁-C₄-alkyl, C₃-C₆-cycloalkoxy-C₁-C₄-alkyl, which groups are unsubstituted or substituted with halogen;
phenyl or benzyl, wherein the phenyl ring is unsubstituted or substituted with one or more R^{F};

each R^{B}
is independently H, C₁-C₆-alkyl, C₂-C₆-alkenyl, C₂-C₆-alkynyl, C₁-C₆-alkoxy-C₁-C₄-alkyl, C₃-C₆-cycloalkyl, C₃-C₆-cycloalkyl-C₁-C₄-alkyl, C₃-C₆-cycloalkoxy-C₁-C₄-alkyl, C₁-C₆-alkyl-carbonyl, C₁-C₆-alkoxy-carbonyl, which groups are unsubstituted or substituted with halogen;
phenyl or benzyl, which groups are unsubstituted or substituted with one or more R^{F};

each R^{C}
is independently H, C₁-C₆-alkyl, C₂-C₆-alkenyl, C₂-C₆-alkynyl, C₁-C₆-alkoxy-C₁-C₄-alkyl, C₃-C₆-cycloalkyl, C₃-C₆-cycloalkyl-C₁-C₄-alkyl, C₃-C₆-cycloalkoxy-C₁-C₄-alkyl, C₁-C₆-alkyl-carbonyl, or C₁-C₆-alkoxy-carbonyl, which groups are unsubstituted or substituted with halogen;
phenyl or benzyl, wherein the phenyl ring is unsubstituted or substituted with one or more R^{F};

each moiety NR^{B}R^{C} may also form an N-bound, saturated 5- to 8-membered heterocycle, which in addition to the nitrogen atom may have 1 or 2 further heteroatoms or heteroatom moieties selected from O, S(=O)ₘ and N-R', wherein R' is H or C₁-C₆-alkyl and wherein the N-bound heterocycle is unsubstituted or substituted with one or more substituents selected from halogen, C₁-C₄-alkyl, C₁-C₄-haloalkyl, C₁-C₄-alkoxy and C₁-C₄-haloalkoxy;
each R^{D}
is independently H, CN, OH, C₁-C₆-alkyl, C₂-C₆-alkenyl, C₂-C₆-alkynyl, C₁-C₆-alkoxy-C₁-C₄-alkyl, C₃-C₆-cycloalkyl, C₃-C₆-cycloalkyl-C₁-C₄-alkyl, C₃-C₆-cycloalkoxy-C₁-C₄-alkyl, which groups are unsubstituted or substituted with halogen;
phenyl or benzyl, wherein the phenyl ring is unsubstituted or substituted with one or more R^{F};

each R^{E}
is independently C₁-C₆-alkyl, C₃-C₆-cycloalkyl, C₃-C₆-cycloalkyl-C₁-C₄-alkyl, which are unsubstituted or substituted with halogen; or
phenyl or benzyl, wherein the phenyl ring is unsubstituted or substituted with one or more R^{F};

each R^{F} is independently halogen, N₃, OH, CN, NO₂, SCN, SF₃, C₁-C₆ alkyl, C₁-C₆-alkoxy, C₂-C₆-alkenyl, C₂-C₆-alkynyl, C₁-C₆-alkoxy-C₁-C₄-alkyl, C₁-C₆-alkoxy-C₁-C₄-alkoxy, C₃-C₆-cycloalkyl, C₃-C₆-cycloalkoxy, C₃-C₆-cycloalkyl-C₁-C₄-alkyl, C₃-C₆-cycloalkoxy-C₁-C₄-alkyl, which groups are unsubstituted or substituted with halogen;
each R^{G}
is independently halogen, OH, CN, NC, NO₂;
C₁-C₆-alkyl, C₃-C₆-cycloalkyl, C₃-C₆-cycloalkenyl, which groups are unsubstituted, or substituted with one or more substituents selected from halogen, OH, CN, C₁-C₃-alkoxy, C₁-C₃-haloalkoxy, and C₁-C₃-alkyl-carbonyl; a 3- to 12-membered saturated, partially unsaturated, or fully unsaturated heterocyclic ring or ring system, wherein said heterocyclic ring or ring system comprises one or more, same or different heteroatoms O, N, or S, and is unsubstituted, or substituted with one or more substituents selected from halogen, OH, CN, C₁-C₃-alkoxy, C₁-C₃-haloalkoxy, and C₁-C₃-alkyl-carbonyl; phenyl, which is unsubstituted, or substituted with one or more substituents selected from halogen, OH, CN, NO₂, C₁-C₃-alkyl, C₁-C₃-haloalkyl, C₁-C₃-alkoxy, C₁-C₃-haloalkoxy, and C₁-C₃-alkyl-carbonyl;
OR^{K}, SR^{K}, OC(=O)R^{K}, OC(=O)OR^{K}, OC(=O)NR^{L}R^{M}, OC(=O)SR^{K}, OC(=S)NR^{L}R^{M}, OC(=S)SR^{K}, OS(=O)ₘR^{K}, OS(=O)ₘNR^{L}R^{M}, ONR^{L}R^{M}, ON=CR^{N}R^{O}, NR^{L}R^{M}, NOR^{K}, ONR^{L}R^{M}, N=CR^{N}R^{O}, NNR^{L}, N(R^{L})C(=O)R^{K}, N(R^{L})C(=O)OR^{K}, S(=O)ₘR^{V}, SC(=O)SR^{K}, SC(=O)NR^{L}R^{M}, S(=O)ₘNR^{L}R^{M}, C(=O)R^{P}, C(=S)R^{P}, C(=O)NR^{L}R^{M}, C(=O)OR^{K}, C(=S)NR^{L}R^{M}, C(=S)OR^{K}, C(=S)SR^{K}, C(=NR^{L})R^{M}, C(=NR^{L})NR^{M}R^{R}, Si(R^{S})₂R^{T};

each R^{H}
is independently halogen, CN, NC, NO₂, SCN, NCS, NCO;
C₁-C₆-alkyl, C₃-C₆-cycloalkyl, C₃-C₆-cycloalkenyl, which groups are unsubstituted, or substituted with one or more substituents selected from halogen, OH, CN, C₁-C₃-alkoxy, C₁-C₃-haloalkoxy, and C₁-C₃-alkyl-carbonyl; phenyl, which is unsubstituted, or substituted with one or more substituents selected from halogen, OH, CN, NO₂, C₁-C₃-alkyl, C₁-C₃-haloalkyl, OR^{K}, SR^{K}, OC(=O)R^{K}, OC(=O)OR^{K}, OC(=O)NR^{L}R^{M}, OC(=O)SR^{K}, OC(=S)NR^{L}R^{M}, OC(=S)SR^{K}, OS(=O)ₘR^{K}, OS(=O)ₘNR^{L}R^{M}, ONR^{L}R^{M}, ON=CR^{N}R^{O}, NR^{L}R^{M}, NOR^{K}, ONR^{L}R^{M}, N=CR^{N}R^{O}, NNR^{L}, N(R^{L})C(=O)R^{K}, N(R^{L})C(=O)OR^{K}, S(=O)ₘR^{V}, SC(=O)SR^{K}, SC(=O)NR^{L}R^{M}, S(=O)ₘNR^{L}R^{M}, C(=O)R^{P}, C(=S)R^{P}, C(=O)NR^{L}R^{M}, C(=O)OR^{K}, C(=S)NR^{L}R^{M}, C(=S)OR^{K}, C(=S)SR^{K}, C(=NR^{L})R^{M}, C(=NR^{L})NR^{M}R^{R}, Si(R^{S})₂R^{T}; or
two geminal substituents R^{H} form together with the atom to which they are bound a group =O, =S, or =NR^{L}.

each R^{J}
is independently halogen, CN, NC, NO₂, SCN, NCS, NCO;
C₁-C₆-alkyl, C₃-C₆-cycloalkyl, C₃-C₆-cycloalkenyl, which groups are unsubstituted, or substituted with one or more substituents selected from halogen, OH, CN, C₁-C₃-alkoxy, C₁-C₃-haloalkoxy, and C₁-C₃-alkyl-carbonyl; phenyl, which is unsubstituted, or substituted with one or more substituents selected from halogen, OH, CN, NO₂, C₁-C₃-alkyl, C₁-C₃-haloalkyl, OR^{K}, SR^{K}, OC(=O)R^{K}, OC(=O)OR^{K}, OC(=O)NR^{L}R^{M}, OC(=O)SR^{K}, OC(=S)NR^{L}R^{M}, OC(=S)SR^{K}, OS(=O)ₘR^{K}, OS(=O)ₘNR^{L}R^{M}, ONR^{L}R^{M}, ON=CR^{N}R^{O}, NR^{L}R^{M}, NOR^{K}, ONR^{L}R^{M}, N=CR^{N}R^{O}, NNR^{L}, N(R^{L})C(=O)R^{K}, N(R^{L})C(=O)OR^{K}, S(=O)ₘR^{V}, SC(=O)SR^{K}, SC(=O)NR^{L}R^{M}, S(=O)ₘNR^{L}R^{M}, C(=O)R^{P}, C(=S)R^{P}, C(=O)NR^{L}R^{M}, C(=O)OR^{K}, C(=S)NR^{L}R^{M}, C(=S)OR^{K}, C(=S)SR^{K}, C(=NR^{L})R^{M}, C(=NR^{L})NR^{M}R^{R}, Si(R^{S})₂R^{T};

each R^{K}
is independently H;
C₁-C₆-alkyl, C₂-C₆-alkenyl, C₂-C₆-alkynyl, C₁-C₆-alkoxy-C₁-C₄-alkyl, C₃-C₆-cycloalkyl, C₃-C₆-cycloalkyl-C₁-C₄-alkyl, C₃-C₆-cycloalkoxy-C₁-C₄-alkyl, which groups are unsubstituted or substituted with one or more substituents selected from halogen, CN, NR^{L}R^{M}; C(=O)NR^{L}R^{M}, C(=O)R^{T}; or
phenyl or benzyl, wherein the phenyl ring is unsubstituted or substituted with one or more R^{F};

each R^{L}, R^{R}
is independently H;
C₁-C₆-alkyl, C₂-C₆-alkenyl, C₂-C₆-alkynyl, C₁-C₆-alkoxy-C₁-C₄-alkyl, C₃-C₆-cycloalkyl, C₃-C₆-cycloalkyl-C₁-C₄-alkyl, C₃-C₆-cycloalkoxy-C₁-C₄-alkyl, which groups are unsubstituted or substituted with halogen;
phenyl and benzyl, which groups are unsubstituted or substituted with one or more R^{F};

each R^{M}
is independently H, C₁-C₆-alkyl, C₂-C₆-alkenyl, C₂-C₆-alkynyl, C₁-C₆-alkoxy-C₁-C₄-alkyl, C₃-C₆-cycloalkyl, C₃-C₆-cycloalkyl-C₁-C₄-alkyl, C₃-C₆-cycloalkoxy-C₁-C₄-alkyl, C₁-C₆-alkyl-carbonyl, C₁-C₆-alkoxy-carbonyl, which groups are unsubstituted or substituted with halogen;
C₁-C₆-alkylen-CN;
phenyl or benzyl, which groups are unsubstituted or substituted with one or more R^{F};

each R^{N}
is independently H, C₁-C₆-alkyl, C₂-C₆-alkenyl, C₂-C₆-alkynyl, C₁-C₆-alkoxy-C₁-C₄-alkyl, C₃-C₆-cycloalkyl, C₃-C₆-cycloalkyl-C₁-C₄-alkyl, C₃-C₆-cycloalkoxy-C₁-C₄-alkyl, C₁-C₆-alkyl-carbonyl, C₁-C₆-alkoxy-carbonyl, which groups are unsubstituted or substituted with halogen;
phenyl or benzyl, wherein the phenyl ring is unsubstituted or substituted with one or more R^{F};

each moiety NR^{M}R^{N}, R^{M}R^{R}, and R^{L}R^{M} may also form an N-bound, saturated 5- to 8-membered heterocycle, which in addition to the nitrogen atom may have 1 or 2 further heteroatoms or heteroatom moieties selected from O, S(=O)ₘ and N-R", wherein R" is H or C₁-C₆-alkyl and wherein the N-bound heterocycle is unsubstituted or substituted with one or more substituents selected from halogen, C₁-C₄-alkyl, C₁-C₄-haloalkyl, C₁-C₄-alkoxy and C₁-C₄-haloalkoxy;
each R^{O}
is independently H, CN, OH, C₁-C₆-alkyl, C₂-C₆-alkenyl, C₂-C₆-alkynyl, C₁-C₆-alkoxy-C₁-C₄-alkyl, C₃-C₆-cycloalkyl, C₃-C₆-cycloalkyl-C₁-C₄-alkyl, C₃-C₆-cycloalkoxy-C₁-C₄-alkyl, which groups are unsubstituted or substituted with halogen;
phenyl or benzyl, wherein the phenyl ring is unsubstituted or substituted with one or more R^{F};

each R^{P}
is independently H;
C₁-C₆-alkyl, C₂-C₆-alkenyl, C₂-C₆-alkynyl, C₁-C₆-alkoxy-C₁-C₄-alkyl, C₃-C₆-cycloalkyl, C₃-C₆-cycloalkyl-C₁-C₄-alkyl, C₃-C₆-cycloalkoxy-C₁-C₄-alkyl, which groups are unsubstituted or substituted with halogen;
phenyl or benzyl, wherein the phenyl ring is unsubstituted or substituted with one or more R^{F};

each R^{S}, R^{T} is independently H, C₁-C₆-alkyl, C₁-C₆-haloalkyl, C₁-C₆-alkoxy, C₁-C₄-alkoxy-C₁-C₄-alkyl, C₃-C₆-cycloalkyl, C₃-C₆-halocycloalkyl, C₁-C₄-haloalkoxy-C₁-C₄-alkyl, or phenyl;
each R^{V}
is indepentently C₁-C₆-alkyl, C₃-C₆-cycloalkyl, C₃-C₆-cycloalkyl-C₁-C₄-alkyl, which are unsubstituted or substituted with halogen; or
phenyl or benzyl, wherein the phenyl ring is unsubstituted or substituted with R^{F};

each R^{W}
is independently H, C₁-C₆-alkyl, C₃-C₆-cycloalkyl, C₃-C₆-cycloalkyl-C₁-C₄-alkyl, which groups are halogenated or non-halogenated;
benzyl, or phenyl, which is unsubstituted or substituted with R^{F};

each R^{X}, R^{Y} is independently H, halogen, CN, C₁-C₆-alkyl, C₁-C₆-haloalkyl, C₃-C₆-cycloalkyl, C₁-C₄-alkoxy, C₁-C₄-alkoxy-C₁-C₄-alkyl, C₁-C₄-alkylsulfanyl, C₁-C₄-alkylsulfanyl-C₁-C₄-alkyl, C₁-C₄-alkylsulfinyl-C₁-C₄-alkyl, C₁-C₄-alkylsulfonyl-C₁-C₄-alkyl or C₁-C₄-alkoxycarbonyl;
each R^{Z}
is halogen, CN, NH₂, C(=O)H, OH, C₃-C₆-cycloalkyl, C(=O)OH, C(=O)NH₂, C₁-C₄-haloalkoxy, C₁-C₄-alkoxy, C₁-C₄-haloalkylsulfanyl, C₁-C₄-haloalkylsulfinyl, C₁-C₄-haloalkylsulfonyl, C₁-C₄-alkoxycarbonyl, C₁-C₄-haloalkoxycarbonyl, C₁-C₄-alkylcarbonyl, C₁-C₄-haloalkylcarbonyl, di-(C₁-C₄)alkylaminocarbonyl, C₁-C₄-alkylaminocarbonyl, C₁-C₄-alkylcarbonylamino, di-(C₁-C₄)alkylcarbonylamino, C₁-C₄-alkoxycarbonylamino, or a group -C(R^{Z1})=NOR^{Z2};
phenyl, which is unsubstituted or substituted with one or more substituents selected from halogen, CN, C₁-C₄-alkyl, C₁-C₄-haloalkyl, C₁-C₄-haloalkoxy, C₁-C₄-alkoxy, C₁-C₄-haloalkylsulfanyl, C₁-C₄-haloalkylsulfinyl, C₁-C₄-haloalkylsulfonyl and C(=O)C₁-C₄-haloalkyl;
C₁-C₄-alkyl which is unsubstituted or substituted with one or more R^{Z3};
R^{Z1} and R^{Z2} are independently H, C₁-C₄-alkyl, or C₁-C₄-haloalkyl;
R^{Z3} is halogen, CN, NH₂, C(=O)H, OH, C₃-C₆-cycloalkyl, hydroxycarbonyl, aminocarbonyl, C₁-C₄-haloalkoxy, C₁-C₄-alkoxy, C₁-C₄-haloalkylsulfanyl, C₁-C₄-haloalkylsulfinyl, C₁-C₄-haloalkylsulfonyl, C₁-C₄-alkoxycarbonyl, C₁-C₄-haloalkoxycarbonyl, C₁-C₄-alkylcarbonyl, C₁-C₄-haloalkylcarbonyl, di-(C₁-C₄)alkylaminocarbonyl, C₁-C₄alkylaminocarbonyl, C₁-C₄-alkylcarbonylamino, di-(C₁-C₄)alkylcarbonylamino, C₁-C₄-alkoxycarbonylamino, a group -C(R^{Z1})=NOR^{Z2};
the index n is 1, 2, 3, or 4 if G is phenyl or a 6-membered hetaryl;
or 1, 2, or 3 if X is a 5-membered hetaryl; and
the index m is 0, 1 or 2;
and the N-oxides, stereoisomers, tautomers and agriculturally or veterinarily acceptable salts thereof.

The compounds of the formula (I), and their agriculturally acceptable salts are highly active against animal pest, *i.e.* harmful arthropodes and nematodes, especially against insects and acaridae which are difficult to control by other means.

Moreover, the invention relates to and includes the following embodiments:
- compositions comprising at least one compound of formula (I) as defined above and a liquid or solid carrier;
- agricultural and veterinary compositions comprising an amount of at least one compound of formula (I) or an enantiomer, diasteromer or salt thereof as defined above;
- methods for combating invertebrate pests, infestation, or infection by invertebrate pests, which method comprises contacting said pest or its food supply, habitat or breeding grounds with a pesticidally effective amount of at least one compound of formula (I) as defined above or a composition thereof;
- methods for controlling invertebrate pests, infestation, or infection by invertebrate pests, which method comprises contacting said pest or its food supply, habitat or breeding grounds with a pesticidally effective amount of at least one compound of formula (I) as defined above or a composition comprising at least one compound of formula (I);
- methods for preventing or protecting against invertebrate pests comprising contacting the invertebrate pests, or their food supply, habitat or breeding grounds with substituted imidazolium compounds of the general formula (I) as defined above or a composition comprising at least one compound of formula (I) as defined above or a composition comprising at least one compound of formula (I);
- methods for protecting crops, plants, plant propagation material and/or growing plants from attack or infestation by invertebrate pests comprising contacting or treating the crops, plants, plant propagation material and growing plants, or soil, material, surface, space, area or water in which the crops, plants, plant propagation material is stored or the plant is growing, with a pesticidally effective amount of at least one compound of formula (I) as defined above or a composition comprising at least one compound of formula (I);
- non-therapeutic methods for treating animals infested or infected by parasites or preventing animals of getting infected or infested by parasites or protecting animals against infestation or infection by parasites which comprises orally, topically or parenterally administering or applying to the animals a parasiticidally effective amount of a compound of formula (I) as defined above or a composition comprising at least one compound of formula (I);
- methods for treating, controlling, preventing or protecting animals against infestation or infection by parasites by administering or applying orally, topically or parenterally to the animals a compound of the general formula (I) as defined above or a composition comprising at least one compound of formula (I);
- seed comprising a compound of formula (I) as defined above, in an amount of from 0.1g to 10kg per 100kg of seed;
- the use of the compounds of formula (I) as defined above for protecting growing plants or plant propagation material from attack or infestation by invertebrate pests;
- the use of compounds of formula (I) or the enantiomers, diastereomers or veterinary acceptable salts thereof for combating parasites in and on animals;
- a process for the preparation of a veterinary composition for treating, controlling, preventing or protecting animals against infestation or infection by parasites which comprises adding a parasiticidally effective amount of a compound of formula (I) or the enantiomers, diastereomers and/or veterinary acceptable salt thereof to a carrier composition suitable for veterinary use;
- the use of a compound of formula (I) or the enantiomers, diastereomers and/or veterinary acceptable salt thereof for the preparation of a medicament for treating, controlling, preventing or protecting animals against infestation or infection by parasites.

All the compounds of formula (I) and, if applicable, their stereoisomers, their tautomers, their salts or their N-oxides as well as compositions thereof are particularly useful for controlling invertebrate pests, in particular for controlling arthropods and nematodes and especially insects. Therefore, the invention relates to the use of a compound of formula (I) as an agrochemical pesticide, preferably for combating or controlling invertebrate pests, in particular invertebrate pests of the group of insects, arachnids or nematodes.

The term "compound(s) according to the invention" or "compound(s) of formula (I)" as used in the invention refers to and comprises the compound(s) as defined herein and/or stereoisomer(s), salt(s), tautomer(s) or N-oxide(s) thereof. The term "compound(s) of the invention" is to be understood as equivalent to the term "compound(s) according to the invention", therefore also comprising stereoisomer(s), salt(s), tautomer(s) or N-oxide(s) of compounds of formula (I). As used herein, the term "compound(s) of the invention" or "compound(s) according to the invention" refers to the compound(s) of formula (I) as defined above, which are also referred to as "compound(s) of formula I" or "compound(s) I" or "formula I compound(s)", and includes their salt(s), tautomer(s), stereoisomer(s), and N-oxide(s).

The term "composition(s) according to the invention" or "composition(s) of the invention" encompasses composition(s) comprising at least one compound of formula (I) according to the invention as defined above, therefore also including a stereoisomer, an agriculturally or veterinary acceptable salt, tautomer or an N-oxide of the compounds of formula (I).

The compounds of the invention may be amorphous or may exist in one or more different crystalline states (polymorphs) or modifications which may have a different macroscopic properties such as stability or show different biological properties such as activities. The invention includes both amorphous and crystalline compounds of the formula (I), mixtures of different crystalline states or modifications of the respective compound I, as well as amorphous or crystalline salts thereof.

The compounds of the formula (I) may have one or, depending on the substitution pattern, more centers of chirality, in which case they are present as mixtures of enantiomers or diastereomers. The invention provides both the single pure enantiomers or pure diastereomers of the compounds of formula (I), and their mixtures and the use according to the invention of the pure enantiomers or pure diastereomers of the compound of formula (I) or its mixtures. Suitable compounds of the formula (I) also include all possible geometrical stereoisomers (cis/trans isomers) and mixtures thereof. Cis/trans isomers may be present with respect to an alkene, carbon-nitrogen double-bond or amide group. The term "stereoisomer(s)" encompasses both optical isomers, such as enantiomers or diastereomers, the latter existing due to more than one center of chirality in the molecule, as well as geometrical isomers (cis/trans isomers). The invention relates to every possible stereoisomer of the compounds of formula (I), *i.e.* to single enantiomers or diastereomers, as well as to mixtures thereof.

Depending on the substitution pattern, the compounds of the formula (I) may be present in the form of their tautomers. Hence the invention also relates to the tautomers of the formula (I) and the stereoisomers, salts, tautomers and N-oxides of said tautomers.

Salts of the compounds of the formula (I) are preferably agriculturally and/or veterinary acceptable salts. They can be formed in a customary method, e.g. by reacting the compound with an acid of the anion in question if the compound of formula (I) has a basic functionality or by reacting an acidic compound of formula (I) with a suitable base.

Suitable agriculturally or veterinary useful salts are especially the salts of those cations or the acid addition salts of those acids whose cations and anions, respectively, do not have any adverse effect on the action of the compounds according to the invention. Suitable cations are in particular the ions of the alkali metals, preferably lithium, sodium and potassium, of the alkaline earth metals, preferably calcium, magnesium, and barium, and of the transition metals, preferably manganese, copper, zinc and iron, and also ammonium (NH₄⁺) and substituted ammonium in which one to four of the hydrogen atoms are replaced by C₁-C₄-alkyl, C₁-C₄-hydroxyalkyl, C₁-C₄-alkoxy, C₁-C₄-alkoxy-C₁-C₄-alkyl, hydroxy-C₁-C₄-alkoxy-C₁-C₄-alkyl, phenyl or benzyl. Examples of substituted ammonium ions comprise methylammonium, isopropylammonium, dimethylammonium, diisopropylammonium, trimethylammonium, tetramethylammonium, tetraethylammonium, tetrabutylammonium, 2-hydroxyethylammonium, 2-(2-hydroxyethoxy)ethyl-ammonium, bis(2-hydroxyethyl)ammonium, benzyltrimethylammonium and benzyltriethylammonium, furthermore phosphonium ions, sulfonium ions, preferably tri(C₁-C₄-alkyl)sulfonium, and sulfoxonium ions, preferably tri(C₁-C₄-alkyl)sulfoxonium.

Anions of useful acid addition salts are primarily chloride, bromide, fluoride, hydrogen sulfate, sulfate, dihydrogen phosphate, hydrogen phosphate, phosphate, nitrate, hydrogen carbonate, carbonate, hexafluorosilicate, hexafluorophosphate, benzoate, and the anions of C₁-C₄-alkanoic acids, preferably formate, acetate, propionate and butyrate. They can be formed by reacting the compounds of the formulae I with an acid of the corresponding anion, preferably of hydrochloric acid, hydrobromic acid, sulfuric acid, phosphoric acid or nitric acid.

The term "N-oxide" includes any compound of the invention which has at least one tertiary nitrogen atom that is oxidized to an N-oxide moiety.

The organic moieties groups mentioned in the above definitions of the variables are - like the term halogen - collective terms for individual listings of the individual group members. The prefix Cₓ-C_{y} indicates in each case the possible number of carbon atoms in the group. "Halogen" will be taken to mean F, Cl, Br, and I, preferably F.

The terms compound(s) of formula (x) and compound(s) (x), wherein x is a roman or arab number are used herein interchangeably and refer to a single or several compounds as defined by the respective formula (x).

The term "substituted with", e.g. as used in "partially, or fully substituted with" means that one or more, e.g. 1, 2, 3, 4 or 5 or all of the hydrogen atoms of a given radical have been replaced by one or more, same or different substituents as subsequently defined. Accordingly, for substituted cyclic moieties, e.g. 1-cyanocyclopropyl, one or more of the hydrogen atoms of the cyclic moiety may be replaced by one or more, same or different substituents.

The term "Cₓ-C_{y}-alkyl" as used herein (and also in Cₓ-C_{y}-alkylamino, di-Cₓ-C_{y}-alkylamino, Cₓ-C_{y}-alkylaminocarbonyl, di-(Cₓ-C_{y}-alkylamino)carbonyl, Cₓ-C_{y}-alkylthio, Cₓ-C_{y}-alkylsulfinyl and Cₓ-C_{y}-alkylsulfonyl) refers to a branched or unbranched saturated hydrocarbon group having n to m, e.g. 1 to 10 carbon atoms, preferably 1 to 6 carbon atoms, e.g. methyl, ethyl, propyl, 1-methylethyl, butyl, 1-methylpropyl, 2-methylpropyl, 1,1-dimethylethyl, pentyl, 1-methylbutyl, 2-methylbutyl, 3-methylbutyl, 2,2-dimethylpropyl, 1-ethylpropyl, hexyl, 1,1-dimethylpropyl, 1,2-dimethylpropyl, 1-methylpentyl, 2-methylpentyl, 3-methylpentyl, 4-methylpentyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl, 1-ethyl-2-methylpropyl, heptyl, octyl, 2-ethylhexyl, nonyl and decyl and their isomers. C₁-C₄-alkyl means for example methyl, ethyl, propyl, 1-methylethyl, butyl, 1-methylpropyl, 2-methylpropyl or 1,1-dimethylethyl.

The term "Cₓ-C_{y}-haloalkyl" as used herein (and also in Cₓ-C_{y}-haloalkylsulfinyl and Cₓ-C_{y}-halo-alkylsulfonyl) refers to a straight-chain or branched alkyl group having n to m carbon atoms, e.g. 1 to 10 in particular 1 to 6 carbon atoms (as mentioned above), where some or all of the hydrogen atoms in these groups may be replaced by halogen atoms as mentioned above, e.g. C₁-C₄-haloalkyl, such as chloromethyl, bromomethyl, dichloromethyl, trichloromethyl, fluoromethyl, difluoromethyl, trifluoromethyl, chlorofluoromethyl, dichlorofluoromethyl, chlorodifluoromethyl, 1-chloroethyl, 1-bromoethyl, 1-fluoroethyl, 2-fluoroethyl, 2,2-difluoroethyl, 2,2,2-trifluoroethyl, 2-chloro-2-fluoroethyl, 2-chloro-2,2-difluoroethyl, 2,2-dichloro-2-fluoroethyl, 2,2,2-trichloroethyl, pentafluoroethyl and the like. The term C₁-C₁₀-haloalkyl in particular comprises C₁-C₂-fluoroalkyl, which is synonym with methyl or ethyl, wherein 1, 2, 3, 4 or 5 hydrogen atoms are substituted with fluorine atoms, such as fluoromethyl, difluoromethyl, trifluoromethyl, 1-fluoroethyl, 2-fluoroethyl, 2,2-difluoroethyl, 2,2,2-trifluoroethyl and pentafluoromethyl.

Similarly, "Cₓ-C_{y}-alkoxy" and "Cₓ-C_{y}-alkylthio" (or Cₓ-C_{y}-alkylsulfenyl, respectively) refer to straight-chain or branched alkyl groups having n to m carbon atoms, e.g. 1 to 10, in particular 1 to 6 or 1 to 4 carbon atoms (as mentioned above) bonded through oxygen (or sulfur linkages, respectively) at any bond in the alkyl group. Examples include C₁-C₄-alkoxy such as methoxy, ethoxy, propoxy, isopropoxy, butoxy, sec-butoxy, isobutoxy and tert-butoxy, further C₁-C₄-alkylthio such as methylthio, ethylthio, propylthio, isopropylthio, and n-butylthio.

Accordingly, the terms "Cₓ-C_{y}-haloalkoxy" and "Cₓ-C_{y}-haloalkylthio" (or Cₓ-C_{y}-haloalkylsulfenyl, resp.) refer to straight-chain or branched alkyl groups having n to m carbon atoms, e.g. 1 to 10, in particular 1 to 6 or 1 to 4 carbon atoms (as mentioned above) bonded through oxygen or sulfur linkages, resp., at any bond in the alkyl group, where some or all of the hydrogen atoms in these groups may be replaced by halogen atoms as mentioned above, e.g. C₁-C₂-haloalkoxy, such as chloromethoxy, bromomethoxy, dichloromethoxy, trichloromethoxy, fluoromethoxy, difluoromethoxy, trifluoromethoxy, chlorofluoromethoxy, dichlorofluoromethoxy, chlorodifluoro-methoxy, 1-chloroethoxy, 1-bromoethoxy, 1-fluoroethoxy, 2-fluoroethoxy, 2,2-difluoroethoxy, 2,2,2-trifluoroethoxy, 2-chloro-2-fluoroethoxy, 2-chloro-2,2-difluoroethoxy, 2,2-dichloro-2-fluoroethoxy, 2,2,2-trichloroethoxy and pentafluoroethoxy, further C₁-C₂-haloalkylthio, such as chloromethylthio, bromomethylthio, dichloromethylthio, trichloromethylthio, fluoromethylthio, difluoromethylthio, trifluoromethylthio, chlorofluoromethylthio, dichlorofluoromethylthio, chlorodifluoromethylthio, 1-chloroethylthio, 1-bromoethylthio, 1-fluoroethylthio, 2-fluoroethylthio, 2,2-difluoroethylthio, 2,2,2-trifluoroethylthio, 2-chloro-2-fluoroethylthio, 2-chloro-2,2-difluoroethylthio, 2,2-dichloro-2-fluoroethylthio, 2,2,2-trichloroethylthio and pentafluoroethylthio and the like. Similarly, the terms C₁-C₂-fluoroalkoxy and C₁-C₂-fluoroalkylthio refer to C₁-C₂-fluoroalkyl which is bound to the remainder of the molecule via an oxygen atom or a sulfur atom, respectively.

The term "C₂-C_{y}-alkenyl" as used herein intends a branched or unbranched unsaturated hydrocarbon group having 2 to m, e.g. 2 to 10 or 2 to 6 carbon atoms and a double bond in any position, such as ethenyl, 1-propenyl, 2-propenyl, 1-methyl-ethenyl, 1-butenyl, 2-butenyl, 3-butenyl, 1-methyl-1-propenyl, 2-methyl-1-propenyl, 1-methyl-2-propenyl, 2-methyl-2-propenyl, 1-pentenyl, 2-pentenyl, 3-pentenyl, 4-pentenyl, 1-methyl-1-butenyl, 2-methyl-1-butenyl, 3-methyl-1-butenyl, 1-methyl-2-butenyl, 2-methyl-2-butenyl, 3-methyl-2-butenyl, 1-methyl-3-butenyl, 2-methyl-3-butenyl, 3-methyl-3-butenyl, 1,1-dimethyl-2-propenyl, 1,2-dimethyl-1-propenyl, 1,2-dimethyl-2-propenyl, 1-ethyl-1-propenyl, 1-ethyl-2-propenyl, 1-hexenyl, 2-hexenyl, 3-hexenyl, 4-hexenyl, 5-hexenyl, 1-methyl-1-pentenyl, 2-methyl-1-pentenyl, 3-methyl-1-pentenyl, 4-methyl-1-pentenyl, 1-methyl-2-pentenyl, 2-methyl-2-pentenyl, 3-methyl-2-pentenyl, 4-methyl-2-pentenyl, 1-methyl-3-pentenyl, 2-methyl-3-pentenyl, 3-methyl-3-pentenyl, 4-methyl-3-pentenyl, 1-methyl-4-pentenyl, 2-methyl-4-pentenyl, 3-methyl-4-pentenyl, 4-methyl-4-pentenyl, 1,1-dimethyl-2-butenyl, 1,1-dimethyl-3-butenyl, 1,2-dimethyl-1-butenyl, 1,2-dimethyl-2-butenyl, 1,2-dimethyl-3-butenyl, 1,3-dimethyl-1-butenyl, 1,3-dimethyl-2-butenyl, 1,3-dimethyl-3-butenyl, 2,2-dimethyl-3-butenyl, 2,3-dimethyl-1-butenyl, 2,3-dimethyl-2-butenyl, 2,3-dimethyl-3-butenyl, 3,3-dimethyl-1-butenyl, 3,3-dimethyl-2-butenyl, 1-ethyl-1-butenyl, 1-ethyl-2-butenyl, 1-ethyl-3-butenyl, 2-ethyl-1-butenyl, 2-ethyl-2-butenyl, 2-ethyl-3-butenyl, 1,1,2-trimethyl-2-propenyl, 1-ethyl-1-methyl-2-propenyl, 1-ethyl-2-methyl-1-propenyl and 1-ethyl-2-methyl-2-propenyl.

The term "C₂-C_{y}-alkynyl" as used herein refers to a branched or unbranched unsaturated hydrocarbon group having 2 to m, e.g. 2 to 10 or 2 to 6 carbon atoms and containing at least one triple bond, such as ethynyl, propynyl, 1-butynyl, 2-butynyl, and the like.

The term "Cₓ-C_{y}-alkoxy-Cₓ-C_{y}-alkyl" as used herein refers to alkyl having n to m carbon atoms, e.g. like specific examples mentioned above, wherein one hydrogen atom of the alkyl radical is replaced by an Cₓ-C_{y}-alkoxy group; wherein the value of x and y of the alkoxy group are independently chosen from that of the alkyl group.

Accordingly, the term "Cₓ-C_{y}-alkoxy-Cₓ-C_{y}-alkoxy" as used herein refers to alkoxy group having n to m carbon atoms, e.g. like methoxy or ethoxy, wherein one hydrogen atom of the alkoxyl radical is replaced by an Cₓ-C_{y}-alkoxy group; wherein the value of x and y of the alkoxy group are independently chosen from that of the alkoxyl group.

The suffix "-carbonyl" in a group or "C(=O)" denotes in each case that the group is bound to the remainder of the molecule via a carbonyl C=O group. This is the case e.g. in alkylcarbonyl, haloalkylcarbonyl, aminocarbonyl, alkylaminocarbonyl, dialkylaminocarbonyl, alkoxycarbonyl, haloalkoxycarbonyl, alkylcarbonylamino, and hydroxycarbonyl. For example, a hydroxycarbonyl group would refer to a carbonic acid group -C(=O)OH, and an aminocyrbonyl group would refer to an amide group -C(=O)NH₂, both of which are bound to the remainder of the molecule via the carbonyl "C=O" group.

The term "aryl" as used herein refers to a mono-, bi- or tricyclic aromatic hydrocarbon radical such as phenyl or naphthyl, in particular phenyl (also referred as to C₆H₅ as subsitituent).

The term "C₃-C_{y}-cycloalkyl" as used herein refers to a monocyclic ring of 3- to y-membered saturated cycloaliphatic radicals, e.g. cyclopropyl (cC₃H₃), cyclobutyl (cC₄H₇), cyclopentyl (cC₃H₉), cyclohexyl (cC₆H₁₁), cycloheptyl, cyclooctyl and cyclodecyl.

Accordingly, the term "C₃-C_{y}-cycloalkoxy" as used herein refers to a "C₃-C_{y}"-cycloalkyl moiety which is bonded to the rest of the molecule via an oxygen atom, such as in cyclopropoxy, cyclobutoxy, cyclopentoxy and cyclohexoxy.

The term "C₃-C_{y}-cycloalkoxy-Cₓ-C_{y}-alkyl" refers to a Cₓ-C_{y}-alkyl moiety, wherein one hydrogen atom is substituted by a C₃-C_{y}-alkoxy group and wherein the indices x and y are indepently chosen according to the indicated meaning.

The term "cycloalkylalkyl" denotes as well as the term "alkyl which may be substituted with cycloalkyl" an alkyl group which is substituted with a cycloalkyl ring, wherein alkyl and cycloakyl are as herein defined.

The term "C₃-C_{y}-cycloalkenyl" as used herein refers to a monocyclic ring of 3- to y-membered partially unsaturated cycloaliphatic radicals.

The term "cycloalkylcycloalkyl" denotes as well as the term "cycloalkyl which may be substituted with cycloalkyl" a cycloalkyl substitution on another cycloalkyl ring, wherein each cycloalkyl ring independently has from 3 to 7 carbon atom ring members and the cycloalkyls are linked through one single bond or have one common carbon atom. Examples of cycloalkylcycloalkyl include cyclopropylcyclopropyl (e.g. 1,1'-bicyclopropyl-2-yl), cyclohexylcyclohexyl wherein the two rings are linked through one single common carbon atom (e.g. 1,1'-bicyclo-hexyl-2-yl), cyclohexylcyclopentyl wherein the two rings are linked through one single bond (e.g. 4-cyclopentylcyclohexyl) and their different stereoisomers such as (1R,2S)-1, 1'-bicyclopropyl-2-yl and (1R,2R)-1,1'-bicyclopropyl-2-yl.The term "carbocycle" or "carbocyclyl" includes, unless otherwise indicated, in general a 3- to 12-membered, preferably a 3- to 8-membered or a 5- to 8-membered, more preferably a 5- or 6-membered mono-cyclic, ring comprising 3 to 12, preferably 3 to 8 or 5 to 8, more preferably 5 or 6 carbon atoms.

The carbocyclic radicals may be saturated, partially unsaturated, or fully unsaturated. Preferably, the term "carbocycle" covers cycloalkyl and cycloalkenyl groups as defined above, for example cyclopropane, cyclobutane, cyclopentane and cyclohexane rings. When it is referred to "fully unsaturated" carbocycles, this term also includes "aromatic" carbocycles. In certain preferred embodiments, a fully unsaturated carbocycle is an aromatic carbocycle as defined below, preferably a 6-membered aromatic carbocycle.

The term "hetaryl" or "aromatic heterocycle" or "aromatic heterocyclic ring" includes mono-cyclic 5- or 6-membered heteroaromatic radicals comprising as ring members 1, 2, 3, or 4 heteroatoms selected from N, O, and S. Examples of 5- or 6-membered heteroaromatic radicals include pyridyl, i.e. 2-, 3-, or 4-pyridyl, pyrimidinyl, i.e. 2-, 4- or 5-pyrimidinyl, pyrazinyl, pyridazinyl, i.e. 3- or 4-pyridazinyl, thienyl, i.e. 2- or 3-thienyl, furyl, i.e. 2-or 3-furyl, pyrrolyl, i.e. 2- or 3-pyrrolyl, oxazolyl, i.e. 2-, 3- or 5-oxazolyl, isoxazolyl, i.e. 3-, 4- or 5-isoxazolyl, thiazolyl, i.e. 2-, 3- or 5-thiazolyl, isothiazolyl, i.e. 3-, 4- or 5-isothiazolyl, pyrazolyl, i.e. 1-, 3-, 4- or 5-pyrazolyl, i.e. 1-, 2-, 4- or 5-imidazolyl, oxadiazolyl, e.g. 2- or 5-[1,3,4]oxadiazolyl, 4- or 5-(1,2,3-oxadiazol)yl, 3- or 5-(1,2,4-oxadiazol)yl, 2- or 5-(1,3,4-thiadiazol)yl, thiadiazolyl, e.g. 2- or 5-(1,3,4-thiadiazol)yl, 4- or 5-(1,2,3-thiadiazol)yl, 3- or 5-(1,2,4-thiadiazol)yl, triazolyl, e.g. 1H-, 2H- or 3H-1,2,3-triazol-4-yl, 2H-triazol-3-yl, 1H-, 2H-, or 4H-1,2,4-triazolyl and tetrazolyl, i.e. 1H- or 2H-tetrazolyl.

The terms "heterocycle", "heterocyclyl" or "heterocyclic ring" includes, unless otherwise indicated, in general 3- to 12-membered, preferably 3- to 8-membered, 3- to 7-membered, or 5-to 8-membered, more preferably 5- or 6-membered, in particular 6-membered monocyclic heterocyclic radicals. The heterocyclic radicals may be saturated, partially unsaturated, or fully unsaturated. As used in this context, the term "fully unsaturated" also includes "aromatic". In a preferred embodiment, a fully unsaturated heterocycle is thus an aromatic heterocycle, preferably a 5- or 6-membered aromatic heterocycle comprising one or more, e.g. 1, 2, 3, or 4, preferably 1, 2, or 3 heteroatoms selected from N, O and S as ring members. Examples of aromatic heterocycles are provided above in connection with the definition of "hetaryl". Unless otherwise indicated, "hetaryls" are thus covered by the term "heterocycles". The heterocyclic non-aromatic radicals usually comprise 1, 2, 3, 4 or 5, preferably 1, 2 or 3 heteroatoms selected from N, O, and S as ring members, where S-atoms as ring members may be present as S, SO or SO₂, and N-atoms may be oxidized, or non-oxidized. Examples of 5- or 6-membered heterocyclic radicals comprise saturated or unsaturated, non-aromatic heterocyclic rings, such as oxiranyl, oxetanyl, thietanyl, thietanyl-S-oxid (S-oxothietanyl), thietanyl-S-dioxid (S-dioxothiethanyl), pyrrolidinyl, pyrrolinyl, pyrazolinyl, tetrahydrofuranyl, dihydrofuranyl, 1,3-dioxolanyl, thiolanyl, S-oxothiolanyl, S-dioxothiolanyl, dihydrothienyl, S-oxodihydrothienyl, S-dioxodihydrothienyl, oxazolidinyl, oxazolinyl, thiazolinyl, oxathiolanyl, piperidinyl, piperazinyl, pyranyl, dihydropyranyl, tetrahydropyranyl, 1,3- and 1,4-dioxanyl, thiopyranyl, S.oxothiopyranyl, S-dioxothiopyranyl, dihydrothiopyranyl, S-oxodihydrothiopyranyl, S-dioxodihydrothiopyranyl, tetrahydrothiopyranyl, S-oxotetrahydrothiopyranyl, S-dioxotetrahydrothiopyranyl, morpholinyl, thiomorpholinyl, S-oxothiomorpholinyl, S-dioxothiomorpholinyl, thiazinyl and the like. Examples for heterocyclic ring also comprising 1 or 2 carbonyl groups as ring members comprise pyrrolidin-2-onyl, pyrrolidin-2,5-dionyl, imidazolidin-2-onyl, oxazolidin-2-onyl, thiazolidin-2-onyl and the like.

The erms "alkylene", "alkenylene", and "alkynylene" refer to alkyl, alkenyl, and alkynyl as defined above, respectively, which are bonded to the remainder of the molecule, via two atoms, preferably via two carbon atoms, of the respective group, so that they represent a linker between two moieties of the molecule. In particular, the term "alkylene" may refer to alkyl chains such as CH₂CH₂, -CH(CH₃)-, CH₂CH₂CH₂, CH(CH₃)CH₂, CH₂CH(CH₃), CH₂CH₂CH₂CH₂, CH₂CH₂CH₂CH₂CH₂, CH₂CH₂CH₂CH₂CH₂CH₂, and CH₂CH₂CH₂CH₂CH₂CH₂CH₂. Similarly, "alkenylene" and "alkynylene" may refer to alkenyl and alkynyl chains, respectively.

The term "5- to 6-membered carbocyclic ring" as used herein refers to cyclopentane and cyclohexane rings.

Examples of 5- or 6-membered saturated heterocyclic rings include: 2-tetrahydrofuranyl, 3-tetrahydrofuranyl, 2-tetrahydrothienyl, 3-tetrahydrothienyl, 2-pyrrolidinyl, 3-pyrrolidinyl, 3-pyrazolidinyl, 4-pyrazolidinyl, 5-pyrazolidinyl, 2-imidazolidinyl, 4-imidazolidinyl, 2-oxazolidinyl, 4-oxazolidinyl, 5-oxazolidinyl, 3-isoxazolidinyl, 4-isoxazolidinyl, 5-isoxazolidinyl, 2-thiazolidinyl, 4-thiazolidinyl, 5-thiazolidinyl, 3-isothiazolidinyl, 4-isothiazolidinyl, 5-isothiazolidinyl, 1,2,4-oxadiazolidin-3-yl, 1,2,4-oxadiazolidin 5 yl, 1,2,4-thiadiazolidin-3-yl, 1,2,4-thiadiazolidin-5-yl, 1,2,4-triazolidin-3-yl,-1,3,4-oxadiazolidin-2-yl, 1,3,4-thiadiazolidin-2-yl, 1,3,4-triazolidin-2-yl, 2-tetrahydropyranyl, 4-tetrahydropyranyl, 1,3-dioxan-5-yl, 1,4-dioxan-2-yl, 2-piperidinyl, 3-piperidinyl, 4-piperidinyl, 3-hexahydropyridazinyl, 4-hexahydropyridazinyl, 2-hexahydropyrimidinyl, 4-hexahydropyrimidinyl, 5-hexahydropyrimidinyl, 2-piperazinyl, 1,3,5-hexahydrotriazin-2-yl and 1,2,4-hexahydrotriazin-3-yl, 2-morpholinyl, 3-morpholinyl, 2-thiomorpholinyl, 3-thiomorpholinyl, 1-oxothiomorpholin-2-yl, 1-oxothiomorpholin-3-yl, 1,1-dioxothiomorpholin-2-yl, 1,1-dioxothio-morpholin-3-yl.

Examples of 5- or 6-membered partially unsaturated heterocyclyl or heterocyclic rings include: 2,3-dihydrofur-2-yl, 2,3-dihydrofur-3-yl, 2,4-dihydrofur-2-yl, 2,4-dihydrofur-3-yl, 2,3-dihydrothien-2-yl, 2,3-dihydrothien-3-yl, 2,4-dihydrothien-2-yl, 2,4-dihydrothien-3-yl, 2-pyrrolin-2-yl, 2-pyrrolin-3-yl, 3-pyrrolin-2-yl, 3-pyrrolin-3-yl, 2-isoxazolin-3-yl, 3-isoxazolin-3-yl, 4-isoxazolin 3 yl, 2-isoxazolin-4-yl, 3-isoxazolin-4-yl, 4-isoxazolin-4-yl, 2-isoxazolin-5-yl, 3-isoxazolin-5-yl, 4-isoxazolin-5-yl, 2-isothiazolin-3-yl, 3-isothiazolin-3-yl, 4-isothiazolin-3-yl, 2-isothiazolin-4-yl, 3-isothiazolin-4-yl, 4-isothiazolin-4-yl, 2-isothiazolin-5-yl, 3-isothiazolin-5-yl, 4-isothiazolin-5-yl, 2,3 dihydropyrazol-1-yl, 2,3-dihydropyrazol-2-yl, 2,3-dihydropyrazol-3-yl, 2,3-dihydropyrazol-4-yl, 2,3-dihydropyrazol-5-yl, 3,4-dihydropyrazol-1-yl, 3,4-dihydropyrazol-3-yl, 3,4-dihydropyrazol-4-yl, 3,4-dihydropyrazol-5-yl, 4,5-dihydropyrazol-1-yl, 4,5-dihydropyrazol-3-yl, 4,5-dihydropyrazol-4-yl, 4,5-dihydropyrazol-5-yl, 2,3-dihydrooxazol-2-yl, 2,3-dihydrooxazol-3-yl, 2,3-dihydrooxazol-4-yl, 2,3-dihydrooxazol-5-yl, 3,4-dihydrooxazol-2-yl, 3,4-dihydrooxazol-3-yl, 3,4-dihydrooxazol-4-yl, 3,4-dihydrooxazol-5-yl, 3,4-dihydrooxazol-2-yl, 3,4-dihydrooxazol-3-yl, 3,4-dihydrooxazol-4-yl, 2-, 3-, 4-, 5- or 6-di- or tetrahydropyridinyl, 3-di- or tetrahydropyridazinyl, 4-di- or tetrahydropyridazinyl, 2-di- or tetrahydropyrimidinyl, 4-di- or tetrahydropyrimidinyl, 5-di- or tetrahydropyrimidinyl, di- or tetrahydropyrazinyl, 1,3,5-di- or tetrahydrotriazin-2-yl.

Examples of 5- or 6-membered fully unsaturated heterocyclic (hetaryl) or heteroaromatic rings are: 2-furyl, 3-furyl, 2-thienyl, 3-thienyl, 2-pyrrolyl, 3-pyrrolyl, 3-pyrazolyl, 4-pyrazolyl, 5-pyrazolyl, 2-oxazolyl, 4-oxazolyl, 5-oxazolyl, 2-thiazolyl, 4-thiazolyl, 5-thiazolyl, 2-imidazolyl, 4-imidazolyl, 1,3,4-triazol-2-yl, 2-pyridinyl, 3-pyridinyl, 4-pyridinyl, 3-pyridazinyl, 4-pyridazinyl, 2-pyrimidinyl, 4-pyrimidinyl, 5-pyrimidinyl and 2-pyrazinyl.

A "C₂-C_{y}-alkylene" is divalent branched or preferably unbranched saturated aliphatic chain having 2 to m, e.g. 2 to 7 carbon atoms, for example CH₂CH₂, -CH(CH₃)-, CH₂CH₂CH₂, CH(CH₃)CH₂, CH₂CH(CH₃), CH₂CH₂CH₂CH₂, CH₂CH₂CH₂CH₂CH₂, CH₂CH₂CH₂CH₂CH₂CH₂, and CH₂CH₂CH₂CH₂CH₂CH₂CH₂.

The term "alkylamino" as used herein refers to a straight-chain or branched saturated alkyl group having 1 to 10 carbon atoms, preferably 1 to 4 carbon atoms, more preferably 1 to 3 carbon atoms, which is bonded via a nitrogen atom, e.g. an -NH- group.

The term "dialkylamino" as used herein refers to a straight-chain or branched saturated alkyl group having 1 to 10 carbon atoms, preferably 1 to 4 carbon atoms, more preferably 1 to 3 carbon atoms, which is bonded via a nitrogen atom, which is substituted by another straight-chain or branched saturated alkyl group having 1 to 10 carbon atoms, preferably 1 to 4 carbon atoms, more preferably 1 to 3 carbon atoms, e.g. a methylamino or ethylamino group.

The term "alkylthio "(alkylsulfanyl: alkyl-S-)" as used herein refers to a straight-chain or branched saturated alkyl group having 1 to 10 carbon atoms, preferably 1 to 4 carbon atoms (= C₁-C₄-alkylthio), more preferably 1 to 3 carbon atoms, which is attached via a sulfur atom. Examples include methylthio, ethylthio, propylthio, isopropylthio, and n-butylthio.

The term "haloalkylthio" as used herein refers to an alkylthio group as mentioned above wherein the hydrogen atoms are partially or fully substituted by fluorine, chlorine, bromine and/or iodine. Examples include chloromethylthio, bromomethylthio, dichloromethylthio, trichloromethylthio, fluoromethylthio, difluoromethylthio, trifluoromethylthio, chlorofluoromethylthio, dichlorofluoromethylthio, chlorodifluoromethylthio, 1-chloroethylthio, 1-bromoethylthio, 1-fluoroethylthio, 2-fluoroethylthio, 2,2-difluoroethylthio, 2,2,2-trifluoroethylthio, 2-chloro-2-fluoroethylthio, 2-chloro-2,2-difluoroethylthio, 2,2-dichloro-2-fluoroethylthio, 2,2,2-trichloroethylthio and pentafluoroethylthio and the like.

The term "alkylsulfinyl" (alkylsulfoxyl: C₁-C₆-alkyl-S(=O)-), as used herein refers to a straight-chain or branched saturated alkyl group (as mentioned above) having 1 to 10 carbon atoms, preferably 1 to 4 carbon atoms (= C₁-C₄-alkylsulfinyl), more preferably 1 to 3 carbon atoms bonded through the sulfur atom of the sulfinyl group at any position in the alkyl group.

The term "(halo)alkylsulfonyl" (alkyl-S(=O)₂-) as used herein refers to a straight-chain or branched saturated (and halogenated) alkyl group having 1 to 10 carbon atoms, preferably 1 to 4 carbon atoms (= C₁-C₄-(halo)alkylsulfonyl), preferably 1 to 3 carbon atoms, which is bonded via the sulfur atom of the sulfonyl group at any position in the (halo)alkyl group.

The term "(halo)alkylsulfanyl" (alkyl-S-) as used herein refers to a straight-chain or branched saturated (and halogenated) alkyl group having 1 to 10 carbon atoms, preferably 1 to 4 carbon atoms (= C₁-C₄-(halo)alkylsulfanyl), preferably 1 to 3 carbon atoms, which is bonded via the sulfur atom of the sulfanyl group at any position in the (halo)alkyl group.

The term "(halo)alkylsulfinyl" (alkyl-S(=O)-) as used herein refers to a straight-chain or branched saturated (and halogenated) alkyl group having 1 to 10 carbon atoms, preferably 1 to 4 carbon atoms (= C₁-C₄-(halo)alkylsulfinyl), preferably 1 to 3 carbon atoms, which is bonded via the sulfur atom of the sulfinyl group at any position in the (halo)alkyl group.

Accordingly, the terms (halo)alkylsulfanylalkyl, (halo)alkylsulfinylalkyl, and (halo)sulfonylalkyl as used for example in "C₁-C₄-alkylsulfanyl-C₁-C₄-alkyl", "C₁-C₄-alkylsulfinyl-C₁-C₄-alkyl", and "C₁-C₄-alkylsulfonyl-C₁-C₄-alkyl" relate to (halo)alkylsulfanyl-, (halo)alkylsulfinyl-, or (halo)alkylsulfonyl-groups that are bonded via the sulfur atom of the sulfanyl, the sulfinyl, or the sulfonyl group, respectively, to the alkyl group, which is in turn bonded to the rest of the molecule.

The term "S(O)(NR^{W})" refers to a group 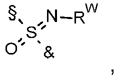 wherein R^{W} is as defined for formula (I) and wherein the symbols "&" and "§" mean the link to the remainder of the molecule. Accordingly, a group R^{W}-W as attached to ring "G" may have the meaning R^{W}-S(O)(NR^{W}), which would refer to a group 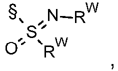 wherein R^{W} is as defined for formula (I) and wherein the symbol "§" means the link to the remainder of the molecule, i.e. ring "G".

The term "alkylcarbonyl" (C₁-C₆-C(=O)-) refers to a straight-chain or branched alkyl group as defined above, which is bonded via the carbon atom of a carbonyl group (C=O) to the remainder of the molecule.

The term "alkoxycarbonyl" refers to an alkoxygroup group as defined above, which is bonded via the carbon atom of a carbonyl group (C=O) to the remainder of the molecule.

The term "alkylaminocarbonyl" (C₁-C₆-NH-C(=O)-) refers to a straight-chain or branched alkylamino group as defined above, which is bonded via the carbon atom of a carbonyl group (C=O) to the remainder of the molecule. Similarly, the term "dialkylaminocarbonyl" refers to a straight-chain or branched saturated alkyl group as defined above, which is bonded to a nitrogen atom, which is substituted with another straight-chain or branched saturated alkyl group as defined above, which nitrogen atom in turn is bonded via a carbonyl group (C=O) to the remainder of the molecule.

The compounds of formula (I) can be prepared by standard methods of organic chemistry. If certain derivatives cannot be prepared by the processes outlined below, they can be obtained by derivatization of other compounds of formula (I) that are accessible by these methods.

Preparation methods that are generally useful for the preparation of compounds of formula (I) have been disclosed in WO2017/167832, especially p.4-6 and in the experimental section, WO2021/204577, especially p.15-26 and in the experimental section, and in the international patent application number WO2021/099240. In the following depicted Processes and Schemes, variables of formulae have a meaning as defined for formula (I) if not described otherwise. The variable "LG" refers to a leaving group, such as CI, Br, I, triflate, tosylate etc..

Compounds (3), falling under the definition of compounds (I) wherein Q is C(R⁶), may be prepared by reaction of compounds (1) with compounds (2) as displayed under Process 1. Process 1:

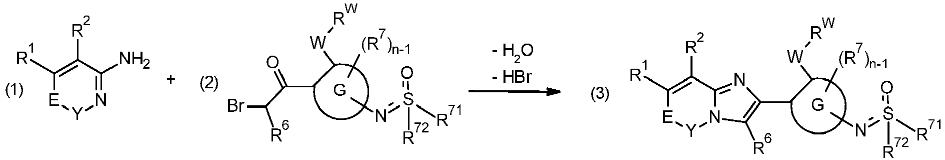

Reactions of this type have been described in EP3257853A1 and WO2018206479. The reaction is typically carried out under elevated temperatures of from 50-160 °C in an inert solvent. Suitable solvents are aliphatic hydrocarbons, such as pentane, hexane, cyclohexane, or petrol ether; aromatic hydrocarbons, such as benzene, toluene, o-, m-, and p-xylene; halogenated hydrocarbons, or halogenated aromatic C₆-C₁₀-hydrocarbons, such as CH₂Cl₂, CHCl₃, CCl₄, CH₂ClCH₂Cl, CCl₃CH₃, CHCl₂CH₂Cl, CCl₂CCl₂, or chlorobenzene; ethers, such as CH₃CH₂OCH₂CH₃, (CH₃)₂CHOCH(CH₃)₂, CH₃OC(CH₃)₃ (MTBE), CH₃OCH₃ (DME), CH₃OCH₂CH₂OCH₃, CH₃OC(CH₃)₂CH₂CH₃, dioxane, anisole, 2-methyltetrahydrofuran, tetrahydrofurane (THF), and diethylene glycol; nitriles, such as CH₃CN, and CH₃CH₂CN; alcohols, such as CH₃OH, CH₃CH₂OH, CH₃CH₂CH₂OH, CH₃CH(OH)CH₃, CH₃(CH₂)₃OH, and C(CH₃)₃OH, CH₂(OH)CH₂(OH), CH₃CH(OH)CH₂OH; amides and urea derivatives, such as dimethyl formamide (DMF), N-methyl-2-pyrrolidone (NMP), dimethyl acetamide (DMA), 1,3-dimethyl-2-imidazolidinone (DMI), 1,3-dimethyl-3,4,5,6-tetrahydro-2(1H)-pyrimidinone (DMPU), hexamethylphosphamide (HMPA); moreover dimethyl sulfoxide (DMSO), sulfolane, and water. Mixtures of the above solvents are also possible.

The reaction may be carried out in the presence of a catalyst, such as an acid or a base, preferably a base. Suitable bases are, in general, inorganic bases, such as LiOH, NaOH, KOH, and Ca(OH)₂; alkali metal and alkaline earth metal oxides, such as Li₂O, Na₂O, CaO, and MgO; alkali metal and alkaline earth metal hydrides, such as LiH, NaH, KH and CaH₂; alkali metal and alkaline earth metal carbonates, such as Li₂CO₃, K₂CO₃ and CaCO₃; alkali metal bicarbonates, such as NaHCO₃; organic bases, such as pyrrolidine; tertiary amines, such as diisopropylethylamine, trimethylamine, triethylamine, triisopropylamine and N-methylpiperidine, imidazol, pyridine; substituted pyridines, such as collidine, lutidine and 4-dimethylaminopyridine, and polycyclic amides and amidines, such as 1,8-diazabicycloundec-7-ene (DBU), 1,4-Diazabicyclo[2.2.2]octane (DABCO); alkali metal salts of secondary amines, such as alkali diisopropylamide, alkali bis(trimethylsilyl)amide, alkali tetramethylpiperidene; alcoholates, such as alkali methanolate, alkali ethanolate, alkali isopropanolate, alkali tert-butanolate; alkali metal - alkyl, and alkali metal - aryl salts, such as n-butyl lithium, tert-butyl lithium, phenyl lithium. Mixtures of the aforementioned bases are also possible. The bases are generally employed in catalytic amounts; however, they can also be used in equimolar amounts, in excess or, if appropriate, as solvent.

Compounds (1) can be prepared as described in WO2017/167832A1, e.g. Example C-1. Compounds (1) and compounds (2) are typically reacted with one another in equimolar amounts. In terms of yield, it may be advantageous to employ an excess of compounds (2).

Accordingly, compounds (2), may for example be prepared as depicted under Scheme-1. Scheme-1:

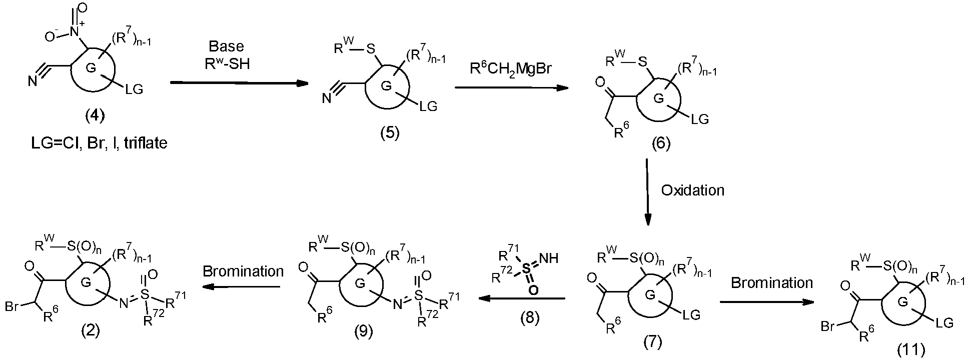

Compounds (5) can be obtained by the treatment of compound (4) by a displacement reaction of the NO₂-group (which can also be any other leaving group such as Cl,Br, I, F, triflate, tosylate etc.) with HS-R^{W} in presence of a base, preferably an inorganic base like K₂CO₃, Na₂CO₃, Cs₂CO₃, or NaH, in an inert solvent like DMF, THF and DMSO at a temperature of -70 to 30 °C. Such methods have been described in Tetrahedron Letters, 2014, vol. 55, 22, p. 3295 - 3298.

Compound (6) may then be prepared via reaction of Compound (5) with Grignard reagents R⁶CH₂MgBr. Such reactions are typically carried out at a temperature of -10 to 25 °C in an inert solvent as described in WO 2018095795, WO 2016012395 and Tetrahedron Letters 1981, vol. 22, 3815-3818. Suitable solvents are for example ethers as listed for Process 1, especially THF and MTBE, and aliphatic and aromatic hydrocarbons like toluene.

This Compound (6) may be further oxidised to SO (sulfoxide) and/or SO₂ (sulfone), in an oxidation reaction involving reagents such as, m-chloroperoxybenzoic acid, hydrogen peroxide, potassium peroxysulfate, sodium periodate, sodium hypochlorite or tert-butyl hypochlorite at a typical temperature of from 0 to 40 °C. Typical solvents used for the oxidations include aliphatic halogenated hydrocarbons and alcohols as listed for Process 1, such as CH₂CL₂ or CHCL₃, CH₃OH and CH₃CH₂OH, as well as CH₃COOH and H₂O. The amount of the oxidant to be used in the reaction is generally 1 to 3 moles, preferably 1 to 1.2 moles, relative to 1 mole of compound (6) as descried in WO2015/091945, WO2016107742 and WO2018095795.

Compound (9) may be prepared by reacting compounds (7) with a reagent (8) in a Buchwald-Hartwig-type amination reaction.

The reaction may be carried out at a temperature of from 20°C to the boiling point of the reaction mixture (such as from 80 to 130°C) over a period of from 1 to 12 hours in an inert solvent in the presence of a catalyst and a base, preferably under inert atmosphere. Typically, the reaction is carried out in a microwave reactor or a closed sealed reactor.

The catalyst may be a palladium based catalyst, involving for example bis(dibenzylideneacetone)palladium(0)(Pd(dba)2), tris(dibenzylideneacetone)dipalladium(0)(Pd₂(dba)₃; optionally in form of its chloroform adduct) or palladium(II) acetate, and a ligand, for example XantPhos ((5-diphenylphosphanyl-9,9-dimethyl-xanthen-4-yl)diphenylphosphane), RuPhos (2-dicyclohexyl-phosphino2',6'-diisopropoxybiphenyl), JohnPhos ([1,1-biphenyl]-2-ylbis(1,1-dimethyl-ethyl)phosphine), BINAP (2,2'-bis(diphenylphosphino)-1,1'-binaphthalene), tol-BINAP ([2,2'-bis(di-p-tolyl-phosphino)-1,1'-binaphthyl]) or tri-(o-tolyl)phosphine. In case the catalyst is palladium based, suitable bases are those as listed under Process 1, preferably inorganic bases like alkali metal and earth alkali metal carbonates such as Na₂CO₃, K₂CO₃, or Cs₂CO₃, or an organic base like sodium or potassium tert-butylate, or mixtures thereof; and suitable solvents are for example ethers and aliphatic and aromatic hydrocarbons as listed for Process 1, preferably dioxane, 1,2-dimethoxyethane or toluene, or mixtures thereof. Such reactions have been described, for example, in Journal of Organic Chemistry, 65(1), 169-175; 2000, Tetrahedron Letters, 39(32), 5731-5734; 1998, Chem. Commun., 47, 7665-7667; 2011 or Tetrahedron, 70(37), 6613-6622; 2014.

Alternatively, the reaction may be catalyzed by an iron-based catalyst, e.g. Fe(lll) chloride, in presence of a ligand, such as N,N'-dimethyl-1,2-ethanediamine. In case the catalyst is iron-based, the base is typical an inorganic base, preferably an alkali or earth alkali carbonate, such as Na₂CO₃, K₂CO₃, or Cs₂CO₃; and the solvent is for example selected from ethers and aliphatic and aromatic hydrocarbons as listed for Process 1, preferably dioxane, 1,2-dimethoxyethane or toluene, or mixtures thereof. Such reactions have been described, for example, in Advanced Synthesis & Catalysis, 350(3), 391-394; 2008.

As a further alternative, the reaction may be catalyzed by a copper-based catalyst, for example Cu(I)- iodide, Cu(I)-acetate or Cu₂O, optionally in presence of a ligand, such as N,N'-dimethyl-1,2-ethanediamine. In case the catalyst is a copper-based catalyst, the base is typically an inorganic base, like alkali metal and earth alkali metal carbonates such as Na₂CO₃, K₂CO₃, or Cs₂CO₃, or alkali metal phosphates like K₃PO₄, or mixtures thereof; and suitable solvents are for example ethers, amides and urea derivatives, aliphatic and aromatic hydrocarbons, as listed for Process 1, preferably dioxane, 1,2-dimethoxyethane, toluene, DMF; as well as DMSO, or mixtures thereof. Such reactions have been described, e.g., in Advanced Synthesis & Catalysis, 349(17+18), 2673- 2676; 2007, or Angewandte Chemie, International Edition, 48, 5691-5693; 2009.

Compounds (2) can then be prepared by halogenation of compounds 9 in an inert solvent at a temperature of from -10 to 80°C. Typical solvents are aliphatic hydrocarbons, aromatic hydrocarbons, halogenated hydrocarbons, ethers, esters, carbonates, nitriles, alcohols, amides and urea derivatives as listed for Process 1, preferably CHCl₃, CH₃COOCH₂CH₃, or CH₂Cl₂. Suitable brominating agentes include CuBr₂, Br₂, HBr in CH₃COOH, and trimethyl phenyl ammonium tribromide. Such procedures have been described in WO2016107742. These conditions may also be used for the bromination of compounds (7) to compounds (11) as displayed in Scheme-1.

Compounds (8), wherein R⁷¹ and R⁷² are as defined in formula (I), are either known compounds, commercially available or can be prepared by known methods, described in the literature, as for example in Journal of the Chemical Society, 3004-5; 1965. Of advantage are preparation methods for compounds (8) starting from readily available sulfide compounds (12) as described in WO2018130174, or Chem. Comm. 53, 348-351; 2017, or from the corresponding sulfoxide compounds (13) as described in J. Org. Chem, 81, 5886-5894, 2016, Angewandte Chemie, International Edition, 55, 7203-7207; 2016, WO2018130174, wherein R⁷¹ and R⁷² are as defined in formula I.

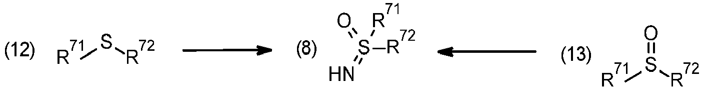

Compounds (3) may also be prepared by reacting compounds (1) with compounds (11) to generate compounds (14), followed by coupling with compounds (8) as displayed under Process 2.

### Process 2:

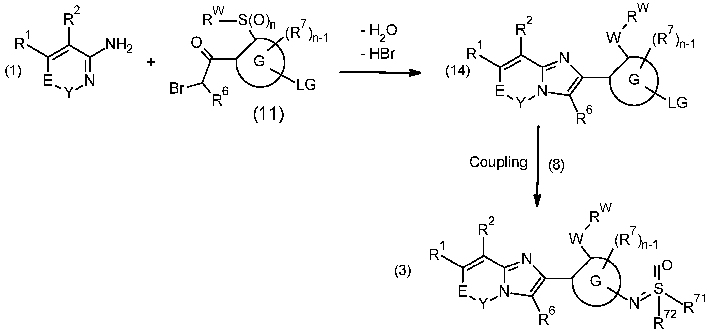

The reaction conditions for the condensation reaction of compounds (1) with compounds (11) can be performed as described above for Process 1. The coupling reaction of compound (14) with compounds (8) in a Buchwald-type process may be performed as described above for the synthesis of compounds (9) in Process 1.

### Process 3:

Compounds of formula (3) may also be prepared by a process of multiple conversions of compounds (14) as displayed under Process 3.

### Process 3:

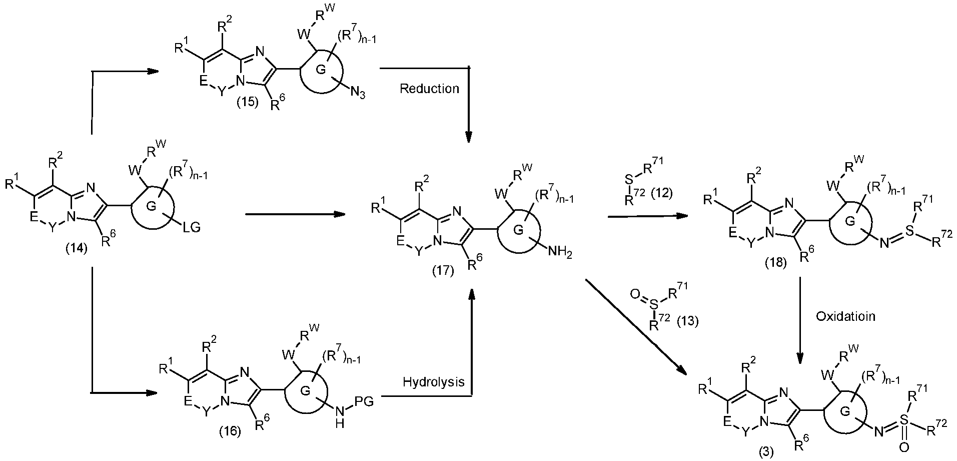

The variable "PG" in formula (16) is a protective group, e.g. imine, diphenylene, tert-butoxycarbonyl, or carboxybenzyl.

Compounds (17) may be prepared in two step process from compounds (14) involving a Buchwald-Hartwig-type amination followed by a hydrolysis step to remove the protective group PG. In the first step, compounds (14) may be reacted with an amine PG-NH₂ in the presence of a catalyst in an inert solvent. PG-NH₂ typically relates to tert-butylcarbamate, benzyl carbamate (wherein the phenyl may be optionally substituted by one or two methoxy), diphenylmethan-imine or phenylmethanimine.

The reaction may be carried out at a temperature of from 20°C to the boiling point of the reaction mixture (such as from 80 to 130°C) over a period of from 1 to 6 hours in an inert solvent in the presence of a palladium-based catalyst and a base, preferably under inert atmosphere. Typically, the reaction is carried out in a microwave reactor or a closed sealed reactor.

The palladium-based catalyst may be e.g. bis(dibenzylideneacetone)palladium(O)(Pd(dba)₂), tris(dibenzylideneacetone)dipalladium(O)(Pd₂(dba)₃; optionally in form of its chloroform adduct) or palladium(II) acetate, and a ligand, e.g. XantPhos ((5-diphenylphosphanyl-9,9-dimethyl-xanthen-4-yl)diphenylphosphane), RuPhos (2-dicyclohexylphosphino2',6'-diisopropoxybiphenyl), JohnPhos ([1,1-biphenyl]-2-ylbis(1,1-dimethyl-ethyl)phosphine), BINAP, tol-BINAP, or tri-(o-tolyl)phosphine. In case the catalyst is palladium based, suitable bases are those as listed under Process 1, preferably inorganic bases like alkali metal and earth alkali metal carbonates such as Na₂CO₃, K₂CO₃, or Cs₂CO₃, or an organic base like sodium or potassium tert-butylate, or mixtures thereof; and suitable solvents are for example ethers and aliphatic and aromatic hydrocarbons as listed for Process 1, preferably dioxane, 1,2-dimethoxyethane or toluene, or mixtures thereof. Such reactions have been described, for example, in Journal of Organic Chemistry, 65(1), 169-175; 2000, Tetrahedron Letters, 39(32), 5731-5734; 1998, Chem. Commun., 47, 7665-7667; 2011 or Tetrahedron, 70(37), 6613-6622; 2014.

The reaction may be alternatively carried out at a temperature of from 100°C to 180°C in an inert solvent in the presence of a Cu-based catalyst and a base, preferably under inert atmosphere. Typically, the reaction is carried out in a microwave reactor.

The Cu-based catalyst may for example involve Cul or copper(II) trifluoromethanesulfonate, optionally in the presence of a ligand, such as 2,2'-bipyridine, proline, N,N'-dimethyl glycine or ethylene glycol. The base is typically an inorganic base, like alkali metal and earth alkali metal carbonates such as Na₂CO₃, K₂CO₃, or Cs₂CO₃, or organic bases, preferably tertiary amines such as triethylamine, and alkali metal alcoholates such as sodium methanolate, sodium tert-butanolate, or. potassium tert-butanolate, or a mixture thereof. Suitable solvents are for example ethers, amides and urea derivatives, as listed for Process 1, preferably dioxane, DMF and N-methylpyrrolidinone; as well as DMSO, or mixtures thereof.

Hydrolysis of compounds (16) may be conducted usually under aqueous, either acidic or basic, conditions. Typically, compounds of the formula (16) are treated with aqueous hydrogen chloride, or trifluoroacetic acid, optionally in the presence of an organic solvent, such as 1,4-dioxane, THF or dichloromethane, at reaction temperatures ranging preferentially from 10°C to the boiling point of the reaction mixture, or the reaction may be performed under microwave irradiation.

Alternatively, compounds (14) may be converted to compounds (17) by a two-step process involving azidation followed by reduction of the azide group to the corresponding amine moiety. Azidation to compounds (15) may be achieved by reacting compound (14) with, for example, sodium- or trimethylsilylazide. Such transformations may be conducted in an inert solvent, in the presence of a copper-based catalyst, such as copper iodide, optionally in the further presence of a ligand, such as proline or N,N'-dimethylethylenediamine at temperatures of from 50 to 150°C. Typicyl solvents are DMSO and urea or amide based solvents like DMF.

Reduction may be conducted under conditions as described in (R.C. Larock, Synthetic Organic Methodology: Comprehensive Organic Transformations. A Guide to Functional Group Preparations, 1989; p. 409) in various organic or aqueous solvents compatible to these conditions, at temperatures from below 0 °C up to the boiling point of the solvent system. Alternatively, the conversion of compounds (14) to compounds (17) may be done in one step, whereby the intermediate azide (15) is reduced in situ under copper catalyst conditions as described in, Journal of Organic Chemistry (2010), 75(14), 4887-4890 and WO 2016/091731.

Compounds (3) may then be prepared from compounds (17) as displayed under Process 3. In one alternative, compounds (18) may be prepared by reacting compounds (17) with a sulfide reagent of the formula (12) under imination reaction conditions. Typical reaction conditions on such procedures can be found in Org. Lett. 2004, 6, 1305-1307, or Chem. Lett. 2004, 33, 482-487, or J. Org. Chem., 40(19), 2758-64; 1975, Synthesis, 2000, 1-64.

In a second alternative, compounds of the formula (3) may be prepared by reacting compounds (17) with a sulfoxide reagent of the formula (13) under similar imination reaction conditions. Typical preparation methods and reaction conditions may be found in Org. Lett. 2004, 6, 1305-1307 Chem. Eur. J. 2004, 10, 906-916.

Of particular interest are metal-free imination methods, wherein compounds (17) are reacted with either the compounds (12) or compounds (13) in the presence of an oxidant like iodbenzene-I,I-diacetate (hypervalent iodine) as described in Tet. Lett. 2005, 46, 8007-8008; or N-bromosuccinimide (NBS) and an organic base such as sodium or potassium tert-butoxide as described in Synthesis 2010, No 17, 2922-2925. Oxidants such as N-iodosuccinimide (NIS) or iodine may be also used alternatively as described, e.g., in Org. Lett. 2007, 9, 3809-3811. An example of hypochlorite salts being used as oxidant, such as NaOCl or Ca(OCl)₂, was described in WO2008/106006.

Alternatively, compounds (3) can also be prepared from compounds (17) by reaction it with sulfoxide reagent (13) by involving phosphorous pentoxide reagent as described in Monatsh. Chem. 101, 396-404; 1970), or (J. Org. Chem. 40, 2758-764; 1975).

Last, compounds (18) may be oxidized to yield compounds (3) by conditions as described above for the oxidation of compounds (6) to yield compounds (7).

Compounds (22), falling under the definition of compounds (I) wherein Q is N(R⁵), may be prepared by reaction of compounds (19) with compounds (20) via amide formation and condensation or direct condensation reaction as displayed under Process 4.

### Process 4:

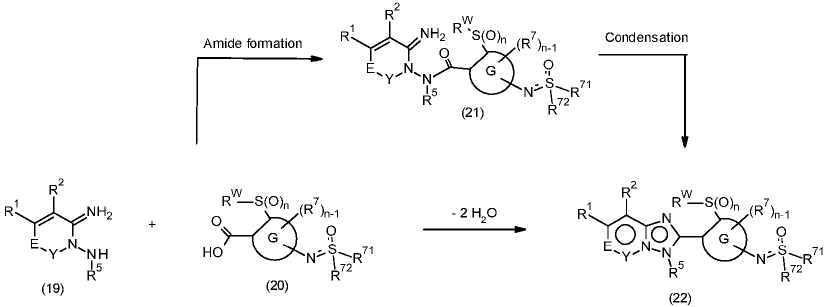

Reactions of this type have been described in EP3257853A1, WO2019/234160 and WO2016162318A1. The reaction is typically carried out at elevated temperatures, e.g. 60 to 160°C, in an inert solvent, optionally in the presence of an acid, or a coupling agent and a base. Suitable solvents are aliphatic hydrocarbons, such as pentane, hexane, cyclohexane, or petrol ether; aromatic hydrocarbons, such as benzene, toluene, o-, m-, and p-xylene; halogenated hydrocarbons, or halogenated aromatic C₆-C₁₀-hydrocarbons, such as CH₂Cl₂, CHCl₃, CCl₄, CH₂ClCH₂Cl, CCl₃CH₃, CHCl₂CH₂Cl, CCl₂CCl₂, or chlorobenzene; ethers, such as CH₃CH₂OCH₂CH₃, (CH₃)₂CHOCH(CH₃)₂, MTBE, DME, CH₃OCH₂CH₂OCH₃, CH₃OC(CH₃)₂CH₂CH₃, dioxane, anisole, 2-methyltetrahydrofuran, THF, and diethylene glycol; nitriles, such as CH₃CN, and CH₃CH₂CN; alcohols, such as CH₃OH, CH₃CH₂OH, CH₃CH₂CH₂OH, CH₃CH(OH)CH₃, CH₃(CH₂)₃OH, and C(CH₃)₃OH, CH₂(OH)CH₂(OH), and CH₃CH(OH)CH₂OH. Mixtures of the above solvents are also possible. Suitable acids are in general inorganic acids such as HF, HCl, HBr, H₂SO₄ and HClO₄; Lewis acids, such as BF₃, AlCl₃, FeCl₃, SnCl₄, TiCl₄ and ZnCl₂, moreover organic acids such as HCOOH, CH₃COOH, CH₃CH₂COOH, oxalic acid, toluene sulphonic acid, benzene sulphonic acid, camphor sulphonic acid, citric acid, and CF₃COOH.

Suitable coupling agents are selected from carbodiimides, such as DCC (dicyclohexylcarbodiimide) and DIC (diisopropylcarbodiimide), benzotriazole derivatives, such as HATU (O-(7-azabenzotriazol-1-yl)-N,N,N',N'-tetramethyluronium hexafluorophosphate), HBTU ((Obenzo-triazol-1-yl)-N,N,N',N'-tetramethyluronium hexafluorophosphate) and HCTU (1H-benzotriazolium-1-[bis(dimethylamino)methylene]-5-chloro tetrafluoroborate) and phosphonium-derived activators, such as BOP ((benzotriazol-1-yloxy)-tris(dimethylamino) phosphonium hexafluorophosphate), PyBOP ((benzotriazol-1-yloxy)-tripyrrolidinphosphonium hexafluorophosphate), and PyBrOP (bromotripyrrolidinphosphonium hexafluorophosphate). In case a coupling agent is applied, suitable solvents are aliphatic hydrocarbons, such as pentane, hexane, cyclohexane, or petrol ether; aromatic hydrocarbons, such as benzene, toluene, o-, m-, and p-xylene; halogenated hydrocarbons, or halogenated aromatic C₆-C₁₀-hydrocarbons, such as CH₂Cl₂, CHCl₃, CCl₄, CH₂ClCH₂Cl, CCl₃CH₃, CHCl₂CH₂Cl, CCl₂CCl₂, or chlorobenzene; ethers, such as CH₃CH₂OCH₂CH₃, (CH₃)₂CHOCH(CH₃)₂, MTBE, DME, CH₃OCH₂CH₂OCH₃, CH₃OC(CH₃)₂CH₂CH₃, dioxane, anisole, 2-methyltetrahydrofuran, THF, and diethylene glycol; nitriles, such as CH₃CN, and CH₃CH₂CN; alcohols, such as CH₃OH, CH₃CH₂OH, CH₃CH₂CH₂OH, CH₃CH(OH)CH₃, CH₃(CH₂)₃OH, and C(CH₃)₃OH, CH₂(OH)CH₂(OH), and CH₃CH(OH)CH₂OH. Mixtures of the above solvents are also possible. Suitable bases are those listed for Process 1.

Alternatively, compounds (20) may be replaced by their corresponding carbonic acid halogenides, e.g. acid chlorides. In this case the reaction is typically carried out in the presence of a base. Suitable bases are those listed for Process 1 above.

In a first step, compounds (21) are thus obtained, which undergo in a second step a condensation reaction as described for Process 1 to yield compounds (22).

Compounds (19) and compounds (20) are typically reacted with one another in equimolar amounts. In terms of yield, it may be advantageous to employ an excess of compounds (19). Compounds (20) may be prepared as described in WO2019/234160. Compounds (19) can be prepared as described in WO2017/167832A1, Example C-4, or Bashandy et al. Journal of Enzyme Inhibition and Medicinal Chemistry, 29(5), 619-627, 2014.

Accordingly, compounds (20) may for example be prepared as follows and depicted under Process 4a.

### Process 4a:

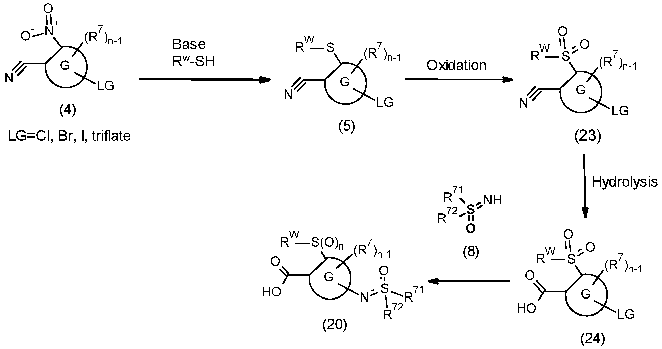

Compound (5) can be obtained from compounds (4) as described above. Compounds (5) may then be oxidised to SO (sulfoxide) and/or SO₂ (sulfone), by oxidation reaction involving reagents such as, m-chloroperoxybenzoic acid, H₂O₂, potassium peroxymonosulfate, NalO₄, NaClO or tert-butyl hypochlorite at a typical temperature of from 0 to 40 °C in an inert solvent. Suitable solvents used for the oxidations include aliphatic halogenated hydrocarbons such as CH₂Cl₂ and CHCL₃; alcohols such as CH₃OH and CH₃CH₂OH; CH₃COOH; and H₂O. The amount of the oxidant to be used in the reaction is generally 1 to 3 moles, preferably 1 to 1.2 moles, relative to 1 mole of the sulfide compounds (5) to produce the sulfoxide compounds, and preferably 2 to 2.2 moles of oxidant, relative to 1 mole of the sulfide compounds (5) as descried in WO2015/091945, WO2016107742, and WO2018095795.

Hydrolysis of compounds (23) can be achieved for example through heating compounds (23) in concentrated acid, such as concentrated HCl, or H₂SO₄., preferably in the presence of water, optionally in the presence of an inert solvent, such as CH₃COOH or ethers (for example THF, ethylene glycol dimethyl ether or 1,4-dioxane) generates compounds (24). The hydrolysis is typically carried out at elevated temperatrues of 40 to 120 °C. Such hydrolysis conditions, and variants thereof, are known to a skilled person.

Buchwald-Hartwig type amination (as described for the synthesis of compound of formulae (3) or (9)) can be used to generate compounds (20) by reaction of compounds (24) with compounds (8).

Compounds (20) may alternatively be prepared according to Process 4b displayed below.

### Process 4b:

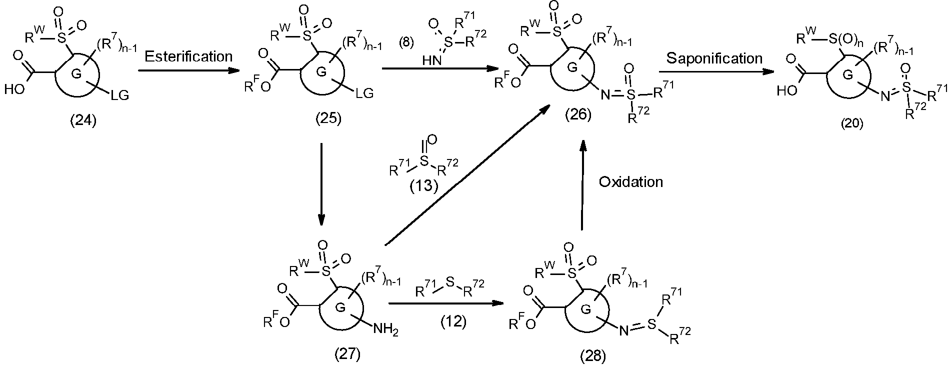

The process involves esterification of compounds (24) to generate compounds (25) in which R^{F} is C₁-C₆-alkyl, under conditions known to a person skilled in the art for example under acidic condition by refluxing in CH₃CH₂OH, CH₃OH or other alcoholic solvents; or under basic condition using an inorganic base like alkali carbonates and hydroxides, such as K₂CO₃, Cs₂CO₃, Na₂CO₃, NaOH, KOH and by adding electrophiles containing an alkyl halide. Compounds (25) may then be further converted by reaction with compounds (8) under Buchwald-Hartwig type C-N coupling to compounds (26), wherein RF is C₁-C₆-alkyl, as described in Process 1) for the generation of compounds (3). Then, compounds (26) may be saponified to compounds (20) under conditions known to a person skilled in the art (using for example conditions such as: aqueous NaOH, KOH, or LiOH in CH₃OH, CH₃CH₂OH, or dioxane at 20 to 100°C.

Compounds (26), may also be prepared by oxidation of compounds (28) under conditions already described above (see process 4). Alternatively, compounds (26) may also be prepared from compounds (27), wherein R^{F} is C₁-C₆-alkyl, by reaction with compounds (13) under conditions already described above in Process 3 for the generation of compounds (3) from compounds (17).

Compounds (28), wherein R^{F} is C₁-C₆-alkyl, may be prepared by reacting compounds (27), with a reagent (12), under conditions already described above for the generation of compounds (18) from compounds (17) in Process 3.

Compounds (27), wherein R^{F} is C₁-C₆-alkyl may be prepared from compounds (25), wherein R^{F} is C₁-C₆-alkyl, under conditions already described above in Process 3 for the generation of compounds (17).

Compounds (27), wherein R^{F} is C₁-C₆-alkyl are either known compounds, commercially available or may be prepared by known methods, described in the literature, as for example in WO2016005263, WO2016023954, WO2016026848, and WO2016104746.

Alternatively, compounds (22) falling under the definition of compounds (I), wherein Q is N(R⁵) may be prepared as displayed under Process 5 and Process 5a.

### Process 5:

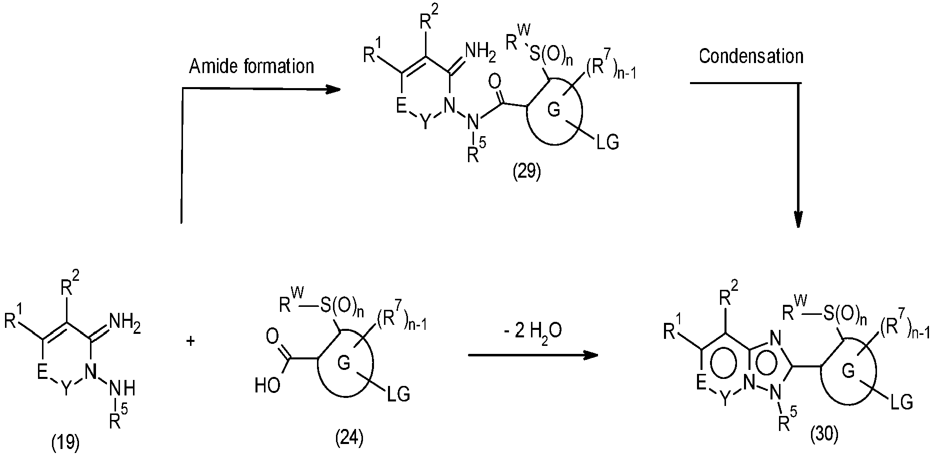

First, compounds (30) may be prepared by reaction of compounds (19) with compounds (24) as displayed under Process 5. The amide formation reaction to generate compounds (29) followed by condensation to generate compounds (30) and/or direct reaction of compounds (19) with compounds (24) to generate compounds (30) can be performed as discussed under Process 4. Subsequently, compounds (22) may be prepared by reaction of compounds (30) with compounds (8) under Buchwald-Hartwig type coupling reaction as described under Process 1 for the generation of compounds of formulae (3) or (9) as displayed under Process 5a.

### Process 5a:

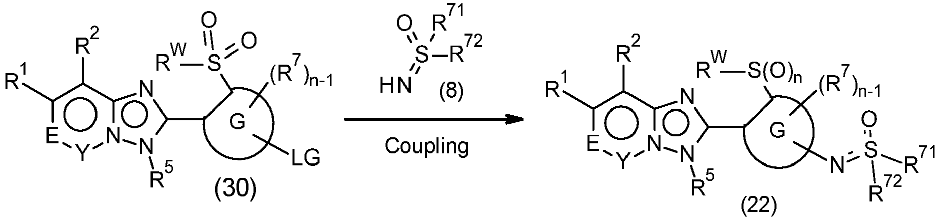

Compounds (22) may also be prepared from compounds (31) as displayed below under Process 7 in a rearrangement reaction.

### Process 7:

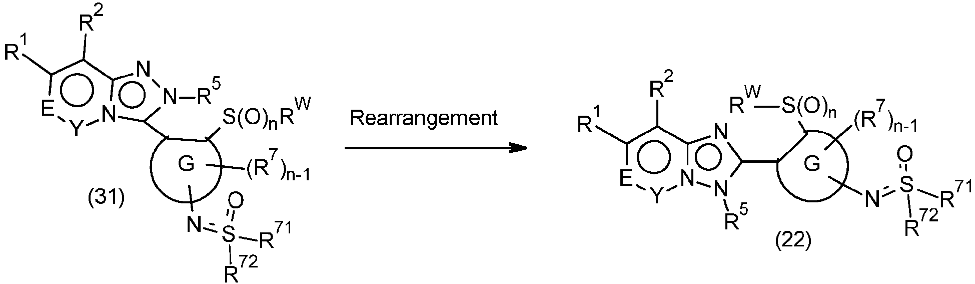

Reactions of this type have been described in Potts K.T., Surapaneni C.R., 1970, Journal of Heterocyclic Chemistry, or Nagamatsu T., Fujita T., 2002, Heterocycles, 57(4), 631-636. The reaction is typically carried out in the presence of a catalyst, usually an acid or a base, such as NaOH or formic acid, in an inert organic solvent or H₂O at a temperature of from 0 to 80°C. If no catalyst is used, the reaction may be carried out at elevated temperatrues, e.g. from 30 to 100°C. Compounds (31) may be prepared in analogy to the methods described in WO 2021/099240 (Processes 17-19) by modification of the educts.

Compounds of formula (I), wherein the variable W is S(O)(NR^{W}) may be produced from compounds of formula (I), wherein the variable W is S by a sequential imidation/oxidation reaction as described by Dannenberg et al., Synthesis (2015), 47, 1951-1959.

The reaction mixtures are worked up in a customary manner, for example by mixing with water, separating the phases and, if appropriate, chromatographic purification of the crude products. Some of the intermediates and end products are obtained in the form of colorless or slightly brownish viscous oils which are purified or freed from volatile components under reduced pressure and at moderately elevated temperature. If the intermediates and end products are obtained as solids, purification can also be carried out by recrystallization or digestion.

The N-oxides may be prepared from the inventive compounds according to conventional oxidation methods, e. g. by treating compounds I with an organic peracid such as metachloroperbenzoic acid (cf. WO03/64572 or J. Med. Chem. 38(11), 1892-903, 1995); or with inorganic oxidizing agents such as hydrogen peroxide (cf. J. Heterocyc. Chem. 18(7), 1305-8, 1981) or potassium peroxymonosulfate (cf. J. Am. Chem. Soc. 123(25), 5962-5973, 2001). The oxidation may lead to pure mono-N-oxides or to a mixture of different N-oxides, which can be separated by conventional methods such as chromatography.

If the synthesis yields mixtures of isomers, a separation is generally not necessarily required since in some cases the individual isomers can be interconverted during work-up for use or during application (for example under the action of light, acids or bases). Such conversions may also take place after use, for example in the treatment of plants in the treated plant, or in the harmful fungus to be controlled.

A skilled person will readily understand that the preferences for the substituents, also in particular the ones given in the tables below for the respective substituents, given herein in connection with compounds (I) apply for the intermediates accordingly. Thereby, the substituents in each case have independently of each other or more preferably in combination the meanings as defined herein.

The variables have, each on their own and in combination, the following preferred meanings. In one embodiment of compounds of formula (I), X is O. In another embodiment of compounds of formula (I), X is S.

In one embodiment of compounds of formula (I), E is NR³ and Q is CR⁶. In another embodiment of compounds of formula (I), E is CR⁴ and Q is NR⁵. In another embodiment of compounds of formula (I), E is NR³ and Q is NR⁵.

In one embodiment of the invention the compound of formula (I) is a compound of formula (I.A), a compound (I.B), or a compound of formula (I.C). 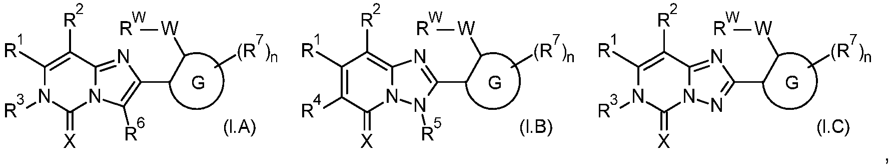 wherein all variables have a meaning as defined for formula (I). In one embodiment the compounds of formula (I) are compounds of formula (I.A). In another embodiment, the compounds of formula (I) are compounds of formula (I.B). In another embodiment, the compounds of formula (I) are compounds of formula (I.C).

In one embodiment R¹ is H, halogen, C₁-C₆-alkyl, C₂-C₆-alkenyl, C₂-C₆-alkynyl, C₁-C₆-alkoxy, C₁-C₆-alkoxy-C₁-C₆-alkyl, C₃-C₆-cycloalkyl, C₃-C₆-cycloalkoxy, C₁-C₆-alkylsulfenyl, C₁-C₆-alkyl-sulfinyl, or C₁-C₆-alkylsulfonyl, which groups are unsubstituted or halogenated.

In one embodiment, R¹ is H, C₁-C₃-alkyl, C₂-C₃-alkenyl, C₂-C₃-alkynyl, C₁-C₃-alkoxy, C₁-C₃-alkoxy-C₁-C₃-alkyl, C₃-C₅-cycloalkyl, C₃-C₅-cycloalkoxy, which groups are unsubstituted or halogenate. In one embodiment, R¹ is H, C₁-C₃-alkyl, or C₁-C₃-alkoxy, which groups are unsubstituted or halogenated. In another embodyment, R¹ is H, C₁-C₃-alkyl, C₂-C₃-alkenyl, C₂-C₃-alkynyl, C₁-C₃-alkoxy, which groups are unsubstituted or halogenated.

In another embodiment, R¹ is C₁-C₃-alkyl, C₁-C₃-alkoxy, which groups are unsubstituted or halogenate. In another embodiment, R¹ is C₁-C₃-haloalkyl, preferably CF₃.

R², R⁴, R⁶ are independently H, halogen, N₃, CN, NO₂, SCN, SF₅; C₁-C₆-alkyl, C₁-C₆-alkoxy, C₂-C₆-alkenyl, tri-C₁-C₆-alkylsilyl, C₂-C₆-alkynyl, C₁-C₆-alkoxy-C₁-C₄-alkyl, C₁-C₆-alkoxy-C₁-C₄-alkoxy, C₃-C₆-cycloalkyl, C₃-C₆-cycloalkoxy, C₃-C₆-cycloalkyl-C₁-C₄-alkyl, C₃-C₆-cycloalkoxyx-C₁-C₄-alkyl, which groups are unsubstituted or substituted with halogen; C(=O)OR^{A}, NR^{B}R^{C}, NOR^{A}, ONR^{B}R^{C}, C₁-C₆-alkylen-NR^{B}R^{C}, O-C₁-C₆-alkylen-NR^{B}R^{C}, C₁-C₆-alkylen-CN, NH-C₁-C₆-alkylen-NR^{B}R^{C}, C(=O)NR^{B}R^{C}, C(=O)R^{P}, C(=S)R^{D}, SO₂NR^{B}R^{C}, S(=O)ₘR^{E}; phenyl or benzyl, wherein the phenyl ring is unsubstituted or substituted with one or more R^{F}.

In one embodiment, R² is H, halogen; C₁-C₃-alkyl, C₁-C₃-alkoxy, C₂-C₃-alkenyl, tri-C₁-C₆-alkyl-silyl, C₂-C₆-alkynyl, C₁-C₃-alkoxy-C₁-C₂-alkyl, C₁-C₃-alkoxy-C₁-C₂-alkoxy, C₃-C₅-cycloalkyl, C₃-C₅-cycloalkoxy, C₃-C₅-cycloalkyl-C₁-C₂-alkyl, C₃-C₅-cycloalkoxyx-C₁-C₂-alkyl, which groups are unsubstituted or substituted with halogen. In another embodiment, R² is H, halogen; C₁-C₃-alkyl, C₁-C₃-alkoxy, C₂-C₃-alkenyl, C₂-C₆-alkynyl, which groups are unsubstituted or substituted with halogen. In another embodiment, R² is H, halogen; C₁-C₃-alkyl, C₁-C₃-alkoxy, which groups are unsubstituted or substituted with halogen. In another embodiment, R² is H, or C₁-C₃-alkyl, preferably H.

In one embodiment, R⁴ is H, halogen; C₁-C₃-alkyl, C₁-C₃-alkoxy, C₂-C₃-alkenyl, tri-C₁-C₆-alkyl-silyl, C₂-C₆-alkynyl, C₁-C₃-alkoxy-C₁-C₂-alkyl, C₁-C₃-alkoxy-C₁-C₂-alkoxy, C₃-C₅-cycloalkyl, C₃-C₅-cycloalkoxy, C₃-C₅-cycloalkyl-C₁-C₂-alkyl, C₃-C₅-cycloalkoxyx-C₁-C₂-alkyl, which groups are unsubstituted or substituted with halogen. In another embodiment, R⁴ is H, halogen; C₁-C₃-alkyl, C₁-C₃-alkoxy, C₂-C₃-alkenyl, C₂-C₆-alkynyl, which groups are unsubstituted or substituted with halogen. In another embodiment, R⁴ is H, halogen; C₁-C₃-alkyl, C₁-C₃-alkoxy, which groups are unsubstituted or substituted with halogen. In another embodiment, R⁴ is H; C₁-C₃-alkyl, or C₁-C₃-haloalkyl. In another embodiment, R⁴ is H, or C₁-C₃-alkyl. In another embodiment, R⁴ is H.

In one embodiment, R⁶ is H, halogen; C₁-C₃-alkyl, C₁-C₃-alkoxy, C₂-C₃-alkenyl, tri-C₁-C₆-alkylsilyl, C₂-C₆-alkynyl, C₁-C₃-alkoxy-C₁-C₂-alkyl, C₁-C₃-alkoxy-C₁-C₂-alkoxy, C₃-C₅-cycloalkyl, C₃-C₅-cycloalkoxy, C₃-C₅-cycloalkyl-C₁-C₂-alkyl, C₃-C₅-Cycloalkoxyx-C₁-C₂-alkyl, which groups are unsubstituted or substituted with halogen. In another embodiment, R⁶ is H, halogen; C₁-C₃-alkyl, C₁-C₃-alkoxy, C₂-C₃-alkenyl, C₂-C₆-alkynyl, which groups are unsubstituted or substituted with halogen. In another embodiment, R⁶ is H, halogen; C₁-C₃-alkyl, C₁-C₃-alkoxy, which groups are unsubstituted or substituted with halogen. In another embodiment, R⁶ is H, C₁-C₃-alkyl, C₁-C₃-haloalkyl. In another embodiment, R⁶ is H, or C₁-C₃-alkyl, preferably H.

Typically, R¹ is H, C₁-C₃-alkyl, or C₁-C₃-alkoxy, which groups are unsubstituted or halogenated; and R² is H, halogen, C₁-C₃-alkyl, C₁-C₃-alkoxy, C₂-C₃-alkenyl, or C₂-C₃-alkynyl, which groups are unsubstituted or halogenated.

R³, R⁵ are independently C₁-C₆-alkyl, C₁-C₆-alkoxy, C₂-C₆-alkenyl, C₂-C₆-alkynyl, C₁-C₆-alkoxy-C₁-C₄-alkyl, C₁-C₆-alkoxy-C₁-C₄-alkoxy, C₃-C₆-cycloalkyl, C₃-C₆-cycloalkyl-C₁-C₄-alkyl, or C₃-C₆-cycloalkoxy-C₁-C₄-alkyl, which are unsubstituted or halogenated; C(=O)OR^{A}, NR^{B}R^{C}, C₁-C₆-alkylen-NR^{B}R^{C}, O-C₁-C₆-alkylen-NR^{B}R^{C}, C₁-C₆-alkylen-CN, NH-C₁-C₆-alkylen-NR^{B}R^{C}, C(=O)NR^{B}R^{C}, C(=O)R^{D}, C(=S)R^{D}, SO₂NR^{B}R^{C}, S(=O)ₘR^{E}; phenyl or benzyl, wherein the phenyl ring is unsubstituted or substituted with one or more R^{F}.

In one embodiment, R³ is C₁-C₆-alkyl, C₁-C₆-alkoxy, C₂-C₆-alkenyl, C₂-C₆-alkynyl, C₁-C₆-alkoxy-C₁-C₄-alkyl, C₃-C₆-cycloalkyl, C₃-C₆-cycloalkyl-C₁-C₄-alkyl, C₃-C₆-cycloalkoxy-C₁-C₄-alkyl, or C₁-C₃-alkoxy-C₁-C₃-alkyl which are unsubstituted or halogenated; phenyl or benzyl, wherein the phenyl ring is unsubstituted or substituted with one or more R^{F}. In another embodiment embodiment, R³ is C₁-C₄-alkyl, C₂-C₄-alkenyl, C₂-C₄-alkynyl, C₃-C₆-cycloalkyl, C₃-C₆-cycloalkyl-C₁-C₂-alkyl, or C₁-C₃-alkoxy-C₁-C₃-alkyl, which are unsubstituted or halogenated; phenyl or benzyl, wherein the phenyl ring is unsubstituted or substituted with one or more R^{F}. In another embodiment, R³ is C₁-C₄-alkyl, C₂-C₄-alkenyl, C₂-C₄-alkynyl, C₃-C₆-cycloalkyl, C₃-C₆-cycloalkyl-C₁-C₂-alkyl, which are unsubstituted or halogenated; phenyl or benzyl, wherein the phenyl ring is unsubstituted or halogenated. In another embodiment, R³ is C₁-C₃-alkyl, C₂-C₃-alkenyl, C₂-C₃-alkynyl, C₃-C₆-cycloalkyl, C₃-C₆-cycloalkyl-C₁-C₂-alkyl, which are unsubstituted or halogenated. In another embodiment, R³ is C₁-C₃-alkyl, C₂-C₃-alkynyl, C₃-C₆-cycloalkyl, C₃-C₆-cycloalkyl-C₁-C₂-alkyl, which are unsubstituted or halogenated. In another embodiment, R³ is C₁-C₃-alkyl, cyclopropyl, or cyclopropylmethyl which are unsubstituted or halogenated. In another embodiment, R³ is C₁-C₃-alkyl, C₃-C₆-cycloalkyl, C₃-C₆-cycloalkyl-C₁-C₂-alkyl, which are unsubstituted or halogenated. In another embodiment, R³ is C₁-C₃-alkyl, preferably methyl, which are unsubstituted or halogenated.

In one embodiment, R⁵ is C₁-C₆-alkyl, C₁-C₆-alkoxy, C₂-C₆-alkenyl, C₂-C₆-alkynyl, C₁-C₆-alkoxy-C₁-C₄-alkyl, C₁-C₆-alkoxy-C₁-C₄-alkoxy, C₃-C₆-cycloalkyl, C₃-C₆-cycloalkyl-C₁-C₄-alkyl, or C₃-C₆-cycloalkoxy-C₁-C₄-alkyl, which are unsubstituted or halogenated. In another embodiment embodiment, R⁵ is C₁-C₃-alkyl, C₁-C₃-alkoxy, C₂-C₃-alkenyl, C₂-C₃-alkynyl, C₃-C₆-cycloalkyl, C₃-C₆-cycloalkyl-C₁-C₂-alkyl, which are unsubstituted or halogenated. In another embodiment, R⁵ is C₁-C₃-alkyl, C₂-C₃-alkenyl, C₂-C₃-alkynyl, which are unsubstituted or halogenated. In another embodiment, R⁵ is C₁-C₃-alkyl, or C₁-C₃-haloalkyl. In another embodiment, R⁵ is C₁-C₃-alkyl, preferably methyl, which are unsubstituted or halogenated.

In one embodiment, R³ is C₁-C₄-alkyl, C₂-C₄alkenyl, C₂-C₄ alkynyl, C₁-C₃-alkoxy-C₁-C₃-alkyl, C₃-C₆ cycloalkyl, C₃-C₆ cycloalkyl-C₁-C₂-alkyl, which groups are unsubstituted or halogenated, and R⁶ is H, C₁-C₃-alkyl or C₁-C₃-haloalkyl.

In another embodiment R⁴ is H, or C₁-C₃ alkyl, or C₁-C₃-haloalkyl; and R⁵ is C₁-C₃-alkyl, or C₁-C₃-haloalkyl.

The cycle G is phenyl, or a 5- or 6-membered hetaryl, preferably 2-pyridyl. For the avoidance of doubt, the cycle G is substituted with n substituents R⁷. Also, for the avoidance of doubt, G is connected to W and to the bicylcic system by direct chemical bonds to two adjacent ring members of G.

In one embodiment, G is phenyl. In another embodiment, G is a 5-membered hetaryl. In another embodiment, G is a 6-membered hetary. In another embodiment, G is a 5-membered hetaryl comprising one N-atom. In another embodiment, G is a 6-membered hetaryl comprising at least one N-atom. In another embodiment, G is a 6-membered hetaryl comprising two N-atoms.

Prefered 5- or 6-membered rings G are depicted below as formulae A1 to A48, wherein "&" stands for the connection to the trycyclic scaffold of compounds of formula (I). For the avoidance of doubt, the formulae A1 to A48 are preferred embodiments on their own and in combination for the following group 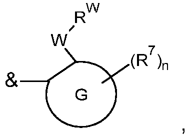 wherein "&" stands for the connection to the tricyclic scaffold in formula (I). In other words, the substituents R^{W}-W and (R⁷)ₙ in formulae A1 to A48 are mere illustrations but are not part of the rings G.

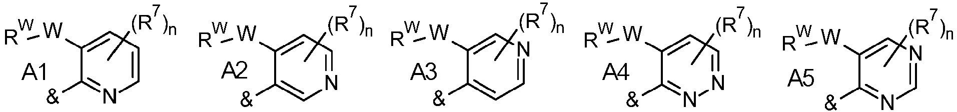

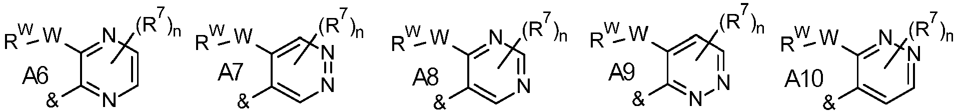

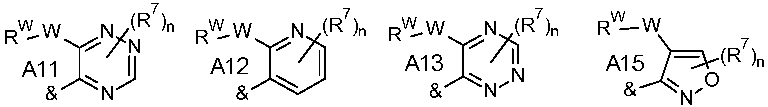

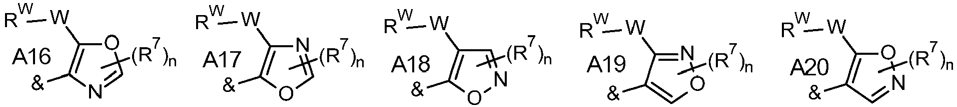

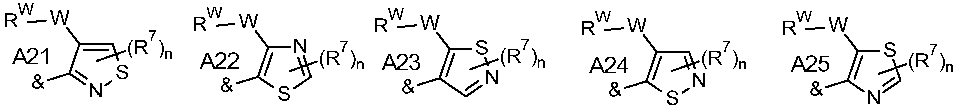

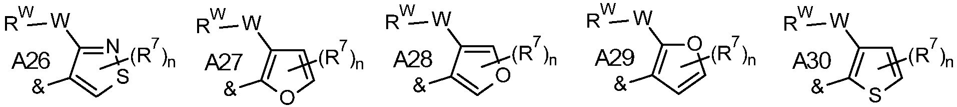

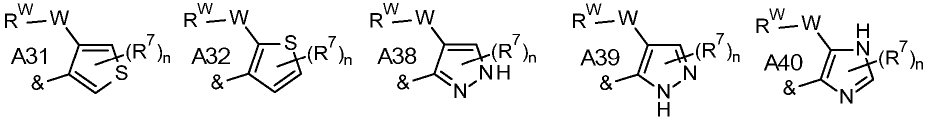

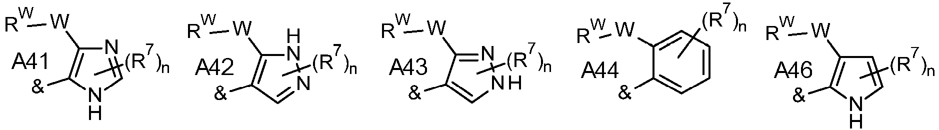

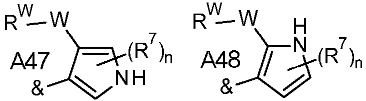

In one embodiment, G is selected from formulae A1 to A14. In one embodiment, G is selected from *formulae* A1 to A3. In another embodiment, G is A1. In another embodiment, G is A2. In another embodiment, G is A3. In another embodiment, G is A44.

A1 and A44 are preferred.

The variable W is S, SO, SO₂, or S(O)(NR^{W}). In one embodiment, the variable W is S. In one embodiment, the variable W is SO. In one embodiment, the variable W is SO₂. In another embodiment, the variable W is S or SO₂. In another embodiment, the variable W is S, SO, or SO₂.

The index n is 1, 2, 3, or 4, if G is phenyl, or a 6-membered hetaryl, or 1, 2, or 3 if G is a 5-membered hetaryl. Typically, n is 1. In another embodiment, n is 2. In another embodiment, n is 3.

The index m is 0, 1, or 2. In one embodiment, m is 0. In another embodiment, m is 2. In another embodiment, m is 1.

Each R⁷ is independently H, halogen, OH, CN, NC, NO₂, N₃, SCN, NCS, NCO, SF₃; C₁-C₆-alkyl, C₃-C₆-cycloalkyl, C₂-C₆-alkenyl, C₃-C₆-cycloalkenyl, C₂-C₆-alkynyl, C₃-C₆-cycloalkyl-C₁-C₃-alkyl, which groups are unsubstituted, or substituted with one or more R^{G}; a 3- to 12-membered saturated, partially unsaturated, or fully unsaturated heterocyclic ring or ring system, wherein said heterocyclic ring or ring system comprises one or more, same or different heteroatoms O, N, or S, and is unsubstituted, or substituted with one or more R^{H}; phenyl, which is unsubstituted, or substituted with one or more R^{J}; OR^{K}, SR^{K}, OC(=O)R^{K}, OC(=O)OR^{K}, OC(=O)NR^{L}R^{M}, OC(=O)SR^{K}, OC(=S)NR^{L}R^{M}, OC(=S)SR^{K}, OS(=O)ₘR^{K}, OS(=O)ₘNR^{L}R^{M}, ONR^{L}R^{M}, N=CR^{N}R^{O}, NR^{L}R^{M}, NOR^{K}, ONR^{L}R^{M}, N=CR^{N}R^{O}, NNR^{L}, N(R^{L})C(=O)R^{K}, N(R^{L})C(=O)OR^{K}, S(=O)ₘR^{V}, SC(=O)SR^{K}, SC(=O)NR^{L}R^{M}, S(=O)ₘNR^{L}R^{M}, C(=O)R^{P}, C(=S)R^{P}, C(=O)NR^{L}R^{M}, C(=O)OR^{K}, C(=S)NR^{L}R^{M}, C(=S)OR^{K}, C(=S)SR^{K}, C(=NR^{L})R^{M}, C(=NR^{L})NR^{M}R^{R}, Si(R^{S})₂R^{T}; or two substituents R⁷ form, together with the ring members of ring G to which they are bound, a 5- or 6- membered saturated, partially unsaturated, or fully unsaturated carbo- or heterocycle, which carbo- or heterocycle is unsubstituted, or substituted with one or more R^{J}, and wherein said heterocycle comprises one or more, same or different heteroatoms O, N, or S; or -C(CN)R^{X}R^{Y}, -C(R^{O})=N-NR^{M}R^{N}, -C(R^{O})=N-OR^{L}, or C₃-C₆-cycloalkyl, which is substituted with CN and which either does not have any further substituents, or which is further substituted with one or more R^{Z}; wherein at least one R⁷ is a group of formula (S) 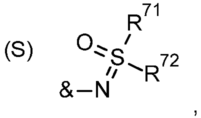 wherein each R⁷¹, R⁷² is independently selected from C₁-C₆-alkyl, C₃-C₆-cycloalkyl, C₂-C₆-alkenyl, C₃-C₆-cycloalkenyl, C₂-C₆-alkynyl, C₃-C₆-cycloalkyl-C₁-C₃-alkyl, which groups are unsubstituted, or substituted with one or more R^{G};
a 3- to 12-membered saturated, partially unsaturated, or fully unsaturated heterocyclic ring or ring system, wherein said heterocyclic ring or ring system comprises one or more, same or different heteroatoms O, N, or S, and is unsubstituted, or substituted with one or more R^{H}; phenyl, which is unsubstituted, or substituted with one or more R^{J}; or two substituents R⁷¹, R⁷² form, together with the sulfur atom to which they are bound, a 5- or 6- membered saturated, partially unsaturated, or fully unsaturated heterocycle, which heterocycle is unsubstituted, or substituted with one or more R^{J}, and wherein said heterocycle comprises no, one or more, same or different heteroatoms O, N, or S in addition to the sulfur atom to which R⁷¹ and R⁷² are bound to.

Accordingly, at least one substituent R⁷ is a group of formula (S) 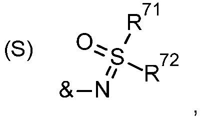 and the cycle G has either no, or n-1 further substituents R⁷.

In one embodiment, each R⁷ is independently H, halogen, OH, CN; C₁-C₃-alkyl, C₃-C₆-cycloalkyl, C₂-C₆-alkenyl, C₃-C₆-cycloalkenyl, C₂-C₆-alkynyl, C₃-C₆-cycloalkyl-C₁-C₃-alkyl, which groups are unsubstituted, or substituted with one or more R^{G}; a 5- to 6-membered saturated, partially unsaturated, or fully unsaturated heterocyclic ring or ring system, wherein said heterocyclic ring or ring system comprises one or more, same or different heteroatoms O, N, or S, and is unsubstituted, or substituted with one or more R^{H}; phenyl, which is unsubstituted, or substituted with one or more R^{J}; OR^{K}, SR^{K}, OC(=O)R^{K}, OC(=O)OR^{K}, OC(=O)NR^{L}R^{M}, OC(=O)SR^{K}, OC(=S)NR^{L}R^{M}, OC(=S)SR^{K}, OS(=O)ₘR^{K}, OS(=O)ₘNR^{L}R^{M}, ONR^{L}R^{M}, N=CR^{N}R^{O}, NR^{L}R^{M}, NOR^{K}, ONR^{L}R^{M}, N=CR^{N}R^{O}, NNR^{L}, N(R^{L})C(=O)R^{K}, N(R^{L})C(=O)OR^{K}, S(=O)ₘR^{V}, SC(=O)SR^{K}, SC(=O)NR^{L}R^{M}, S(=O)ₘNR^{L}R^{M}, C(=O)R^{P}, C(=S)R^{P}, C(=O)NR^{L}R^{M}, C(=O)OR^{K}, C(=S)NR^{L}R^{M}, C(=S)OR^{K}, C(=S)SR^{K}, C(=NR^{L})R^{M}, C(=NR^{L})NR^{M}R^{R}, Si(R^{S})₂R^{T}; wherein at least one R⁷ is a group of formula (S) 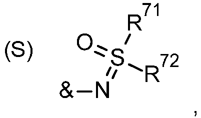
wherein each R⁷¹, R⁷² is independently selected from C₁-C₆-alkyl, C₃-C₆-cycloalkyl, C₂-C₆-alkenyl, C₃-C₆-cycloalkenyl, C₂-C₆-alkynyl, C₃-C₆-cycloalkyl-C₁-C₃-alkyl, which groups are unsubstituted, or substituted with one or more R^{G};
a 3- to 12-membered saturated, partially unsaturated, or fully unsaturated heterocyclic ring or ring system, wherein said heterocyclic ring or ring system comprises one or more, same or different heteroatoms O, N, or S, and is unsubstituted, or substituted with one or more R^{H}; phenyl, which is unsubstituted, or substituted with one or more R^{J};
or two substituents R⁷¹, R⁷² form, together with the sulfur atom to which they are bound, a 5- or 6- membered saturated, partially unsaturated, or fully unsaturated heterocycle, which heterocycle is unsubstituted, or substituted with one or more R^{J}, and wherein said heterocycle comprises no, one or more, same or different heteroatoms O, N, or S in addition to the sulfur atom to which R⁷¹ and R⁷² are bound to.

In another embodiment, each R⁷ is independently H, halogen; C₁-C₃-alkyl, C₁-C₃-alkoxyy, which groups are unsubstituted, or halogenated; and
wherein at least one R⁷ is a group of formula (S) 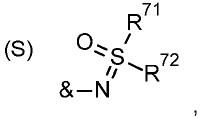
wherein each R⁷¹, R⁷² is independently selected from C₁-C₆-alkyl, C₃-C₆-cycloalkyl, C₂-C₆-alkenyl, C₃-C₆-cycloalkenyl, C₂-C₆-alkynyl, C₃-C₆-cycloalkyl-C₁-C₃-alkyl, which groups are unsubstituted, or substituted with one or more R^{G};
a 3- to 12-membered saturated, partially unsaturated, or fully unsaturated heterocyclic ring or ring system, wherein said heterocyclic ring or ring system comprises one or more, same or different heteroatoms O, N, or S, and is unsubstituted, or substituted with one or more R^{H}; phenyl, which is unsubstituted, or substituted with one or more R^{J};
or two substituents R⁷¹, R⁷² form, together with the sulfur atom to which they are bound, a 5- or 6- membered saturated, partially unsaturated, or fully unsaturated heterocycle, which heterocycle is unsubstituted, or substituted with one or more R^{J}, and wherein said heterocycle comprises no, one or more, same or different heteroatoms O, N, or S in addition to the sulfur atom to which R⁷¹ and R⁷² are bound to.

In one embodiment, each R⁷¹, R⁷² is independently selected from C₁-C₃-alkyl, C₃-C₆-cycloalkyl, C₂-C₆-alkenyl, C₃-C₆-cycloalkenyl, C₂-C₆-alkynyl, C₃-C₆-cycloalkyl-C₁-C₃-alkyl, which groups are unsubstituted, or substituted with one or more, same or different substituents selected from halogen and CN;
a 3- to 6-membered saturated, partially unsaturated, or fully unsaturated heterocyclic ring or ring system, wherein said heterocyclic ring or ring system comprises one or more, same or different heteroatoms O, N, or S, and is unsubstituted, or substituted with one or more, same or different substituents selected from halogen, CN, C₁-C₃-alkyl, and C₁-C₃-haloalkyl;
phenyl, which is unsubstituted, or substituted with one or more, same or different substituents selected from halogen, CN, C₁-C₃-alkyl, and C₁-C₃-haloalkyl; or two substituents R⁷¹, R⁷² form, together with the sulfur atom to which they are bound, a 5- or 6- membered saturated, partially unsaturated, or fully unsaturated heterocycle, which heterocycle is unsubstituted, or substituted with one or more, same or different substituents halogen, CN, C₁-C₃-alkyl, and C₁-C₃-haloalkyl, and wherein said heterocycle comprises no, one or more, same or different heteroatoms O, N, or S in addition to the sulfur atom to which R⁷¹ and R⁷² are bound to.

In another embodiment, each R⁷¹, R⁷² is independently selected from C₁-C₃-alkyl, which is unsubstituted, or substituted with one or more, same or different substituents selected from halogen;
a 3- to 6-membered saturated, partially unsaturated, or fully unsaturated heterocyclic ring or ring system, wherein said heterocyclic ring or ring system comprises one or more, same or different heteroatoms O, N, or S, and is unsubstituted, or substituted with one or more, same or different substituents selected from halogen, CN, C₁-C₃-alkyl, and C₁-C₃-haloalkyl;
phenyl, which is unsubstituted, or substituted with one or more, same or different substituents selected from halogen, CN, C₁-C₃-alkyl, and C₁-C₃-haloalkyl;
or two substituents R⁷¹, R⁷² form, together with the sulfur atom to which they are bound, a 5- or 6- membered saturated, partially unsaturated, or fully unsaturated heterocycle, which heterocycle is unsubstituted, or substituted with one or more, same or different substituents halogen, CN, C₁-C₃-alkyl, and C₁-C₃-haloalkyl, and wherein said heterocycle comprises no, one or more, same or different heteroatoms O, N, or S in addition to the sulfur atom to which R⁷¹ and R⁷² are bound to.

In another embodiment, each R⁷¹, R⁷² is independently selected from C₁-C₃-alkyl, which is unsubstituted, or substituted with one or more, same or different substituents selected from halogen; phenyl, which is unsubstituted, or substituted with one or more, same or different substituents selected from halogen, CN, C₁-C₃-alkyl, and C₁-C₃-haloalkyl;
or two substituents R⁷¹, R⁷² form, together with the sulfur atom to which they are bound, a 5- or 6- membered saturated, partially unsaturated, or fully unsaturated heterocycle, which heterocycle is unsubstituted, or substituted with halogen, and wherein said heterocycle comprises no, one or more, same or different heteroatoms O, N, or S in addition to the sulfur atom to which R⁷¹ and R⁷² are bound to.

In a preferred embodiment each R⁷ is independently H, halogen; C₁-C₃-alkyl, C₁-C₃-alkoxyy, which groups are unsubstituted, or halogenated; and
wherein at least one R⁷ is a group of formula (S) 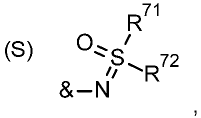
wherein each R⁷¹, R⁷² is independently selected from C₁-C₃-alkyl, which is unsubstituted, or substituted with one or more, same or different substituents selected from halogen;
a 3- to 6-membered saturated, partially unsaturated, or fully unsaturated heterocyclic ring or ring system, wherein said heterocyclic ring or ring system comprises one or more, same or different heteroatoms O, N, or S, and is unsubstituted, or substituted with one or more, same or different substituents selected from halogen, CN, C₁-C₃-alkyl, and C₁-C₃-haloalkyl;
phenyl, which is unsubstituted, or substituted with one or more, same or different substituents selected from halogen, CN, C₁-C₃-alkyl, and C₁-C₃-haloalkyl;
or two substituents R⁷¹, R⁷² form, together with the sulfur atom to which they are bound, a 5- or 6- membered saturated, partially unsaturated, or fully unsaturated heterocycle, which heterocycle is unsubstituted, or substituted with one or more, same or different substituents halogen, CN, C₁-C₃-alkyl, and C₁-C₃-haloalkyl, and wherein said heterocycle comprises no, one or more, same or different heteroatoms O, N, or S in addition to the sulfur atom to which R⁷¹ and R⁷² are bound to.

Each R^{A} is independently H, C₁-C₆-alkyl, C₂-C₆-alkenyl, C₂-C₆-alkynyl, C₁-C₆-alkoxy-C₁-C₄-alkyl, C₃-C₆-cycloalkyl, C₃-C₆-cycloalkyl-C₁-C₄-alkyl, C₃-C₆-cycloalkoxy-C₁-C₄-alkyl, which groups are unsubstituted or substituted with halogen; phenyl or benzyl, wherein the phenyl ring is unsubstituted or substituted with one or more R^{F}.

In one embodiment, each R^{A} is independently H, C₁-C₃-alkyl, C₂-C₃-alkenyl, C₂-C₃-alkynyl, C₁-C₃-alkoxy-C₁-C₂-alkyl, C₃-C₆-cycloalkyl, C₃-C₆-cycloalkyl-C₁-C₂-alkyl, C₃-C₆-cycloalkoxy-C₁-C₂-alkyl, which groups are unsubstituted or substituted with halogen; or phenyl or benzyl, wherein the phenyl ring is unsubstituted or substituted with one or more R^{F}.

In one embodiment, each R^{A} is independently H, C₁-C₃-alkyl, C₂-C₃-alkenyl, C₂-C₃-alkynyl, C₃-C₆-cycloalkyl, which groups are unsubstituted or substituted with halogen; or phenyl or benzyl, wherein the phenyl ring is unsubstituted or substituted with one or more, same or different substitutents selected from halogen, C₁-C₃-alkyl, C₁-C₃-alkoxy, C₁-C₃-haloalkoxy, and C₁-C₃-haloalkyl.

Each R^{B} is independently H, C₁-C₆-alkyl, C₂-C₆-alkenyl, C₂-C₆-alkynyl, C₁-C₆-alkoxy-C₁-C₄-alkyl, C₃-C₆-cycloalkyl, C₃-C₆-cycloalkyl-C₁-C₄-alkyl, C₃-C₆-cycloalkoxy-C₁-C₄-alkyl, C₁-C₆-alkylcarbonyl, C₁-C₆-alkoxy-carbonyl, which groups are unsubstituted or substituted with halogen; C₁-C₆-alkylen-CN; phenyl and benzyl, which groups are unsubstituted or substituted with one or more R^{F}.

In one embodiment, each R^{B} is independently H, C₁-C₃-alkyl, C₂-C₃-alkenyl, C₂-C₃-alkynyl, C₃-C₆-cycloalkyl, C₃-C₆-cycloalkyl-C₁-C₄-alkyl, which groups are unsubstituted or substituted with halogen; phenyl or benzyl, which groups are unsubstituted or substituted with one or more R^{F}.

In another embodiment, each R^{B} is independently H, C₁-C₃-alkyl, C₂-C₃-alkenyl, C₂-C₃-alkynyl, C₃-C₆-cycloalkyl, which groups are unsubstituted or substituted with halogen; phenyl or benzyl, which groups are unsubstituted or substituted with one or more, same or different substituents selected from halogen, C₁-C₃-alkyl, C₁-C₃-alkoxy, C₁-C₃-haloalkoxy, and C₁-C₃-haloalkyl.

Each R^{C} is independently H, C₁-C₆-alkyl, C₂-C₆-alkenyl, C₂-C₆-alkynyl, C₁-C₆-alkoxy-C₁-C₄-alkyl, C₃-C₆-cycloalkyl, C₃-C₆-cycloalkyl-C₁-C₄-alkyl, C₃-C₆-cycloalkoxy-C₁-C₄-alkyl, which groups are unsubstituted or substituted with halogen; phenyl or benzyl, wherein the phenyl ring is unsubstituted or substituted with one or more R^{F}.

In one embodiment, each R^{C} is independently H, C₁-C₃-alkyl, C₂-C₃-alkenyl, C₂-C₃-alkynyl, C₃-C₆-cycloalkyl, C₃-C₆-cycloalkyl-C₁-C₄-alkyl, which groups are unsubstituted or substituted with halogen; phenyl or benzyl, which groups are unsubstituted or substituted with one or more R^{F}.

In another embodiment, each R^{C} is independently H, C₁-C₃-alkyl, C₂-C₃-alkenyl, C₂-C₃-alkynyl, C₃-C₆-cycloalkyl, which groups are unsubstituted or substituted with halogen; phenyl or benzyl, which groups are unsubstituted or substituted with one or more, same or different substituents selected from halogen, C₁-C₃-alkyl, C₁-C₃-alkoxy, C₁-C₃-haloalkoxy, and C₁-C₃-haloalkyl. Alternatively, each moiety NR^{B}R^{C} may also form an N-bound, saturated 5- to 8-membered heterocycle, which in addition to the nitrogen atom may have 1 or 2 further heteroatoms or heteroatom moieties selected from O, S(=O)ₘ and N-R', wherein R' is H or C₁-C₆-alkyl and wherein the N-bound heterocycle is unsubstituted or substituted with one or more, same or different substituents selected from halogen, C₁-C₄-alkyl, C₁-C₄-haloalkyl, C₁-C₄-alkoxy and C₁-C₄-haloalkoxy. In one embodiment, each moiety NR^{B}R^{C} may also form an N-bound, saturated 5-to 6-membered heterocycle, wherein the N-bound heterocycle is unsubstituted or substituted with one or more, same or different substituents selected from halogen, C₁-C₃-alkyl, C₁-C₃-haloalkyl, C₁-C₃-alkoxy and C₁-C₃-haloalkoxy.

Each R^{D} is independently H, CN, OH, C₁-C₆-alkyl, C₂-C₆-alkenyl, C₂-C₆-alkynyl, C₁-C₆-alkoxy-C₁-C₄-alkyl, C₃-C₆-cycloalkyl, C₃-C₆-cycloalkyl-C₁-C₄-alkyl, C₃-C₆-cycloalkoxy-C₁-C₄-alkyl, which groups are unsubstituted or substituted with halogen; phenyl or benzyl, wherein the phenyl ring is unsubstituted or substituted with one or more R^{F}.

In one embodiment, each R^{D} is independently C₁-C₃-alkyl, C₂-C₃-alkenyl, C₂-C₃-alkynyl, C₃-C₆-cycloalkyl, C₃-C₆-cycloalkyl-C₁-C₄-alkyl, which groups are unsubstituted or substituted with halogen; phenyl or benzyl, which groups are unsubstituted or substituted with one or more R^{F}. In another embodiment, each R^{D} is independently C₁-C₃-alkyl, C₂-C₃-alkenyl, C₂-C₃-alkynyl, C₃-C₆-cycloalkyl, which groups are unsubstituted or substituted with halogen; phenyl or benzyl, which groups are unsubstituted or substituted with one or more, same or different substituents selected from halogen, C₁-C₃-alkyl, C₁-C₃-alkoxy, C₁-C₃-haloalkoxy, and C₁-C₃-haloalkyl.

Each R^{E} is indepentently C₁-C₆-alkyl, C₃-C₆-Cycloalkyl, C₃-C₆-cycloalkyl-C₁-C₄-alkyl, which are unsubstituted or substituted with halogen; or phenyl or benzyl, wherein the phenyl ring is unsubstituted or substituted with R^{F}.

In one embodiment, each R^{E} is indepentently C₁-C₃-alkyl, C₃-C₆-cycloalkyl, C₃-C₆-cycloalkyl-C₁-C₂-alkyl, which are unsubstituted or substituted with halogen; or phenyl or benzyl, wherein the phenyl ring is unsubstituted or substituted with one or more, same or different substituents selected from halogen, C₁-C₃-alkyl, C₁-C₃-alkoxy, C₁-C₃-haloalkoxy, and C₁-C₃-haloalkyl. In one embodiment, each R^{E} is indepentently C₁-C₃-alkyl, or C₁-C₃-haloalkyl.

Each R^{F} is independently halogen, N₃, OH, CN, NO₂, SCN, SF₃; C₁-C₆ alkyl, C₁-C₆ alkoxy, C₂-C₆ alkenyl, C₂-C₆ alkynyl, C₁-C₆ alkoxy-C₁-C₄ alkyl, C₁-C₆ alkoxy-C₁-C₄ alkoxy, C₃-C₆ cycloalkyl, C₃-C₆ cycloalkoxy, C₃-C₆ cycloalkyl-C₁-C₄ alkyl, C₃-C₆ cycloalkoxy-C₁-C₄ alkyl, which groups are unsubstituted or substituted with halogen.

In one embdodiment, each R^{F} is independently halogen, OH, CN, NO₂; C₁-C₃-alkyl, C₁-C₃-alkoxy, C₂-C₃ alkenyl, C₂-C₃-alkynyl, C₃-C₆-cycloalkyl, which groups are unsubstituted or substituted with halogen. In another embdodiment, each R^{F} is independently halogen; C₁-C₃-alkyl, C₁-C₃-alkoxy, C₂-C₃ alkenyl, C₂-C₃-alkynyl, which groups are unsubstituted or substituted with halogen. In another embodiment, each R^{F} is independently halogen, C₁-C₃-alkyl, or C₁-C₃-haloalkyl.

Each R^{G} is independently halogen, OH, CN, NC, NO₂; C₁-C₆-alkyl, C₃-C₆-cycloalkyl, C₃-C₆-cycloalkenyl, which groups are unsubstituted, or substituted with one or more, same or different substituents selected from halogen, OH, CN, C₁-C₃-alkoxy, C₁-C₃-haloalkoxy, and C₁-C₃-alkylcarbonyl; a 3- to 12-membered saturated, partially unsaturated, or fully unsaturated heterocyclic ring or ring system, wherein said heterocyclic ring or ring system comprises one or more, same or different heteroatoms O, N, or S, and is unsubstituted, or substituted with one or more, same or different substituents selected from halogen, OH, CN, C₁-C₃-alkoxy, C₁-C₃-haloalkoxy, and C₁-C₃-alkyl-carbonyl; phenyl, which is unsubstituted, or substituted with one or more, same or different substituents selected from halogen, OH, CN, NO₂, C₁-C₃-alkyl, C₁-C₃-haloalkyl, C₁-C₃-alkoxy, C₁-C₃-haloalkoxy, and C₁-C₃-alkyl-carbonyl; OR^{K}, SR^{K}, OC(=O)R^{K}, OC(=O)OR^{K}, OC(=O)NR^{L}R^{M}, OC(=O)SR^{K}, OC(=S)NR^{L}R^{M}, OC(=S)SR^{K}, OS(=O)ₘR^{K}, OS(=O)ₘNR^{L}R^{M}, ONR^{L}R^{M}, ON=CR^{N}R^{O}, NR^{L}R^{M}, NOR^{K}, ONR^{L}R^{M}, N=CR^{N}R^{O}, NNR^{L}, N(R^{L})C(=O)R^{K}, N(R^{L})C(=O)OR^{K}, S(=O)ₙR^{V}, SC(=O)SR^{K}, SC(=O)NR^{L}R^{M}, S(=O)ₘNR^{L}R^{M}, C(=O)R^{P}, C(=S)R^{P}, C(=O)NR^{L}R^{M}, C(=O)OR^{K}, C(=S)NR^{L}R^{M}, C(=S)OR^{K}, C(=S)SR^{K}, C(=NR^{L})R^{M}, C(=NR^{L})NR^{M}R^{R}, Si(R^{S})₂R^{T}.

In one embodiment, each R^{G} is independently halogen, OH, CN; C₁-C₃-alkyl, C₃-C₆-cycloalkyl, C₃-C₆-cycloalkenyl, which groups are unsubstituted, or substituted with one or more, same or different substituents selected from halogen, OH, CN, C₁-C₃-alkoxy, C₁-C₃-haloalkoxy, and C₁-C₃-alkyl-carbonyl; a 5- to 6-membered saturated, partially unsaturated, or fully unsaturated heterocyclic ring or ring system, wherein said heterocyclic ring or ring system comprises one or more, same or different heteroatoms O, N, or S, and is unsubstituted, or substituted with one or more, same or different substituents selected from halogen, OH, CN, C₁-C₃-alkoxy, C₁-C₃-haloalkoxy, and C₁-C₃-alkyl-carbonyl; phenyl, which is unsubstituted, or substituted with one or more, same or different substituents selected from halogen, OH, CN, NO₂, C₁-C₃-alkyl, C₁-C₃-haloalkyl, C₁-C₃-alkoxy, C₁-C₃-haloalkoxy, and C₁-C₃-alkyl-carbonyl; OR^{K}, SR^{K}, OC(=O)R^{K}, OC(=O)OR^{K}, OC(=O)NR^{L}R^{M}, ONR^{L}R^{M}, ON=CR^{N}R^{O}, NR^{L}R^{M}, NOR^{K}, ONR^{L}R^{M}, NNR^{L}, N(R^{L})C(=O)R^{K}, N(R^{L})C(=O)OR^{K}, C(=O)R^{P}, C(=S)R^{P}, C(=O)NR^{L}R^{M}, C(=O)OR^{K}. In one embodiment, each R^{G} is independently halogen, CN; C₁-C₃-alkyl, C₁-C₃-alkoxy, C₁-C₃-haloalkyl, C₁-C₃-haloalkoxy, or phenyl. In another embodiment, each R^{G} is independently halogen, CN; C₁-C₃-alkyl, C₁-C₃-alkoxy, C₁-C₃-haloalkyl, or C₁-C₃-haloalkoxy. In another embodiment, each R^{G} is independently CN or halogen.

Each R^{H} is independently halogen, CN, NC, NO₂, SCN, NCS, NCO; C₁-C₆-alkyl, C₃-C₆-cycloalkyl, C₃-C₆-cycloalkenyl, which groups are unsubstituted, or substituted with one or more, same or different substituents selected from halogen, OH, CN, C₁-C₃-alkoxy, C₁-C₃-haloalkoxy, and C₁-C₃-alkyl-carbonyl;
phenyl, which is unsubstituted, or substituted with one or more, same or different substituents selected from halogen, OH, CN, NO₂, C₁-C₃-alkyl, C₁-C₃-haloalkyl, OR^{K}, SR^{K}, OC(=O)R^{K}, OC(=O)OR^{K}, OC(=O)NR^{L}R^{M}, OC(=O)SR^{K}, OC(=S)NR^{L}R^{M}, OC(=S)SR^{K}, OS(=O)ₘR^{K}, OS(=O)ₘNR^{L}R^{M}, ONR^{L}R^{M}, ON=CR^{N}R^{O}, NR^{L}R^{M}, NOR^{K}, ONR^{L}R^{M}, N=CR^{N}R^{O}, NNR^{L}, N(R^{L})C(=O)R^{K}, N(R^{L})C(=O)OR^{K}, S(=O)ₙR^{V}, SC(=O)SR^{K}, SC(=O)NR^{L}R^{M}, S(=O)ₘNR^{L}R^{M}, C(=O)R^{P}, C(=S)R^{P}, C(=O)NR^{L}R^{M}, C(=O)OR^{K}, C(=S)NR^{L}R^{M}, C(=S)OR^{K}, C(=S)SR^{K}, C(=NR^{L})R^{M}, C(=NR^{L})NR^{M}R^{R}, Si(R^{S})₂R^{T}; or two geminal substituents R^{H} form together with the atom to which they are bound a group =O, =S, or =NR^{L}. In one embodiment, each R^{H} is independently halogen, CN; C₁-C₃-alkyl, C₁-C₃-haloalkyl, C₁-C₃-alkoxy, or C₁-C₃-haloalkoxy. In another embodiment, each R^{H} is independently halogen or CN, preferably CN.

Each R^{J} is independently halogen, CN, NC, NO₂, SCN, NCS, NCO; C₁-C₆-alkyl, C₃-C₆-cycloalkyl, C₃-C₆-cycloalkenyl, which groups are unsubstituted, or substituted with one or more, same or different substituents selected from halogen, OH, CN, C₁-C₃-alkoxy, C₁-C₃-haloalkoxy, and C₁-C₃-alkyl-carbonyl; phenyl, which is unsubstituted, or substituted with one or more, same or different substituents selected from halogen, OH, CN, NO₂, C₁-C₃-alkyl, C₁-C₃-haloalkyl, OR^{K}, SR^{K}, OC(=O)R^{K}, OC(=O)OR^{K}, OC(=O)NR^{L}R^{M}, OC(=O)SR^{K}, OC(=S)NR^{L}R^{M}, OC(=S)SR^{K}, OS(=O)ₘR^{K}, OS(=O)ₘNR^{L}R^{M}, ONR^{L}R^{M}, ON=CR^{N}R^{O}, NR^{L}R^{M}, NOR^{K}, ONR^{L}R^{M}, N=CR^{N}R^{O}, NNR^{L}, N(R^{L})C(=O)R^{K}, N(R^{L})C(=O)OR^{K}, S(=O)ₙR^{V}, SC(=O)SR^{K}, SC(=O)NR^{L}R^{M}, S(=O)ₘNR^{L}R^{M}, C(=O)R^{P}, C(=S)R^{P}, C(=O)NR^{L}R^{M}, C(=O)OR^{K}, C(=S)NR^{L}R^{M}, C(=S)OR^{K}, C(=S)SR^{K}, C(=NR^{L})R^{M}, C(=NR^{L})NR^{M}R^{R},or Si(R^{S})₂R^{T}. In one embodiment, each R^{J} is independently halogen, CN; C₁-C₃-alkyl, C₁-C₃-haloalkyl, C₁-C₃-alkoxy, or C₁-C₃-haloalkoxy. In another embodiment, each R^{J} is independently C₁-C₃-alkyl or C₁-C₃-haloalkyl.

Each R^{K} is independently H; C₁-C₆-alkyl, C₂-C₆-alkenyl, C₂-C₆-alkynyl, C₁-C₆-alkoxy-C₁-C₄-alkyl, C₃-C₆-cycloalkyl, C₃-C₆-cycloalkyl-C₁-C₄-alkyl, C₃-C₆-cycloalkoxy-C₁-C₄-alkyl, which groups are unsubstituted or substituted with one or more, same or different substituents selected from halogen, CN, NR^{M}R^{N}; C(=O)NR^{M}R^{N}, C(=O)R^{T}; or phenyl or benzyl, wherein the phenyl ring is unsubstituted or substituted with one or more R^{F}.

In one embodiment, each R^{K} is independently C₁-C₃-alkyl, C₂-C₃-alkenyl, C₂-C₃-alkynyl, C₃-C₆-cycloalkyl, C₃-C₆-cycloalkyl-C₁-C₄-alkyl, which groups are unsubstituted or substituted with halogen; phenyl or benzyl, which groups are unsubstituted or substituted with one or more R^{F}. In another embodiment, each R^{K} is independently C₁-C₃-alkyl, C₂-C₃-alkenyl, C₂-C₃-alkynyl, C₃-C₆-cycloalkyl, which groups are unsubstituted or substituted with halogen; phenyl or benzyl, which groups are unsubstituted or substituted with one or more, same or different substituents selected from halogen, C₁-C₃-alkyl, C₁-C₃-alkoxy, C₁-C₃-haloalkoxy, and C₁-C₃-haloalkyl.

Each R^{L}, R^{R} is independently H; C₁-C₆-alkyl, C₂-C₆-alkenyl, C₂-C₆-alkynyl, C₁-C₆-alkoxy-C₁-C₄-alkyl, C₃-C₆-cycloalkyl, C₃-C₆-cycloalkyl-C₁-C₄-alkyl, C₃-C₆-cycloalkoxy-C₁-C₄-alkyl, which groups are unsubstituted or substituted with halogen; phenyl and benzyl, which groups are unsubstituted or substituted with one or more R^{F}.

In one embodiment, each R^{L}, R^{R} is independently H; C₁-C₃-alkyl, C₂-C₃-alkenyl, C₂-C₃-alkynyl, C₁-C₃-alkoxy-C₁-C₂-alkyl, C₃-C₆-cycloalkyl, C₃-C₆-cycloalkyl-C₁-C₂-alkyl, C₃-C₆-cycloalkoxy-C₁-C₂-alkyl, which groups are unsubstituted or substituted with halogen; phenyl and benzyl, which groups are unsubstituted or substituted with halogen, C₁-C₃-alkyl, or C₁-C₃-haloalkyl. In another embodiment, each R^{L}, R^{R} is independently H; C₁-C₃-alkyl, or C₁-C₃-haloalkyl.

Each R^{M} is independently H, C₁-C₆-alkyl, C₂-C₆-alkenyl, C₂-C₆-alkynyl, C₁-C₆-alkoxy-C₁-C₄-alkyl, C₃-C₆-cycloalkyl, C₃-C₆-cycloalkyl-C₁-C₄-alkyl, C₃-C₆-cycloalkoxy-C₁-C₄-alkyl, C₁-C₆-alkylcarbonyl, C₁-C₆-alkoxy-carbonyl, which groups are unsubstituted or substituted with halogen;
C₁-C₆-alkylen-CN; phenyl or benzyl, which groups are unsubstituted or substituted with one or more R^{F}. In one embodiment, each R^{M} is independently H; C₁-C₃-alkyl, or C₁-C₃-haloalkyl.

Each R^{N} is independently H, C₁-C₆-alkyl, C₂-C₆-alkenyl, C₂-C₆-alkynyl, C₁-C₆-alkoxy-C₁-C₄-alkyl, C₃-C₆-cycloalkyl, C₃-C₆-cycloalkyl-C₁-C₄-alkyl, C₃-C₆-cycloalkoxy-C₁-C₄-alkyl, C₁-C₆-alkylcarbonyl, C₁-C₆-alkoxy-carbonyl, which groups are unsubstituted or substituted with halogen; phenyl or benzyl, wherein the phenyl ring is unsubstituted or substituted with one or more R^{F}. In one embodiment, each R^{N} is independently H; C₁-C₃-alkyl, or C₁-C₃-haloalkyl.

Alternatively each moiety NR^{M}R^{N}, R^{M}R^{R}, and R^{L}R^{M} may also form an N-bound, saturated 5- to 8-membered heterocycle, which in addition to the nitrogen atom may have 1 or 2 further heteroatoms or heteroatom moieties selected from O, S(=O)ₘ and N-R", wherein R" is H or C₁-C₆-alkyl and wherein the N-bound heterocycle is unsubstituted or substituted with one or more, same or different substituents selected from halogen, C₁-C₄-alkyl, C₁-C₄-haloalkyl, C₁-C₄-alkoxy and C₁-C₄-haloalkoxy. In one embodiment, each moiety NR^{M}R^{N}, R^{M}R^{R}, and R^{L}R^{M} may also form an N-bound, saturated 5- to 6-membered heterocycle, which in addition to the nitrogen atom may have 1 or 2 further heteroatoms or heteroatom moieties selected from O, S(=O)ₘ and N-R", wherein R is H or C₁-C₆-alkyl and wherein the N-bound heterocycle is unsubstituted or substituted with one or more, same or different substituents selected from halogen, C₁-C₃-alkyl, C₁-C₃-haloalkyl, C₁-C₃-alkoxy and C₁-C₃-haloalkoxy.

Each R^{O} is independently H, CN, OH, C₁-C₆-alkyl, C₂-C₆-alkenyl, C₂-C₆-alkynyl, C₁-C₆-alkoxy-C₁-C₄-alkyl, C₃-C₆-cycloalkyl, C₃-C₆-cycloalkyl-C₁-C₄-alkyl, C₃-C₆-cycloalkoxy-C₁-C₄-alkyl, which groups are unsubstituted or substituted with halogen; phenyl or benzyl, wherein the phenyl ring is unsubstituted or substituted with one or more R^{F}. In one embodiment, each R^{O} is independently H, CN, or C₁-C₆-alkyl.

Each R^{P} is independently H; C₁-C₆-alkyl, C₂-C₆-alkenyl, C₂-C₆-alkynyl, C₁-C₆-alkoxy-C₁-C₄-alkyl, C₃-C₆-cycloalkyl, C₃-C₆-cycloalkyl-C₁-C₄-alkyl, C₃-C₆-cycloalkoxy-C₁-C₄-alkyl, which groups are unsubstituted or substituted with halogen; phenyl or benzyl, wherein the phenyl ring is unsubstituted or substituted with one or more R^{F}.

In one embodiment, each R^{P} is independently C₁-C₃-alkyl, C₂-C₃-alkenyl, C₂-C₃-alkynyl, C₃-C₆-cycloalkyl, C₃-C₆-cycloalkyl-C₁-C₄-alkyl, which groups are unsubstituted or substituted with halogen; phenyl or benzyl, which groups are unsubstituted or substituted with one or more R^{F}. In another embodiment, each R^{P} is independently C₁-C₃-alkyl, C₂-C₃-alkenyl, C₂-C₃-alkynyl, C₃-C₆-cycloalkyl, which groups are unsubstituted or substituted with halogen; phenyl or benzyl, which groups are unsubstituted or substituted with one or more, same or different substituents selected from halogen, C₁-C₃-alkyl, C₁-C₃-alkoxy, C₁-C₃-haloalkoxy, and C₁-C₃-haloalkyl.

Each R^{S}, R^{T} is independently H, C₁-C₆-alkyl, C₁-C₆-haloalkyl, C₁-C₆-alkoxy, C₁-C₄-alkoxy-C₁-C₄-alkyl, C₃-C₆-cycloalkyl, C₃-C₆-halocycloalkyl, C₁-C₄-haloalkoxy-C₁-C₄-alkyl, or phenyl. In one embodiment, each R^{S}, R^{T} is independently H, C₁-C₆-alkyl, C₁-C₆-haloalkyl.

Each R^{V} is indepentently C₁-C₆-alkyl, C₃-C₆-Cycloalkyl, C₃-C₆-cycloalkyl-C₁-C₄-alkyl, which are unsubstituted or substituted with halogen; or phenyl or benzyl, wherein the phenyl ring is unsubstituted or substituted with R^{F}. In one embodiment, each R^{V} is indepentently C₁-C₃-alkyl, or C₁-C₃-haloalkyl. In another embodiment, each R^{V} is C₁-C₃-alkyl, preferably ethyl which is unsubstituted;

Each R^{W} is indepentently H, C₁-C₆-alkyl, C₃-C₆-cycloalkyl, C₃-C₆-cycloalkyl-C₁-C₄-alkyl, which groups are halogenated or unsubstituted; benzyl, or phenyl, which is unsubstituted or substituted with R^{F}.

In one embodiment, each R^{W} is indepentently C₁-C₃-alkyl or C₃-C₆-cycloalkyl, which are unsubstituted or substituted with halogen; or phenyl or benzyl, wherein the phenyl ring is unsubstituted or substituted with one or more, same or different substituents selected from halogen, C₁-C₃-alkyl, C₁-C₃-alkoxy, C₁-C₃-haloalkoxy, and C₁-C₃-haloalkyl.

In another embodiment, each R^{W} is indepentently C₁-C₃-alkyl, or C₁-C₃-haloalkyl.

In another embodiment, each R^{W} is C₁-C₃-alkyl, preferably ethyl which is unsubstituted.

R^{X}, R^{Y} are independently H, halogen, CN, C₁-C₆-alkyl, C₁-C₆-haloalkyl, C₃-C₆-cycloalkyl, C₁-C₄-alkoxy, C₁-C₄-alkoxy-C₁-C₄-alkyl, C₁-C₄-alkylsulfanyl, C₁-C₄-alkylsulfanyl-C₁-C₄-alkyl, C₁-C₄-alkylsulfinyl-C₁-C₄-alkyl, C₁-C₄-alkylsulfonyl-C₁-C₄-alkyl or C₁-C₄-alkoxycarbonyl.

Each R^{X} is independently H, halogen, CN, C₁-C₆-alkyl, C₁-C₆-haloalkyl, C₃-C₆-cycloalkyl, C₁-C₄-alkoxy, C₁-C₄-alkoxy-C₁-C₄-alkyl, C₁-C₄-alkylsulfanyl, C₁-C₄-alkylsulfanyl-C₁-C₄-alkyl, C₁-C₄-alkylsulfinyl-C₁-C₄-alkyl, C₁-C₄-alkylsulfonyl-C₁-C₄-alkyl or C₁-C₄-alkoxycarbonyl.

In one embodiment, each R^{X} is independently H, halogen, CN, C₁-C₆-alkyl, C₁-C₆-haloalkyl, or C₃-C₆-cycloalkyl. In another embodiment, each R^{X} is C₁-C₄-alkoxy, C₁-C₄-alkoxy-C₁-C₄-alkyl, C₁-C₄-alkylsulfanyl, C₁-C₄-alkylsulfanyl-C₁-C₄-alkyl, C₁-C₄-alkylsulfinyl-C₁-C₄-alkyl, C₁-C₄-alkylsulfonyl-C₁-C₄-alkyl or C₁-C₄-alkoxycarbonyl. In another embodiment, each R^{X} is H or C₁-C₆-alkyl. In another embodiment, each R^{X} is C₁-C₆-alkyl; preferably CH₃.

In one embodiment, each R^{Y} is independently H, halogen, CN, C₁-C₆-alkyl, C₁-C₆-haloalkyl, C₃-C₆-cycloalkyl, C₁-C₄-alkoxy, C₁-C₄-alkoxy-C₁-C₄-alkyl, C₁-C₄-alkylsulfanyl, C₁-C₄-alkylsulfanyl-C₁-C₄-alkyl, C₁-C₄-alkylsulfinyl-C₁-C₄-alkyl, C₁-C₄-alkylsulfonyl-C₁-C₄-alkyl or C₁-C₄-alkoxycarbonyl. In one embodiment, each R^{Y} is H, halogen, CN, C₁-C₆-alkyl, C₁-C₆-haloalkyl, or C₃-C₆-cycloalkyl. In another embodiment, each R^{'Y} is C₁-C₄-alkoxy, C₁-C₄-alkoxy-C₁-C₄-alkyl, C₁-C₄-alkylsulfanyl, C₁-C₄-alkylsulfanyl-C₁-C₄-alkyl, C₁-C₄-alkylsulfinyl-C₁-C₄-alkyl, C₁-C₄-alkylsulfonyl-C₁-C₄-alkyl or C₁-C₄-alkoxycarbonyl. In another embodiment, each R^{Y} is H or C₁-C₆-alkyl. In another embodiment, each R^{Y} is C₁-C₆-alkyl; preferably CH₃.

Typically, R^{X} and R^{Y} are both not H. Preferably, R^{X} and R^{Y} are independently selected from halogen, CN, C₁-C₆-alkyl, C₁-C₆-haloalkyl, C₃-C₆-cycloalkyl, C₁-C₄-alkoxy, C₁-C₄-alkoxy-C₁-C₄-alkyl, C₁-C₄-alkylsulfanyl, C₁-C₄-alkylsulfanyl-C₁-C₄-alkyl, C₁-C₄-alkylsulfinyl-C₁-C₄-alkyl, C₁-C₄-alkylsulfonyl-C₁-C₄-alkyl or C₁-C₄-alkoxycarbonyl, more preferably selected from C₁-C₆-alkyl, C₁-C₆-haloalkyl, C₃-C₆-cycloalkyl, C₁-C₄-alkoxy, and C₁-C₄-alkoxy-C₁-C₄-alkyl, most preferably from C₁-C₆-alkyl, C₁-C₆-haloalkyl, C₃-C₆-cycloalkyl, and C₁-C₄-alkoxy, especially preferably from C₁-C₆-alkyl and C₁-C₆-haloalkyl, in particular from C₁-C₃-alkyl and C₁-C₃-haloalkyl, such as from C₁-C₃-alkyl. In a particularly preferred embodiment, both R^{X} and R^{Y} are CH₃.

Each R^{Z} is independently halogen, CN, NH₂, C(=O)H, OH, C₃-C₆-cycloalkyl, C(=O)OH, C(=O)NH₂, C₁-C₄-haloalkoxy, C₁-C₄-alkoxy, C₁-C₄-haloalkylsulfanyl, C₁-C₄-haloalkylsulfinyl, C₁-C₄-haloalkylsulfonyl, C₁-C₄-alkoxycarbonyl, C₁-C₄-haloalkoxycarbonyl, C₁-C₄-alkylcarbonyl, C₁-C₄-haloalkylcarbonyl, di-(C₁-C₄)alkylaminocarbonyl, C₁-C₄-alkylaminocarbonyl, C₁-C₄-alkylcarbonylamino, di-(C₁-C₄)alkylcarbonylamino, C₁-C₄-alkoxycarbonylamino, or a group -C(R^{Z1})=NOR^{Z2}; phenyl, which is unsubstituted or substituted with one or more, same or different substituents selected from halogen, CN, C₁-C₄-alkyl, C₁-C₄-haloalkyl, C₁-C₄-haloalkoxy, C₁-C₄-alkoxy, C₁-C₄-haloalkylsulfanyl, C₁-C₄-haloalkylsulfinyl, C₁-C₄haloalkylsulfonyl and C(=O)C₁-C₄-haloalkyl; C₁-C₄-alkyl which is unsubstituted or substituted with one or more R^{Z3}.

R^{Z1} and R^{Z2} are independently H, C₁-C₄-alkyl, or C₁-C₄-haloalkyl;
R^{Z3} is halogen, CN, NH₂, C(=O)H, OH, C₃-C₆-cycloalkyl, hydroxycarbonyl, aminocarbonyl, C₁-C₄-haloalkoxy, C1-C₄-alkoxy, C₁-C₄-haloalkylsulfanyl, C₁-C₄-haloalkylsulfinyl, C₁-C₄-haloalkyl-sulfonyl, C₁-C₄-alkoxycarbonyl, C₁-C₄-haloalkoxycarbonyl, C₁-C₄-alkylcarbonyl, C₁-C₄-haloalkyl-carbonyl, di-(C₁-C₄)alkylaminocarbonyl, C₁-C₄alkylaminocarbonyl, C₁-C₄-alkylcarbonylamino, di-(C₁-C₄)alkylcarbonylamino, C₁-C₄-alkoxycarbonylamino, a group -C(R^{Z1})=NOR^{Z2}.

In one embodiment, each R^{Z} is independently halogen, CN, NH₂, C(=O)H, OH, C₃-C₆-cycloalkyl, C(=O)OH, C(=O)NH₂, C₁-C₄-haloalkoxy, C₁-C₄-alkoxy, C₁-C₄-haloalkylsulfanyl, C₁-C₄-haloalkylsulfinyl, C₁-C₄-haloalkylsulfonyl, C₁-C₄-alkoxycarbonyl, C₁-C₄-haloalkoxycarbonyl, C₁-C₄-alkylcarbonyl, C₁-C₄-haloalkylcarbonyl, di-(C₁-C₄)alkylaminocarbonyl, C₁-C₄-alkylaminocarbonyl, C₁-C₄-alkylcarbonylamino, di-(C₁-C₄)alkylcarbonylamino, C₁-C₄-alkoxycarbonylamino, or a group -C(R^{Z1})=NOR^{Z2}.

In another embodiment, each R^{Z} is independently halogen, CN, NH₂, CHO, OH, C₃-C₆-cycloalkyl, C(=O)OH, C(=O)NH₂, C₁-C₄-haloalkoxy, or C₁-C₄-alkoxy.

In another embodiment, each R^{Z} is independently halogen, CN, NH₂, or OH.

At least one R⁷ is a group of formula (S) 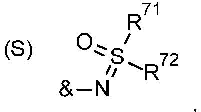 as described above. The position of said group may be described by the following scheme: Formulae (G.A) and (G.B) display the alternatives of the ring G being either a 6-membered hetaryl or phenyl; or a 5-membered hetaryl 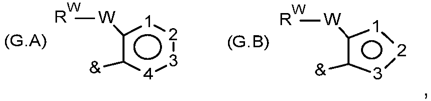 wherein the numbers 1, 2, 3, and 4 each independently denominate the position of a specific ring member, wherein the identity of said ring members is as described herein for formula (I), wherein the "&"-symbol signifies the connection to the remainder of formula (I), and wherein the other variables are defined as for formula (I).

Accordingly, the position x of the following group of formula (S) 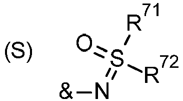 of a ring A1 to A48 will be indicated by the respective suffic ".x", such as A1.1, A1.2, A1.3, or A1.4.

For example, a ring A1 having said substituent of formula (S) at position 2 would correspond to the ring (A1.2) 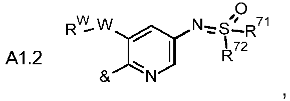 wherein all variables have a meaning as defined for formula (I).

In case the index n is 2, a second residue R⁷ is present, whose location at the ring "G" may likewise be indicated by anoterh suffix ".y" in addition to and after the suffix ".x", such as A1.1.2, A.1.1.3. Accordingly, the nomenclature of such a ring would follow the formula "A.x.y", wherein the "x" indicates the position of the group of formula (S), and wherein the "y" indicates the position of the second substituent R⁷.

For example, a ring A1 having two substituents R⁷, wherein at least one is a group of formula (S) as defined herein at position 2, and a further substituent R⁷ is located at position 3 corresponds to the ring (A1.2.3) 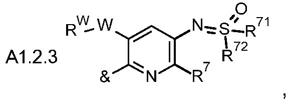 wherein all variables have a meaning as defined for formula (I).

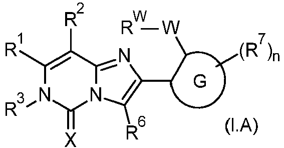

Accordingly, the following Tables 1 to 105 correspond to specific ring systems as combinations of rings G, wherein n is 1, with bicyclic fused rings of formula (I.A):
Table 1: compounds of formula (I.A), wherein G is of formula (A1.1).
Table 2: compounds of formula (I.A), wherein G is of formula (A1.2).
Table 3: compounds of formula (I.A), wherein G is of formula (A1.3).
Table 4: compounds of formula (I.A), wherein G is of formula (A2.1).
Table 5: compounds of formula (I.A), wherein G is of formula (A2.2).
Table 6: compounds of formula (I.A), wherein G is of formula (A2.4).
Table 7: compounds of formula (I.A), wherein G is of formula (A3.1).
Table 8: compounds of formula (I.A), wherein G is of formula (A3.3).
Table 9: compounds of formula (I.A), wherein G is of formula (A3.4).
Table 10: compounds of formula (I.A), wherein G is of formula (A4.1).
Table 11: compounds of formula (I.A), wherein G is of formula (A4.2).
Table 12: compounds of formula (I.A), wherein G is of formula (A5.1).
Table 13: compounds of formula (I.A), wherein G is of formula (A5.3).
Table 14: compounds of formula (I.A), wherein G is of formula (A6.2).
Table 15: compounds of formula (I.A), wherein G is of formula (A6.3).
Table 16: compounds of formula (I.A), wherein G is of formula (A7.1).
Table 17: compounds of formula (I.A), wherein G is of formula (A7.4).
Table 18: compounds of formula (I.A), wherein G is of formula (A8.2).
Table 19: compounds of formula (I.A), wherein G is of formula (A8.4).
Table 20: compounds of formula (I.A), wherein G is of formula (A9.1).
Table 21: compounds of formula (I.A), wherein G is of formula (A9.2).
Table 22: compounds of formula (I.A), wherein G is of formula (A9.1).
Table 23: compounds of formula (I.A), wherein G is of formula (A9.2).
Table 24: compounds of formula (I.A), wherein G is of formula (A10.3).
Table 25: compounds of formula (I.A), wherein G is of formula (A10.4).
Table 26: compounds of formula (I.A), wherein G is of formula (A11.3).
Table 27: compounds of formula (I.A), wherein G is of formula (A12.2).
Table 28: compounds of formula (I.A), wherein G is of formula (A12.3).
Table 29: compounds of formula (I.A), wherein G is of formula (A12.4).
Table 30: compounds of formula (I.A), wherein G is of formula (A15.1).
Table 31: compounds of formula (I.A), wherein G is of formula (A16.2).
Table 32: compounds of formula (I.A), wherein G is of formula (A17.2).
Table 32: compounds of formula (I.A), wherein G is of formula (A18.1).
Table 33: compounds of formula (I.A), wherein G is of formula (A19.3).
Table 34: compounds of formula (I.A), wherein G is of formula (A20.3).
Table 35: compounds of formula (I.A), wherein G is of formula (A21.2).
Table 36: compounds of formula (I.A), wherein G is of formula (A22.3).
Table 37: compounds of formula (I.A), wherein G is of formula (A23.3).
Table 38: compounds of formula (I.A), wherein G is of formula (A24.1).
Table 39: compounds of formula (I.A), wherein G is of formula (A25.2).
Table 40: compounds of formula (I.A), wherein G is of formula (A26.3).
Table 41: compounds of formula (I.A), wherein G is of formula (A27.1).
Table 42: compounds of formula (I.A), wherein G is of formula (A27.2).
Table 43: compounds of formula (I.A), wherein G is of formula (A28.1).
Table 44: compounds of formula (I.A), wherein G is of formula (A28.3).
Table 45: compounds of formula (I.A), wherein G is of formula (A29.2).
Table 46: compounds of formula (I.A), wherein G is of formula (A29.3).
Table 47: compounds of formula (I.A), wherein G is of formula (A30.1).
Table 48: compounds of formula (I.A), wherein G is of formula (A30.2).
Table 49: compounds of formula (I.A), wherein G is of formula (A31.1).
Table 50: compounds of formula (I.A), wherein G is of formula (A31.3).
Table 51: compounds of formula (I.A), wherein G is of formula (A32.2).
Table 52: compounds of formula (I.A), wherein G is of formula (A32.3).
Table 53: compounds of formula (I.A), wherein G is of formula (A32.3).
Table 54: compounds of formula (I.A), wherein G is of formula (A32.4).
Table 55: compounds of formula (I.A), wherein G is of formula (A38.1).
Table 56: compounds of formula (I.A), wherein G is of formula (A39.1).
Table 57: compounds of formula (I.A), wherein G is of formula (A40.2).
Table 58: compounds of formula (I.A), wherein G is of formula (A41.2).
Table 59: compounds of formula (I.A), wherein G is of formula (A42.3).
Table 60: compounds of formula (I.A), wherein G is of formula (A43.3).
Table 61: compounds of formula (I.A), wherein G is of formula (A44.1).
Table 62: compounds of formula (I.A), wherein G is of formula (A44.2).
Table 63: compounds of formula (I.A), wherein G is of formula (A44.3).
Table 64: compounds of formula (I.A), wherein G is of formula (A44.4).
Table 61: compounds of formula (I.A), wherein G is of formula (A46.1).
Table 62: compounds of formula (I.A), wherein G is of formula (A46.2).
Table 63: compounds of formula (I.A), wherein G is of formula (A47.1).
Table 64: compounds of formula (I.A), wherein G is of formula (A47.3).
Table 65: compounds of formula (I.A), wherein G is of formula (A48.2).
Table 66: compounds of formula (I.A), wherein G is of formula (A48.3).
Table 67: compounds of formula (I.A), wherein G is of formula (A1.1.2).
Table 68: compounds of formula (I.A), wherein G is of formula (A1.1.3).
Table 69: compounds of formula (I.A), wherein G is of formula (A1.2.1).
Table 70: compounds of formula (I.A), wherein G is of formula (A1.2.3).
Table 71: compounds of formula (I.A), wherein G is of formula (A1.3.1).
Table 72: compounds of formula (I.A), wherein G is of formula (A1.3.2).
Table 73: compounds of formula (I.A), wherein G is of formula (A2.1.2).
Table 74: compounds of formula (I.A), wherein G is of formula (A2.1.4).
Table 75: compounds of formula (I.A), wherein G is of formula (A2.2.1).
Table 76: compounds of formula (I.A), wherein G is of formula (A2.2.4).
Table 77: compounds of formula (I.A), wherein G is of formula (A2.4.1).
Table 78: compounds of formula (I.A), wherein G is of formula (A2.4.2).
Table 79: compounds of formula (I.A), wherein G is of formula (A3.1.3).
Table 80: compounds of formula (I.A), wherein G is of formula (A3.1.4).
Table 81: compounds of formula (I.A), wherein G is of formula (A3.3.1).
Table 82: compounds of formula (I.A), wherein G is of formula (A3.3.4).
Table 83: compounds of formula (I.A), wherein G is of formula (A3.4.1).
Table 84: compounds of formula (I.A), wherein G is of formula (A3.4.3).
Table 85: compounds of formula (I.A), wherein G is of formula (A4.1.2).
Table 86: compounds of formula (I.A), wherein G is of formula (A4.2.1).
Table 87: compounds of formula (I.A), wherein G is of formula (A5.1.3).
Table 88: compounds of formula (I.A), wherein G is of formula (A5.3.1).
Table 90: compounds of formula (I.A), wherein G is of formula (A6.2.3).
Table 91: compounds of formula (I.A), wherein G is of formula (A6.3.2).
Table 92: compounds of formula (I.A), wherein G is of formula (A7.1.4).
Table 93: compounds of formula (I.A), wherein G is of formula (A7.4.1).
Table 94: compounds of formula (I.A), wherein G is of formula (A8.2.4).
Table 95: compounds of formula (I.A), wherein G is of formula (A8.4.2).
Table 96: compounds of formula (I.A), wherein G is of formula (A9.1.2).
Table 97: compounds of formula (I.A), wherein G is of formula (A9.2.1).
Table 98: compounds of formula (I.A), wherein G is of formula (A10.3.4).
Table 99: compounds of formula (I.A), wherein G is of formula (A10.4.3).
Table 100: compounds of formula (I.A), wherein G is of formula (A12.2.3).
Table 101: compounds of formula (I.A), wherein G is of formula (A12.2.4).
Table 102: compounds of formula (I.A), wherein G is of formula (A12.3.1).
Table 103: compounds of formula (I.A), wherein G is of formula (A12.3.4).
Table 104: compounds of formula (I.A), wherein G is of formula (A12.4.2).
Table 105: compounds of formula (I.A), wherein G is of formula (A12.4.3).

The following Table A represents specific combinations of definitions for substituents R³ and R⁷ in lines L.1 to L.60. Each combination individually and all combinations collectively represent preferred embodiments.

**Table A**

| Line | R³ | Group (S) |
|---|---|---|
| L.1 | cC₃H₅ | 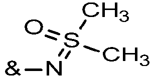 |
| L.2 | cC₃H₅ | 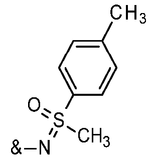 |
| L.3 | cC₃H₅ | 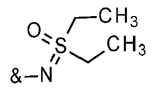 |
| L.4 | cC₃H₅ | 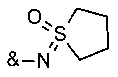 |
| L.5 | CC₃H₅ | 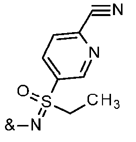 |
| L.6 | cC₃H₅ | 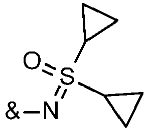 |
| L.7 | cC₃H₅ | 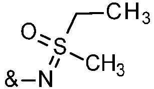 |
| L.8 | cC₃H₅ | 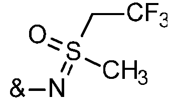 |
| L.9 | cC₃H₅ | 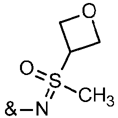 |
| L.10 | cC₃H₅ | 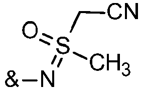 |
| L.11 | cC₃H₅ | 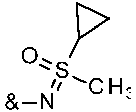 |
| L.12 | cC₃H₅ | 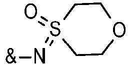 |
| L.13 | CH₂-cC₃H₅ | 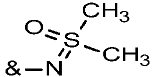 |
| L.14 | CH₂-cC₃H₅ | 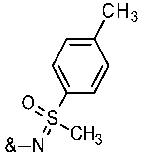 |
| L.15 | CH₂-cC₃H₅ | 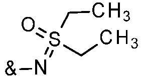 |
| L.16 | CH₂-cC₃H₅ | 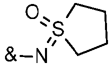 |
| L.17 | CH₂-cC₃H₅ | 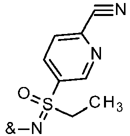 |
| L.18 | CH₂-cC₃H₅ | 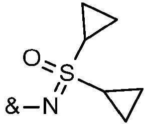 |
| L.19 | CH₂-cC₃H₅ | 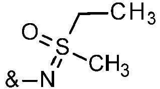 |
| L.20 | CH₂-cC₃H₅ | 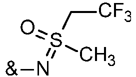 |
| L.21 | CH₂-cC₃H₅ | 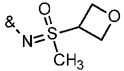 |
| L.22 | CH₂-cC₃H₅ | 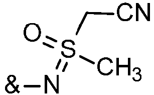 |
| L.23 | CH₂-cC₃H₅ | 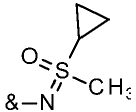 |
| L.24 | CH₂-cC₃H₅ | 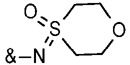 |
| L.25 | CH₃ | 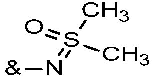 |
| L.26 | CH₃ | 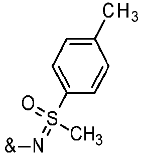 |
| L.27 | CH₃ | 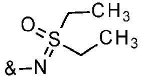 |
| L.28 | CH₃ | 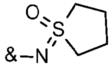 |
| L.29 | CH₃ | 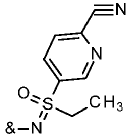 |
| L.30 | CH₃ | 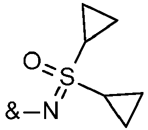 |
| L.31 | CH₃ | 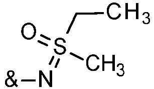 |
| L.32 | CH₃ | 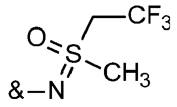 |
| L.33 | CH₃ | 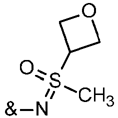 |
| L.34 | CH₃ | 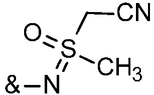 |
| L.35 | CH₃ | 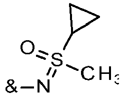 |
| L.36 | CH₃ | 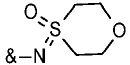 |
| L.37 | CH₂CF₃ | 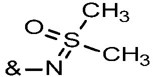 |
| L.38 | CH₂CF₃ | 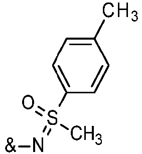 |
| L.39 | CH₂CF₃ | 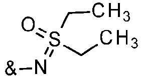 |
| L.40 | CH₂CF₃ | 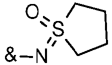 |
| L.41 | CH₂CF₃ | 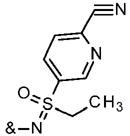 |
| L.42 | CH₂CF₃ | 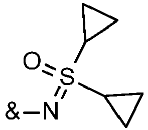 |
| L.43 | CH₂CF₃ | 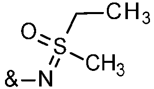 |
| L.44 | CH₂CF₃ | 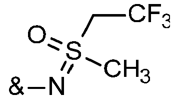 |
| L.45 | CH₂CF₃ | 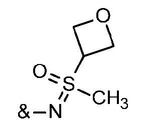 |
| L.46 | CH₂CF₃ | 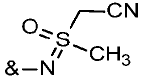 |
| L.47 | CH₂CF₃ | 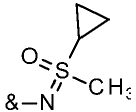 |
| L.48 | CH₂CF₃ | 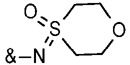 |
| L.49 | OCH₃ | 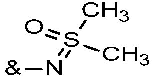 |
| L.50 | OCH₃ | 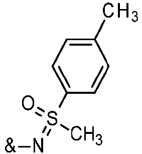 |
| L.51 | OCH₃ | 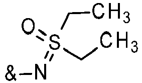 |
| L.52 | OCH₃ | 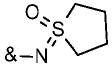 |
| L.53 | OCH₃ | 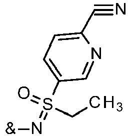 |
| L.54 | OCH₃ | 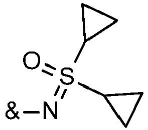 |
| L.55 | OCH₃ | 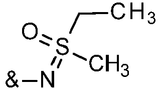 |
| L.56 | OCH₃ | 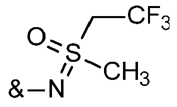 |
| L.57 | OCH₃ | 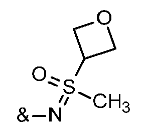 |
| L.58 | OCH₃ | 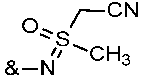 |
| L.59 | OCH₃ | 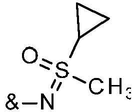 |
| L.60 | OCH₃ | 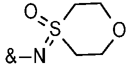 |

The following Embodiments 1 to 60 represent specific combinations of definitions for all substituents and Tables. Each combination individually and all combinations collectively represent preferred embodiments.
Embodiment 1: R¹ is CF₃, R² is H, X is O, R⁶ is H, W is SO₂, R^{W} is CH₃CH₂, the combination of R³ and the group of formula (S) is as in line L.1 of Table A, the ring system is as indicated in a table selected from Table 1 to Table 105, and the second substituent R⁷, if present, is CH₃.
Embodiment 2: R¹ is CF₃, R² is H, X is O, R⁶ is H, W is SO₂, R^{W} is CH₃CH₂, the combination of R³ and the group of formula (S) is as in line L.2 of Table A, the ring system is as indicated in a table selected from Table 1 to Table 105, and the second substituent R⁷, if present, is CH₃.
Embodiment 3: R¹ is CF₃, R² is H, X is O, R⁶ is H, W is SO₂, R^{W} is CH₃CH₂, the combination of R³ and the group of formula (S) is as in line L.3 of Table A, the ring system is as indicated in a table selected from Table 1 to Table 105, and the second substituent R⁷, if present, is CH₃.
Embodiment 4: R¹ is CF₃, R² is H, X is O, R⁶ is H, W is SO₂, R^{W} is CH₃CH₂, the combination of R³ and the group of formula (S) is as in line L.4 of Table A, the ring system is as indicated in a table selected from Table 1 to Table 105, and the second substituent R⁷, if present, is CH₃.
Embodiment 5: R¹ is CF₃, R² is H, X is O, R⁶ is H, W is SO₂, R^{W} is CH₃CH₂, the combination of R³ and the group of formula (S) is as in line L.5 of Table A, the ring system is as indicated in a table selected from Table 1 to Table 105, and the second substituent R⁷, if present, is CH₃.
Embodiment 6: R¹ is CF₃, R² is H, X is O, R⁶ is H, W is SO₂, R^{W} is CH₃CH₂, the combination of R³ and the group of formula (S) is as in line L.6 of Table A, the ring system is as indicated in a table selected from Table 1 to Table 105, and the second substituent R⁷, if present, is CH₃.
Embodiment 7: R¹ is CF₃, R² is H, X is O, R⁶ is H, W is SO₂, R^{W} is CH₃CH₂, the combination of R³ and the group of formula (S) is as in line L.7 of Table A, the ring system is as indicated in a table selected from Table 1 to Table 105, and the second substituent R⁷, if present, is CH₃.
Embodiment 8: R¹ is CF₃, R² is H, X is O, R⁶ is H, W is SO₂, R^{W} is CH₃CH₂, the combination of R³ and the group of formula (S) is as in line L.8 of Table A, the ring system is as indicated in a table selected from Table 1 to Table 105, and the second substituent R⁷, if present, is CH₃.
Embodiment 9: R¹ is CF₃, R² is H, X is O, R⁶ is H, W is SO₂, R^{W} is CH₃CH₂, the combination of R³ and the group of formula (S) is as in line L.9 of Table A, the ring system is as indicated in a table selected from Table 1 to Table 105, and the second substituent R⁷, if present, is CH₃.
Embodiment 10: R¹ is CF₃, R² is H, X is O, R⁶ is H, W is SO₂, R^{W} is CH₃CH₂, the combination of R³ and the group of formula (S) is as in line L.10 of Table A, the ring system is as indicated in a table selected from Table 1 to Table 105, and the second substituent R⁷, if present, is CH₃.
Embodiment 11: R¹ is CF₃, R² is H, X is O, R⁶ is H, W is SO₂, R^{W} is CH₃CH₂, the combination of R³ and the group of formula (S) is as in line L.11 of Table A, the ring system is as indicated in a table selected from Table 1 to Table 105, and the second substituent R⁷, if present, is CH₃.
Embodiment 12: R¹ is CF₃, R² is H, X is O, R⁶ is H, W is SO₂, R^{W} is CH₃CH₂, the combination of R³ and the group of formula (S) is as in line L.12 of Table A, the ring system is as indicated in a table selected from Table 1 to Table 105, and the second substituent R⁷, if present, is CH₃.
Embodiment 13: R¹ is CF₃, R² is H, X is O, R⁶ is H, W is SO₂, R^{W} is CH₃CH₂, the combination of R³ and the group of formula (S) is as in line L.13 of Table A, the ring system is as indicated in a table selected from Table 1 to Table 105, and the second substituent R⁷, if present, is CH₃.
Embodiment 14: R¹ is CF₃, R² is H, X is O, R⁶ is H, W is SO₂, R^{W} is CH₃CH₂, the combination of R³ and the group of formula (S) is as in line L.14 of Table A, the ring system is as indicated in a table selected from Table 1 to Table 105, and the second substituent R⁷, if present, is CH₃.
Embodiment 15: R¹ is CF₃, R² is H, X is O, R⁶ is H, W is SO₂, R^{W} is CH₃CH₂, the combination of R³ and the group of formula (S) is as in line L.15 of Table A, the ring system is as indicated in a table selected from Table 1 to Table 105, and the second substituent R⁷, if present, is CH₃.
Embodiment 16: R¹ is CF₃, R² is H, X is O, R⁶ is H, W is SO₂, R^{W} is CH₃CH₂, the combination of R³ and the group of formula (S) is as in line L.16 of Table A, the ring system is as indicated in a table selected from Table 1 to Table 105, and the second substituent R⁷, if present, is CH₃.
Embodiment 17: R¹ is CF₃, R² is H, X is O, R⁶ is H, W is SO₂, R^{W} is CH₃CH₂, the combination of R³ and the group of formula (S) is as in line L.17 of Table A, the ring system is as indicated in a table selected from Table 1 to Table 105, and the second substituent R⁷, if present, is CH₃.
Embodiment 18: R¹ is CF₃, R² is H, X is O, R⁶ is H, W is SO₂, R^{W} is CH₃CH₂, the combination of R³ and the group of formula (S) is as in line L.18 of Table A, the ring system is as indicated in a table selected from Table 1 to Table 105, and the second substituent R⁷, if present, is CH₃.
Embodiment 19: R¹ is CF₃, R² is H, X is O, R⁶ is H, W is SO₂, R^{W} is CH₃CH₂, the combination of R³ and the group of formula (S) is as in line L.19 of Table A, the ring system is as indicated in a table selected from Table 1 to Table 105, and the second substituent R⁷, if present, is CH₃.
Embodiment 20: R¹ is CF₃, R² is H, X is O, R⁶ is H, W is SO₂, R^{W} is CH₃CH₂, the combination of R³ and the group of formula (S) is as in line L.20 of Table A, the ring system is as indicated in a table selected from Table 1 to Table 105, and the second substituent R⁷, if present, is CH₃.
Embodiment 21: R¹ is CF₃, R² is H, X is O, R⁶ is H, W is SO₂, R^{W} is CH₃CH₂, the combination of R³ and the group of formula (S) is as in line L.21 of Table A, the ring system is as indicated in a table selected from Table 1 to Table 105, and the second substituent R⁷, if present, is CH₃.
Embodiment 22: R¹ is CF₃, R² is H, X is O, R⁶ is H, W is SO₂, R^{W} is CH₃CH₂, the combination of R³ and the group of formula (S) is as in line L.22 of Table A, the ring system is as indicated in a table selected from Table 1 to Table 105, and the second substituent R⁷, if present, is CH₃.
Embodiment 23: R¹ is CF₃, R² is H, X is O, R⁶ is H, W is SO₂, R^{W} is CH₃CH₂, the combination of R³ and the group of formula (S) is as in line L.23 of Table A, the ring system is as indicated in a table selected from Table 1 to Table 105, and the second substituent R⁷, if present, is CH₃.
Embodiment 24: R¹ is CF₃, R² is H, X is O, R⁶ is H, W is SO₂, R^{W} is CH₃CH₂, the combination of R³ and the group of formula (S) is as in line L.24 of Table A, the ring system is as indicated in a table selected from Table 1 to Table 105, and the second substituent R⁷, if present, is CH₃.
Embodiment 25: R¹ is CF₃, R² is H, X is O, R⁶ is H, W is SO₂, R^{W} is CH₃CH₂, the combination of R³ and the group of formula (S) is as in line L.25 of Table A, the ring system is as indicated in a table selected from Table 1 to Table 105, and the second substituent R⁷, if present, is CH₃.
Embodiment 26: R¹ is CF₃, R² is H, X is O, R⁶ is H, W is SO₂, R^{W} is CH₃CH₂, the combination of R³ and the group of formula (S) is as in line L.26 of Table A, the ring system is as indicated in a table selected from Table 1 to Table 105, and the second substituent R⁷, if present, is CH₃.
Embodiment 27: R¹ is CF₃, R² is H, X is O, R⁶ is H, W is SO₂, R^{W} is CH₃CH₂, the combination of R³ and the group of formula (S) is as in line L.27 of Table A, the ring system is as indicated in a table selected from Table 1 to Table 105, and the second substituent R⁷, if present, is CH₃.
Embodiment 28: R¹ is CF₃, R² is H, X is O, R⁶ is H, W is SO₂, R^{W} is CH₃CH₂, the combination of R³ and the group of formula (S) is as in line L.28 of Table A, the ring system is as indicated in a table selected from Table 1 to Table 105, and the second substituent R⁷, if present, is CH₃.
Embodiment 29: R¹ is CF₃, R² is H, X is O, R⁶ is H, W is SO₂, R^{W} is CH₃CH₂, the combination of R³ and the group of formula (S) is as in line L.29 of Table A, the ring system is as indicated in a table selected from Table 1 to Table 105, and the second substituent R⁷, if present, is CH₃.
Embodiment 30: R¹ is CF₃, R² is H, X is O, R⁶ is H, W is SO₂, R^{W} is CH₃CH₂, the combination of R³ and the group of formula (S) is as in line L.30 of Table A, the ring system is as indicated in a table selected from Table 1 to Table 105, and the second substituent R⁷, if present, is CH₃.
Embodiment 31: R¹ is CF₃, R² is H, X is O, R⁶ is H, W is SO₂, R^{W} is CH₃CH₂, the combination of R³ and the group of formula (S) is as in line L.31 of Table A, the ring system is as indicated in a table selected from Table 1 to Table 105, and the second substituent R⁷, if present, is CH₃.
Embodiment 32: R¹ is CF₃, R² is H, X is O, R⁶ is H, W is SO₂, R^{W} is CH₃CH₂, the combination of R³ and the group of formula (S) is as in line L.32 of Table A, the ring system is as indicated in a table selected from Table 1 to Table 105, and the second substituent R⁷, if present, is CH₃.
Embodiment 33: R¹ is CF₃, R² is H, X is O, R⁶ is H, W is SO₂, R^{W} is CH₃CH₂, the combination of R³ and the group of formula (S) is as in line L.33 of Table A, the ring system is as indicated in a table selected from Table 1 to Table 105, and the second substituent R⁷, if present, is CH₃.
Embodiment 34: R¹ is CF₃, R² is H, X is O, R⁶ is H, W is SO₂, R^{W} is CH₃CH₂, the combination of R³ and the group of formula (S) is as in line L.34 of Table A, the ring system is as indicated in a table selected from Table 1 to Table 105, and the second substituent R⁷, if present, is CH₃.
Embodiment 35: R¹ is CF₃, R² is H, X is O, R⁶ is H, W is SO₂, R^{W} is CH₃CH₂, the combination of R³ and the group of formula (S) is as in line L.35 of Table A, the ring system is as indicated in a table selected from Table 1 to Table 105, and the second substituent R⁷, if present, is CH₃.
Embodiment 36: R¹ is CF₃, R² is H, X is O, R⁶ is H, W is SO₂, R^{W} is CH₃CH₂, the combination of R³ and the group of formula (S) is as in line L.36 of Table A, the ring system is as indicated in a table selected from Table 1 to Table 105, and the second substituent R⁷, if present, is CH₃.
Embodiment 37: R¹ is CF₃, R² is H, X is O, R⁶ is H, W is SO₂, R^{W} is CH₃CH₂, the combination of R³ and the group of formula (S) is as in line L.37 of Table A, the ring system is as indicated in a table selected from Table 1 to Table 105, and the second substituent R⁷, if present, is CH₃.
Embodiment 38: R¹ is CF₃, R² is H, X is O, R⁶ is H, W is SO₂, R^{W} is CH₃CH₂, the combination of R³ and the group of formula (S) is as in line L.38 of Table A, the ring system is as indicated in a table selected from Table 1 to Table 105, and the second substituent R⁷, if present, is CH₃.
Embodiment 39: R¹ is CF₃, R² is H, X is O, R⁶ is H, W is SO₂, R^{W} is CH₃CH₂, the combination of R³ and the group of formula (S) is as in line L.39 of Table A, the ring system is as indicated in a table selected from Table 1 to Table 105, and the second substituent R⁷, if present, is CH₃.
Embodiment 40: R¹ is CF₃, R² is H, X is O, R⁶ is H, W is SO₂, R^{W} is CH₃CH₂, the combination of R³ and the group of formula (S) is as in line L.40 of Table A, the ring system is as indicated in a table selected from Table 1 to Table 105, and the second substituent R⁷, if present, is CH₃.
Embodiment 41: R¹ is CF₃, R² is H, X is O, R⁶ is H, W is SO₂, R^{W} is CH₃CH₂, the combination of R³ and the group of formula (S) is as in line L.41 of Table A, the ring system is as indicated in a table selected from Table 1 to Table 105, and the second substituent R⁷, if present, is CH₃.
Embodiment 42: R¹ is CF₃, R² is H, X is O, R⁶ is H, W is SO₂, R^{W} is CH₃CH₂, the combination of R³ and the group of formula (S) is as in line L.42 of Table A, the ring system is as indicated in a table selected from Table 1 to Table 105, and the second substituent R⁷, if present, is CH₃.
Embodiment 43: R¹ is CF₃, R² is H, X is O, R⁶ is H, W is SO₂, R^{W} is CH₃CH₂, the combination of R³ and the group of formula (S) is as in line L.43 of Table A, the ring system is as indicated in a table selected from Table 1 to Table 105, and the second substituent R⁷, if present, is CH₃.
Embodiment 44: R¹ is CF₃, R² is H, X is O, R⁶ is H, W is SO₂, R^{W} is CH₃CH₂, the combination of R³ and the group of formula (S) is as in line L.44 of Table A, the ring system is as indicated in a table selected from Table 1 to Table 105, and the second substituent R⁷, if present, is CH₃.
Embodiment 45: R¹ is CF₃, R² is H, X is O, R⁶ is H, W is SO₂, R^{W} is CH₃CH₂, the combination of R³ and the group of formula (S) is as in line L.45 of Table A, the ring system is as indicated in a table selected from Table 1 to Table 105, and the second substituent R⁷, if present, is CH₃.
Embodiment 46: R¹ is CF₃, R² is H, X is O, R⁶ is H, W is SO₂, R^{W} is CH₃CH₂, the combination of R³ and the group of formula (S) is as in line L.46 of Table A, the ring system is as indicated in a table selected from Table 1 to Table 105, and the second substituent R⁷, if present, is CH₃.
Embodiment 47: R¹ is CF₃, R² is H, X is O, R⁶ is H, W is SO₂, R^{W} is CH₃CH₂, the combination of R³ and the group of formula (S) is as in line L.47 of Table A, the ring system is as indicated in a table selected from Table 1 to Table 105, and the second substituent R⁷, if present, is CH₃.
Embodiment 48: R¹ is CF₃, R² is H, X is O, R⁶ is H, W is SO₂, R^{W} is CH₃CH₂, the combination of R³ and the group of formula (S) is as in line L.48 of Table A, the ring system is as indicated in a table selected from Table 1 to Table 105, and the second substituent R⁷, if present, is CH₃.
Embodiment 49: R¹ is CF₃, R² is H, X is O, R⁶ is H, W is SO₂, R^{W} is CH₃CH₂, the combination of R³ and the group of formula (S) is as in line L.49 of Table A, the ring system is as indicated in a table selected from Table 1 to Table 105, and the second substituent R⁷, if present, is CH₃.
Embodiment 50: R¹ is CF₃, R² is H, X is O, R⁶ is H, W is SO₂, R^{W} is CH₃CH₂, the combination of R³ and the group of formula (S) is as in line L.50 of Table A, the ring system is as indicated in a table selected from Table 1 to Table 105, and the second substituent R⁷, if present, is CH₃.
Embodiment 51: R¹ is CF₃, R² is H, X is O, R⁶ is H, W is SO₂, R^{W} is CH₃CH₂, the combination of R³ and the group of formula (S) is as in line L.51 of Table A, the ring system is as indicated in a table selected from Table 1 to Table 105, and the second substituent R⁷, if present, is CH₃.
Embodiment 52: R¹ is CF₃, R² is H, X is O, R⁶ is H, W is SO₂, R^{W} is CH₃CH₂, the combination of R³ and the group of formula (S) is as in line L.51 of Table A, the ring system is as indicated in a table selected from Table 1 to Table 105, and the second substituent R⁷, if present, is CH₃.
Embodiment 53: R¹ is CF₃, R² is H, X is O, R⁶ is H, W is SO₂, R^{W} is CH₃CH₂, the combination of R³ and the group of formula (S) is as in line L.53 of Table A, the ring system is as indicated in a table selected from Table 1 to Table 105, and the second substituent R⁷, if present, is CH₃.
Embodiment 54: R¹ is CF₃, R² is H, X is O, R⁶ is H, W is SO₂, R^{W} is CH₃CH₂, the combination of R³ and the group of formula (S) is as in line L.54 of Table A, the ring system is as indicated in a table selected from Table 1 to Table 105, and the second substituent R⁷, if present, is CH₃.
Embodiment 55: R¹ is CF₃, R² is H, X is O, R⁶ is H, W is SO₂, R^{W} is CH₃CH₂, the combination of R³ and the group of formula (S) is as in line L.55 of Table A, the ring system is as indicated in a table selected from Table 1 to Table 105, and the second substituent R⁷, if present, is CH₃.
Embodiment 56: R¹ is CF₃, R² is H, X is O, R⁶ is H, W is SO₂, R^{W} is CH₃CH₂, the combination of R³ and the group of formula (S) is as in line L.56 of Table A, the ring system is as indicated in a table selected from Table 1 to Table 105, and the second substituent R⁷, if present, is CH₃.
Embodiment 57: R¹ is CF₃, R² is H, X is O, R⁶ is H, W is SO₂, R^{W} is CH₃CH₂, the combination of R³ and the group of formula (S) is as in line L.57 of Table A, the ring system is as indicated in a table selected from Table 1 to Table 105, and the second substituent R⁷, if present, is CH₃.
Embodiment 58: R¹ is CF₃, R² is H, X is O, R⁶ is H, W is SO₂, R^{W} is CH₃CH₂, the combination of R³ and the group of formula (S) is as in line L.58 of Table A, the ring system is as indicated in a table selected from Table 1 to Table 105, and the second substituent R⁷, if present, is CH₃.
Embodiment 59: R¹ is CF₃, R² is H, X is O, R⁶ is H, W is SO₂, R^{W} is CH₃CH₂, the combination of R³ and the group of formula (S) is as in line L.59 of Table A, the ring system is as indicated in a table selected from Table 1 to Table 105, and the second substituent R⁷, if present, is CH₃.
Embodiment 60: R¹ is CF₃, R² is H, X is O, R⁶ is H, W is SO₂, R^{W} is CH₃CH₂, the combination of R³ and the group of formula (S) is as in line L.60 of Table A, the ring system is as indicated in a table selected from Table 1 to Table 105, and the second substituent R⁷, if present, is CH₃.

In a preferred embodiment, the compound of formula (I) is a compound of formula (I.A) wherein G is a ring selected from A1 to A48, and wherein
- R¹: is H, C₁-C₃-alkyl, or C₁-C₃-haloalkyl;
- R²: is H, or C₁-C₃-alkyl, preferably H;
- R³: is C₁-C₃-alkyl, C₃-C₅-cycloalkyl, C₃-C₆-cycloalkyl-C₁C₂-alkyl, which groups are unsubstituted or halogenated;
- R⁶: is H, or C₁-C₃-alkyl, preferably H;
- X: is O or S, preferably O;
- n: is 1 or 2;
each R⁷ is is independently H, halogen;
C₁-C₆-alkyl, C₁-C₃-haloalkyl
wherein at least one R⁷ is a group of formula (S) 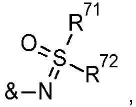 wherein each R⁷¹, R⁷² is independently selected from C₁-C₃-alkyl, C₃-C₆-cycloalkyl, which groups are unsubstituted, or substituted with one or more, same or different substituents selected from halogen and CN;
a 5- to 6-membered saturated, partially unsaturated, or fully unsaturated heterocyclic ring, wherein said heterocyclic ring comprises one or more, same or different heteroatoms O, N, or S, and is unsubstituted, or substituted with one or more, same or different substituents selected from halogen, CN and C₁-C₃-alkyl;
phenyl, which is unsubstituted, or substituted with one or more, same or different substituents selected from halogen, CN and C₁-C₃-alkyl;
or two substituents R⁷¹, R⁷² form, together with the sulfur atom to which they are bound, a 5- or 6- membered saturated, partially unsaturated, or fully unsaturated heterocycle, which heterocycle is unsubstituted, or substituted with one or more, same or different substituents selected from halogen, CN, and C₁-C₃-alkyl, and wherein said heterocycle comprises no, one or more, same or different heteroatoms O, N, or S in addition to the sulfur atom to which R⁷¹ and R⁷² are bound to.

In another preferred embodiment, the compound of formula (I) is a compound of formula (I.A) wherein G is a ring A1, and wherein
- R¹: is C₁-C₃-alkyl, or C₁-C₃-haloalkyl;
- R²: is H, or C₁-C₃-alkyl, preferably H;
- R³: is C₁-C₃-alkyl, cyclopropyl, or cyclopropylmethyl, which groups are unsubstituted or halogenated;
- R⁶: is H, or C₁-C₃-alkyl, preferably H;
- X: is O or S, preferably O;
- n: is 1 or 2;
each R⁷ is is independently H;
C₁-C₆-alkyl, C₁-C₃-haloalkyl,
wherein at least one R⁷ is a group of formula (S) 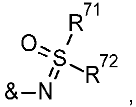
wherein each R⁷¹, R⁷² is independently selected from C₁-C₃-alkyl, or C₃-C₆-cycloalkyl, which groups are unsubstituted, or substituted with one or more, substituents selected from halogen and CN;
a 5- to 6-membered saturated, partially unsaturated, or fully unsaturated heterocyclic ring, wherein said heterocyclic ring comprises one or more, same or different heteroatoms O, N, or S, and is unsubstituted, or substituted with one or more, same or different substituents selected from halogen, CN and C₁-C₃-alkyl;
phenyl, which is unsubstituted, or substituted with one or more, same or different substituents selected from halogen, CN and C₁-C₃-alkyl;
or two substituents R⁷¹, R⁷² form, together with the sulfur atom to which they are bound, a 5- or 6- membered saturated, partially unsaturated, or fully unsaturated heterocycle, which heterocycle is unsubstituted, or substituted with one or more, same or different substituents selected from halogen, CN, and C₁-C₃-alkyl, and wherein said heterocycle comprises no, one or more, same or different heteroatoms O, N, or S in addition to the sulfur atom to which R⁷¹ and R⁷² are bound to.

In another preferred embodiment, the compound of formula (I) is a compound of formula (I.A) wherein G is a ring A44, and wherein
- R¹: is C₁-C₃-alkyl, or C₁-C₃-haloalkyl;
- R²: is H, or C₁-C₃-alkyl, preferably H;
- R³: is C₁-C₃-alkyl, cyclopropyl, or cyclopropylmethyl, which groups are unsubstituted or halogenated;
- R⁶: is H, or C₁-C₃-alkyl, preferably H;
- X: is O or S, preferably O;
- n: is 1 or 2;
each R⁷ is is independently H;
C₁-C₆-alkyl, C₁-C₃-haloalkyl,
wherein at least one R⁷ is a group of formula (S),
wherein each R⁷¹, R⁷² is independently selected from C₁-C₃-alkyl, or C₃-C₆-cycloalkyl, which groups are unsubstituted, or substituted with one or more, substituents selected from halogen and CN;
a 5- to 6-membered saturated, partially unsaturated, or fully unsaturated heterocyclic ring, wherein said heterocyclic ring comprises one or more, same or different heteroatoms O, N, or S, and is unsubstituted, or substituted with one or more, same or different substituents selected from halogen, CN and C₁-C₃-alkyl;
phenyl, which is unsubstituted, or substituted with one or more, same or different substituents selected from halogen, CN and C₁-C₃-alkyl;
or two substituents R⁷¹, R⁷² form, together with the sulfur atom to which they are bound, a 4-, 5- or 6- membered saturated, partially unsaturated, or fully unsaturated heterocycle, which heterocycle is unsubstituted, or substituted with one or more, same or different substituents selected from halogen, CN, and C₁-C₃-alkyl, and wherein said heterocycle comprises no, one or more, same or different heteroatoms O, N, or S in addition to the sulfur atom to which R⁷¹ and R⁷² are bound to.

In another preferred embodiment of compounds I both groups R⁷¹, R⁷² form, together with the sulfur atom to which they are bound, a 4-, 5- or 6- membered saturated, partially unsaturated, or fully unsaturated heterocycle, which heterocycle is unsubstituted, or substituted with one or more, same or different substituents selected from halogen, CN, and C₁-C₃-alkyl, and wherein said heterocycle comprises no, one or more, same or different heteroatoms O, N, or S in addition to the sulfur atom to which R⁷¹ and R⁷² are bound to, and the other variables are as defined and preferred as outlined in the outset and the claims.

In another preferred embodiment, the compound of formula (I) is a compound of formula (I.A) wherein G is a ring A1, and wherein
- R¹: is C₁-C₃-alkyl, or C₁-C₃-haloalkyl;
- R²: is H, or C₁-C₃-alkyl;
- R³: is C₁-C₃-alkyl, cyclopropyl, or cyclopropylmethyl, which groups are unsubstituted or halogenated;
- R⁶: is H, or C₁-C₃-alkyl;
- X: is O or S;
- n: is 1 or 2;
each R⁷ is is independently H;
C₁-C₆-alkyl, C₁-C₃-haloalkyl
wherein at least one R⁷ is a group of formula (S) 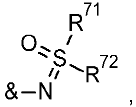
   wherein each R⁷¹, R⁷² is independently selected from C₁-C₃-alkyl, or C₃-C₆-cycloalkyl, which groups are unsubstituted, or substituted with one or more, same or different substituents selected from halogen and CN;
   or two substituents R⁷¹, R⁷² form, together with the sulfur atom to which they are bound, a 5- or 6- membered saturated, partially unsaturated, or fully unsaturated heterocycle, which heterocycle is unsubstituted, or substituted with one or more, same or different substituents selected from halogen, CN, and C₁-C₃-alkyl, and wherein said heterocycle comprises no, one or more, same or different heteroatoms O, N, or S in addition to the sulfur atom to which R⁷¹ and R⁷² are bound to.

In another preferred embodiment, the compound of formula (I) is a compound of formula (I.A) wherein G is a ring A1 or A44, and wherein
- R¹: is C₁-C₃-alkyl, or C₁-C₃-haloalkyl;
- R²: is H, or C₁-C₃-alkyl, preferably H;
- R³: is C₁-C₃-alkyl, cyclopropyl, cyclopropylmethyl, or benzyl, which groups are unsubstituted or halogenated;
- R⁶: is H, or C₁-C₃-alkyl, preferably H
- X: is O or S, preferably O;
- n: is 1 or 2, preferably 1;
each R⁷ is independently H, C₁-C₆-alkyl, C₁-C₃-haloalkyl,
wherein at least one R⁷ is a group of formula (S) 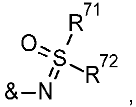
wherein each R⁷¹, R⁷² is independently selected from
C₁-C₃-alkyl, or C₃-C₆-cycloalkyl, which groups are unsubstituted, or substituted with one or more, substituents selected from halogen and CN;
a 4-, 5- or 6-membered saturated, partially unsaturated, or fully unsaturated heterocyclic ring, wherein said heterocyclic ring comprises one or more, same or different heteroatoms O, N, or S, and is unsubstituted, or substituted with one or more, same or different substituents selected from halogen, CN and C₁-C₃-alkyl;
phenyl, which is unsubstituted, or substituted with one or more, same or different substituents selected from halogen, CN, and C₁-C₃-alkyl;
or two substituents R⁷¹, R⁷² form, together with the sulfur atom to which they are bound, a 4-, 5- or 6- membered saturated, partially unsaturated, or fully unsaturated heterocycle, which heterocycle is unsubstituted, or substituted with one or more, same or different substituents selected from halogen, CN, and C₁-C₃-alkyl, and wherein said heterocycle comprises no, one or more, same or different heteroatoms O, N, or S in addition to the sulfur atom to which R⁷¹ and R⁷² are bound to.

The invention also relates to a mixture of at least one compound of the invention with at least one mixing partner. Preferred are binary mixtures of one compound of the invention as component I with one mixing partner herein as component II. Preferred weight ratios for such binary mixtures are from 5000:1 to 1:5000, preferably from 1000:1 to 1:1000, more preferably from 100:1 to 1:100, particularly from 10:1 to 1:10. In such binary mixtures, components I and II may be used in equal amounts, or an excess of component I, or an excess of component II may be used.

Mixing partners can be selected from pesticides, in particular insecticides, nematicides, and acaricides, fungicides, herbicides, plant growth regulators, fertilizers. Preferred mixing partners are insecticides, nematicides, and fungicides.

The invention also relates to agrochemical compositions comprising an auxiliary and at least one compound of the invention or a mixture thereof.

An agrochemical composition comprises a pesticidally effective amount of a compound of the invention or a mixture thereof.

The compounds of the invention or the mixtures thereof can be converted into customary types of agro-chemical compositions, e.g. solutions, emulsions, suspensions, dusts, powders, pastes, granules, pressings, capsules, and mixtures thereof. Examples for composition types are suspensions (e.g. SC, OD, FS), emulsifiable concentrates (e.g. EC), emulsions (e.g. EW, EO, ES, ME), capsules (e.g. CS, ZC), pastes, pastilles, wettable powders or dusts (e.g. WP, SP, WS, DP, DS), pressings (e.g. BR, TB, DT), granules (e.g. WG, SG, GR, FG, GG, MG), insecticidal articles (e.g. LN), as well as gel formulations for the treatment of plant propagation materials e.g. seeds (e.g. GF). These and further compositions types are defined in the "Catalogue of pesticide formulation types and international coding system", Technical Monograph No. 2, 6th Ed. May 2008, CropLife International.

The compositions are prepared in a known manner, e.g. described by Mollet and Grubemann, Formulation technology, Wiley VCH, Weinheim, 2001; or Knowles, New developments in crop protection product formulation, Agrow Reports DS243, T&F Informa, London, 2005.

Examples for suitable auxiliaries are solvents, liquid carriers, solid carriers or fillers, surfactants, dispersants, emulsifiers, wetters, adjuvants, solubilizers, penetration enhancers, protective colloids, adhesion agents, thickeners, humectants, repellents, attractants, feeding stimulants, compatibilizers, bactericides, anti-freezing agents, anti-foaming agents, colorants, tackifiers and binders.

Suitable solvents and liquid carriers are water and organic solvents, e.g. mineral oil fractions of medium to high boiling point, e.g. kerosene, diesel oil; oils of vegetable or animal origin; hydrocarbons, e.g. toluene, paraffin, tetrahydronaphthalene, alkylated naphthalenes; alcohols, e.g. ethanol, propanol, butanol, benzylalcohol, cyclohexanol; glycols; DMSO; ketones, e.g. cyclohexanone; esters, e.g. lactates, carbonates, fatty acid esters, gamma-butyrolactone; fatty acids; phosphonates; amines; amides, e.g. N-methylpyrrolidone, fatty acid dimethylamides; and mixtures thereof.

Suitable solid carriers or fillers are mineral earths, e.g. silicates, silica gels, talc, kaolins, limestone, lime, chalk, clays, dolomite, diatomaceous earth, bentonite, CaSO₄, MgSO₄, MgO; polysaccharide powders, e.g. cellulose, starch; fertilizers, e.g. (NH₄)₂SO₄, (NH₄)₃PO₄, NH₄NO₃, ureas; products of vegetable origin, e.g. cereal meal, tree bark meal, wood meal, nutshell meal, and mixtures thereof.

Suitable surfactants are surface-active compounds, e.g. anionic, cationic, nonionic and amphoteric surfactants, block polymers, polyelectrolytes, and mixtures thereof. Such surfactants can be used as emusifier, dispersant, solubilizer, wetter, penetration enhancer, protective colloid, or adjuvant. Examples of surfactants are listed in McCutcheon's, Vol.1: Emulsifiers & Detergents, McCutcheon's Directories, Glen Rock, USA, 2008 (International or North American Ed.).

Suitable anionic surfactants are alkali, alkaline earth or ammonium salts of sulfonates, sulfates, phosphates, carboxylates, and mixtures thereof. Examples of sulfonates are alkylarylsulfonates, diphenylsulfonates, alpha-olefin sulfonates, lignine sulfonates, sulfonates of fatty acids and oils, sulfonates of ethoxylated alkylphenols, sulfonates of alkoxylated arylphenols, sulfonates of condensed naphthalenes, sulfonates of dodecyl- and tridecylbenzenes, sulfonates of naphthalenes and alkylnaphthalenes, sulfosuccinates or sulfosuccinamates. Examples of sulfates are sulfates of fatty acids and oils, of ethoxylated alkylphenols, of alcohols, of ethoxylated alcohols, or of fatty acid esters. Examples of phosphates are phosphate esters. Examples of carboxylates are alkyl carboxylates, and carboxylated alcohol or alkylphenol ethoxylates.

Suitable nonionic surfactants are alkoxylates, N-subsituted fatty acid amides, amine oxides, esters, sugar-based surfactants, polymeric surfactants, and mixtures thereof. Examples of alkoxylates are compounds e.g. alcohols, alkylphenols, amines, amides, arylphenols, fatty acids or fatty acid esters which have been alkoxylated with 1 to 50 equivalents. Ethylene oxide and/or propylene oxide may be employed for the alkoxylation, preferably ethylene oxide. Examples of N-subsititued fatty acid amides are fatty acid glucamides or fatty acid alkanolamides. Examples of esters are fatty acid esters, glycerol esters or monoglycerides. Examples of sugar-based surfactants are sorbitans, ethoxylated sorbitans, sucrose and glucose esters or alkylpolyglucosides. Examples of polymeric surfactants are homo- or copolymers of vinylpyrrolidone, vinylalcohols, or vinylacetate.

Suitable cationic surfactants are quaternary surfactants, e.g. quaternary ammonium compounds with one or two hydrophobic groups, or salts of long-chain primary amines. Suitable amphoteric surfactants are alkylbetains and imidazolines. Suitable block polymers are block polymers of the A-B or A-B-A type comprising blocks of polyethylene oxide and polypropylene oxide, or of the A-B-C type comprising alkanol, polyethylene oxide and polypropylene oxide. Suitable polyelectrolytes are polyacids or polybases. Examples of polyacids are alkali salts of polyacrylic acid or polyacid comb polymers. Examples of polybases are polyvinylamines or polyethyleneamines.

Suitable adjuvants are compounds, which have a neglectable or even no pesticidal activity themselves, and which improve the biological performance of the compounds of the invention on the target. Examples are surfactants, mineral or vegetable oils, and other auxilaries. Further examples are listed by Knowles, Adjuvants and additives, Agrow Reports DS256, T&F Informa UK, 2006, chapter 5.

Suitable thickeners are polysaccharides (e.g. xanthan gum, carboxymethylcellulose), anorganic clays (organically modified or unmodified), polycarboxylates, and silicates.

Suitable bactericides are bronopol and isothiazolinone derivatives e.g. alkylisothiazoli-nones and benzisothiazolinones.

Suitable anti-freezing agents are ethylene glycol, propylene glycol, urea and glycerin. Suitable anti-foaming agents are silicones, long chain alcohols, and salts of fatty acids. Suitable colorants (e.g. in red, blue, or green) are pigments of low water solubility and water-soluble dyes. Examples are inorganic colorants (e.g. iron oxide, titan oxide, iron hexacyanoferrate) and organic colorants (e.g. alizarin-, azo-, and phthalocyanine colorants).

Suitable tackifiers or binders are polyvinylpyrrolidons, polyvinylacetates, polyvinyl alcohols, polyacrylates, biological or synthetic waxes, and cellulose ethers.

Examples for composition types and their preparation are:
i) Water-soluble concentrates (SL, LS)
   10-60 wt% of a compound I according to the invention and 5-15 wt% wetting agent (e.g. alcohol alkoxylates) are dissolved in water and/or in a water-soluble solvent (e.g. alcohols) up to 100 wt%. The active substance dissolves upon dilution with water.
ii) Dispersible concentrates (DC)
   5-25 wt% of a compound I according to the invention and 1-10 wt% dispersant (e.g. polyvinylpyrrolidone) are dissolved in up to 100 wt% organic solvent (e.g. cyclohexanone). Dilution with water gives a dispersion.
iii) Emulsifiable concentrates (EC)
   15-70 wt% of a compound I according to the invention and 5-10 wt% emulsifiers (e.g. calcium dodecylbenzenesulfonate and castor oil ethoxylate) are dissolved in up to 100 wt% water-insoluble organic solvent (e.g. aromatic hydrocarbon). Dilution with water gives an emulsion.
iv) Emulsions (EW, EO, ES)
   5-40 wt% of a compound I according to the invention and 1-10 wt% emulsifiers (e.g. calcium dodecylbenzenesulfonate and castor oil ethoxylate) are dissolved in 20-40 wt% water-insoluble organic solvent (e.g. aromatic hydrocarbon). This mixture is introduced into up to 100 wt% water by means of an emulsifying machine and made into a homogeneous emulsion. Dilution with water gives an emulsion.
v) Suspensions (SC, OD, FS)
   In an agitated ball mill, 20-60 wt% of a compound I according to the invention are comminuted with addition of 2-10 wt% dispersants and wetting agents (e.g. sodium lignosulfonate and alcohol ethoxylate), 0,1-2 wt% thickener (e.g. xanthan gum) and up to 100 wt% water to give a fine active substance suspension. Dilution with water gives a stable suspension of the active sub-stance. For FS type composition up to 40 wt% binder (e.g. polyvinylalcohol) is added.
vi) Water-dispersible granules and water-soluble granules (WG, SG)
   50-80 wt% of a compound I according to the invention are ground finely with addition of up to 100 wt% dispersants and wetting agents (e.g. sodium lignosulfonate and alcohol ethoxylate) and prepared as water-dispersible or water-soluble granules by means of technical appliances (e.g. extrusion, spray tower, fluidized bed). Dilution with water gives a stable dispersion or solution of the active substance.
vii) Water-dispersible powders and water-soluble powders (WP, SP, WS)
   50-80 wt% of a compound I according to the invention are ground in a rotor-stator mill with addition of 1-5 wt% dispersants (e.g. sodium lignosulfonate), 1-3 wt% wetting agents (e.g. alcohol ethoxylate) and up to 100 wt% solid carrier, e.g. silica gel. Dilution with water gives a stable dispersion or solution of the active substance.
viii) Gel (GW, GF)
   In an agitated ball mill, 5-25 wt% of a compound I according to the invention are comminuted with addition of 3-10 wt% dispersants (e.g. sodium lignosulfonate), 1-5 wt% thickener (e.g. carboxymethylcellulose) and up to 100 wt% water to give a fine suspension of the active sub-stance. Dilution with water gives a stable suspension of the active substance.
ix) Microemulsion (ME)
   5-20 wt% of a compound I according to the invention are added to 5-30 wt% organic solvent blend (e.g. fatty acid dimethylamide and cyclohexanone), 10-25 wt% surfactant blend (e.g. alkohol ethoxylate and arylphenol ethoxylate), and water up to 100 %. This mixture is stirred for 1 h to produce spontaneously a thermodynamically stable microemulsion.
x) Microcapsules (CS)
   An oil phase comprising 5-50 wt% of a compound I according to the invention, 0-40 wt% water insoluble organic solvent (e.g. aromatic hydrocarbon), 2-15 wt% acrylic monomers (e.g. methylmethacrylate, methacrylic acid and a di- or triacrylate) are dispersed into an aqueous solution of a protective colloid (e.g. polyvinyl alcohol). Radical polymerization initiated by a radi-cal initiator results in the formation of poly(meth)acrylate microcapsules. Alternatively, an oil phase comprising 5-50 wt% of a compound I according to the invention, 0-40 wt% water insolu-ble organic solvent (e.g. aromatic hydrocarbon), and an isocyanate monomer (e.g. diphenylme-thene-4,4'-diisocyanatae) are dispersed into an aqueous solution of a protective colloid (e.g. polyvinyl alcohol). The addition of a polyamine (e.g. hexamethylenediamine) results in the for-mation of a polyurea microcapsule. The monomers amount to 1-10 wt%. The wt% relate to the total CS composition.
xi) Dustable powders (DP, DS)
   1-10 wt% of a compound I according to the invention are ground finely and mixed intimately with up to 100 wt% solid carrier, e.g. finely divided kaolin.
xii) Granules (GR, FG)
   0.5-30 wt% of a compound I according to the invention is ground finely and associated with up to 100 wt% solid carrier (e.g. silicate). Granulation is achieved by extrusion, spray-drying or the fluidized bed.
xiii) Ultra-low volume liquids (UL)
   1-50 wt% of a compound I according to the invention are dissolved in up to 100 wt% organic solvent, e.g. aromatic hydrocarbon.

The compositions types i) to xi) may optionally comprise further auxiliaries, e.g. 0.1-1 wt% bactericides, 5-15 wt% anti-freezing agents, 0.1-1 wt% anti-foaming agents, and 0.1-1 wt% colorants.

The agrochemical compositions generally comprise between 0.01 and 95%, preferably between 0.1 and 90%, and most preferably between 0.5 and 75%, by weight of active substance. The active substances are employed in a purity of from 90% to 100%, preferably from 95% to 100% (according to NMR spectrum).

Various types of oils, wetters, adjuvants, fertilizer, or micronutrients, and other pesticides (e.g. herbicides, insecticides, fungicides, growth regulators, safeners) may be added to the active substances or the compositions comprising them as premix or, if appropriate not until immediately prior to use (tank mix). These agents can be admixed with the compositions according to the invention in a weight ratio of 1:100 to 100:1, preferably 1:10 to 10:1.

The user applies the composition according to the invention usually from a predosage device, a knapsack sprayer, a spray tank, a spray plane, or an irrigation system. Usually, the agro-chemical composition is made up with water, buffer, and/or further auxiliaries to the desired application concentration and the ready-to-use spray liquor or the agrochemical composition according to the invention is thus obtained. Usually, 20 to 2000 liters, preferably 50 to 400 liters, of the ready-to-use spray liquor are applied per hectare of agricultural useful area.

According to one embodiment, individual components of the composition of the invention e.g. parts of a kit or parts of a binary or ternary mixture may be mixed by the user himself in a spray tank and further auxiliaries may be added, if appropriate.

In a further embodiment, either individual components of the composition according to the invention or partially premixed components, e.g. components comprising compounds of the invention and/or mixing partners as defined above, may be mixed by the user in a spray tank and further auxiliaries and additives may be added.

In a further embodiment, either individual components of the composition according to the invention or partially premixed components, e.g. components comprising compounds of the invention and/or mixing partners as defined above, can be applied jointly (e.g. after tank mix) or consecutively.

The compounds of the invention are suitable for use in protecting crops, plants, plant propagation materials, e.g. seeds, or soil or water, in which the plants are growing, from attack or infestation by animal pests. Therefore, the invention also relates to a plant protection method, which comprises contacting crops, plants, plant propagation materials, e.g. seeds, or soil or water, in which the plants are growing, to be protected from attack or infestation by animal pests, with a pesticidally effective amount of a compound of the invention.

The compounds of the invention are also suitable for use in combating or controlling animal pests. Therefore, the invention also relates to a method of combating or controlling animal pests, which comprises contacting the animal pests, their habitat, breeding ground, or food supply, or the crops, plants, plant propagation materials, e.g. seeds, or soil, or the area, material or environment in which the animal pests are growing or may grow, with a pesticidally effective amount of a compound of the invention.

The compounds of the invention are effective through both contact and ingestion. Furthermore, the compounds of the invention can be applied to any and all developmental stages, e.g. egg, larva, pupa, and adult.

The compounds of the invention can be applied as such or in form of compositions comprising them as defined above. Furthermore, the compounds of the invention can be applied together with a mixing partner or in form of compositions comprising said mixtures. The components of said mixture can be applied simultaneously, jointly or separately, or in succession, that is immediately one after another and thereby creating the mixture "in situ" on the desired location, e.g. the plant, the sequence, in the case of separate application, generally not having any effect on the result of the control measures.

The application can be carried out both before and after the infestation of the crops, plants, plant propagation materials, e.g. seeds, soil, or the area, material or environment by the pests.

Suitable application methods include i.a. soil treatment, seed treatment, in furrow application, and foliar application. Soil treatment methods include drenching the soil, drip irrigation (drip application onto the soil), dipping roots, tubers or bulbs, or soil injection. Seed treatment techniques include seed dressing, seed coating, seed dusting, seed soaking, and seed pelleting. In furrow applications typically include the steps of making a furrow in cultivated land, seeding the furrow with seeds, applying the pesticidally active compound to the furrow, and closing the furrow. Foliar application refers to the application of the pesticidally active compound to plant foliage, e.g. through spray equipment. For foliar applications, it can be advantageous to modify the behavior of the pests by use of pheromones in combination with the compounds of the invention. Suitable pheromones for specific crops and pests are known and publicly available from databases of pheromones and semiochemicals, e.g. http://www.pherobase.com. As used herein, the term "contacting" includes both direct contact (applying the compounds/compositions directly on the animal pest or plant - typically to the foliage, stem or roots of the plant) and indirect contact (applying the compounds/compositions to the locus, i.e. habitat, breeding ground, plant, seed, soil, area, material, or environment in which a pest is growing or may grow, of the animal pest or plant).

The term "animal pest" includes arthropods, gastropods, and nematodes. Preferred animal pests according to the invention are arthropods, preferably insects and arachnids, in particular insects. Insects, which are of particular relevance for crops, are typically referred to as crop insect pests.

The term "crop" refers to both, growing and harvested crops.

The term "plant" includes cereals, e.g. durum and other wheat, rye, barley, triticale, oats, rice, or maize (fodder maize and sugar maize / sweet and field corn); beet, e.g. sugar beet, or fodder beet; fruits, e.g. pomes, stone fruits, or soft fruits, e.g. apples, pears, plums, peaches, nectarines, almonds, cherries, papayas, strawberries, raspberries, blackberries or gooseberries; leguminous plants, e.g. beans, lentils, peas, alfalfa, or soybeans; oil plants, e.g. rapeseed (oilseed rape), turnip rape, mustard, olives, sunflowers, coconut, cocoa beans, castor oil plants, oil palms, ground nuts, or soybeans; cucurbits, e.g. squashes, pumpkins, cucumber or melons; fiber plants, e.g. cotton, flax, hemp, or jute; citrus fruit, e.g. oranges, lemons, grapefruits or mandarins; vegetables, e.g. eggplant, spinach, lettuce (e.g. iceberg lettuce), chicory, cabbage, asparagus, cabbages, carrots, onions, garlic, leeks, tomatoes, potatoes, cucurbits or sweet peppers; lauraceous plants, e.g. avocados, cinnamon, or camphor; energy and raw material plants, e.g. corn, soybean, rapeseed, sugar cane or oil palm; tobacco; nuts, e.g. walnuts; pistachios; coffee; tea; bananas; vines; hop; sweet leaf (Stevia); natural rubber plants or ornamental and forestry plants, , shrubs, broad-leaved trees or evergreens, eucalyptus; turf; lawn; grass. Preferred plants include potatoes sugar beets, tobacco, wheat, rye, barley, oats, rice, corn, cotton, soybeans, rapeseed, legumes, sunflowers, coffee, or sugar cane; fruits; vines; ornamentals; or vegetables, e.g. cucumbers, tomatoes, beans or squashes.

The term "cultivated plants" is to be understood as including plants which have been modified by mutagenesis or genetic engineering in order to provide a new trait to a plant or to modify an already present trait.

Mutagenesis includes techniques of random mutagenesis using X-rays or mutagenic chemicals, but also techniques of targeted mutagenesis, in order to create mutations at a specific locus of a plant genome. Targeted mutagenesis techniques frequently use oligonucleotides or proteins like CRISPR/Cas, zinc-finger nucleases, TALENs or meganucleases to achieve the targeting effect.

Genetic engineering usually uses recombinant DNA techniques to create modifications in a plant genome which under natural circumstances cannot readily be obtained by cross breeding, mutagenesis or natural recombination. Typically, one or more genes are integrated into the genome of a plant in order to add a trait or improve a trait. These integrated genes are also referred to as transgenes in the art, while plant comprising such transgenes are referred to as transgenic plants. The process of plant transformation usually produces several transformation events, wich differ in the genomic locus in which a transgene has been integrated. Plants comprising a specific transgene on a specific genomic locus are usually described as comprising a specific "event", which is referred to by a specific event name. Traits which have been introduced in plants or have been modified include in particular herbicide tolerance, insect resistance, increased yield and tolerance to abiotic conditions, like drought.

Herbicide tolerance has been created by using mutagenesis as well as using genetic engineering. Plants which have been rendered tolerant to ALS inhibitor herbicides by conventional methods of mutagenesis and breeding comprise plant varieties commercially available under the name Clearfield^{®}.

Herbicide tolerance has been created to glyphosate, glufosinate, 2,4-D, dicamba, oxynil herbicides, like bromoxynil and ioxynil, sulfonylurea herbicides, ALS inhibitor herbicides and HPPD inhibitors, like isoxaflutole and mesotrione.

Transgenes wich have been used to provide herbicide tolerance traits comprise: for tolerance to glyphosate: cp4 epsps, epsps grg23ace5, mepsps, 2mepsps, gat4601, gat4621 and goxv247, for tolerance to glufosinate: pat and bar, for tolerance to 2,4-D: aad-1 and aad-12, for tolerance to dicamba: dmo, for tolerance to oxynil herbicies: bxn, for tolerance to sulfonylurea herbicides: zm-hra, csr1-2, gm-hra, S4-HrA, for tolerance to ALS inhibitor herbicides: csr1-2, for tolerance to HPPD inhibitor herbicides: hppdPF, W336 and avhppd-03.

Transgenic corn events comprising herbicide tolerance genes are e.g., but not excluding others, DAS40278, MON801, MON802, MON809, MON810, MON832, MON87411, MON87419, MON87427, MON88017, MON89034, NK603, GA21, MZHG0JG, HCEM485, VCO-Ø1981-5, 676, 678, 680, 33121, 4114, 59122, 98140, Bt10, Bt176, CBH-351, DBT418, DLL25, MS3, MS6, MZIR098, T25, TC1507 and TC6275.

Transgenic soybean events comprising herbicide tolerance genes are e.g., but not excluding others, GTS 40-3-2, MON87705, MON87708, MON87712, MON87769, MON89788, A2704-12, A2704-21, A5547-127, A5547-35, DP356043, DAS44406-6, DAS68416-4, DAS-81419-2, GU262, SYHTØH2, W62, W98, FG72 and CV127.

Transgenic cotton events comprising herbicide tolerance genes are e.g., but not excluding others, 19-51a, 31707, 42317, 81910, 281-24-236, 3006-210-23, BXN10211, BXN10215, BXN10222, BXN10224, MON1445, MON1698, MON88701, MON88913, GHB119, GHB614, LLCotton25, T303-3 and T304-40.

Transgenic canola events comprising herbicide tolerance genes are e.g., but not excluding others, MON88302, HCR-1, HCN10, HCN28, HCN92, MS1, MS8, PHY14, PHY23, PHY35, PHY36, RF1, RF2 and RF3.

Insect resistance has mainly been created by transferring bacterial genes for insecticidal proteins to plants. Transgenes which have most frequently been used are toxin genes of *Bacillus spec.* and synthetic variants thereof, like cry1A, cry1Ab, cry1Ab-Ac, cry1Ac, cry1A.105, cry1F, cry1Fa2, cry2Ab2, cry2Ae, mcry3A, ecry3.1Ab, cry3Bb1, cry34Ab1, cry35Ab1, cry9C, vip3A(a), vip3Aa20. However, also genes of plant origin have been transferred to other plants. In particular genes coding for protease inhibitors, like CpTI and pinll. A further approach uses transgenes in order to produce double stranded RNA in plants to target and downregulate insect genes. An example for such a transgene is dvsnf7.

Transgenic corn events comprising genes for insecticidal proteins or double stranded RNA are e.g., but not excluding others, Bt10, Bt11, Bt176, MON801, MON802, MON809, MON810, MON863, MON87411, MON88017, MON89034, 33121, 4114, 5307, 59122, TC1507, TC6275, CBH-351, MIR162, DBT418 and MZIR098.

Transgenic soybean events comprising genes for insecticidal proteins are e.g., but not excluding others, MON87701, MON87751 and DAS-81419.

Transgenic cotton events comprising genes for insecticidal proteins are e.g., but not excluding others, SGK321, MON531, MON757, MON1076, MON15985, 31707, 31803, 31807, 31808, 42317, BNLA-601, Event1, COT67B, COT102, T303-3, T304-40, GFM Cry1A, GK12, MLS 9124, 281-24-236, 3006-210-23, GHB119 and SGK321.

Increased yield has been created by increasing ear biomass using the transgene athb17, being present in corn event MON87403, or by enhancing photosynthesis using the transgene bbx32, being present in the soybean event MON87712.

Cultivated plants comprising a modified oil content have been created by using the transgenes: gm-fad2-1, Pj.D6D, Nc.Fad3, fad2-1A and fatb1-A. Soybean events comprising at least one of these genes are: 260-05, MON87705 and MON87769.

Tolerance to abiotic conditions, in particular to tolerance to drought, has been created by using the transgene cspB, comprised by the corn event MON87460 and by using the transgene Hahb-4, comprised by soybean event IND-ØØ41Ø-5.

Traits are frequently combined by combining genes in a transformation event or by combining different events during the breeding process. Preferred combination of traits are herbicide tolerance to different groups of herbicides, insect tolerance to different kind of insects, in particular tolerance to lepidopteran and coleopteran insects, herbicide tolerance with one or several types of insect resistance, herbicide tolerance with increased yield as well as a combination of herbicide tolerance and tolerance to abiotic conditions.

Plants comprising singular or stacked traits as well as the genes and events providing these traits are known (http://www.isaaa.org/gmapprovaldatabase) and (http://cera-qmc.org/GMCropDatabase).

Further information on specific events and methods to detect them can be found for canola events MS1, MS8, RF3, GT73, MON88302, KK179 in WO01/031042, WO01/041558, WO01/041558, WO02/036831, WO11/153186, WO13/003558, for cotton events MON1445, MON15985, MON531(MON15985), LLCotton25, MON88913, COT102, 281-24-236, 3006-210-23, COT67B, GHB614, T304-40, GHB119, MON88701, 81910 in WO02/034946, WO02/100163, WO02/100163, WO03/013224, WO04/072235, WO04/039986, WO05/103266, WO05/103266, WO06/128573, WO07/017186, WO08/122406, WO08/151780, WO12/134808, WO13/112527, for corn events GA21, MON810, DLL25, TC1507, MON863, MIR604, LY038, MON88017, 3272, 59122, NK603, MIR162, MON89034, 98140, 32138, MON87460, 5307, 4114, MON87427, DAS40278, MON87411, 33121, MON87403, MON87419 in WO98/044140, US02/102582, US03/126634, WO04/099447, WO04/011601, WO05/103301, WO05/061720, WO05/059103, WO06/098952, WO06/039376, US2007/292854, WO07/142840, WO07/140256, WO08/112019, WO09/103049, WO09/111263, WO10/077816, WO11/084621, WO11/062904, WO11/022469, WO13/169923, WO14/116854, WO15/053998, WO15/142571, for potato events E12, F10, J3, J55, V11, X17, Y9 in WO14/178910, WO14/178913, WO14/178941, WO14/179276, WO16/183445, WO17/062831, WO17/062825, for rice events LLRICE06, LLRICE601, LLRICE62 in WO00/026345, WO00/026356, WO00/026345 for soybean events H7-1, MON89788, A2704-12, A5547-127, DP305423, DP356043, MON87701, MON87769, CV127, MON87705, DAS68416-4, MON87708, MON87712, SYHT0H2, DAS81419, DAS81419 x DAS44406-6, MON87751 in WO04/074492, WO06/130436, WO06/108674, WO06/108675, WO08/054747, WO08/002872, WO09/064652, WO09/102873, WO10/080829, WO10/037016, WO11/066384, WO11/034704, WO12/051199, WO12/082548, WO13/016527, WO13/016516, WO14/201235.

The use of compositions according to the invention on cultivated plants may result in effects which are specific to a cultivated plant comprising a certain gene or event. These effects may comprise enhanced yield, enhanced resistance or tolerance to insects, nematodes, fungal, bacterial, mycoplasma, viral or viroid pathogens as well as early vigour, early or delayed ripening, cold or heat tolerance as well as changed amino acid or fatty acid spectrum or content.

It has been found that the pesticidal activity of the compounds of the invention may be enhanced by the insecticidal trait of a modified plant. Furthermore, it has been found that the compounds of the invention are suitable for preventing insects to become resistant to the insecticidal trait or for combating pests, which already have become resistant to the insecticidal trait of a modified plant. Moreover, the compounds of the invention are suitable for combating pests, against which the insecticidal trait is not effective, so that a complementary insecticidal activity can advantageously be used.

The term "plant propagation material" refers to all the generative parts of the plant e.g. seeds and vegetative plant material e.g. cuttings and tubers (e.g. potatoes), which can be used for the multiplication of the plant. This includes seeds, roots, fruits, tubers, bulbs, rhizomes, shoots, sprouts and other parts of plants. Seedlings and young plants, which are to be transplanted after germination or after emergence from soil, may also be included. These plant propagation materials may be treated prophylactically with a plant protection compound either at or before planting or transplanting.

The term "seed" embraces seeds and plant propagules of all kinds including but not limited to true seeds, seed pieces, suckers, corms, bulbs, fruit, tubers, grains, cuttings, cut shoots and the like, and means in a preferred embodiment true seeds.

In general, "pesticidally effective amount" means the amount of active ingredient needed to achieve an observable effect on growth, including the effects of necrosis, death, retardation, prevention, and removal, destruction, or otherwise diminishing the occurrence and activity of the target organism. The pesticidally effective amount can vary for the various compounds/compositions used in the invention. A pesticidally effective amount of the compositions will also vary according to the prevailing conditions e.g. desired pesticidal effect and duration, weather, target species, locus, mode of application.

In the case of soil treatment, in furrow application or of application to the pests dwelling place or nest, the quantity of active ingredient ranges from 0.0001 to 500 g per 100 m², preferably from 0.001 to 20 g per 100 m².

For use in treating crop plants, e.g. by foliar application, the rate of application of the active ingredients of this invention may be in the range of 0.0001 g to 4000 g per hectare, e.g. from 1 g to 2 kg per hectare or from 1 g to 750 g per hectare, desirably from 1 g to 100 g per hectare, more desirably from 10 g to 50 g per hectare, e.g., 10 to 20 g per hectare, 20 to 30 g per hectare, 30 to 40 g per hectare, or 40 to 50 g per hectare.

The compounds of the invention are particularly suitable for use in the treatment of seeds in order to protect the seeds from insect pests, in particular from soil-living insect pests, and the resulting seedling's roots and shoots against soil pests and foliar insects. The invention therefore also relates to a method for the protection of seeds from insects, in particular from soil insects, and of the seedling's roots and shoots from insects, in particular from soil and foliar insects, said method comprising treating the seeds before sowing and/or after pregermination with a compound of the invention. The protection of the seedling's roots and shoots is preferred. More preferred is the protection of seedling's shoots from piercing and sucking insects, chewing insects and nematodes.

The term "seed treatment" comprises e.g. seed dressing, seed coating, seed dusting, seed soaking, seed pelleting, and in-furrow application methods. Preferably, the seed treatment application of the active compound is carried out by spraying or by dusting the seeds before sowing of the plants and before emergence of the plants.

The invention also comprises seeds coated with or containing the active compound. The term "coated with and/or containing" generally signifies that the active ingredient is for the most part on the surface of the propagation product at the time of application, although a greater or lesser part of the ingredient may penetrate into the propagation product, depending on the method of application. When the said propagation product is (re)planted, it may absorb the active ingredient.

Suitable seed is e.g. seed of cereals, root crops, oil crops, vegetables, spices, ornamentals, e.g. seed of durum and other wheat, barley, oats, rye, maize (fodder maize and sugar maize / sweet and field corn), soybeans, oil crops, crucifers, cotton, sunflowers, bananas, rice, oilseed rape, turnip rape, sugarbeet, fodder beet, eggplants, potatoes, grass, lawn, turf, fodder grass, tomatoes, leeks, pumpkin/squash, cabbage, iceberg lettuce, pepper, cucumbers, melons, Brassica species, melons, beans, peas, garlic, onions, carrots, tuberous plants e.g. potatoes, sugar cane, tobacco, grapes, petunias, geranium/pelargoniums, pansies and impatiens.

In addition, the active compound may also be used for the treatment of seeds from plants, which have been modified by mutagenisis or genetic engineering, and which e.g. tolerate the action of herbicides or fungicides or insecticides.

Conventional seed treatment formulations include e.g. flowable concentrates FS, solutions LS, suspoemulsions (SE), powders for dry treatment DS, water dispersible powders for slurry treatment WS, water-soluble powders SS and emulsion ES and EC and gel formulation GF. These formulations can be applied to the seed diluted or undiluted. Application to the seeds is carried out before sowing, either directly on the seeds or after having pregerminated the latter. Preferably, the formulations are applied such that germination is not included.

The active substance concentrations in ready-to-use formulations, which may be obtained after two-to-tenfold dilution, are preferably from 0.01 to 60% by weight, more preferably from 0.1 to 40% by weight.

In a preferred embodiment a FS formulation is used for seed treatment. Typically, a FS formulation may comprise 1-800 g/l of active ingredient, 1-200 g/l Surfactant, 0 to 200 g/l antifreezing agent, 0 to 400 g/l of binder, 0 to 200 g/l of a pigment and up to 1 liter of a solvent, preferably water.

Especially preferred FS formulations of the compounds of the invention for seed treatment usually comprise from 0.1 to 80% by weight (1 to 800 g/l) of the active ingredient, from 0.1 to 20% by weight (1 to 200 g/l) of at least one surfactant, e.g. 0.05 to 5% by weight of a wetter and from 0.5 to 15% by weight of a dispersing agent, up to 20% by weight, e.g. from 5 to 20% of an anti-freeze agent, from 0 to 15% by weight, e.g. 1 to 15% by weight of a pigment and/or a dye, from 0 to 40% by weight, e.g. 1 to 40% by weight of a binder (sticker/adhesion agent), optionally up to 5% by weight, e.g. from 0.1 to 5% by weight of a thickener, optionally from 0.1 to 2% of an anti-foam agent, and optionally a preservative e.g. a biocide, antioxidant or the like, e.g. in an amount from 0.01 to 1% by weight and a filler/vehicle up to 100% by weight.

In the treatment of seed, the application rates of the compounds of the invention are generally from 0.1 g to 10 kg per 100 kg of seed, preferably from 1 g to 5 kg per 100 kg of seed, more preferably from 1 g to 1000 g per 100 kg of seed and in particular from 1 g to 200 g per 100 kg of seed, e.g. from 1 g to 100 g or from 5 g to 100 g per 100 kg of seed.

The invention therefore also relates to seed comprising a compound of the invention, or an agriculturally useful salt thereof, as defined herein. The amount of the compound of the invention or the agriculturally useful salt thereof will in general vary from 0.1 g to 10 kg per 100 kg of seed, preferably from 1 g to 5 kg per 100 kg of seed, in particular from 1 g to 1000 g per 100 kg of seed. For specific crops e.g. lettuce the rate can be higher.

The compounds of the invention may also be used for improving the health of a plant. Therefore, the invention also relates to a method for improving plant health by treating a plant, plant propagation material and/or the locus where the plant is growing or is to grow with an effective and non-phytotoxic amount of a compound of the invention.

As used herein "an effective and non-phytotoxic amount" means that the compound is used in a quantity which allows to obtain the desired effect, but which does not give rise to any phytotoxic symptom on the treated plant or on the plant grown from the treated propagule or treated soil.

"Plant health" is defined as a condition of the plant and/or its products which is determined by several aspects alone or in combination with each other e.g. yield (e.g. increased biomass and/or increased content of valuable ingredients), quality (e.g. improved content or composition of certain ingredients or shelf life), plant vigour (e.g. improved plant growth and/or greener leaves ("greening effect"), tolerance to abiotic (e.g. drought) and/or biotic stress (e.g. disease) and production efficiency (e.g., harvesting efficiency, processability).

The above identified indicators for the health condition of a plant may be interdependent and may result from each other. Each indicator is defined in the art and can be determined by methods known to a skilled person.

The compounds of the invention are also suitable for use against non-crop insect pests. For use against said non-crop pests, compounds of the invention can be used as bait composition, gel, general insect spray, aerosol, as ultra-low volume application and bed net (impregnated or surface applied). Furthermore, drenching and rodding methods can be used.

As used herein, the term "non-crop insect pest" refers to pests, which are particularly relevant for non-crop targets, e.g. ants, termites, wasps, flies, ticks, mosquitoes, bed bugs, crickets, or cockroaches.

The bait can be a liquid, a solid or a semisolid preparation (e.g. a gel). The bait employed in the composition is a product, which is sufficiently attractive to incite insects e.g. ants, termites, wasps, flies, mosquitoes, crickets etc. or cockroaches to eat it. The attractiveness can be manipulated by using feeding stimulants or sex pheromones. Food stimulants are preferably chosen from animal and/or plant proteins (meat-, fish- or blood meal, insect parts, egg yolk), from fats and oils of animal and/or plant origin, or mono-, oligo- or polyorganosaccharides, especially from sucrose, lactose, fructose, dextrose, glucose, starch, pectin or even molasses or honey. Fresh or decaying parts of fruits, crops, plants, animals, insects or specific parts thereof can also serve as a feeding stimulant. Sex pheromones are known to be more insect specific. Specific pheromones are known (http://www.pherobase.com).

For use in bait compositions, the typical content of active ingredient is from 0.001 wt% to 15 wt%, desirably from 0.001 wt% to 5 wt% of active compound.

Formulations of the compounds of the invention as aerosols (e.g in spray cans), oil sprays or pump sprays are highly suitable for professional or non-professional users for controlling pests e.g. flies, fleas, ticks, bed bugs, mosquitoes or cockroaches. Aerosol recipes are preferably composed of the active compound, solvents, furthermore auxiliaries e.g. emulsifiers, perfume oils, if appropriate stabilizers, and, if required, propellants.

The oil spray formulations differ from the aerosol recipes in that no propellants are used.

For use in spray compositions, the content of active ingredient is from 0.001 to 80 wt%, preferably from 0.01 to 50 wt% and most preferably from 0.01 to 15 wt%.

The compounds of the invention and its respective compositions can also be used in mosquito and fumigating coils, smoke cartridges, vaporizer plates or long-term vaporizers and also in moth papers, moth pads or other heat-independent vaporizer systems.

Methods to control infectious diseases transmitted by insects (e.g. malaria, dengue and yellow fever, lymphatic filariasis, and leishmaniasis) with compounds of the invention and its respective compositions also comprise treating surfaces of huts and houses, air spraying and impregnation of curtains, tents, clothing items, bed nets, tsetse-fly trap. Insecticidal compositions for application to fibers, fabric, knitgoods, nonwovens, netting material or foils and tarpaulins preferably comprise a mixture including the insecticide, optionally a repellent and at least one binder.

The compounds of the invention and its compositions can be used for protecting wooden materials e.g. trees, board fences, sleepers, frames, artistic artifacts, etc. and buildings, but also construction materials, furniture, leathers, fibers, vinyl articles, electric wires and cables etc. from ants, termites and/or wood or textile destroying beetles, and for controlling ants and termites from doing harm to crops or human beings (e.g. when the pests invade into houses and public facilities or nest in yards, orchards or parks).

Customary application rates in the protection of materials are, e.g., from 0.001 g to 2000 g or from 0.01 g to 1000 g of active compound per m² treated material, desirably from 0.1 g to 50 g per m².

Insecticidal compositions for use in the impregnation of materials typically contain from 0.001 to 95 wt%, preferably from 0.1 to 45 wt%, and more preferably from 1 to 25 wt% of at least one repellent and/or insecticide.

The compounds of the invention are especially suitable for efficiently combating animal pests e.g. arthropods, gastropods and nematodes including:
insects from the order of **Lepidoptera,** e.g. *Achroia grisella, Acleris* spp. e.g. *A. fimbriana, A. gloverana, A. variana; Acrolepiopsis assectella, Acronicta major, Adoxophyes* spp. e.g. *A. cyrtosema, A. orana; Aedia leucomelas, Agrotis* spp. e.g. *A. exclamationis, A. fucosa, A. ipsilon, A. orthogoma, A. segetum, A. subterranea; Alabama argillacea, Aleurodicus dispersus, Alsophila pometaria, Ampelophaga rubiginosa, Amyelois transitella, Anacampsis sarcitella, Anagasta kuehniella, Anarsia lineatella, Anisota senatoria, Antheraea pernyi, Anticarsia (=Thermesia)* spp. e.g. *A. gemmatalis; Apamea* spp., *Aproaerema modicella, Archips* spp. e.g. *A. argyrospila, A. fuscocupreanus, A. rosana, A. xyloseanus; Argyresthia conjugella, Argyroploce* spp., *Argyrotaenia* spp. e.g. *A. velutinana; Athetis mindara, Austroasca viridigrisea, Autographa gamma, Autographa nigrisigna, Barathra brassicae, Bedellia* spp., *Bonagota salubricola, Borbo cinnara, Bucculatrix thurberiella, Bupalus piniarius, Busseola* spp., *Cacoecia* spp. e.g. *C*. *murinana, C. podana; Cactoblastis cactorum, Cadra cautella, Calingo braziliensis, Caloptilis theivora, Capua reticulana, Carposina* spp. e.g. *C*. *niponensis, C*. *sasakii; Cephus* spp., *Chaetocnema aridula, Cheimatobia brumata, Chilo* spp. e.g. *C*. *Indicus, C. suppressalis, C. partellus; Choreutis pariana, Choristoneura* spp. e.g. *C*. *conflictana, C. fumiferana, C. longicellana, C. murinana, C. occidentalis, C*. *rosaceana; Chrysodeixis (=Pseudoplusia)* spp. e.g. *C*. *eriosoma, C. includens; Cirphis unipuncta, Clysia ambiguella, Cnaphalocerus* spp., *Cnaphalocrocis medinalis, Cnephasia* spp., *Cochylis hospes, Coleophora* spp., *Colias eurytheme, Conopomorpha* spp., *Conotrachelus* spp., *Copitarsia* spp., *Corcyra cephalonica, Crambus caliginosellus, Crambus teterrellus, Crocidosema (=Epinotia) aporema, Cydalima (=Diaphania) perspectalis, Cydia (=Carpocapsa)* spp. e.g. *C*. *pomonella, C. latiferreana; Dalaca noctuides, Datana integerrima, Dasychira pinicola, Dendrolimus* spp. e.g. *D. pini, D. spectabilis, D. sibiricus; Desmia funeralis, Diaphania* spp. e.g. *D. nitidalis, D. hyalinata; Diatraea grandiosella, Diatraea saccharalis, Diphthera festiva, Earias* spp. e.g. *E. insulana, E. vittella; Ecdytolopha aurantianu, Egira (=Xylomyges) curialis, Elasmopalpus lignosellus, Eldana saccharina, Endopiza viteana, Ennomos subsignaria, Eoreuma loftini, Ephestia* spp. e.g. *E. cautella, E. elutella, E. kuehniella; Epinotia aporema, Epi-phyas postvittana, Erannis tiliaria, Erionota thrax, Etiella* spp., *Eulia* spp., *Eupoecilia ambiguella, Euproctis chrysorrhoea, Euxoa* spp., *Evetria bouliana, Faronta albilinea, Feltia* spp. e.g. *F*. *subterranean; Galleria mellonella, Gracillaria* spp., *Grapholita* spp. e.g. *G. funebrana, G. molesta, G. inopinata; Halysidota* spp., *Harrisina americana, Hedylepta* spp., *Helicoverpa* spp. e.g. *H. armigera (=Heliothis armigera), H. zea (=Heliothis zea); Heliothis* spp. e.g. *H. assulta, H. subflexa, H. virescens; Hellula* spp. e.g. *H. undalis, H. rogatalis; Helocoverpa gelotopoeon, Hemileuca oliviae, Herpetogramma licarsisalis, Hibernia defoliaria, Hofmannophila pseudospretella, Homoeosoma electellum, Homona magnanima, Hypena scabra, Hyphantria cunea, Hyponomeuta padella, Hyponomeuta malinellus, Kakivoria flavofasciata, Keiferia lycopersicella, Lambdina fiscellaria fiscellaria, Lambdina fiscellaria lugubrosa, Lamprosema indicata, Laspeyresia molesta, Leguminivora glycinivorella, Lerodea eufala, Leucinodes orbonalis, Leucoma salicis, Leucoptera* spp. e.g. *L. coffeella, L. scitella; Leuminivora lycinivorella, Lithocolletis blancardella, Lithophane antennata, Llattia octo (=Amyna axis), Lobesia botrana, Lophocampa* spp., *Loxagrotis albicosta, Loxostege* spp. e.g. *L. sticticalis, L. cereralis; Lymantria* spp. e.g. *L. dispar, L. monacha; Lyonetia clerkella, Lyonetia prunifoliella, Malacosoma* spp. e.g. *M. americanum, M. californicum, M. constrictum, M. neustria; Mamestra* spp. e.g. *M. brassicae, M. configurata; Mamstra brassicae, Manduca* spp. e.g. *M. quinquemaculata, M. sexta; Marasmia spp, Marmara* spp., *Maruca testulalis, Megalopyge lanata, Melanchra picta, Melanitis leda, Mocis* spp. e.g. *M*. *lapites, M. repanda; Mocis latipes, Monochroa fragariae, Mythimna separata, Nemapogon cloacella, Neoleucinodes elegantalis, Nepytia* spp., *Nymphula* spp., *Oiketicus* spp., *Omiodes indicata, Omphisa anastomosalis, Operophtera brumata, Orgyia pseudotsugata, Oria* spp., *Orthaga thyrisalis, Ostrinia* spp. e.g. *O. nubilalis; Oulema oryzae, Paleacrita vernata, Panolis flammea, Parnara* spp., *Papaipema nebris, Papilio cresphontes, Paramyelois transitella, Paranthrene regalis, Paysandisia archon, Pectinophora* spp. e.g. *P. gossypiella; Peridroma saucia, Perileucoptera* spp., e.g. *P. coffeella; Phalera bucephala, Phryganidia californica, Phthorimaea* spp. e.g. P. *operculella; Phyllocnistis citrella, Phyllonorycter* spp. e.g. *P. blancardella, P. crataegella, P. issikii, P. ringoniella; Pieris* spp. e.g. *P. brassicae, P. rapae, P. napi; Pilocrocis tripunctata, Plathypena scabra, Platynota* spp. e.g. *P. flavedana, P. idaeusalis, P. stultana; Platyptilia carduidactyla, Plebejus argus, Plodia interpunctella, Plusia spp, Plutella maculipennis, Plutella xylostella, Pontia protodica, Prays* spp., *Prodenia* spp., *Proxenus lepigone, Pseudaletia* spp. e.g. *P. sequax, P. unipuncta; Pyrausta nubilalis, Rachiplusia nu, Richia albicosta, Rhizobius ventralis, Rhyacionia frustrana, Sabulodes aegrotata, Schizura concinna, Schoenobius* spp., *Schreckensteinia festaliella, Scirpophaga* spp. e.g. *S. incertulas, S. innotata; Scotia segetum, Sesamia* spp. e.g. *S. inferens, Seudyra subflava, Sitotroga cerealella, Sparganothis pilleriana, Spilonota lechriaspis, S. ocellina, Spodoptera (=Lamphygma)* spp. e.g. *S. cosmoides, S. eridania, S. exigua, S. frugiperda, S. latisfascia, S. littoralis, S. litura, S. omithogalli; Stigmella* spp., *Stomopteryx subsecivella, Strymon bazochii, Sylepta derogata, Synanthedon* spp. e.g. S. exitiosa, *Tecia solanivora, Telehin licus, Thaumatopoea pityocampa, Thaumatotibia (=Cryptophlebia) leucotreta, Thaumetopoea pityocampa, Thecla* spp., *Theresimima ampelophaga, Thyrinteina* spp, *Tildenia inconspicuella, Tinea* spp. e.g. *T. cloacella, T. pellionella; Tineola bisselliella, Tortrix* spp. e.g. *T. viridana; Trichophaga tapetzella, Trichoplusia* spp. e.g. *T. ni; Tuta (=Scrobipalpula) absoluta, Udea* spp. e.g. *U. rubigalis, U. rubigalis; Virachola* spp., *Yponomeuta padella, and Zeiraphera canadensis;*
insects from the order of Coleoptera, e.g. *Acalymma vittatum, Acanthoscehdes obtectus, Adoretus* spp., *Agelastica alni, Agrilus* spp. e.g. *A. anxius, A. planipennis, A. sinuatus; Agriotes* spp. e.g. *A. fuscicollis, A. lineatus, A. obscurus; Alphitobius diaperinus, Amphimallus solstitialis, Anisandrus dispar, Anisoplia austriaca, Anobium punctatum, Anomala corpulenta, Anomala rufocuprea, Anoplophora* spp. e.g. *A. glabripennis; Anthonomus* spp. e.g. *A. eugenii, A. grandis, A. pomorum; Anthrenus* spp., *Aphthona euphoridae, Apion* spp., *Apogonia* spp., *Athous haemorrhoidalis, Atomaria* spp. e.g. *A. linearis; Attagenus* spp., *Aulacophora femoralis, Blastophagus piniperda, Blitophaga undata, Bruchidius obtectus, Bruchus* spp. e.g. *B. lentis, B. pisorum, B. rufimanus; Byctiscus betulae, Callidiellum rufipenne, Callopistria floridensis, Callosobruchus chinensis, Cameraria ohridella, Cassida nebulosa, Cerotoma trifurcata, Cetonia aurata, Ceuthorhynchus* spp. e.g. C. *assimilis, C. napi; Chaetocnema tibialis, Cleonus mendicus, Conoderus* spp. e.g. C. *vespertinus; Conotrachelus nenuphar, Cosmopolites* spp., *Costelytra zealandica, Crioceris asparagi, Cryptolestes ferrugineus, Cryptorhynchus lapathi, Ctenicera* spp. e.g. C. *destructor; Curculio* spp., *Cylindrocopturus* spp., *Cyclocephala* spp., *Dactylispa balyi, Dectes texanus, Dermestes* spp., *Diabrotica* spp. e.g. *D. undecimpunctata, D. speciosa, D. longicornis, D. semipunctata, D. virgifera; Diaprepes abbreviates, Dichocrocis* spp., *Dicladispa armigera, Diloboderus abderus, Diocalandra frumenti (Diocalandra stigmaticollis), Enaphalodes rufulus, Epilachna* spp. e.g. *E. varivestis, E. vigintioctomaculata; Epitrix* spp. e.g. *E. hirtipennis, E. similaris; Eutheola humilis, Eutinobothrus brasiliensis, Faustinus cubae, Gibbium psylloides, Gnathocerus cornutus, Hellula undalis, Heteronychus arator, Hylamorpha elegans, Hylobius abietis, Hylotrupes bajulus, Hypera* spp. e.g. *H. brunneipennis, H. postica; Hypomeces squamosus, Hypothenemus* spp., *Ips typographus, Lachnosterna consanguinea, Lasioderma serricorne, Latheticus oryzae, Lathridius* spp., *Lema* spp. e.g. *L. bilineata, L. melanopus; Leptinotarsa* spp. e.g. *L. decemlineata; Leptispa pygmaea, Limonius californicus, Lissorhoptrus oryzophilus, Lixus* spp., *Luperodes* spp., *Lyctus* spp. e.g. *L. bruneus; Liogenys fuscus, Macrodactylus* spp. e.g. *M. subspinosus; Maladera matrida, Megaplatypus mutates, Megascelis* spp., *Melanotus communis, Meligethes* spp. e.g. *M. aeneus; Melolontha* spp. e.g. *M. hippocastani, M. me*/*olontha; Metamasius hemipterus, Microtheca* spp., *Migdolus* spp. e.g. *M. fryanus, Monochamus* spp. e.g. *M. alternatus; Naupactus xanthographus, Niptus hololeucus, Oberia brevis, Oemona hirta, Oryctes rhinoceros, Oryzaephilus surinamensis, Oryzaphagus oryzae, Otiorrhynchus sulcatus, Otiorrhynchus ovatus, Otiorrhynchus sulcatus, Oulema melanopus, Oulema oryzae, Oxycetonia jucunda, Phaedon* spp. e.g. *P. brassicae, P. cochleariae; Phoracantha recurva, Phyllobius pyri, Phyllopertha horticola, Phyllophaga* spp. e.g. *P. helleri; Phyllotreta* spp. e.g. *P. chrysocephala, P. nemorum, P. striolata, P. vittula; Phyllopertha horticola, Popillia japonica, Premnotrypes* spp., *Psacothea hilaris, Psylliodes chrysocephala, Prostephanus truncates, Psylliodes* spp., *Ptinus* spp., *Pulga saltona, Rhizopertha dominica, Rhynchophorus* spp. e.g. *R. billineatus, R. ferrugineus, R. palmarum, R. phoenicis, R. vulneratus; Saperda candida, Scolytus schevyrewi, Scyphophorus acupunctatus, Sitona lineatus, Sitophilus* spp. e.g. *S*. *granaria, S*. *oryzae, S*. *zeamais; Sphenophorus* spp. e.g. *S. levis; Stegobium paniceum, Sternechus* spp. e.g. *S. subsignatus; Strophomorphus ctenotus, Symphyletes* spp., *Tanymecus* spp., *Tenebrio molitor, Tenebrioides mauretanicus, Tribolium* spp. e.g. *T. castaneum; Trogoderma* spp., *Tychius* spp., *Xylotrechus* spp. e.g. *X. pyrrhoderus; and, Zabrus* spp. *e.g. Z. tenebrioides;* insects from the order of Diptera e.g. *Aedes* spp. e.g. *A. aegypti, A. albopictus, A. vexans; Anastrepha ludens, Anopheles* spp. e.g. *A. albimanus, A. crucians, A. freeborni, A. gambiae, A. leucosphyrus, A. maculipennis, A. minimus, A. quadrimaculatus, A. sinensis; Bactrocera invadens, Bibio hortulanus, Calliphora erythrocephala, Calliphora vicina, Ceratitis capitata, Chrysomyia* spp. e.g. *C*. *bezziana, C. hominivorax, C. macellaria; Chrysops atlanticus, Chrysops discalis, Chrysops silacea, Cochliomyia* spp. e.g. *C*. *hominivorax; Contarinia* spp. e.g. *C*. *sorghicola; Cordylobia anthropophaga, Culex* spp. e.g. *C*. *nigripalpus, C. pipiens, C. quinquefasciatus, C. tarsalis, C. tritaeniorhynchus; Culicoides furens, Culiseta inornata, Culiseta melanura, Cuterebra* spp., *Dacus cucurbitae, Dacus oleae, Dasineura brassicae, Dasineura oxycoccana, Delia* spp. e.g. *D*. *antique, D. coarctata, D. platura, D. radicum; Dermatobia hominis, Drosophila* spp. e.g. *D. suzukii, Fannia* spp. e.g. *F. canicularis; Gastraphilus* spp. e.g. *G. intestinalis; Geomyza tipunctata, Glossina* spp. e.g. *G. fuscipes, G. morsitans, G. palpalis, G. tachinoides; Haematobia irritans, Haplodiplosis equestris, Hippelates* spp., Hylemyia spp. e.g. *H. platura; Hypoderma* spp. e.g. *H. lineata; Hyppobosca* spp., *Hydrellia philippina, Leptoconops torrens, Liriomyza* spp. e.g. *L. sativae, L. trifolii; Lucilia* spp. e.g. *L. caprina, L. cuprina, L. sericata; Lycoria pectoralis, Mansonia titillanus, Mayetiola* spp. e.g. *M. destructor; Musca* spp. e.g. *M. autumnalis, M. domestica; Muscina stabulans, Oestrus* spp. e.g. *O*. *ovis; Opomyza florum, Oscinella* spp. e.g. *O. frit; Orseolia oryzae, Pegomya hysocyami, Phlebotomus argentipes, Phorbia* spp. e.g. *P*. *antiqua, P. brassicae, P. coarctata; Phytomyza gymnostoma, Prosimulium mixtum, Psila rosae, Psorophora columbiae, Psorophora discolor, Rhagoletis* spp. e.g. *R. cerasi, R. cingulate, R. indifferens, R. mendax, R. pomonella; Rivellia quadrifasciata, Sarcophaga* spp. e.g. *S. haemorrhoidalis; Simulium vittatum, Sitodiplosis mosellana, Stomoxys* spp. e.g. *S. calcitrans; Tabanus* spp. e.g. *T. atratus, T. bovinus, T. lineola, T. similis; Tannia spp., Thecodiplosis japonensis, Tipula oleracea, Tipula paludosa,* and *Wohlfahrtia* spp;
insects from the order of Thysanoptera e.g., *Baliothrips biformis, Dichromothrips corbetti, Dichromothrips* ssp., *Echinothrips americanus, Enneothrips flavens, Frankliniella* spp. e.g. *F*. *fusca, F. occidentalis, F. tritici; Heliothrips* spp., *Hercinothrips femoralis, Kakothrips* spp., *Microcephalothrips abdominalis, Neohydatothrips samayunkur, Pezothrips kellyanus, Rhipiphorothrips cruentatus, Scirtothrips* spp. e.g. *S. citri,* S. *dorsalis, S. perseae; Stenchaetothrips spp, Taeniothrips cardamoni, Taeniothrips inconsequens, Thrips* spp. e.g. *T. imagines, T. hawaiiensis, T. oryzae, T. palmi, T. parvispinus, T. tabaci;*
insects from the order of Hemiptera e.g., *Acizzia jamatonica, Acrosternum* spp. e.g. *A. hilare;* Acyrthosipon spp. e.g. *A. onobrychis, A. pisum; Adelges laricis, Adelges tsugae, Adelphocoris* spp., e.g. *A. rapidus, A. superbus; Aeneolamia* spp., *Agonoscena* spp., *Aulacorthum solani, Aleurocanthus woglumi, Aleurodes* spp., *Aleurodicus disperses, Aleurolobus barodensis, Aleurothrixus* spp., *Amrasca* spp., *Anasa tristis, Antestiopsis* spp., *Anuraphis cardui, Aonidiella* spp., *Aphanostigma piri, Aphidula nasturtii, Aphis* spp. e.g. *A. craccivora, A. fabae, A. forbesi, A. gossypii, A. grossulariae, A. maidiradicis, A. pomi, A. sambuci, A. schneideri, A. spiraecola; Arboridia apicalis, Arilus critatus, Aspidiella* spp., *Aspidiotus* spp., *Atanus* spp., *Aulacaspis yasumatsui, Aulacorthum solani, Bactericera cockerelli (Paratrioza cockerelli), Bemisia* spp. e.g. *B. argentifolii, B. tabaci (Aleurodes tabaci); Blissus* spp. e.g. *B. leucopterus; Brachycaudus* spp. e.g. *B. cardui, B. helichrysi, B. persicae, B. prunicola; Brachycolus* spp., *Brachycorynella asparagi, Brevicoryne brassicae, Cacopsylla* spp. e.g. C. *fulguralis, C. pyricola (Psylla piri); Calligypona marginata, Calocoris* spp., *Campylomma livida, Capitophorus horni, Carneocephala fulgida, Cavelerius* spp., *Ceraplastes* spp., *Ceratovacuna lanigera, Ceroplastes ceriferus,* Cero*sipha gossypii, Chaetosiphon fragaefolii, Chionaspis tegalensis, Chlorita onukii, Chromaphis juglandicola, Chrysomphalus ficus, Cicadulina mbila, Cimex* spp. e.g. *C*. *hemipterus, C. lectularius; Coccomytilus halli, Coccus* spp. e.g. *C. hesperidum, C. pseudomagnoliarum; Corythucha arcuata, Creontiades dilutus, Cryptomyzus ribis, Chrysomphalus aonidum, Cryptomyzus ribis, Ctenarytaina spatulata, Cyrtopeltis notatus, Dalbulus* spp., *Dasynus piperis, Dialeurodes* spp. e.g. *D. citrifolii; Dalbulus maidis, Diaphorina* spp. e.g. *D. citri; Diaspis* spp. e.g. *D. bromeliae; Dichelops furcatus, Diconocoris hewetti, Doralis* spp., *Dreyfusia nordmannianae, Dreyfusia piceae, Drosicha* spp., *Dysaphis* spp. e.g. *D. plantaginea, D. pyri, D. radicola; Dysaulacorthum pseudosolani, Dysdercus* spp. e.g. *D*. *cingulatus, D. intermedius; Dysmicoccus* spp., *Edessa* spp., *Geocoris* spp., *Empoasca* spp. e.g. *E. fabae, E. solana; Epidiaspis leperii, Eriosoma* spp. e.g. *E. lanigerum, E. pyricola; Erythroneura* spp., *Eurygaster spp.* e.g. *E. integriceps; Euscelis bilobatus, Euschistus* spp. e.g. *E. heros, E. impictiventris, E. servus; Fiorinia theae, Geococcus coffeae, Glycaspis brimblecombei, Halyomorpha* spp. e.g. *H. halys; Heliopeltis* spp., *Homalodisca vitripennis (=H. coagulata), Horcias nobilellus, Hyalopterus pruni, Hyperomyzus lactucae, Icerya* spp. e.g. *I. purchase; Idiocerus* spp., *Idioscopus* spp., *Laodelphax striatellus, Lecanium* spp., *Lecanoideus floccissimus, Lepidosaphes* spp. e.g. *L. ulmi; Leptocorisa* spp., *Leptoglossus phyllopus, Lipaphis erysimi, Lygus* spp. e.g. *L. hesperus, L. lineolaris, L. pratensis; Maconellicoccus hirsutus, Marchalina hellenica, Macropes excavatus, Macrosiphum* spp. e.g. *M. rosae, M. avenae, M. euphorbiae; Macrosteles quadrilineatus, Mahanarva fimbriolata, Megacopta cribraria, Megoura viciae, Melanaphis pyrarius, Melanaphis sacchari, Melanocallis (=Tinocallis) caryaefoliae, Metcafiella* spp., *Metopolophium dirhodum, Monellia costalis, Monelliopsis pecanis, Myzocallis coryli, Murgantia* spp., *Myzus* spp. e.g. *M. ascalonicus, M. cerasi, M. nicotianae, M. persicae, M. varians; Nasonovia ribis-nigri, Neotoxoptera formosana, Neomegalotomus spp, Nephotettix* spp. e.g. *N. malayanus, N. nigropictus, N. parvus, N. virescens; Nezara* spp. e.g. *N*. *viridula; Nilaparvata lugens, Nysius huttoni, Oebalus* spp. e.g. *O. pugnax; Oncometopia* spp., *Orthezia praelonga, Oxycaraenus hyalinipennis, Parabemisia myricae, Parlatoria* spp., *Parthenolecanium* spp. e.g. *P. corni, P. persicae; Pemphigus* spp. e.g. *P*. *bursarius, P. populivenae; Peregrinus maidis, Perkinsiella saccharicida, Phenacoccus* spp. e.g. *P. aceris, P. gossypii; Phloeomyzus passerinii, Phorodon humuli, Phylloxera* spp. e.g. *P. devastatrix, Piesma quadrata, Piezodorus* spp. e.g. *P. guildinii; Pinnaspis aspidistrae, Planococcus* spp. e.g. *P. citri, P. ficus; Prosapia bicincta, Protopulvinaria pyriformis, Psallus seriatus, Pseudacysta persea, Pseudaulacaspis pentagona, Pseudococcus* spp. e.g. *P. comstocki; Psylla* spp. e.g. *P. mali; Pteromalus* spp., *Pulvinaria amygdali, Pyrilla* spp., *Quadraspidiotus* spp., e.g. Q. *perniciosus; Quesada gigas, Rastrococcus* spp., *Reduvius senilis, Rhizoecus americanus, Rhodnius* spp., *Rhopalomyzus ascalonicus, Rhopalosiphum* spp. e.g. *R. pseudobrassicas, R. insertum, R. maidis, R. padi; Sagatodes* spp., *Sahlbergella singularis, Saissetia* spp., *Sappaphis mala, Sappaphis mali, Scaptocoris* spp., *Scaphoides titanus, Schizaphis graminum, Schizoneura lanuginosa, Scotinophora* spp., *Selenaspidus articulatus, Sitobion avenae, Sogata* spp., *Sogatella furcifera, Solubea insularis, Spissistilus festinus (=Stictocephala festina), Stephanitis nashi, Stephanitis pyrioides, Stephanitis takeyai, Tenalaphara malayensis, Tetraleurodes perseae, Therioaphis maculate, Thyanta* spp. e.g. *T. accerra, T. perditor; Tibraca* spp., *Tomaspis* spp., *Toxoptera* spp. e.g. *T. aurantii; Trialeurodes* spp. e.g. *T. abutilonea, T. ricini, T. vaporariorum; Triatoma* spp., *Trioza* spp., *Typhlocyba* spp., *Unaspis* spp. e.g. *U. citri, U. yanonensis; and Viteus vitifolii,*
Insects from the order **Hymenoptera** e.g. *Acanthomyops interjectus, Athalia rosae, Atta* spp. *e.g. A. capiguara, A. cephalotes, A. cephalotes, A. laevigata, A. robusta, A. sexdens, A. texana, Bombus* spp., *Brachymyrmex* spp., *Camponotus* spp. e.g. C. *floridanus, C. pennsylvanicus, C. modoc; Cardiocondyla nuda, Chalibion sp, Crematogaster* spp., *Dasymutilla occidentalis, Diprion* spp., *Dolichovespula maculata, Dorymyrmex* spp., *Dryocosmus kuriphilus, Formica* spp., *Hoplocampa* spp. e.g. *H. minuta, H. testudinea; Iridomyrmex humilis,* Lasius spp. e.g. *L*. *niger, Linepithema humile, Liometopum* spp., *Leptocybe invasa, Monomorium* spp. e.g. *M*. *pharaonis, Monomorium, Nylandria fulva, Pachycondyla chinensis, Paratrechina longicornis, Paravespula* spp., e.g. *P. germanica, P. pennsylvanica, P. vulgaris; Pheidole* spp. e.g. *P*. *megacephala; Pogonomyrmex* spp. e.g. *P. barbatus, P. californicus, Polistes rubiginosa, Prenolepis impairs, Pseudomyrmex gracilis, Schelipron* spp., *Sirex cyaneus, Solenopsis* spp. e.g. *S. geminata, S.invicta, S. molesta, S. richteri, S. xyloni, Sphecius speciosus, Sphex* spp., *Tapinoma* spp. e.g. *T. melanocephalum, T. sessile; Tetramorium* spp. e.g. *T. caespitum, T. bicarinatum, Vespa* spp. e.g. *V. crabro; Vespula* spp. e.g. *V. squamosal; Wasmannia auropunctata, Xylocopa* sp;
Insects from the order **Orthoptera** e.g. *Acheta domesticus, Calliptamus italicus, Chortoicetes terminifera, Ceuthophilus* spp., *Diastrammena asynamora, Dociostaurus maroccanus, Gryllotalpa* spp. e.g. *G. africana, G. gryllotalpa; Gryllus* spp., *Hieroglyphus daganensis, Kraussaria angulifera, Locusta* spp. e.g. *L. migratoria, L. pardalina; Melanoplus* spp. e.g. *M. bivittatus, M. femurrubrum, M. mexicanus, M. sanguinipes, M. spretus; Nomadacris septemfasciata, Oedaleus senegalensis, Scapteriscus* spp., *Schistocerca* spp. e.g. *S. americana, S. gregaria, Stemope*/*matus* spp., *Tachycines asynamorus,* and *Zonozerus variegatus;*
Pests from the Class **Arachnida** e.g. **Acari,e.g.** of the families Argasidae, Ixodidae and Sarcoptidae, e.g. *Amblyomma* spp. (e.g. *A. americanum, A. variegatum, A. maculatum*)*,* Argas spp. e.g. *A. persicu*)*, Boophilus* spp. e.g. *B. annulatus, B. decoloratus, B. microplus, Dermacentor* spp. e.g. *D.silvarum, D. andersoni, D. variabilis, Hyalomma* spp. e.g. *H. truncatum, Ixodes* spp. e.g. *I. ricinus, I. rubicundus, I. scapularis, I. holocyclus, I. pacificus, Rhipicephalus sanguineus, Ornithodorus* spp. e.g. *O. moubata, O. hermsi, O. turicata, Ornithonyssus bacoti, Otobius megnini, Dermanyssus gallinae, Psoroptes* spp. e.g. *P. ovis, Rhipicephalus* spp. e.g. *R*. *sanguineus, R. appendiculatus, Rhipicephalus evertsi, Rhizoglyphus* spp., *Sarcoptes* spp. e.g.S. *Scabiei;* and Family **Eriophyidae** including *Aceria* spp. e.g. *A. sheldoni, A. anthocoptes, Acallitus* spp., *Aculops* spp. e.g. *A. lycopersici, A. pelekassi; Aculus* spp. e.g. *A. schlechtendali; Colomerus vitis, Epitrimerus pyri, Phyllocoptruta oleivora; Eriophytes ribis* and *Eriophyes* spp. e.g. *Eriophyes sheldoni;* Family **Tarsonemidae** including *Hemitarsonemus* spp., *Phytonemus pallidus and Polyphagotarsonemus latus, Stenotarsonemus* spp. *Steneotarsonemus spinki;* Family **Tenuipalpidae** including Brevipalpus spp. e.g. *B. phoenicis;* Family **Tetranychidae** including *Eotetranychus* spp., *Eutetranychus* spp., *Oligonychus* spp., *Petrobia latens, Tetranychus* spp. e.g. *T. cinnabarinus, T. evansi, T. kanzawai, T, pacificus, T. phaseulus, T. telarius* and *T. urticae; Bryobia praetiosa; Panonychus* spp. e.g. *P*. *ulmi, P. citri; Metatetranychus* spp. and *Oligonychus* spp. e.g. *O. pratensis, O. perseae, Vasates lycopersici, Raoiella indica, Family* **Carpoglyphidae** including *Carpoglyphus* spp.; *Penthaleidae* spp. e.g. *Halotydeus destructor,* Family **Demodicidae** with species e.g. *Demodex* spp.; Family **Trombicidea** including *Trombicula* spp.; Family **Macronyssidae** including *Ornothonyssus* spp.; Family **Pyemotidae** including *Pyemotes tritici; Tyrophagus putrescentiae;* Family **Acaridae** including *Acarus siro;* Family **Araneida** including *Latrodectus mactans, Tegenaria agrestis, Chiracanthium sp, Lycosa sp Achaearanea tepidariorum* and *Loxosceles reclusa;*
Pests from the Phylum **Nematoda,** e.g. plant parasitic nematodes e.g. root-knot nematodes, *Meloidogyne* spp. e.g. *M. hapla, M. incognita, M. javanica;* cyst-forming nematodes, *Globodera* spp. e.g. *G. rostochiensis; Heterodera* spp. e.g. *H. avenae, H. glycines, H. schachtii, H. trifolii;* Seed gall nematodes, *Anguina* spp.; Stem and foliar nematodes, *Aphelenchoides* spp. e.g. *A. besseyi;* Sting nematodes, *Belonolaimus* spp. e.g. *B. longicaudatus;* Pine nematodes, *Bursaphelenchus* spp. e.g. *B. lignicolus, B. xylophilus;* Ring nematodes, *Criconema* spp., *Criconemella* spp. e.g. *C*. *xenoplax* and *C*. *ornata;* and, *Criconemoides* spp. e.g. *Criconemoides informis; Mesocriconema* spp.; Stem and bulb nematodes, *Ditylenchus* spp. e.g. *D*. *destructor, D. dipsaci;* Awl nematodes, *Dolichodorus* spp.; Spiral nematodes, *Heliocotylenchus multicinctus;* Sheath and sheathoid nematodes, *Hemicycliophora* spp. and *Hemicriconemoides* spp.; *Hirshmanniella* spp.; Lance nematodes, *Hoploaimus* spp.; False rootknot nematodes, *Nacobbus* spp.; Needle nematodes, *Longidorus* spp. e.g. *L. elongatus;* Lesion nematodes, *Pratylenchus* spp. e.g. *P*. *brachyurus, P. neglectus, P. penetrans, P. curvitatus, P. goodeyi;* Burrowing nematodes, *Radopholus* spp. e.g. *R. similis; Rhadopholus* spp.; *Rhodopholus* spp.; Reniform nematodes, *Rotylenchus* spp. e.g. *R. robustus, R. reniformis; Scutellonema* spp.; Stubby-root nematode, *Trichodorus* spp. e.g. *T. obtusus, T. primitivus; Paratrichodorus* spp. e.g. *P. minor;* Stunt nematodes, *Tylenchorhynchus* spp. e.g. *T. claytoni, T. dubius;* Citrus nematodes, *Tylenchulus* spp. e.g. *T. semipenetrans;* Dagger nematodes, *Xiphinema* spp.; and other plant parasitic nematode species;
Insects from the order **Blattodea** e.g. *Macrotermes* spp. e.g. *M. natalensis; Cornitermes cumulans, Procomitermes* spp., *Globitermes sulfureus, Neocapritermes* spp. e.g. *N. opacus, N. parvus; Odontotermes spp., Nasutitermes* spp. e.g. *N. corniger, Coptotermes* spp. e.g. *C*. *formosanus, C. gestroi, C. acinaciformis; Reticulitermes* spp. e.g. *R. hesperus, R. tibialis, R. speratus, R. flavipes, R. grassei, R. lucifugus, R. virginicus; Heterotermes* spp. e.g. *H. aureus, H. longiceps, H. tenuis; Cryptotermes* spp. e.g. *C*. *brevis, C. cavifrons; Incisitermes* spp. e.g. *I. minor, I. snyderi; Marginitermes hubbardi, Kalotermes flavicollis, Neotermes* spp. e.g. *N*. *castaneus, Zootermopsis* spp. *e.g. Z. angusticollis, Z. nevadensis, Mastotermes* spp. e.g. *M*. *darwiniensis; Blatta* spp. e.g. *B. orientalis, B. lateralis; Blattella* spp. e.g. *B. asahinae, B. germanica; Rhyparobia maderae, Panchlora nivea, Periplaneta* spp. e.g. *P. americana, P. australasiae, P. brunnea, P. fuliginosa, P. japonica; Supella longipalpa, Parcoblatta pennsylvanica, Eurycotis floridana, Pycnoscelus surinamensis,*
Insects from the order **Siphonoptera** e.g. *Cediopsylla simples, Ceratophyllus* spp., *Ctenocephalides* spp. e.g. *C*. *felis, C. canis, Xenopsylla cheopis, Pulex irritans, Trichodectes canis, Tunga penetrans,* and *Nosopsyllus fasciatus,*
Insects from the order **Thysanura** e.g. *Lepisma saccharina, Ctenolepisma urbana,* and *Thermobia domestica,*
Pests from the class **Chilopoda** e.g. *Geophilus* spp., Scutigera spp. e.g. *Scutigera coleoptrata;*
Pests from the class **Diplopoda** e.g. *Blaniulus guttulatus, Julus* spp., *Narceus* spp.,
Pests from the class **Symphyla** e.g. *Scutigerella immaculata,*
Insects from the order **Dermaptera,** e.g. *Forficula auricularia,*
Insects from the order **Collembola,** e.g. *Onychiurus* spp., e.g. *Onychiurus armatus,*
Pests from the order **Isopoda** e.g., *Armadillidium vulgare, Oniscus asellus, Porcellio scaber,* Insects from the order **Phthiraptera,** e.g. *Damalinia* spp., Pediculus spp. e.g. *Pediculus humanus capitis, Pediculus humanus corporis, Pediculus humanus humanus; Pthirus pubis,* Haematopinus spp. e.g. *Haematopinus eurysternus, Haematopinus suis;* Linognathus spp. e.g. *Linognathus vituli; Bovicola bovis, Menopon gallinae, Menacanthus stramineus* and *Solenopotes capillatus, Trichodectes* spp.,
Further pest species which may be controlled by compounds I include: from the Phylum **Mollusca,** class **Bivalvia,** e.g., *Dreissena* spp.; class **Gastropoda,** e.g., *Arion* spp., *Biomphalaria* spp., *Bulinus* spp., *Deroceras* spp., *Galba* spp., *Lymnaea* spp., *Oncomelania* spp., *Pomacea canaliclata, Succinea* spp.; from the class of the **helminths,** e.g., *Ancylostoma duodenale, Ancylostoma ceylanicum, Acylostoma braziliensis, Ancylostoma* spp., *Ascaris lubricoides, Ascaris spp., Brugia malayi, Brugia timori, Bunostomum* spp., *Chabertia* spp., *Clonorchis* spp., *Cooperia* spp., *Dicrocoelium* spp., *Dictyocaulus filaria, Diphyllobothrium latum, Dracunculus medinensis, Echinococcus granulosus, Echinococcus multilocularis, Enterobius vermicularis, Faciola* spp., Haemonchus spp. e.g. *Haemonchus contortus; Heterakis* spp., *Hymenolepis nana, Hyostrongulus* spp., *Loa Loa, Nematodirus* spp., *Oesophagostomum* spp., *Opisthorchis* spp., *Onchocerca volvulus, Ostertagia* spp., *Paragonimus* spp., *Schistosomen* spp., *Strongyloides fuelleborni, Strongyloides stercora lis, Stronyloides* spp., *Taenia saginata, Taenia solium, Trichinella spiralis, Trichinella nativa, Trichinella britovi, Trichinella nelsoni, Trichinella pseudopsiralis, Trichostrongulus spp., Trichuris trichuria, Wuchereria bancrofti.*

The compounds of the invention are particularly suitable for efficiently combating insects from the sub-order of **Auchenorrhyncha,** e.g. *Amrasca biguttula, Empoasca spp., Nephotettix virescens, Sogatella furcifera, Mahanarva spp., Laodelphax striatellus, Nilaparvata lugens, Diaphorina citri;*
Lepidoptera, e.g. *Helicoverpa spp., Heliothis virescens, Lobesia botrana, Ostrinia nubilalis, Plutella xylostella, Pseudoplusia includens, Scirpophaga incertulas, Spodoptera spp., Trichoplusia ni, Tuta absoluta, Cnaphalocrocis medialis, Cydia pomonella, Chilo suppressalis, Anticarsia gemmatalis, Agrotis ipsilon, Chrysodeixis includens;*
True bugs, e.g. *Lygus spp.,* Stink bugs such as *Euschistus spp., Halyomorpha halys, Nezara viridula, Piezodorus guildinii, Dichelops furcatus;*
Thrips, e.g. *Frankliniella spp., Thrips spp., Dichromothrips corbettii;*
Aphids, e.g. *Acyrthosiphon pisum, Aphis spp., Myzus persicae, Rhopalosiphum spp., Schizaphis graminum, Megoura viciae;*
Whiteflies, e.g. *Trialeurodes vaporariorum, Bemisia spp.;*
Coleoptera, e.g. *Phyllotreta spp., Melanotus spp., Meligethes aeneus, Leptinotarsa decimlineata, Ceutorhynchus spp., Diabrotica spp., Anthonomus grandis, Atomaria linearia, Agriotes spp., Epilachna spp.;*
Flies, e.g. *Delia spp., Ceratitis capitate, Bactrocera spp., Liriomyza spp.;*
Coccoidea, e.g. *Aonidiella aurantia, Ferrisia virgate;*
Anthropods of class Arachnida (Mites), e.g. *Penthaleus major, Tetranychus spp.;*
Nematodes, e.g. *Heterodera glycines, Meloidogyne sp., Pratylenchus spp., Caenorhabditis elegans.*

The compounds of the invention are suitable for use in treating or protecting animals against infestation or infection by parasites. Therefore, the invention also relates to the use of a compound of the invention for the manufacture of a medicament for the treatment or protection of animals against infestation or infection by parasites. Furthermore, the invention relates to a compound of the formula (I) for use in a method of treating or protecting animals against infestation and infection by parasites, which comprises orally, topically or parenterally administering or applying to the animals a parasiticidally effective amount of a compound of the invention.

The invention also relates to the non-therapeutic use of compounds of the invention for treating or protecting animals against infestation and infection by parasites. Moreover, the invention relates to a non-therapeutic method of treating or protecting animals against infestation and infection by parasites, which comprises applying to a locus a parasiticidally effective amount of a compound of the invention.

The compounds of the invention are further suitable for use in combating or controlling parasites in and on animals. Furthermore, the invention relates to a method of combating or controlling parasites in and on animals, which comprises contacting the parasites with a parasitically effective amount of a compound of the invention.

The invention also relates to the non-therapeutic use of compounds of the invention for controlling or combating parasites. Moreover, the invention relates to a non-therapeutic method of combating or controlling parasites, which comprises applying to a locus a parasiticidally effective amount of a compound of the invention.

The compounds of the invention can be effective through both contact (via soil, glass, wall, bed net, carpet, blankets or animal parts) and ingestion (e.g. baits). Furthermore, the compounds of the invention can be applied to any and all developmental stages.

The compounds of the invention can be applied as such or in form of compositions comprising the compounds of the invention.

The compounds of the invention can also be applied together with a mixing partner, which acts against pathogenic parasites, e.g. with synthetic coccidiosis compounds, polyetherantibiotics e.g. Amprolium, Robenidin, Toltrazuril, Monensin, Salinomycin, Maduramicin, Lasalocid, Narasin or Semduramicin, or with other mixing partners as defined above, or in form of compositions comprising said mixtures.

The compounds of the invention and compositions comprising them can be applied orally, parenterally or topically, e.g. dermally. The compounds of the invention can be systemically or non-systemically effective.

The application can be carried out prophylactically, therapeutically or non-therapeutically. Furthermore, the application can be carried out preventively to places at which occurrence of the parasites is expected.

As used herein, the term "contacting" includes both direct contact (applying the compounds/compositions directly on the parasite, including the application directly on the animal or excluding the application directly on the animal, e.g. at it's locus for the latter) and indirect contact (applying the compounds/compositions to the locus of the parasite). The contact of the parasite through application to its locus is an example of a non-therapeutic use of the compounds of the invention.

The term "locus" means the habitat, food supply, breeding ground, area, material or environment in which a parasite is growing or may grow outside of the animal.

As used herein, the term "parasites" includes endo- and ectoparasites. In some embodiments of the invention, endoparasites can be preferred. In other embodiments, ectoparasites can be preferred. Infestations in warm-blooded animals and fish include lice, biting lice, ticks, nasal bots, keds, biting flies, muscoid flies, flies, myiasitic fly larvae, chiggers, gnats, mosquitoes and fleas.

The compounds of the invention are especially useful for combating parasites of the following orders and species, respectively: **fleas (Siphonaptera),** e.g. *Ctenocephalides felis, C. canis, Xenopsylla cheopis, Pulex irritans, Tunga penetrans,* and *Nosopsyllus fasciatus;* **cockroaches (Blattaria - Blattodea),** e.g. *Blattella germanica, B. asahinae, Periplaneta americana, P. japonica, P. brunnea, P. fuligginosa, P. australasiae,* and *Blatta orientalis;* **flies, mosquitoes (Diptera),** e.g. *Aedes aegypti, A. albopictus, A. vexans, Anastrepha ludens, Anopheles maculipennis, A. crucians, A. albimanus, A. gambiae, A. freeborni, A. leucosphyrus, A. minimus, A. quadrimaculatus, Calliphora vicina, Chrysomya bezziana, C. hominivorax, C. macellaria, Chrysops discalis, C. silacea, C. atlanticus, Cochliomyia hominivorax, Cordylobia anthropophaga, Culicoides furens, Culex pipiens, C. nigripalpus, C. quinquefasciatus, C. tarsalis, Culiseta inornata, C. melanura, Dermatobia hominis, Fannia canicularis, Gasterophilus intestinalis, Glossina morsitans, G. palpalis, G. fuscipes, G. tachinoides, Haematobia irritans, Haplodiplosis equestris, Hippelates spp., Hypoderma lineata, Leptoconops torrens, Lucilia caprina, L. cuprina, L. sericata, Lycoria pectoralis, Mansonia spp., Musca domestica, M. stabulans, Oestrus ovis, Phlebotomus argentipes, Psorophora columbiae, P. discolor, Prosimulium mixtum, Sarcophaga spp., S. haemorrhoidalis, Simulium vittatum, Stomoxys calcitrans, Tabanus bovinus, T. atratus, T. lineola,* and *T. similis;* **lice (Phthiraptera),** e.g. *Pediculus humanus capitis, P. humanus humanus, Pthirus pubis, Haematopinus eurysternus, H. suis, Linognathus vituli, Bovicola bovis, Menopon gallinae, Menacanthus stramineus,* and *Solenopotes capillatus;* **ticks and parasitic mites (Parasitiformes): ticks (Ixodida),** e.g. *Ixodes scapularis, I. holocyclus, I. pacificus, Rhiphicephalus sanguineus, Dermacentor andersoni, D. variabilis, Amblyomma americanum, A. maculatum, Ornithodorus hermsi, O. turicata* and **parasitic mites (Mesostigmata),** e.g. *Ornithonyssus bacoti, Dermanyssus gallinae;* **Actinedida (Prostigmata) and Acaridida (Astigmata),** e.g. *Acarapis spp., Cheyletiella spp., Ornithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp.,* and *Laminosioptes spp;* **Bugs (Heteropterida):** *Cimex lectularius, C. hemipterus, Reduvius senilis, Triatoma spp., Rhodnius ssp., Panstrongylus ssp.,* and *Arilus critatus;* **Anoplurida,** e.g. *Haematopinus spp., Linognathus spp., Pediculus spp., Phtirus spp.,* and *Solenopotes spp.;* **Mallophagida (suborders Arnblycerina and Ischnocerina),** e.g. *Trimenopon spp., Menopon spp., Trinoton spp., Bovicola spp., Werneckiella spp., Lepikentron spp., Trichodectes spp.,* and *Felicola spp.;* **Roundworms Nematoda: Wipeworms and Trichinosis (Trichosyringida),** e.g. Trichinellidae *(Trichinella spp.),* (Trichuridae) *Trichuris spp., Capillaria spp.;* **Rhabditida,** e.g. *Rhabditis spp., Strongyloides spp., Helicephalobus spp.;* Strongylida, e.g. *Strongylus spp., Ancylostoma spp., Necator americanus, Bunostomum spp. (Hookworm), Trichostrongylus spp., Haemonchus contortus, Ostertagia spp., Cooperia spp., Nematodirus spp., Dictyocaulus spp., Cyathostoma spp., Oesophagostomum spp., Stephanurus dentatus, Ollulanus spp., Chabertia spp., Stephanurus dentatus, Syngamus trachea, Ancylostoma spp., Uncinaria spp., Globocephalus spp., Necator spp., Metastrongylus spp., Muellerius capillaris, Protostrongylus spp., Angiostrongylus spp., Parelaphostrongylus spp., Aleurostrongylus abstrusus,* and *Dioctophyma renale;* **Intestinal roundworms (Ascaridida),** e.g. *Ascaris lumbricoides, Ascaris suum, Ascaridia galli, Parascaris equorum, Enterobius vermicularis (Threadworm), Toxocara canis, Toxascaris leonine, Skrjabinema spp.,* and *Oxyuris equi;* **Camallanida,** e.g. *Dracunculus medinensis* (guinea worm); **Spirurida,** e.g. *Thelazia spp., Wuchereria spp., Brugia spp., Onchocerca spp., Dirofilari spp.a, Dipetalonema spp., Setaria spp., Elaeophora spp., Spirocerca lupi,* and *Habronema spp.;* **Thorny headed worms (Acanthocephala),** e.g. *Acanthocephalus spp., Macracanthorhynchus hirudinaceus* and *Oncicola spp.;* **Planarians (Plathelminthes): Flukes (Trematoda),** e.g. *Faciola spp., Fascioloides magna, Paragonimus spp., Dicrocoelium spp., Fasciolopsis buski, Clonorchis sinensis, Schistosoma spp., Trichobilharzia spp., Alaria alata, Paragonimus spp.,* and *Nanocyetes spp.;* **Cercomeromorpha,** in particular **Cestoda (Tapeworms),** e.g. *Diphyllobothrium spp., Tenia spp., Echinococcus spp., Dipylidium caninum, Multiceps spp., Hymenolepis spp., Mesocestoides spp., Vampirolepis spp., Moniezia spp., Anoplocephala spp., Sirometra spp., Anoplocephala spp.,* and *Hymenolepis spp..* The term "animal" includes warm-blooded animals (including humans) and fish. Preferred are mammals, e.g. cattle, sheep, swine, camels, deer, horses, pigs, poultry, rabbits, goats, dogs and cats, water buffalo, donkeys, fallow deer and reindeer, and also in fur-bearing animals e.g. mink, chinchilla and raccoon, birds e.g. hens, geese, turkeys and ducks and fish e.g. fresh- and salt-water fish e.g. trout, carp and eels. Particularly preferred are domestic animals, e.g. dogs or cats.

Generally, "parasiticidally effective amount" means the amount of active ingredient needed to achieve an observable effect on growth, including the effects of necrosis, death, retardation, prevention, and removal, destruction, or otherwise diminishing the occurrence and activity of the target organism. The parasiticidally effective amount can vary for the various compounds/compositions used in the invention. A parasiticidally effective amount of the compositions will also vary according to the prevailing conditions e.g. desired parasiticidal effect and duration, target species, mode of application.

Generally, it is favorable to apply the compounds of the invention in total amounts of 0.5 mg/kg to 100 mg/kg per day, preferably 1 mg/kg to 50 mg/kg per day.

For oral administration to warm-blooded animals, the compounds I may be formulated as animal feeds, animal feed premixes, animal feed concentrates, pills, solutions, pastes, suspensions, drenches, gels, tablets, boluses and capsules. In addition, the compounds I may be administered to the animals in their drinking water. For oral administration, the dosage form chosen should provide the animal with 0.01 mg/kg to 100 mg/kg of animal body weight per day of the compounds I, preferably with 0.5 mg/kg to 100 mg/kg of animal body weight per day. Alternatively, the compounds I may be administered to animals parenterally, e.g., by intraruminal, intramuscular, intravenous or subcutaneous injection. The compounds I may be dispersed or dissolved in a physiologically acceptable carrier for subcutaneous injection. Alternatively, the compounds I may be formulated into an implant for subcutaneous administration. In addition the compounds I may be transdermally administered to animals. For parenteral administration, the dosage form chosen should provide the animal with 0.01 mg/kg to 100 mg/kg of animal body weight per day of the compounds *I.*

The compounds I may also be applied topically to the animals in the form of dips, dusts, powders, collars, medallions, sprays, shampoos, spot-on and pour-on formulations and in ointments or oil-in-water or water-in-oil emulsions. For topical application, dips and sprays usually contain 0.5 ppm to 5,000 ppm and preferably 1 ppm to 3,000 ppm of the compounds *I.* In addition, the compounds I may be formulated as ear tags for animals, particularly quadrupeds e.g. cattle and sheep.

Suitable preparations are:
- Solutions e.g. oral solutions, concentrates for oral administration after dilution, solutions for use on the skin or in body cavities, pouring-on formulations, gels;
- Emulsions and suspensions for oral or dermal administration; semi-solid preparations;
- Formulations in which the active compound is processed in an ointment base or in an oil-in-water or water-in-oil emulsion base;
- Solid preparations e.g. powders, premixes or concentrates, granules, pellets, tablets, boluses, capsules; aerosols and inhalants, and active compound-containing shaped articles.

Compositions suitable for injection are prepared by dissolving the active ingredient in a suitable solvent and optionally adding further auxiliaries e.g. acids, bases, buffer salts, preservatives, and solubilizers. Suitable auxiliaries for injection solutions are known in the art. The solutions are filtered and filled sterile.

Oral solutions are administered directly. Concentrates are administered orally after prior dilution to the use concentration. Oral solutions and concentrates are prepared according to the state of the art and as described above for injection solutions, sterile procedures not being necessary.

Solutions for use on the skin are trickled on, spread on, rubbed in, sprinkled on or sprayed on. Solutions for use on the skin are prepared according to the state of the art and according to what is described above for injection solutions, sterile procedures not being necessary.

Gels are applied to or spread on the skin or introduced into body cavities. Gels are prepared by treating solutions which have been prepared as described in the case of the injection solutions with sufficient thickener that a clear material having an ointment-like consistency results. Suitable thickeners are known in the art.

Pour-on formulations are poured or sprayed onto limited areas of the skin, the active compound penetrating the skin and acting systemically. Pour-on formulations are prepared by dissolving, suspending or emulsifying the active compound in suitable skin-compatible solvents or solvent mixtures. If appropriate, other auxiliaries e.g. colorants, bioabsorption-promoting substances, antioxidants, light stabilizers, adhesives are added. Suitable such auxiliaries are known in the art.

Emulsions can be administered orally, dermally or as injections. Emulsions are either of the water-in-oil type or of the oil-in-water type. They are prepared by dissolving the active compound either in the hydrophobic or in the hydrophilic phase and homogenizing this with the solvent of the other phase with the aid of suitable emulsifiers and, if appropriate, other auxiliaries e.g. colorants, absorption-promoting substances, preservatives, antioxidants, light stabilizers, viscosity-enhancing substances. Suitable hydrophobic phases (oils), suitable hydrophilic phases, suitable emulsifiers, and suitable further auxiliaries for emulsions are known in the art.

Suspensions can be administered orally or topically/dermally. They are prepared by suspending the active compound in a suspending agent, if appropriate with addition of other auxiliaries e.g. wetting agents, colorants, bioabsorption-promoting substances, preservatives, antioxidants, light stabilizers. Suitable suspending agents, and suitable other auxiliaries for suspensions including wetting agents are known in the art.

Semi-solid preparations can be administered orally or topically/dermally. They differ from the suspensions and emulsions described above only by their higher viscosity.

For the production of solid preparations, the active compound is mixed with suitable excipients, if appropriate with addition of auxiliaries, and brought into the desired form. Suitable auxiliaries for this purpose are known in the art.

The compositions which can be used in the invention can comprise generally from about 0.001 to 95% of the compound of the invention.

Ready-to-use preparations contain the compounds acting against parasites, preferably ectoparasites, in concentrations of 10 ppm to 80% by weight, preferably from 0.1 to 65% by weight, more preferably from 1 to 50% by weight, most preferably from 5 to 40% by weight.

Preparations which are diluted before use contain the compounds acting against ectoparasites in concentrations of 0.5 to 90% by weight, preferably of 1 to 50% by weight.

Furthermore, the preparations comprise the compounds of formula I against endoparasites in concentrations of 10 ppm to 2% by weight, preferably of 0.05 to 0.9% by weight, very particularly preferably of 0.005 to 0.25% by weight.

Topical application may be conducted with compound-containing shaped articles e.g. collars, medallions, ear tags, bands for fixing at body parts, and adhesive strips and foils.

Generally it is favorable to apply solid formulations which release compounds of the invention in total amounts of 10 mg/kg to 300 mg/kg, preferably 20 mg/kg to 200 mg/kg, most preferably 25 mg/kg to 160 mg/kg body weight of the treated animal in the course of three weeks.

The following examples illustrate the invention.

### A. Preparation of Compounds

Materials: Unless otherwise noted, reagents and solvents were purchased at highest commercial quality and used without further purification.

All reactions were monitored by thin-layer chromatography (TLC) using Merck silica gel 60 F₂54 pre-coated plates (0.25 mm). Flash chromatography was carried out with Agela technologies silica gel (Agela techno, silica irregular 40-60µm, 60A, Cat.-No. C-CS1400).

¹H NMR spectra were recorded on Bruker (500 MHz). Chemical shifts are expressed in ppm downfield from the internal solvent peaks for DMSO-d₆ (¹H; δ = 2.50ppm) (¹H; δ = 2.50ppm) and CD₃OD (¹H; δ = 3.30ppm), and J values are given in Hertz. The following abbreviations were used to explain the multiplicities: s = singlet, d = doublet, t = triplet, q = quartet, dd = double doublet, dt = double triplet, m = multiplet, br = broad. High-resolution mass spectra were measured on a JEOL JMS-T100LP.

Characterization: The compounds were characterized by coupled High Performance Liquid Chromatography with mass spectrometry (HPLC/MS).

Method A: UHPLC-MS on Shimadzu LCMS 2020 ESI. Analytical UHPLC column: C-18, 50mm, 4.6mm, 5micron; mobile phase: 100mM Ammonium Formate, B: Acetonitrile, Flow Rate: 1.2mL/min, Injection Vol: 1µL in 1.50min; Gradient: 10% B to 100% B in 1.5min, Hold 100% B for 1min, 2.51min 10% B Run time: 3min at 400°C. MS-method: ESI positive; mass range (m/z) 100-800.

Method B: UHPLC-MSD 5 on Shimadzu Naxera LC 30 LCMS 2020 ESI. Analytical UHPLC column: Kinetex SB C-18, 50mm, 2.1mm, 1.7micron; mobile phase: water 0.1% TFA, B: Acetonitrile, Flow Rate: 0.8 mL/min, Injection Vol: 0.3µL in 1.50min; Gradient: 5% B to 100% B in 1.5min.

Abbreviations used: min is minutes; DCM is dichlormethane; mL is milliliters

### Synthesis Example 1: 6-cyclopropyl-2-[5-[[dimethyl(oxo)-λ-6-sulfanylidene]amino]-3-ethylsulfonyl-2-pyridyl]-7-(trifluoromethyl)imidazo[1,2-c]pyrimidin-5-one (compound I.1)

### Step-1: Manufacture of 5-bromo-3-ethylsulfanyl-pyridine-2-carbonitrile

To a stirred solution of 5-bromo-3-nitro-pyridine-2-carbonitrile (0.087mol) in DMF (200mL) at -40°C, was added sodium ethane thiolate (0.105mol) portion wise over a period of 30min under N₂-atmosphere at a maintained temperature of between -40 to -50°C. The resultant reaction mixture was stirred at the same temperature for 10min, then gradually allowed to reach a temperature of 20 to 25°C with continued stirring over the next hour. The reaction mixture was then quenched by addition of a saturated aqueous solution of NH₄Cl and was extracted with ethyl acetate. The combined organic phases were dried, and concentrated under reduced pressure to get crude mass, which was purified by column chromatography to afford 5-bromo-3-ethylsulfanyl-pyridine-2-carbonitrile as a yellow solid (17g; 82% yield). ¹ H-NMR (500MHz, CDCl₃): δ 8.52 (s, 1H), 7.85 (s, 1H), 3.07 (q, 2H, *J*=10Hz), 1.43 (t, 3H, *J*=7.3 Hz). LC-MS mass found [M+H]⁺ was 244.

### Step-2: Manufacture of 1-(5-bromo-3-ethylsulfanyl-2-pyridyl)ethanone

To a stirred solution of 5-bromo-3-ethylsulfanyl-pyridine-2-carbonitrile (17g) in THF (170mL) at 0°C, was added methyl magnesium bromide (2eq 3M in diethyletherDEE, 46.6mL) dropwise over a period of 30min at 0°C to -5°C under N₂-atmosphere. The resultant reaction mixture was allowed to stir at 0°C for 2 hours. Then, the resulting reaction mixture was quenched with an aqueous 1N solution of HCI and was extracted. The combined organic layers were dried and concentrated under reduced pressure to get a crude mass. Treating of the crude mass with ethyl acetate and heptane at -30°C afforded a precipitate, which was filtered and concentrated under reduced pressure to afford 1-(5-bromo-3-ethylsulfanyl-2-pyridyl)ethanone as a yellow solid (14g, 82% yield). ¹H-NMR (500MHz, DMSO-d₆): δ 8.57 (s, 1H), 8.05 (s, 1H), 3.01 (q, 2H, *J*=10Hz), 2.59 (s, 3H), 1.26 (t, 3H, *J*=7.3Hz). LC-MS: mass found [M+H]⁺ was 261.

### Step-3: Manufacture of 1-(5-bromo-3-ethylsulfonyl-2-pyridyl)ethanone

To a stirred solution of 1-(5-bromo-3-ethylsulfanyl-2-pyridyl)ethanone (0.018mol) in DCM (40mL) at 0°C, was added *m*-chloroperoxy benzoic acid (9.7g). Resultant reaction mixture was stirred at a temperature of 20 to 25°C for 3 to 4 hours. Then, the reaction mixture was quenched with an aqueous saturated bicarbonate solution and extracted. The combined organic layers were dried and concentrated under reduced pressure to get 1-(5-bromo-3-ethylsulfonyl-2-pyridyl)ethanone (3.2g, 82% yield) as an off white solid. ¹H-NMR (500MHz, CDCl₃): δ 9.12 (s, 1H), 8.55 (s, 1H), 3.55 (q, 2H *J*=12Hz), 2.5 (s, 3H),1.20 (t, 3H, *J*=7Hz). LC-MS: mass found for [M+H]⁺ was 292.

### Step-4: Manufacture of 2-bromo-1-(5-bromo-3-ethylsulfonyl-2-pyridyl)ethanone

To a stirred solution of 1-(5-bromo-3-ethylsulfonyl-2-pyridyl)ethanone (0.010mol) in CHCl₃ (30mL) at 0 °C, was added CH₃COOH (30mL) and HBr in CH₃COOH (33wt.-% in CH₃COOH, 30mL) and stirred for 1 to 10mins. To the resulting reaction mixture was subsequently added Br₂ (0.012mol) in CHCl₃. Resultant reaction mixture was heated to 60°C for 1 hour. Then, the reaction mixture was quenched with aqueous saturated bicarbonate solution and was extracted. The combined organic layers were dried and concentrated under reduced pressure to get a crude mass, which was purified by column chromatography to afford 2-bromo-1-(5-bromo-3-ethylsulfonyl-2-pyridyl)ethanone as an off white solid (2.2g, 82% yield). ¹H-NMR (500MHz, CDCl₃): δ 9.17 (s, 1H), 8.63 (s, 1H), 4.92 (s, 2H), 3.59 (q, 2H J=12Hz), 1.20 (t, 3H, J=7Hz). LC-MS: mass found for [M+H]⁺ was 372.

### Step-5: Manufacture of 2-(5-bromo-3-ethylsulfonyl-2-pyridyl)-6-cyclopropyl-7-(trifluoromethyl)imidazo[1,2-c]pyrimidin-5-one

To a stirred solution of 2-bromo-1-(5-bromo-3-ethylsulfonyl-2-pyridyl)ethanone (0.006mol) in tert-butanol (4mL) was added 1-cyclopropyl-4-imino-6-(trifluoromethyl)pyrimidin-2-one (0.036mol). A molecular sieve was added to this reaction mixture (2g), which was subsequently heated to 120°C for 24 hours. Then, the reaction mixture was filtered through a celite bed, and the filtrate was collected and concentrated under reduced pressure to get a crude mass, which was purified by column chromatography to afford 2-(5-bromo-3-ethylsulfonyl-2-pyridyl)-6-cyclopropyl-7-(trifluoromethyl)imidazo[1,2-c]pyrimidin-5-one as a yellow solid (1.5g, 57% yield). ¹H-NMR (500MHz, DMSO-d₆): δ 9.10 (s, 1H), 8.53 (s, 1H), 8.28 (s, 1H), 7.52 (s, 1H), 4.06 (q, 2H, *J*=10Hz), 3.17 (s, 1H), 1.23 (t, 3H, *J*=7Hz), 1.09 (dd, 4H, *J*=4.5Hz). LC-MS: mass found for [M+H]⁺ was 492.

### Step-6: Manufacture of 6-cyclopropyl-2-[5-[[dimethyl(oxo)-λ-6-sulfanylidene]amino]-3-ethylsulfonyl-2-pyridyl]-7-(trifluoromethyl)imidazo[1,2-c]pyrimidin-5-one (compound I.1)

A solution of 2-(5-bromo-3-ethylsulfonyl-2-pyridyl)-6-cyclopropyl-7-(trifluoromethyl)imidazo[1,2-c]pyrimidin-5-one (0.609mmol), S,S-dimethyl-sulfoximine (0.914mmol), CsCO₃ (0.914mmol) and (5-diphenylphosphanyl-9,9-dimethyl-xanthen-4-yl)-diphenyl-phosphane (Xantphos, 0.061mmol) in 1,4-dioxane was flushed with N₂ for 10 mins. Tris(dibenzylideneacetone)dipaliadium(0) (0.030mmol) was added, the vial sealed and the mixture stirred in the microwave at 110°C for 120mins or a closed sealed reactor at 110°C for 180mins. The reaction mixture was cooled and filtered through celite and washed with DCM. The organic layer was diluted with water and the product extracted with DCM. The combined organic layers were dried, filtered and concentrated under reduced pressure. The resulting residue was purified by column chromatography to afford 6-cyclopropyl-2-[5-[[dimethyl(oxo)-λ-6-sulfanylidene]amino]-3-ethylsulfonyl-2-pyridyl]-7-(trifluoromethyl)imidazo[1,2-c]pyrimidin-5-one with approx. 85% yield. ¹H-NMR (500MHz, CDCl₃) δ 8.61 (q, *J*=2.0Hz, 1H), 8.24 (d, *J*=1.9Hz, 1H), 8.11 (q, *J*=2.0Hz, 1H), 7.14 (s, 1H), 3.93-3.75 (m, 2H), 3.26 (t, *J*=1.6Hz, 6H), 3.16 (s, 1H), 1.40-1.21 (m, 5H), 1.12 (s, 2H). LC-MS: mass found for [M+H]⁺ was 504

With appropriate modification of the starting materials or intermediates thereof, the procedures as described in the preparation example above were used to obtain further compounds of formulae I.A.A1 and I.A.A44 listed in Table B with their physical data.

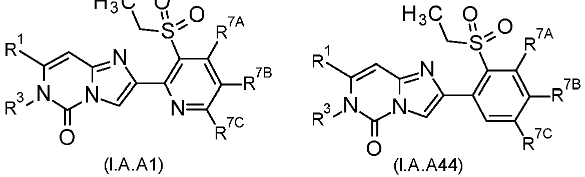

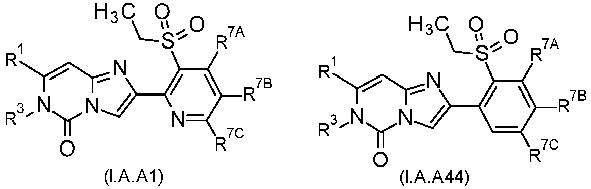

**Table B**

| No. | Formula | R¹ | R³ | R^{7A} | R^{7B} | R^{7C} | ¹H-NMR data (δ); LCMS [RT (min), M⁺] |
|---|---|---|---|---|---|---|---|
| I.1 | I.A.A1 | CF₃ | cC₃H₅ | H | 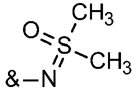 | H | δ (CDCI₃): 8.61 (q, *J*=2.0Hz, 1H), 8.24 (d, *J*=1.9Hz, 1H), 8.11 (q, *J*=2.0Hz, 1H), 7.14 (s, 1H), 3.93-3.75 (m, 2H), 3.26 (t, *J*=1.6Hz, 6H), 3.16 (s, 1H), 1.40-1.21 (m, 5H), 1.12 (s, 2H); |
| I.2 | I.A.A1 | CF₃ | CH₃ | H | 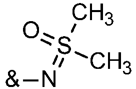 | H | δ (CDCl₃): 8.22 (s, 1H), 8.12 (d, *J*=2.8Hz, 1H), 7.53 (d, *J*=2.8Hz, 1H), 7.14 (s, 1H), 3.85 (q, *J*=7.4Hz, 2H), 3.74 (s, 3H), 3.23(s, 6H), 1.28 (t, *J*=7.4 Hz, 3H) |
| I.3 | I.A.A1 | CF₃ | CH₂-cC₃H₅ | H | 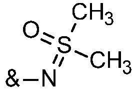 | H | δ (DMSO-d6): 8.48 (d, J=2.6Hz, 1H), 8.19 (s, 1H), 7.91 (d, *J* =2.6Hz, 1H), 7.55 (s, 1H), 3.98 (m, *J=14.8,* 7.0Hz, 4H), 3.40 (s, 6H), 1.22 (t, *J*=7.4Hz, 4H), 0.53 (td, *J*=6.6, 2.3Hz, 4H) |
| I.4 | I.A.A1 | CF₃ | cC₃H₅ | H | 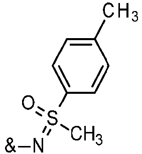 | H | δ (CDCl₃): 8.41 (d, *J*=2.6Hz, 1H), 8.07 (s, 1H), 7.94-7.84 (m, 2H), 7.71 (d, *J*=2.6Hz, 1H), 7.47 (d, *J*=8.1Hz, 2H), 7.42 (s, 1H), 3.58 (s, 3H), 3.14 (s, 1H), 2.39 (s, 3H), 1.64 (d, *J*=15.5Hz, 2H), 1.30-1.17 (m, 2H), 1.17-1.03 (m, 2H), 0.93 (t, *J*=7.3Hz, 3H) |
| I.5 | I.A.A1 | CF₃ | cC₃H₅ | H | 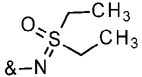 | H | δ (CDCl₃): 8.22 (s, 1H), 8.12 (d, *J*=2.8 Hz, 1H), 7.53 (d, *J*=2.8 Hz, 1H), 7.14 (s, 1H), 4.43 (q, *J*=11.5Hz, 4H), 3.85 (q, *J*=7.4 Hz, 2H), 3.16 (s, 1H), 1.74 (*J*=7.9Hz, 2H), 1.38-1.24 (m, 6H), 1.12 (d, *J*=7.9Hz, 2H), 0.84 (t, *J*=17.9Hz, 3H) |
| I.6 | I.A.A1 | CF₃ | cC₃H₅ | H | 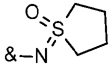 | H | δ (CDCl₃): 8.61 (s, 1H), 8.26 (s, 1H), 8.09 (d, *J*=2.4Hz, 1H), 7.17 (s, 1H), 3.86 (q, *J*=7.4Hz, 2H), 3.47 (dt, *J*=13.2, 6.7Hz, 2H), 3.33 (dt, *J*=13.6, 7.0Hz, 2H), 3.26-3.06 (m, 2H), 2.49-2.39 (m, 2H), 2.39-2.20 (m, 2H), 1.42-1.24 (m, 4H), 1.14 (s, 2H) |
| I.7 | I.A.A1 | CF₃ | CH₃ | H | 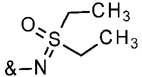 | H | δ (DMSO-d₆): 8.49 (d, *J*=2.5Hz, 1H), 8.15 (s, 1H), 7.94 (d, *J*=2.6Hz, 1H), 7.52 (s, 1H), 3.94 (t, *J*=7.4Hz, 2H), 3.61 (s, 3H), 3.48 (qd, *J*=7.2, 2.1Hz, 4H), 1.32 (t, *J*=7.4 Hz, 6H), 1.26-1.17 (m, 3H) |
| I.8 | I.A.A1 | CF₃ | cC₃H₅ | H | 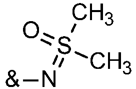 | CH₃ | δ (DMSO-d₆)⁻ 8.08 (s, 1H), 7.94 (s, 1H), 7.46 (s, 1H), 3.91 (q, *J*=7.4Hz, 2H), 3.36 (s, 6H), 3.14 (s, 1H), 2.48 (s, 3H), 1.19 (t, *J*=7.3 Hz, 3H), 1.15 (dd, *J*=6.9, 2.0Hz, 2H), 1.09 (d, *J*=4.4Hz, 2H) |
| I.9 | I.A.A1 | CF₃ | cC₃H₅ | H | 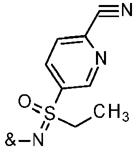 | H | δ (DMSO-d₆): 9.21 (dd, *J*=2.3, 0.9Hz, 1H), 8.54 (d, *J*=2.5Hz, 1H), 8.43 (dd, *J*=8.1, 2.2Hz, 1H), 8.22 (s, 1H), 8.06 (d, *J*=2.5Hz, 1H), 7.94 (dd, *J*=8.1, 0.9Hz, 1H), 7.14 (s, 1H), 3.83 (dddt, *J* = 28.8, 21.4, 14.7, 7.4Hz, 2H), 3.59 (dq, *J*=14.8, 7.5Hz, 1H), 3.49 (dq, *J*=14.6, 7.4Hz, 1H), 3.19-3.14 (m, 1H), 1.48 (t, *J*=7.4Hz, 3H), 1.35-1.22 (m, 5H), 1.13 (m, 2H) |
| I.10 | I.A.A1 | CF₃ | cC₃H₅ | H | 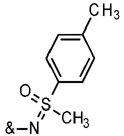 | H | δ (CDCl₃): 8.41 (d, *J*=2.5Hz, 1H), 8.09 (s, 1H), 7.90 (d, *J*=2.5Hz, 1H), 7.80-7.74 (m, 2H), 7.30 (d, *J*=8.1Hz, 2H), 7.03 (s, 1H), 3.74 (dq, *J*=14.8, 7.5Hz, 1H), 3.60 (dq, *J*=14.7, 7.4Hz, 1H), 3.27 (s, 3H), 3.06 (td, *J*=7.2, 3.7Hz, 1H), 2.36 (s, 3H), 1.24-1.17 (m, 2H), 1.13 (t, *J*=7.4Hz, 3H), 1.03 (d, *J*=4.3Hz, 2H). |
| I.11 | I.A.A1 | CF₃ | cC₃H₅ | H | 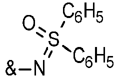 | H | δ (CDCl₃): 8.64 (d, *J*=2.4 Hz, 1H), 8.18 (s, 1H), 8.14 (d, *J*=2.6Hz, 1H), 8.11-8.05 (m, 5H), 7.61-7.51 (m, 5H), 7.11 (s, 1H), 3.77 (q, *J*=7.7Hz, 2H), 3.14 (s, 1H), 1.30-1.18 (m, 7H), |
| I.12 | I.A.A1 | CF₃ | cC₃H₅ | H | 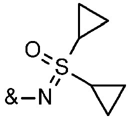 | H | δ (CDCl₃): 8.44 (d, *J*=2.5Hz, 1H), 8.11 (s, 1H), 7.94 (d, *J*=2.5Hz, 1H), 7.46 (s, 1H), 3.96 (q, *J*=7.4Hz, 2H), 3.17 (m, 1H), 2.97 (tt, *J*=7.6, 4.8Hz, 2H), 1.28-1.20 (m, 4H), 1.19 (t, *J*=7.4 Hz, 3H), 1.18-1.06 (m, 8H). |
| I.13 | I.A.A1 | CF₃ | CH₃ | H | 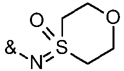 | H | δ (CDCl₃): 8.56 (d, *J*=2.5 Hz, 1H), 8.17 (s, 1H), 7.95 (d, *J*=2.6 Hz, 1H), 7.53 (s, 1H), 4.12 (dd, *J*=10.7, 6.4Hz, 2H), 4.05-3.89 (m, 4H), 3.62 (m, 7H), 1.21 (m, 3H) |
| I.14 | I.A.A1 | CF₃ | cC₃H₅ | H | 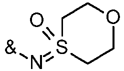 | H | δ (CDCl₃): 8.55 (d, *J*=2.5Hz, 1H), 8.11 (s, 1H), 7.94 (d, *J*=2.5Hz, 1H), 7.47 (s, 1H), 4.13 (d, *J*=12.9Hz, 2H), 4.08-3.91 (m, 4H), 3.71-3.48 (m, 4H), 3.16 (s, 1H), 1.21 (t, *J*=7.4Hz, 3H), 1.12 (t, *J*=8.7Hz, 4H). |
| I.15 | I.A.A1 | CF₃ | cC₃H₅ | H | 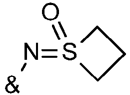 | H | (Method A):1.011, 516 (M+1) |
| I.16 | I.A.A1 | CF₃ | benzyl | H | 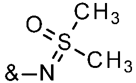 | H | δ (DMSO-d₆): 8.46 (d, *J*=2.5Hz, 1H), 8.18 (s, 1H), 7.90 (d, *J*=2.5Hz, 1H), 7.62 (s, 1H), 7.23 (m, 5H), 5.30 (s, 2H), 3.96 (q, *J*=7.4Hz, 2H), 2.50 (d, *J*=3.8Hz, 6H), 1.21 (t, *J*=7.4Hz, 3H). |
| I.17 | I.A.A1 | CH₂CH₃ | CH₃ | H | 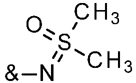 | H | δ (DMSO-d₆): 8.45 (d, *J*=2.4Hz, 1H), 7.93 (d, *J*=2.0Hz, 1H), 7.89 (s, 1H), 6.52 (s, 1H), 4.00 (d, *J*=7.4Hz, 2H), 3.56 (d, *J*=1.9Hz, 3H), 3.38 (d, *J*=2.0 Hz, 6H), 2.96 (d, *J*=1.9Hz, 3H), 2.76 (d, *J*=7.4Hz, 2H), 1.23 (t, *J*=7.4Hz, 3H). |
| I.18 | I.A.A1 | CHF₂ | cC₃H₅ | H | 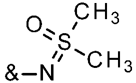 | H | (Method B): 0.872, 486.1 (M+1) |
| I.19 | I.A.A44 | CF₃ | cC₃H₅ | H | 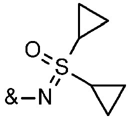 | H | δ (DMSO-*d*₆): 8.14 (s, 1H), 7.65 (d, *J*=2.3Hz, 1H), 7.56 (d, *J*=8.3Hz, 1H), 7.44 (s, 1H), 7.26 (dd, *J*=8.3, 2.4Hz, 1H), 3.53 (q, *J*=7.3Hz, 2H), 3.16 (m, 1H), 2.89 (dd, *J*=12.6, 2.6Hz, 2H), 1.21 (t, J=7.4Hz, 3H), 1.10 (p, *J*=6.7Hz, 12H). |
| I.20 | I.A.A44 | CF₃ | cC₃H₅ | H | 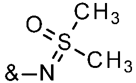 | H | δ (DMSO-d₆): 8.11 (s, 1H), 7.60-7.54 (m, 2H), 7.42 (s, 1H), 7.29 (dd, *J*=8.3, 2.4Hz, 1H), 3.61 (d, *J*=7.4Hz, 2H), 3.19 (s, 6H), 3.18 (s, 1H), 1.13 (d, *J*=6.8Hz, 3H), 1.07 (t, *J*=7.3Hz, 4H). |
| I.21 | I.A.A44 | CF₃ | CH₂-cC₃H₅ | H | 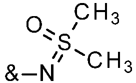 | H | δ (DMSO-d₆): 8.21 (s, 1H), 7.64-7.58 (m, 2H), 7.53 (s, 1H), 7.32 (dd, *J*=8.2, 2.5Hz, 1H), 3.99 (d, *J*=6.8Hz, 2H), 3.56 (q, *J*=7.4Hz, 2H), 3.19 (s, 6H), 3.18 (s, 1H), 1.10 (t, *J*=7.4Hz, 3H), 0.57-0.50 (m, 4H). |
| 1.22 | I.A.A1 | CF₃ | CH₂-cC₃H₅ | H | 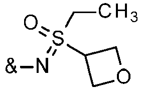 | H | δ (CDCl₃): 8.64 (d, *J*=2.5Hz, 1H), 8.26 (s, 1H), 8.13 (d, *J*=2.5Hz, 1H), 7.16 (s, 1H), 3.85 (q, *J*=7.5Hz, 2H), 3.38 (dd, *J*=7.5, 3.5Hz, 2H), 3.19 (t, *J*=7.4Hz, 3H), 3.18 (s, 2H), 1.54 (t, *J*=7.4Hz, 3H), 1.41-1.25 (m, 6H), 1.14 (m, 2H). |

| | | | | | | | |
|---|---|---|---|---|---|---|---|
| & denotes the bond to the remainder of the molecule | | | | | | | |

### B. Biological Examples

The activity of the compounds of formula (I) of the invention could be demonstrated and evaluated in biological tests described in the following. If not otherwise specified, the test solutions are prepared as follows: The active compound is dissolved at the desired concentration in a mixture of 1:1 (vol:vol) distilled water : acteone. The test solution is prepared at the day of use. Test solutions are prepared in general at concentrations of 2500ppm, 800ppm, and 300ppm (wt/vol).

### B.1 Boll weevil (Anthonomus grandis)

For evaluating control of boll weevil (*Anthonomus grandis*) the test unit consisted of 96-well-microtiter plates containing an insect diet and 5-10 *A. grandis* eggs. The compounds were formulated using a solution containing 75% v/v water and 25% v/v DMSO. Different concentrations of formulated compounds were sprayed onto the insect diet at 5 µl, using a custom-built micro atomizer, at two replications. After application, microtiter plates were incubated at about 25 ± 1°C and about 75 ± 5 % relative humidity for 5 days. Egg and larval mortality was then visually assessed. In this test, compounds I.1, I.2, I.3, I.5, I.6, I.7, I.8, I.9, I.11, I.13, I.14, I.15, I.16, I.19, I.20, I.21, I.22, resp., at 800ppm showed over 75% mortality in comparison with untreated controls.

### B.2 Tobacco budworm (Heliothis virescens)

For evaluating control of tobacco budworm (*Heliothis virescens*) the test unit consisted of 96-well-microtiter plates containing an insect diet and 15-25 *H. virescens* eggs. The compounds were formulated using a solution containing 75% v/v water and 25% v/v DMSO. Different concentrations of formulated compounds were sprayed onto the insect diet at 10 µl, using a custom-built micro atomizer, at two replications. After application, microtiter plates were incubated at about 28 ± 1°C and about 80 ± 5 % relative humidity for 5 days. Egg and larval mortality was then visually assessed. In this test, compounds I.1, I.3, I.6, I.8, I.9, I.11, I.14, I.15, I.19, I.20, I.21, I.22, resp., at 800ppm showed over 75% mortality in comparison with untreated controls.

### B.3 Green Peach Aphid (Myzus persicae)

For evaluating control of green peach aphid (*Myzus persicae*) through systemic means the test unit consisted of 96-well-microtiter plates containing liquid artificial diet under an artificial membrane. The compounds were formulated using a solution containing 75% v/v water and 25% v/v DMSO. Different concentrations of formulated compounds were pipetted into the aphid diet, using a custom built pipetter, at two replications. After application, 5 - 8 adult aphids were placed on the artificial membrane inside the microtiter plate wells. The aphids were then allowed to suck on the treated aphid diet and incubated at about 23 ± 1°C and about 50 ± 5 % relative humidity for 3 days. Aphid mortality and fecundity was then visually assessed. In this test, compounds I.1, I.2, I.3, I.4, I.5, I.6, I.7, I.8, I.9, I.10, I.11, I.13, I.14, I.15, I.16, I.19, I.20, I.21, I.22, resp., at 800ppm showed over 75 % mortality in comparison with untreated controls.

### B.4 Greenhouse Whitefly (Trialeurodes vaporarirorum)

For evaluating control of Greenhouse Whitefly (*Trialeurodes vaporariorum*) the test unit consisted of 96-well-microtiter plates containing a leaf disk of egg plant leaf disk with white fly eggs. The compounds or mixtures were formulated using a solution containing 75% water and 25% DMSO. Different concentrations of formulated were sprayed onto the insect diet at 2.5µl, using a custom-built micro atomizer, at two replications. After application, microtiter plates were incubated at 23 ± 1°C, 65 ± 5 % RH for 6 days. Mortality of hatched crawlers was then visually assessed. In this test, compounds I.1, I.2, I.5, I.6, I.7, I.8, I.15, I.19, I.20, I.21, I.22, resp., at 800ppm showed over 75 % mortality in comparison with untreated controls.

### B.5 Yellow fever mosquito (Aedes aegypti)

For evaluating control of yellow fever mosquito (*Aedes aegypti*) the test unit consisted of 96-well-microtiter plates containing 200µl of tap water per well and 5-15 freshly hatched *A. aegypti* larvae. The active compounds were formulated using a solution containing 75% (v/v) water and 25% (v/v) DMSO. Different concentrations of formulated compounds or mixtures were sprayed onto the insect diet at 2.5µl, using a custom-built micro atomizer, at two replications. After application, microtiter plates were incubated at 28 ± 1°C, 80 ± 5 % RH for 2 days. Larval mortality was then visually assessed. In this test, compounds I.1, I.2, I.3, I.8, I.9, I.12, I.13, I.14, I.15, I.19, I.20, I.21, resp., at 800ppm showed over 75% mortality in comparison with untreated controls.

### B.6 Green Soldier Stink Bug (Nezara viridula)

The active compound is dissolved at the desired concentration in a mixture of 1:1 (vol:vol) distilled water : aceteone. Surfactant (Kinetic) is added at a rate of 0.01% (vol/vol). The test solution is prepared at the day of use. Soybean pods are placed in 90 x 50 mm glass Petri dishes lined with moistened filter paper and inoculated with ten late 3rd instar *N. viridula.* Using a hand atomizer, an approximately 2 ml solution is sprayed into each Petri dish. Treated set-up is kept at about 25-26°C and relative humidity of about 65-70%. Percent mortality is recorded after 5 days. In this test, compounds I.1, I.2, I.3, I.8, I.12, I.13, I.14, I.15, I.20, I.21, I.22, resp., at 300ppm showed over 75% mortality in comparison with untreated controls.

## Claims

1. A compound of formula (I) 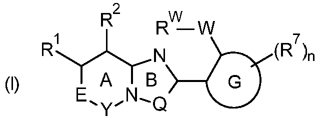 wherein
the rings A and B are fully unsaturated;
Y is C=X, wherein X is O or S;
E is N(R³) or C(R⁴);
Q is N, N(R⁵) or C(R⁶);
G is phenyl, or a 5- or 6-membered hetaryl;
W is S, S(O), S(O)₂, or S(O)(NR^{W});
R¹ is H, halogen, C₁-C₆-alkyl, C₂-C₆-alkenyl, C₂-C₆-alkynyl, C₁-C₆-alkoxy, C₁-C₆-alkoxy-C₁-C₆-alkyl, C₃-C₆-cycloalkyl, C₃-C₆-cycloalkoxy, C₁-C₆-alkylsulfenyl, C₁-C₆-alkylsulfinyl, or C₁-C₆-alkylsulfonyl, which groups are unsubstituted or halogenated;
R³, R⁵ are
independently C₁-C₆-alkyl, C₁-C₆-alkoxy, C₂-C₆-alkenyl, C₂-C₆-alkynyl, C₁-C₆-alkoxy-C₁-C₄-alkyl, C₁-C₆-alkoxy-C₁-C₄-alkoxy, C₃-C₆-cycloalkyl, C₃-C₆-cycloalkyl-C₁-C₄-alkyl, C₃-C₆-cycloalkoxy-C₁-C₄-alkyl, which are unsubstituted or halogenated;
C(=O)OR^{A}, NR^{B}R^{C}, C₁-C₆-alkylen-NR^{B}R^{C}, O-C₁-C₆-alkylen-NR^{B}R^{C}, C₁-C₆-alkylen-CN, NH-C₁-C₆-alkylen-NR^{B}R^{C}, C(=O)NR^{B}R^{C}, C(=O)R^{D}, C(=S)R^{D}, SO₂NR^{B}R^{C}, S(=O)ₘR^{E};
phenyl or benzyl, wherein the phenyl ring is unsubstituted or substituted with one or more R^{F};
R², R⁴, R⁶
are independently H, halogen, N₃, CN, NO₂, SCN, SF₃, C₁-C₆-alkyl, C₁-C₆-alkoxy, C₂-C₆-alkenyl, tri-C₁-C₆-alkylsilyl, C₂-C₆-alkynyl, C₁-C₆-alkoxy-C₁-C₄-alkyl, C₁-C₆-alkoxy-C₁-C₄-alkoxy, C₃-C₆-cycloalkyl, C₃-C₆-cycloalkoxy, C₃-C₆-cycloalkyl-C₁-C₄-alkyl, C₃-C₆-cycloalkoxyx-C₁-C₄-alkyl, which groups are unsubstituted or substituted with halogen,
C(=O)OR^{A}, NR^{B}R^{C}, NOR^{A}, ONR^{B}R^{C}, C₁-C₆-alkylen-NR^{B}R^{C}, O-C₁-C₆-alkylen-NR^{B}R^{C}, C₁-C₆-alkylen-CN, NH-C₁-C₆-alkylen-NR^{B}R^{C}, C(=O)NR^{B}R^{C}, C(=O)R^{D}, C(=S)R^{D}, SO₂NR^{B}R^{C}, S(=O)ₘR^{E};
phenyl or benzyl, wherein the phenyl ring is unsubstituted or substituted with one or more R^{F};
each R⁷
is independently H, halogen, OH, CN, NC, NO₂, N₃, SCN, NCS, NCO, SF₅;
C₁-C₆-alkyl, C₃-C₆-cycloalkyl, C₂-C₆-alkenyl, C₃-C₆-cycloalkenyl, C₂-C₆-alkynyl, C₃-C₆-cycloalkyl-C₁-C₃-alkyl, which groups are unsubstituted, or substituted with one or more R^{G};
a 3- to 12-membered saturated, partially unsaturated, or fully unsaturated heterocyclic ring or ring system, wherein said heterocyclic ring or ring system comprises one or more, same or different heteroatoms O, N, or S, and is unsubstituted, or substituted with one or more R^{H};
phenyl, which is unsubstituted, or substituted with one or more R^{J};
OR^{K}, SR^{K}, OC(=O)R^{K}, OC(=O)OR^{K}, OC(=O)NR^{L}R^{M}, OC(=O)SR^{K}, OC(=S)NR^{L}R^{M}, OC(=S)SR^{K}, OS(=O)ₘR^{K}, OS(=O)ₘNR^{L}R^{M}, ONR^{L}R^{M}, ON=CR^{N}R^{O}, NR^{L}R^{M}, NOR^{K}, ONR^{L}R^{M}, N=CR^{N}R^{O}, NNR^{L}, N(R^{L})C(=O)R^{K}, N(R^{L})C(=O)OR^{K}, S(=O)ₘR^{V}, SC(=O)SR^{K}, SC(=O)NR^{L}R^{M}, S(=O)ₘNR^{L}R^{M}, C(=O)R^{P}, C(=S)R^{P}, C(=O)NR^{L}R^{M}, C(=O)OR^{K}, C(=S)NR^{L}R^{M}, C(=S)OR^{K}, C(=S)SR^{K}, C(=NR^{L})R^{M}, C(=NR^{L})NR^{M}R^{R}, Si(R^{S})₂R^{T};
or two substituents R⁷ form, together with the ring members of ring G to which they are bound, a 5- or 6- membered saturated, partially unsaturated, or fully unsaturated carbo- or heterocycle, which carbo- or heterocycle is unsubstituted, or substituted with one or more R^{J}, and wherein said heterocycle comprises one or more, same or different heteroatoms O, N, or S; or
-C(CN)R^{X}R^{Y}, -C(R^{O})=N-NR^{M}R^{N}, -C(R^{O})=N-OR^{L}, or C₃-C₆-cycloalkyl, which is substituted with CN and which either does not have any further substituents, or which is further substituted with one or more R^{Z};
wherein at least one R⁷ is a group of formula (S) 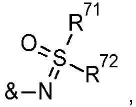
wherein each R⁷¹, R⁷² is independently selected from C₁-C₆-alkyl, C₃-C₆-cycloalkyl, C₂-C₆-alkenyl, C₃-C₆-cycloalkenyl, C₂-C₆-alkynyl, C₃-C₆-cycloalkyl-C₁-C₃-alkyl, which groups are unsubstituted, or substituted with one or more R^{G};
a 3- to 12-membered saturated, partially unsaturated, or fully unsaturated heterocyclic ring or ring system, wherein said heterocyclic ring or ring system comprises one or more, same or different heteroatoms O, N, or S, and is unsubstituted, or substituted with one or more R^{H};
phenyl, which is unsubstituted, or substituted with one or more R^{J};
or two substituents R⁷¹, R⁷² form, together with the sulfur atom to which they are bound, a 4-, 5-, or 6- membered saturated, partially unsaturated, or fully unsaturated heterocycle, which heterocycle is unsubstituted, or substituted with one or more R^{J}, and wherein said heterocycle comprises no, one or more, same or different heteroatoms O, N, or S in addition to the sulfur atom to which R⁷¹ and R⁷² are bound to;
each R^{A} is
independently H, C₁-C₆-alkyl, C₂-C₆-alkenyl, C₂-C₆-alkynyl, C₁-C₆-alkoxy-C₁-C₄-alkyl, C₃-C₆-cycloalkyl, C₃-C₆-cycloalkyl-Cₗ-C₄-alkyl, C₃-C₆-cycloalkoxy-C₁-C₄-alkyl, which groups are unsubstituted or substituted with halogen;
phenyl or benzyl, wherein the phenyl ring is unsubstituted or substituted with one or more R^{F};
each R^{B}
is independently H, C₁-C₆-alkyl, C₂-C₆-alkenyl, C₂-C₆-alkynyl, C₁-C₆-alkoxy-C₁-C₄-alkyl, C₃-C₆-cycloalkyl, C₃-C₆-cycloalkyl-Cₗ-C₄-alkyl, C₃-C₆-cycloalkoxy-C₁-C₄-alkyl, C₁-C₆-alkyl-carbonyl, C₁-C₆-alkoxy-carbonyl, which groups are unsubstituted or substituted with halogen;
phenyl or benzyl, which groups are unsubstituted or substituted with one or more R^{F};
each R^{C}
is independently H, C₁-C₆-alkyl, C₂-C₆-alkenyl, C₂-C₆-alkynyl, C₁-C₆-alkoxy-C₁-C₄-alkyl, C₃-C₆-cycloalkyl, C₃-C₆-cycloalkyl-Cₗ-C₄-alkyl, C₃-C₆-cycloalkoxy-C₁-C₄-alkyl, C₁-C₆-alkyl-carbonyl, or C₁-C₆-alkoxy-carbonyl, which groups are unsubstituted or substituted with halogen;
phenyl or benzyl, wherein the phenyl ring is unsubstituted or substituted with one or more R^{F};
each moiety NR^{B}R^{C} may also form an N-bound, saturated 5- to 8-membered heterocycle, which in addition to the nitrogen atom may have 1 or 2 further heteroatoms or heteroatom moieties selected from O, S(=O)ₘ and N-R', wherein R' is H or C₁-C₆-alkyl and wherein the N-bound heterocycle is unsubstituted or substituted with one or more, same or different substituents selected from halogen, C₁-C₄-alkyl, C₁-C₄-haloalkyl, C₁-C₄-alkoxy and C₁-C₄-haloalkoxy;
each R^{D}
is independently H, CN, OH, C₁-C₆-alkyl, C₂-C₆-alkenyl, C₂-C₆-alkynyl, C₁-C₆-alkoxy-C₁-C₄-alkyl, C₃-C₆-cycloalkyl,C₃-C₆-cycloalkyl-C₁-C₄-alkyl, C₃-C₆-cycloalkoxy-C₁-C₄-alkyl, which groups are unsubstituted or substituted with halogen;
phenyl or benzyl, wherein the phenyl ring is unsubstituted or substituted with one or more R^{F};
each R^{E}
is independently C₁-C₆-alkyl, C₃-C₆-cycloalkyl, C₃-C₆-cycloalkyl-C₁-C₄-alkyl, which are unsubstituted or substituted with halogen; or
phenyl or benzyl, wherein the phenyl ring is unsubstituted or substituted with one or more R^{F};
each R^{F} is independently halogen, N₃, OH, CN, NO₂, SCN, SF₃, C₁-C₆ alkyl, C₁-C₆-alkoxy, C₂-C₆-alkenyl, C₂-C₆-alkynyl, C₁-C₆-alkoxy-C₁-C₄ alkyl, C₁-C₆-alkoxy-C₁-C₄-alkoxy, C₃-C₆-cycloalkyl, C₃-C₆ cycloalkoxy, C₃-C₆-cycloalkyl-C₁-C₄ alkyl, C₃-C₆-cycloalkoxy-C₁-C₄ alkyl, which groups are unsubstituted or substituted with halogen;
each R^{G}
is independently halogen, OH, CN, NC, NO₂;
C₁-C₆-alkyl, C₃-C₆-cycloalkyl, C₃-C₆-cycloalkenyl, which groups are unsubstituted, or substituted with one or more, same or different substituents selected from halogen, OH, CN, C₁-C₃-alkoxy, C₁-C₃-haloalkoxy, and C₁-C₃-alkyl-carbonyl;
a 3- to 12-membered saturated, partially unsaturated, or fully unsaturated heterocyclic ring or ring system, wherein said heterocyclic ring or ring system comprises one or more, same or different heteroatoms O, N, or S, and is unsubstituted, or substituted with one or more, same or different substituents selected from halogen, OH, CN, C₁-C₃-alkoxy, C₁-C₃-haloalkoxy, and C₁-C₃-alkyl-carbonyl;
phenyl, which is unsubstituted, or substituted with one or more, same or different substituents selected from halogen, OH, CN, NO₂, C₁-C₃-alkyl, C₁-C₃-haloalkyl, C₁-C₃-alkoxy, C₁-C₃-haloalkoxy, and C₁-C₃-alkyl-carbonyl;
OR^{K}, SR^{K}, OC(=O)R^{K}, OC(=O)OR^{K}, OC(=O)NR^{L}R^{M}, OC(=O)SR^{K}, OC(=S)NR^{L}R^{M}, OC(=S)SR^{K}, OS(=O)ₘR^{K}, OS(=O)ₘNR^{L}R^{M}, ONR^{L}R^{M}, ON=CR^{N}R^{O}, NR^{L}R^{M}, NOR^{K}, ONR^{L}R^{M}, N=CR^{N}R^{O}, NNR^{L}, N(R^{L})C(=O)R^{K}, N(R^{L})C(=O)OR^{K}, S(=O)ₘR^{V}, SC(=O)SR^{K}, SC(=O)NR^{L}R^{M,} S(=O)ₘNR^{L}R^{M}, C(=O)R^{P}, C(=S)R^{P}, C(=O)NR^{L}R^{M}, C(=O)OR^{K}, C(=S)NR^{L}R^{M}, C(=S)OR^{K}, C(=S)SR^{K}, C(=NR^{L})R^{M}, C(=NR^{L})NR^{M}R^{R}, Si(R^{S})₂R^{T};
each R^{H}
is independently halogen, CN, NC, NO₂, SCN, NCS, NCO;
C₁-C₆-alkyl, C₃-C₆-cycloalkyl, C₃-C₆-cycloalkenyl, which groups are unsubstituted, or substituted with one or more, same or different substituents selected from halogen, OH, CN, C₁-C₃-alkoxy, C₁-C₃-haloalkoxy, and C₁-C₃-alkyl-carbonyl;
phenyl, which is unsubstituted, or substituted with one or more, same or different substituents selected from halogen, OH, CN, NO₂, C₁-C₃-alkyl, C₁-C₃-haloalkyl, OR^{K}, SR^{K}, OC(=O)R^{K}, OC(=O)OR^{K}, OC(=O)NR^{L}R^{M}, OC(=O)SR^{K}, OC(=S)NR^{L}R^{M}, OC(=S)SR^{K}, OS(=O)ₘR^{K}, OS(=O)ₘNR^{L}R^{M}, ONR^{L}R^{M}, ON=CR^{N}R^{O}, NR^{L}R^{M}, NOR^{K}, ONR^{L}R^{M}, N=CR^{N}R^{O}, NNR^{L}, N(R^{L})C(=O)R^{K}, N(R^{L})C(=O)OR^{K}, S(=O)ₘR^{V}, SC(=O)SR^{K}, SC(=O)NR^{L}R^{M,} S(=O)ₘNR^{L}R^{M}, C(=O)R^{P}, C(=S)R^{P}, C(=O)NR^{L}R^{M}, C(=O)OR^{K}, C(=S)NR^{L}R^{M}, C(=S)OR^{K}, C(=S)SR^{K}, C(=NR^{L})R^{M}, C(=NR^{L})NR^{M}R^{R}, Si(R^{S})₂R^{T}; or
two geminal substituents R^{H} form together with the atom to which they are bound a group =O, =S, or =NR^{L}.
each R^{J}
is independently halogen, CN, NC, NO₂, SCN, NCS, NCO;
C₁-C₆-alkyl, C₃-C₆-cycloalkyl, C₃-C₆-cycloalkenyl, which groups are unsubstituted, or substituted with one or more, same or different substituents selected from halogen, OH, CN, C₁-C₃-alkoxy, C₁-C₃-haloalkoxy, and C₁-C₃-alkyl-carbonyl;
phenyl, which is unsubstituted, or substituted with one or more, same or different substituents selected from halogen, OH, CN, NO₂, C₁-C₃-alkyl, C₁-C₃-haloalkyl, OR^{K}, SR^{K}, OC(=O)R^{K}, OC(=O)OR^{K}, OC(=O)NR^{L}R^{M}, OC(=O)SR^{K}, OC(=S)NR^{L}R^{M}, OC(=S)SR^{K}, OS(=O)ₘR^{K}, OS(=O)ₘNR^{L}R^{M}, ONR^{L}R^{M}, ON=CR^{N}R^{O}, NR^{L}R^{M}, NOR^{K}, ONR^{L}R^{M}, N=CR^{N}R^{O}, NNR^{L}, N(R^{L})C(=O)R^{K}, N(R^{L})C(=O)OR^{K}, S(=O)ₘR^{V}, SC(=O)SR^{K}, SC(=O)NR^{L}R^{M}, S(=O)ₘNR^{L}R^{M}, C(=O)R^{P}, C(=S)R^{P}, C(=O)NR^{L}R^{M}, C(=O)OR^{K}, C(=S)NR^{L}R^{M}, C(=S)OR^{K}, C(=S)SR^{K}, C(=NR^{L})R^{M}, C(=NR^{L})NR^{M}R^{R}, Si(R^{S})₂R^{T};
each R^{K}
is independently H;
C₁-C₆-alkyl, C₂-C₆-alkenyl, C₂-C₆-alkynyl, C₁-C₆-alkoxy-C₁-C₄-alkyl, C₃-C₆-cycloalkyl, C₃-C₆-cycloalkyl-C₁-C₄-alkyl, C₃-C₆-cycloalkoxy-C₁-C₄-alkyl, which groups are unsubstituted or substituted with one or more, same or different substituents selected from halogen, CN, NR^{L}R^{M}; C(=O)NR^{L}R^{M}, C(=O)R^{T}; or phenyl or benzyl, wherein the phenyl ring is unsubstituted or substituted with one or more R^{F};
each R^{L}, R^{R}
is independently H;
C₁-C₆-alkyl, C₂-C₆-alkenyl, C₂-C₆-alkynyl, C₁-C₆-alkoxy-C₁-C₄-alkyl, C₃-C₆-cycloalkyl, C₃-C₆-cycloalkyl-C₁-C₄-alkyl, C₃-C₆-cycloalkoxy-C₁-C₄-alkyl, which groups are unsubstituted or substituted with halogen;
phenyl and benzyl, which groups are unsubstituted or substituted with one or more R^{F};
each R^{M}
is independently H, C₁-C₆-alkyl, C₂-C₆-alkenyl, C₂-C₆-alkynyl, C₁-C₆-alkoxy-C₁-C₄-alkyl, C₃-C₆-cycloalkyl, C₃-C₆-cycloal kyl-C₁-C₄-alkyl, C₃-C₆-cycloalkoxy-C₁-C₄-alkyl, C₁-C₆-alkyl-carbonyl, C₁-C₆-alkoxy-carbonyl, which groups are unsubstituted or substituted with halogen;
C₁-C₆-alkylen-CN;
phenyl or benzyl, which groups are unsubstituted or substituted with one or more R^{F};
each R^{N}
is independently H, C₁-C₆-alkyl, C₂-C₆-alkenyl, C₂-C₆-alkynyl, C₁-C₆-alkoxy-C₁-C₄-alkyl, C₃-C₆-cycloalkyl, C₃-C₆-cycloalkyl-C₁-C₄-alkyl, C₃-C₆-cycloalkoxy-C₁-C₄-alkyl, C₁-C₆-alkyl-carbonyl, C₁-C₆-alkoxy-carbonyl, which groups are unsubstituted or substituted with halogen;
phenyl or benzyl, wherein the phenyl ring is unsubstituted or substituted with one or more R^{F};
each moiety NR^{M}R^{N}, R^{M}R^{R}, and R^{L}R^{M} may also form an N-bound, saturated 5- to 8-membered heterocycle, which in addition to the nitrogen atom may have 1 or 2 further heteroatoms or heteroatom moieties selected from O, S(=O)ₘ and N-R", wherein R" is H or C₁-C₆-alkyl and wherein the N-bound heterocycle is unsubstituted or substituted with one or more, same or different substituents selected from halogen, C₁-C₄-alkyl, C₁-C₄-haloalkyl, C₁-C₄-alkoxy and C₁-C₄-haloalkoxy;
each R^{O}
is independently H, CN, OH, C₁-C₆-alkyl, C₂-C₆-alkenyl, C₂-C₆-alkynyl, C₁-C₆-alkoxy-C₁-C₄-alkyl, C₃-C₆-cycloalkyl, C₃-C₆-cycloalkyl-C₁-C₄-alkyl, C₃-C₆-cyclo-alkoxy-C₁-C₄-alkyl, which groups are unsubstituted or substituted with halogen;
phenyl or benzyl, wherein the phenyl ring is unsubstituted or substituted with one or more R^{F};
each R^{P}
is independently H;
C₁-C₆-alkyl, C₂-C₆-alkenyl, C₂-C₆-alkynyl, C₁-C₆-alkoxy-C₁-C₄-alkyl, C₃-C₆-cycloalkyl, C₃-C₆-cycloalkyl-C₁-C₄-alkyl, C₃-C₆-cycloalkoxy-C₁-C₄-alkyl, which groups are unsubstituted or substituted with halogen;
phenyl or benzyl, wherein the phenyl ring is unsubstituted or substituted with one or more R^{F};
each R^{S}, R^{T} is independently H, C₁-C₆-alkyl, C₁-C₆-haloalkyl, C₁-C₆-alkoxy, C₁-C₄-alkoxy-C₁-C₄-alkyl, C₃-C₆-cycloalkyl, C₃-C₆-halocycloalkyl, C₁-C₄-haloalkoxy-C₁-C₄-alkyl, or phenyl;
each R^{V}
is indepentently C₁-C₆-alkyl, C₃-C₆-cycloalkyl, C₃-C₆-cycloalkyl-C₁-C₄-alkyl, which are unsubstituted or substituted with halogen; or
phenyl or benzyl, wherein the phenyl ring is unsubstituted or substituted with R^{F};
each R^{W} is independently H, C₁-C₆-alkyl, C₃-C₆-cycloalkyl, C₃-C₆-cycloalkyl-C₁-C₄-alkyl, which groups are halogenated or non-halogenated; benzyl, or phenyl, which is unsubstituted or substituted with R^{F};
each R^{X}, R^{Y} is independently H, halogen, CN, C₄-C₆-alkyl, C₁-C₆-haloalkyl, C₃-C₆-cycloalkyl, C₁-C₄-alkoxy, C₁-C₄-alkoxy-C₁-C₄-alkyl, C₁-C₄-alkylsulfanyl, C₁-C₄-alkyl-sulfanyl-C₁-C₄-alkyl, C₁-C₄-alkylsulfinyl-C₁-C₄-alkyl, C₁-C₄-alkylsulfonyl-C₁-C₄-alkyl, or C₁-C₄-alkoxycarbonyl;
each R^{Z}
is halogen, CN, NH₂, C(=O)H, OH, C₃-C₆-cycloalkyl, C(=O)OH, C(=O)NH₂, C₁-C₄-haloalkoxy, C₁-C₄-alkoxy, C₁-C₄-haloalkylsulfanyl, C₁-C₄-haloalkylsulfinyl, C₁-C₄-haloalkylsulfonyl, C₁-C₄-alkoxycarbonyl, C₁-C₄-haloalkoxycarbonyl, C₁-C₄-alkylcarbonyl, C₁-C₄-haloalkylcarbonyl, di-(C₁-C₄)alkylaminocarbonyl, C₁-C₄-alkylaminocarbonyl, C₁-C₄-alkylcarbonylamino, di-(C₁-C₄)alkylcarbonylamino, C₁-C₄-alkoxycarbonylamino, or a group -C(R^{Z1})=NOR^{Z2};
phenyl, which is unsubstituted or substituted with one or more, same or different substituents selected from halogen, CN, C₁-C₄-alkyl, C₁-C₄-haloalkyl, C₁-C₄-haloalkoxy, C₁-C₄-alkoxy, C₁-C₄-haloalkylsulfanyl, C₁-C₄-haloalkylsulfinyl, C₁-C₄haloalkylsulfonyl and C(=O)C₁-C₄-haloalkyl; C₁-C₄-alkyl which is unsubstituted or substituted with one or more R^{Z3};
R^{Z1} and R^{Z2} are independently H, C₁-C₄-alkyl, or C₁-C₄-haloalkyl;
R^{Z3} is halogen, CN, NH₂, C(=O)H, OH, C₃-C₆-cycloalkyl, hydroxycarbonyl, aminocarbonyl, C₁-C₄-haloalkoxy, C₁-C₄-alkoxy, C₁-C₄-haloalkylsulfanyl, C₁-C₄-haloalkylsulfinyl, C₁-C₄-haloalkylsulfonyl, C₁-C₄-alkoxycarbonyl, C₁-C₄-haloalkoxycarbonyl, C₄-C₄-alkylcarbonyl, C₁-C₄-haloalkylcarbonyl, di-(C₁-C₄)alkylaminocarbonyl, C₁-C₄alkylaminocarbonyl, C₁-C₄-alkylcarbonylamino, di-(C₁-C₄)alkylcarbonylamino, C₁-C₄-alkoxycarbonylamino, a group -C(R^{Z1})=NOR^{Z2};
the index n is 1, 2, 3, or 4 if G is phenyl or a 6-membered hetaryl;
or 1, 2, or 3 if X is a 5-membered hetaryl; and
the index m is 0, 1 or 2;
and the N-oxides, stereoisomers, tautomers and agriculturally or veterinarily acceptable salts thereof.

2. The compound of formula (I) according to claim 1, wherein
R¹ is H, C₁-C₃-alkyl, or C₁-C₃-alkoxy, which groups are unsubstituted or halogenated;
R² is H, halogen, C₁-C₃-alkyl, C₁-C₃-alkoxy, C₂-C₃-alkenyl, or C₂-C₃-alkynyl, which groups are unsubstituted or halogenated.

3. The compound of formula (I) according to claim 1 or 2, wherein the compound of formula
(I) is a compound of formula (I.A), (I.B) or (I.C); 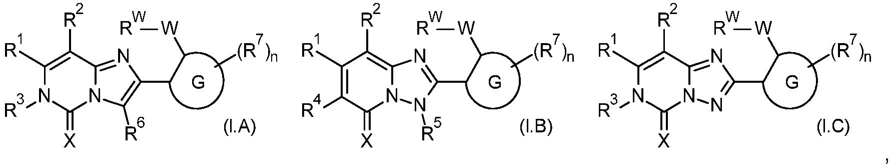 wherein all variables have a meaning as defined for formula (I).

4. The compound of formula (I.A) according to claim 3, wherein
R³ C₁-C₄ -alkyl, C₂-C₄ alkenyl, C₂-C₄ alkynyl, C₁-C₃-alkoxy-C₁-C₃-alkyl, C₃-C₆ cycloalkyl, C₃-C₆ cycloalkyl-C₁-C₂-alkyl, which groups are unsubstituted or halogenated;
R⁶ is H, C₁-C₃-alkyl or C₁-C₃-haloalkyl.

5. The compound of formula (I.B) according to claim 3 or 4, wherein
R⁴ is H, or C₁-C₃ alkyl, or C₁-C₃-haloalkyl;
R⁵ is C₁-C₃-alkyl, or C₁-C₃-haloalkyl.

6. The compound of formula (I) according to any of claims 1 to 5, wherein G is pyridyl.

7. The compound of formula (I) according to any of claims 1 to 6, wherein
each R^{W} is independently C₁-C₃-alkyl or C₁-C₃-haloalkyl, and
m is 0 or 2.

8. The compound of formula (I) according to any of claims 1 to 7, wherein the index n is 1 or 2.

9. A pesticidal mixture comprising the compound of formula (I), as defined in any of claims 1 to 8, an N-oxide or an agriculturally acceptable salt thereof and a further pesticidal ingredient.

10. Use of compounds of formula (I) as defined in any of claims 1 to 8 as an agrochemical pesticide.

11. A compound of the formula (I) as defined in any of claims 1 to 8, or or a composition as defined in claim 9 for use in a method for combating or controlling invertebrate pests, which method comprises contacting said pest or its food supply, habitat or breeding grounds with a pesticidally effective amount of at least one compound of the formula (I) according to any of claims 1 to 8 or the composition according to claim 9.

12. A method for protecting growing plants from attack or infestation by invertebrate pests, which method comprises contacting a plant, or soil or water in which the plant is growing, with a pesticidally effective amount of at least one compound of the formula (I), according to any of the claims 1 to 8 or the composition according to claim 9.

13. Seed comprising a compound of the formula (I), as defined in any of claims 1 to 8, or the composition, as defined in claim 9, in an amount of from 0.1 g to 10 kg per 100 kg of seed.

14. Use of a compound of the formula (I), as defined in any of the claims 1 to 8, or of the compositions as defined in claim 9, for protecting growing plants from attack or infestation by invertebrate pests.

15. A compound of the formula (I) as defined in any of claims 1 to 8, or or a composition as defined in claim 9 for use in a method for treating or protecting an animal from infestation or infection by invertebrate pests which comprises bringing the animal in contact with a pesticidally effective amount of a compound of the formula (I) as defined in any of claims 1 to 8, or or a composition as defined in claim 9.

## Patentansprüche

1. Verbindung der Formel (I) 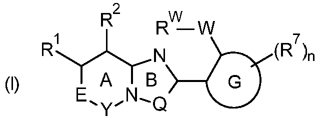 wobei
die Ringe A und B vollständig ungesättigt sind;
Y für C=X steht, wobei X für O oder S steht;
E für N(R³) oder C(R⁴) steht;
Q für N, N(R⁵) oder C(R⁶) steht;
G für Phenyl oder ein 5- oder 6-gliedriges Heteroaryl steht;
W für S, S(O), S(O)₂ oder S(O) (NR^{W}) steht;
R¹ für H, Halogen, C₁-C₆-Alkyl, C₂-C₆-Alkenyl, C₂-C₆-Alkinyl, C₁-C₆-Alkoxy, C₁-C₆-Alkoxy-C₁-C₆-alkyl, C₃-C₆-Cycloalkyl, C₃-C₆-Cycloalkoxy, C₁-C₆-Alkylsulfenyl, C₁-C₆-Alkylsulfinyl oder C₁-C₆-Alkylsulfonyl steht, wobei diese Gruppen unsubstituiert oder halogeniert sind;
R³, R⁵ unabhängig für Folgendes stehen: C₁-C₆-Alkyl, C₁-C₆-Alkoxy, C₂-C₆-Alkenyl, C₂-C₆-Alkinyl, C₁-C₆-Alkoxy-C₁-C₄-alkyl, C₁-C₆-Alkoxy-C₁-C₄-alkoxy, C₃-C₆-Cycloalkyl, C₃-C₆-Cycloalkyl-C₁-C₄-alkyl, C₃-C₆-Cycloalkoxy-C₁-C₄-alkyl, wobei diese Gruppen unsubstituiert oder halogeniert sind;
C(=O)OR^{A}, NR^{B}R^{C}, C₁-C₆-Alkylen-NR^{B}R^{C}, O-C₁-C₆-Alkylen-NR^{B}R^{C}, C₁-C₆-Alkylen-CN, NH-C₁-C₆-Alkylen-NR^{B}R^{C}, C (=O) NR^{B}R^{C}, C(=O)R^{D}, C(=S)R^{D}, SO₂NR^{B}R^{C}, S(=O)ₘR^{E};
Phenyl oder Benzyl, wobei der Phenylring unsubstituiert oder durch ein oder mehrere R^{F} substituiert ist;
R², R⁴, R⁶ unabhängig für Folgendes stehen: H, Halogen, N₃, CN, NO₂, -SCN, -SF₅, C₁-C₆-Alkyl, C₁-C₆-Alkoxy, C₂-C₆-Alkenyl, Tri-C₁-C₆-alkylsilyl, C₂-C₆-Alkinyl, C₁-C₆-Alkoxy-C₁-C₄-alkyl, C₁-C₆-Alkoxy-C₁-C₄-alkoxy, C₃-C₆-Cycloalkyl, C₃-C₆-Cycloalkoxy, C₃-C₆-Cycloalkyl-C₁-C₄-alkyl, C₃-C₆-Cycloalkoxy-C₁-C₄-alkyl, wobei diese Gruppen unsubstituiert oder durch Halogen substituiert sind, C(=O)OR^{A}, NR^{B}R^{C}, NOR^{A}, ONR^{B}R^{C}, C₁-C₆-Alkylen-NR^{B}R^{C}, O-C₁-C₆-Alkylen-NR^{B}R^{C}, C₁-C₆-Alkylen-CN, NH-C₁-C₆-Alkylen-NR^{B}R^{C}, C (=O) NR^{B}R^{C}, C(=O)R^{D}, C(=S)R^{D}, SO₂NR^{B}R^{C}, S(=O)ₙ,R^{E};
Phenyl oder Benzyl, wobei der Phenylring unsubstituiert oder durch ein oder mehrere R^{F} substituiert ist;
R⁷ jeweils unabhängig für Folgendes steht: H, Halogen, OH, CN, NC, NO₂, N₃, SCN, NCS, NCO, SF₅;
C₁-C₆-Alkyl, C₃-C₆-Cycloalkyl, C₂-C₆-Alkenyl, C₃-C₆-Cycloalkenyl, C₂-C₆-Alkinyl, C₃-C₆-Cycloalkyl-C₁-C₃-alkyl, wobei diese Gruppen unsubstituiert oder durch ein oder mehrere R^{G} substituiert sind;
einen 3- bis 12-gliedrigen gesättigten, teilweise ungesättigten oder vollständig ungesättigten heterocyclischen Ring oder ein 3- bis 12-gliedriges gesättigtes, teilweise ungesättigtes oder vollständig ungesättigtes heterocyclisches Ringsystem, wobei der heterocyclische Ring bzw. das heterocyclische Ringsystem ein oder mehrere, gleiche oder verschiedene Heteroatome O, N oder S umfasst und unsubstituiert oder durch ein oder mehrere R^{H} substituiert ist;
Phenyl, das unsubstituiert oder durch ein oder mehrere R^{J} substituiert ist;
OR^{K}, SR^{K}, OC(=O)R^{K}, OC(=O)OR^{K}, OC(=O)NR^{L}R^{M}, OC(=O)SR^{K}, OC(=S)NR^{L}R^{M}, OC(=S)SR^{K}, OS(=O)ₘR^{K}, OS(=O)ₘNR^{L}R^{M}, ONR^{L}R^{M}, ON=CR^{N}R^{O}, NR^{L}R^{M}, NOR^{K}, ONR^{L}R^{M}, N=CR^{N}R^{O}, NNR^{L}, N(R^{L})C(=O)R^{K}, N(R^{L})C(=O)OR^{K}, S(=O)ₘR^{V}, SC(=O)SR^{K}, SC(=O)NR^{L}R^{M}, S(=O)ₘNR^{L}R^{M}, C(=O)R^{P}, C(=S)R^{P}, C(=O)NR^{L}R^{M}, C(=O)OR^{K}, C(=S)NR^{L}R^{M}, C(=S)OR^{K}, C(=S)SR^{K}, C(=NR^{L})R^{M}, C(=NR^{L})NR^{M}R^{R}, Si(R^{S})₂R^{T};
oder zwei Substituenten R⁷ zusammen mit den Ringgliedern von Ring G, an die sie gebunden sind, einen 5- oder 6-gliedrigen gesättigten, teilweise ungesättigten oder vollständig ungesättigten Carbo- oder Heterocyclus bilden, wobei der Carbo- oder Heterocyclus unsubstituiert oder durch ein oder mehrere R^{J} substituiert ist und wobei der Heterocyclus ein oder mehrere, gleiche oder verschiedene Heteroatome O, N oder S umfasst; oder -C(CN)R^{X}R^{Y}, -C(R^{O})=N-NR^{M}R^{N}, -C(R^{O})=N-OR^{L}oder C₃-C₆-Cycloalkyl, das durch CN substituiert ist und das entweder keine weiteren Substituenten aufweist oder das weiter durch ein oder mehrere R^{z} substituiert ist;
wobei mindestens ein R⁷ für eine Gruppe der Formel (S) steht: 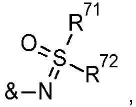
wobei R⁷¹, R⁷² jeweils unabhängig ausgewählt sind aus C₁-C₆-Alkyl, C₃-C₆-Cycloalkyl, C₂-C₆-Alkenyl, C₃-C₆-Cycloalkenyl, C₂-C₆-Alkinyl, C₃-C₆-Cycloalkyl-C₁-C₃-alkyl, wobei diese Gruppen unsubstituiert oder durch ein oder mehrere R^{G} substituiert sind;
einen 3- bis 12-gliedrigen gesättigten, teilweise ungesättigten oder vollständig ungesättigten heterocyclischen Ring oder ein 3- bis 12-gliedriges gesättigtes, teilweise ungesättigtes oder vollständig ungesättigtes heterocyclisches Ringsystem, wobei der heterocyclische Ring bzw. das heterocyclische Ringsystem ein oder mehrere, gleiche oder verschiedene Heteroatome O, N oder S umfasst und unsubstituiert oder durch ein oder mehrere R^{H} substituiert ist;
Phenyl, das unsubstituiert oder durch ein oder mehrere R^{J} substituiert ist;
oder zwei Substituenten R⁷¹, R⁷² zusammen mit dem Schwefelatom, an das sie gebunden sind, einen 4-, 5- oder 6-gliedrigen gesättigten, teilweise ungesättigten oder vollständig ungesättigten Heterocyclus bilden, wobei der Heterocyclus unsubstituiert oder durch ein oder mehrere R^{J} substituiert ist und wobei der Heterocyclus neben dem Schwefelatom, an das R⁷¹ und R⁷² gebunden sind, kein, ein oder mehrere, gleiche oder verschiedene Heteroatome O, N oder S umfasst;
R^{A} jeweils unabhängig für Folgendes steht: H, C₁-C₆-Alkyl, C₂-C₆-Alkenyl, C₂-C₆-Alkinyl, C₁-C₆-Alkoxy-C₁-C₄-alkyl, C₃-C₆-Cycloalkyl, C₃-C₆-Cycloalkyl-C₁-C₄-alkyl, C₃-C₆-Cycloalkoxy-C₁-C₄-alkyl, wobei diese Gruppen unsubstituiert oder durch Halogen substituiert sind; Phenyl oder Benzyl, wobei der Phenylring unsubstituiert oder durch ein oder mehrere R^{F} substituiert ist;
R^{B} jeweils unabhängig für Folgendes steht: H, C₁-C₆-Alkyl, C₂-C₆-Alkenyl, C₂-C₆-Alkinyl, C₁-C₆-Alkoxy-C₁-C₄-alkyl, C₃-C₆-Cycloalkyl, C₃-C₆-Cycloalkyl-C₁-C₄-alkyl, C₃-C₆-Cycloalkoxy-C₁-C₄-alkyl, C₁-C₆-Alkyl-carbonyl, C₁-C₆-Alkoxy-carbonyl, wobei diese Gruppen unsubstituiert oder durch Halogen substituiert sind;
Phenyl oder Benzyl, wobei diese Gruppen unsubstituiert oder durch ein oder mehrere R^{F} substituiert sind;
R^{C} jeweils unabhängig für Folgendes steht: H, C₁-C₆-Alkyl, C₂-C₆-Alkenyl, C₂-C₆-Alkinyl, C₁-C₆-Alkoxy-C₁-C₄-alkyl, C₃-C₆-Cycloalkyl, C₃-C₆-Cycloalkyl-C₁-C₄-alkyl, C₃-C₆-Cycloalkoxy-C₁-C₄-alkyl, C₁-C₆-Alkyl-carbonyl, C₁-C₆-Alkoxy-carbonyl, wobei diese Gruppen unsubstituiert oder durch Halogen substituiert sind;
Phenyl oder Benzyl, wobei der Phenylring unsubstituiert oder durch ein oder mehrere R^{F} substituiert ist;
jede Gruppierung NR^{B}R^{C} auch einen N-gebundenen, gesättigten 5- bis 8-gliedrigen Heterocyclus bilden kann, der zusätzlich zu dem Stickstoffatom 1 oder 2 weitere Heteroatome bzw. Heteroatomgruppierungen, die aus O, S(=O)ₘ und N-R' ausgewählt sind, aufweisen kann, wobei R' für H oder C₁-C₆-Alkyl steht, und wobei der N-gebundene Heterocyclus unsubstituiert oder durch einen oder mehrere, gleiche oder verschiedene Substituenten, die aus Halogen, C₁-C₄-Alkyl, C₁-C₄-Halogenalkyl, C₁-C₄-Alkoxy und C₁-C₄-Halogenalkoxy ausgewählt sind, substituiert ist;
R^{D} jeweils unabhängig für Folgendes steht: H, CN, OH, C₁-C₆-Alkyl, C₂-C₆-Alkenyl, C₂-C₆-Alkinyl, C₁-C₆-Alkoxy-C₁-C₄-alkyl, C₃-C₆-Cycloalkyl, C₃-C₆-Cycloalkyl-C₁-C₄-alkyl, C₃-C₆-Cycloalkoxy-C₁-C₄-alkyl, wobei diese Gruppen unsubstituiert oder durch Halogen substituiert sind; Phenyl oder Benzyl, wobei der Phenylring unsubstituiert oder durch ein oder mehrere R^{F} substituiert ist;
R^{E} jeweils unabhängig für Folgendes steht: C₁-C₆-Alkyl, C₃-C₆-Cycloalkyl, C₃-C₆-Cycloalkyl-C₁-C₄-alkyl, wobei diese Gruppen unsubstituiert oder durch Halogen substituiert sind; oder
Phenyl oder Benzyl, wobei der Phenylring unsubstituiert oder durch ein oder mehrere R^{F} substituiert ist;
R^{F} jeweils unabhängig für Halogen, N₃, OH, CN, NO₂, SCN, SF₅, C₁-C₆-Alkyl, C₁-C₆-Alkoxy, C₂-C₆-Alkenyl, C₂-C₆-Alkinyl, C₁-C₆-Alkoxy-C₁-C₄ alkyl, C₁-C₆-Alkoxy-C₁-C₄-alkoxy, C₃-C₆-Cycloalkyl, C₃-C₆-Cycloalkoxy, C₃-C₆-Cycloalkyl-C₁-C₄ alkyl, C₃-C₆-Cycloalkoxy-C₁-C₄ alkyl steht, wobei diese Gruppen unsubstituiert oder durch Halogen substituiert sind;
R^{G} unabhängig für Folgendes steht: Halogen, OH, CN, NC, NO₂;
C₁-C₆-Alkyl, C₃-C₆-Cycloalkyl, C₃-C₆-Cycloalkenyl, wobei diese Gruppen unsubstituiert oder durch einen oder mehrere, gleiche oder verschiedene Substituenten, die aus Halogen, OH, CN, C₁-C₃-Alkoxy, C₁-C₃-Halogenalkoxy und C₁-C₃-Alkylcarbonyl ausgewählt sind, substituiert sind; einen 3- bis 12-gliedrigen gesättigten, teilweise ungesättigten oder vollständig ungesättigten heterocyclischen Ring oder ein 3- bis 12-gliedriges gesättigtes, teilweise ungesättigtes oder vollständig ungesättigtes heterocyclisches Ringsystem, wobei der heterocyclische Ring bzw. das heterocyclische Ringsystem ein oder mehrere, gleiche oder verschiedene Heteroatome O, N oder S umfasst und unsubstituiert oder durch einen oder mehrere, gleiche oder verschiedene Substituenten, die aus Halogen, OH, CN, C₁-C₃-Alkoxy, C₁-C₃-Halogenalkoxy und C₁-C₃-Alkylcarbonyl ausgewählt sind, substituiert ist;
Phenyl, das unsubstituiert oder durch einen oder mehrere, gleiche oder verschiedene Substituenten, die aus Halogen, OH, CN, NO₂, C₁-C₃-Alkyl, C₁-C₃-Halogenalkyl, C₁-C₃-Alkoxy, C₁-C₃-Halogenalkoxy und C₁-C₃-Alkylcarbonyl ausgewählt sind, substituiert ist;
OR^{K}, SR^{K}, OC(=O)R^{K}, OC(=O)OR^{K}, OC(=O)NR^{L}R^{M}, OC(=O)SR^{K}, OC(=S)NR^{L}R^{M}, OC(=S)SR^{K}, OS(=O)ₘR^{K}, OS(=O)ₘNR^{L}R^{M}, ONR^{L}R^{M}, ON=CR^{N}R^{O}, NR^{L}R^{M}, NOR^{K}, ONR^{L}R^{M}, N=CR^{N}R^{O}, NNR^{L}, N(R^{L})C(=O)R^{K}, N(R^{L})C(=O)OR^{K}, S(=O)ₘR^{V}, SC(=O)SR^{K}, SC(=O)NR^{L}R^{M}, S(=O)ₘNR^{L}R^{M}, C(=O)R^{P}, C(=S)R^{P}, C(=O)NR^{L}R^{M}, C(=O)OR^{K}, C(=S)NR^{L}R^{M}, C(=S)OR^{K}, C(=S)SR^{K}, C(=NR^{L})R^{M}, C(=NR^{L})NR^{M}R^{R}, Si(R^{S})₂R^{T};
R^{H} jeweils unabhängig für Folgendes steht: Halogen, CN, NC, NO₂, SCN, NCS, NCO;
C₁-C₆-Alkyl, C₃-C₆-Cycloalkyl, C₃-C₆-Cycloalkenyl, wobei diese Gruppen unsubstituiert oder durch einen oder mehrere, gleiche oder verschiedene Substituenten, die aus Halogen, OH, CN, C₁-C₃-Alkoxy, C₁-C₃-Halogenalkoxy und C₁-C₃-Alkylcarbonyl ausgewählt sind, substituiert sind; Phenyl, das unsubstituiert oder durch einen oder mehrere, gleiche oder verschiedene Substituenten substituiert ist, die aus Halogen, OH, CN, NO₂, C₁-C₃-alkyl, C₁-C₃-haloalkyl, OR^{K}, SR^{K}, OC(=O)R^{K}, OC(=O)OR^{K}, OC(=O)NR^{L}R^{M}, OC(=O)SR^{K}, OC(=S)NR^{L}R^{M}, OC(=S)SR^{K}, OS(=O)ₘR^{K}, OS(=O)ₘNR^{L}R^{M}, ONR^{L}R^{M}, ON=CR^{N}R^{O}, NR^{L}R^{M}, NOR^{K}, ONR^{L}R^{M}, N=CR^{N}R^{O}, NNR^{L}, N(R^{L})C(=O)R^{K}, N(R^{L})C(=O)OR^{K}, S(=O)_{mR}^{V}, SC(=O)SR^{K}, SC(=O)NR^{L}R^{M}, S(=O)ₘNR^{L}R^{M}, C(=O)R^{P}, C(=S)R^{P}, C(=O)NR^{L}R^{M}, C(=O)OR^{K}, C(=S)NR^{L}R^{M}, C(=S)OR^{K},C(=S)SR^{K}, C (=NR^{L})R^{M}, C(=NR^{L})NR^{M}R^{R}, Si(R^{S})₂R^{T} ausgewählt sind; oder
zwei geminale Substituenten R^{H} zusammen mit dem Atom, an das sie gebunden sind, eine Gruppe =O, =S oder =NR^{L} bilden;
R^{J} jeweils unabhängig für Folgendes steht: Halogen, CN, NC, NO₂, SCN, NCS, NCO;
C₁-C₆-Alkyl, C₃-C₆-Cycloalkyl, C₃-C₆-Cycloalkenyl, wobei diese Gruppen unsubstituiert oder durch einen oder mehrere, gleiche oder verschiedene Substituenten, die aus Halogen, OH, CN, C₁-C₃-Alkoxy, C₁-C₃-Halogenalkoxy und C₁-C₃-Alkylcarbonyl ausgewählt sind, substituiert sind; Phenyl, das unsubstituiert oder durch einen oder mehrere, gleiche oder verschiedene Substituenten substituiert ist, die aus Halogen, OH, CN, NO₂, C₁-C₃-alkyl, C₁-C₃-haloalkyl, OR^{K}, SR^{K}, OC(=O)R^{K}, OC(=O)OR^{K}, OC(=O)NR^{L}R^{M}, OC(=O)SR^{K}, OC(=S)NR^{L}R^{M}, OC(=S) SR^{K}, OS(=O)R^{K}, OS(=O)ₘNR^{L}R^{M}, ONR^{L}R^{M}, ON=CR^{N}R^{O}, NR^{L}R^{M}, NOR^{K}, ONR^{L}R^{M}, N=CR^{N}R^{O}, NNR^{L}, N(R^{L})C(=O)R^{K}, N(R^{L})C(=O)OR^{K}, S(=O)ₘR^{V}, SC(=O)SR^{K}, SC (=O) NR^{L}R^{M}, S(=O)ₘNR^{L}R^{M}, C(=O)R^{P}, C(=S)R^{P}, C(=O)NR^{L}R^{M}, C(=O)OR^{K}, C(=S)NR^{L}R^{M}, C(=S)OR^{K}, C(=S)SR^{K}, C(=NR^{L})R^{M}, C(=NR^{L})NR^{M}R^{R}, Si(R^{S})₂R^{T} ausgewählt sind;
R^{K} jeweils unabhängig für Folgendes steht: H; C₁-C₆-Alkyl, C₂-C₆-Alkenyl, C₂-C₆-Alkinyl, C₁-C₆-Alkoxy-C₁-C₄-alkyl, C₃-C₆-Cycloalkyl, C₃-C₆-Cycloalkyl-C₁-C₄-alkyl, C₃-C₆-Cycloalkoxy-C₁-C₄-alkyl, wobei diese Gruppen unsubstituiert oder durch einen oder mehrere, gleiche oder verschiedene Substituenten, die aus Halogen, CN, NR^{L}R^{M}; C(=O)NR^{L}R^{M}, C(=O)R^{T} ausgewählt sind, substituiert sind; oder Phenyl oder Benzyl, wobei der Phenylring unsubstituiert oder durch ein oder mehrere R^{F} substituiert ist;
R^{L}, R^{R} jeweils unabhängig für Folgendes steht: H; C₁-C₆-Alkyl, C₂-C₆-Alkenyl, C₂-C₆-Alkinyl, C₁-C₆-Alkoxy-C₁-C₄-alkyl, C₃-C₆-Cycloalkyl, C₃-C₆-Cycloalkyl-C₁-C₄-alkyl, C₃-C₆-Cycloalkoxy-C₁-C₄-alkyl, wobei diese Gruppen unsubstituiert oder durch Halogen substituiert sind; Phenyl und Benzyl, wobei diese Gruppen unsubstituiert oder durch ein oder mehrere R^{F} substituiert sind;
R^{M} jeweils unabhängig für Folgendes steht: H, C₁-C₆-Alkyl, C₂-C₆-Alkenyl, C₂-C₆-Alkinyl, C₁-C₆-Alkoxy-C₁-C₄-alkyl, C₃-C₆-Cycloalkyl, C₃-C₆-Cycloalkyl-C₁-C₄-alkyl, C₃-C₆-Cycloalkoxy-C₁-C₄-alkyl, C₁-C₆-Alkyl-carbonyl, C₁-C₆-Alkoxy-carbonyl, wobei diese Gruppen unsubstituiert oder durch Halogen substituiert sind;
C₁-C₆-Alkylen-CN;
Phenyl oder Benzyl, wobei diese Gruppen unsubstituiert oder durch ein oder mehrere R^{F} substituiert sind;
R^{N} jeweils unabhängig für Folgendes steht: H, C₁-C₆-Alkyl, C₂-C₆-Alkenyl, C₂-C₆-Alkinyl, C₁-C₆-Alkoxy-C₁-C₄-alkyl, C₃-C₆-Cycloalkyl, C₃-C₆-Cycloalkyl-C₁-C₄-alkyl, C₃-C₆-Cycloalkoxy-C₁-C₄-alkyl, C₁-C₆-Alkyl-carbonyl, C₁-C₆-Alkoxy-carbonyl, wobei diese Gruppen unsubstituiert oder durch Halogen substituiert sind;
Phenyl oder Benzyl, wobei der Phenylring unsubstituiert oder durch ein oder mehrere R^{F} substituiert ist;
jede Gruppierung NR^{M}R^{N}, R^{M}R^{R} und R^{L}R^{M} auch einen N-gebundenen, gesättigten 5- bis 8-gliedrigen Heterocyclus bilden kann, der zusätzlich zu dem Stickstoffatom 1 oder 2 weitere Heteroatome bzw. Heteroatomgruppierungen, die aus O, S(=O)ₘ und N-R" ausgewählt sind, aufweisen kann, wobei R" für H oder C₁-C₆-Alkyl steht, und wobei der N-gebundene Heterocyclus unsubstituiert oder durch einen oder mehrere, gleiche oder verschiedene Substituenten, die aus Halogen, C₁-C₄-Alkyl, C₁-C₄-Halogenalkyl, C₁-C₄-Alkoxy und C₁-C₄-Halogenalkoxy ausgewählt sind, substituiert ist;
R^{O} steht jeweils unabhängig für H, CN, OH, C₁-C₆-Alkyl, C₂-C₆-Alkenyl, C₂-C₆-Alkinyl, C₁-C₆-Alkoxy-C₁-C₄-alkyl, C₃-C₆-Cycloalkyl, C₃-C₆-Cycloalkyl-C₁-C₄-alkyl, C₃-C₆-Cycloalkoxy-C₁-C₄-alkyl, wobei diese Gruppen unsubstituiert oder durch Halogen substituiert sind; Phenyl oder Benzyl, wobei der Phenylring unsubstituiert oder durch ein oder mehrere R^{F} substituiert ist;
R^{P} jeweils unabhängig für Folgendes steht: H; C₁-C₆-Alkyl, C₂-C₆-Alkenyl, C₂-C₆-Alkinyl, C₁-C₆-Alkoxy-C₁-C₄-alkyl, C₃-C₆-Cycloalkyl, C₃-C₆-Cycloalkyl-C₁-C₄-alkyl, C₃-C₆-Cycloalkoxy-C₁-C₄-alkyl, wobei diese Gruppen unsubstituiert oder durch Halogen substituiert sind; Phenyl oder Benzyl, wobei der Phenylring unsubstituiert oder durch ein oder mehrere R^{F} substituiert ist;
R^{S}, R^{T} jeweils unabhängig für H, C₁-C₆-Alkyl, C₁-C₆-Halogenalkyl, C₁-C₆-Alkoxy, C₁-C₄-Alkoxy-C₁ -C₄ -alkyl, C₃-C₆-Cycloalkyl, C₃-C₆-Halogencycloalkyl, C₁-C₄-Halogenalkoxy-C₁-C₄-alkyl oder Phenyl stehen;
R^{V} jeweils unabhängig für Folgendes steht: C₁-C₆-Alkyl, C₃-C₆-Cycloalkyl, C₃-C₆-Cycloalkyl-C₁-C₄-alkyl, wobei diese Gruppierungen unsubstituiert oder durch Halogen substituiert sind,
Phenyl oder Benzyl, wobei der Phenylring unsubstituiert oder durch R^{F} substituiert ist;
R^{W} jeweils unabhängig für Folgendes steht: H, C₁-C₆-Alkyl, C₃-C₆-Cycloalkyl, C₃-C₆-Cycloalkyl-C₁-C₄-alkyl, wobei diese Gruppen halogeniert oder nicht halogeniert sind; Benzyl oder Phenyl, das unsubstituiert oder durch R^{F} substituiert ist;
R^{X}, R^{Y} jeweils unabhängig für H, Halogen, CN, C₁-C₆-Alkyl, C₁-C₆-Halogenalkyl, C₃-C₆-Cyclo-alkyl, C₁-C₄-Alkoxy, C₁-C₄-Alkoxy-C₁-C₄-alkyl, C₁-C₄-Alkylsulfanyl, C₁-C₄-Alkylsulfanyl-C₁-C₄-alkyl, C₁-C₄-Alkylsulfinyl-C₁-C₄-alkyl, C₁-C₄-Alkylsulfonyl-C₁-C₄-alkyl oder C₁-C₄-Alkoxycarbonyl stehen;
R^{Z} jeweils für Folgendes steht: Halogen, CN, NH₂, C(=O)H, OH, C₃-C₆-Cycloalkyl, C(=O)OH, C(=O)NH₂, C₁-C₄-Halogenalkoxy, C₁-C₄-Alkoxy, C₁-C₄- Halogenalkylsulfanyl, C₁-C₄-Halogenalkylsulfinyl, C₁-C₄-Halogenalkylsulfonyl, C₁-C₄-Alkoxycarbonyl, C₁-C₄-Halogenalkoxycarbonyl, C₁-C₄-Alkylcarbonyl, C₁-C₄-Halogenalkylcarbonyl, Di(C₁-C₄)alkylaminocarbonyl, C₁-C₄-alkylaminocarbonyl, C₁-C₄-Alkylcarbonylamino, Di(C₁-C₄)alkylcarbonylamino, C₁-C₄-Alkoxycarbonylamino oder eine Gruppe -C(R^{Z1}) = NOR^{Z2}; Phenyl, das unsubstituiert oder durch einen oder mehrere, gleiche oder verschiedene Substituenten, die aus Halogen, CN, C₁-C₄-Alkyl, C₁-C₄-Halogenalkyl, C₁-C₄-Halogenalkoxy, C₁-C₄-Alkoxy, C₁-C₄-Halogenalkylsulfanyl, C₁-C₄-Halogenalkylsulfinyl, C₁-C₄-Halogenalkylsulfonyl und C (=O) C₁-C₄-Halogenalkyl ausgewählt sind, substituiert ist;
C₁-C₄-Alkyl, das unsubstituiert oder durch ein oder mehrere R^{Z3} substituiert ist;
R^{Z1} und R^{Z2} unabhängig für H, C₁-C₄-Alkyl oder C₁-C₄-Halogenalkyl stehen;
R^{Z3} für Halogen, CN, NH₂, C(=O)H, OH, C₃-C₆-Cycloalkyl, Hydroxycarbonyl, Aminocarbonyl, C₁-C₄-Halogenalkoxy, C₁-C₄-Alkoxy, C₁-C₄-Halogenalkylsulfanyl, C₁-C₄-Halogenalkylsulfinyl, C₁-C₄-Halogenalkylsulfonyl, C₁-C₄-Alkoxycarbonyl, C₁-C₄-Halogenalkoxycarbonyl, C₁-C₄-Alkylcarbonyl, C₁-C₄-Halogenalkylcarbonyl, Di(C₁-C₄)alkylaminocarbonyl, C₁-C₄alkylaminocarbonyl, C₁-C₄-Alkylcarbonyl-amino, Di (C₁-C₄) alkylcarbonylamino, C₁-C₄-Alkoxycarbonylamino, eine Gruppe -C(R^{Z1}) = NOR^{Z2} steht; der Index n für 1, 2, 3 oder 4 steht, wenn G für Phenyl oder ein 6-gliedriges Hetaryl steht;
oder für 1, 2 oder 3 steht, wenn X für ein 5-gliedriges Hetaryl steht; und
der Index m für 0, 1 oder 2 steht;
und die N-Oxide, Stereoisomere, Tautomere und landwirtschaftlich oder veterinärmedizinisch unbedenklichen Salze davon.

2. Verbindung der Formel (I) nach Anspruch 1, wobei
R¹ für H, C₁-C₃-Alkyl oder C₁-C₃-Alkoxy steht, wobei diese Gruppen unsubstituiert oder halogeniert sind,
R² für H, Halogen, C₁-C₃-Alkyl, C₁-C₃-Alkoxy, C₂-C₃-Alkenyl oder C₂-C₃-Alkinyl steht, wobei diese Gruppen unsubstituiert oder halogeniert sind.

3. Verbindung der Formel (I) nach Anspruch 1 oder 2, wobei es sich bei der Verbindung der Formel (I) um eine Verbindung der Formel (I.A), (I.B) oder (I.C) handelt; 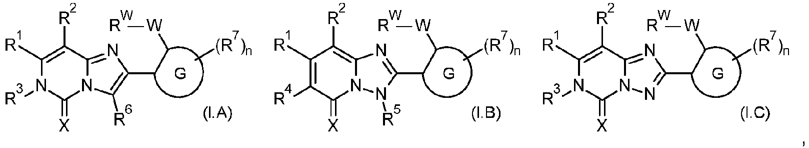 wobei alle Variablen eine wie für Formel (I) definierte Bedeutung haben.

4. Verbindung der Formel (I.A) nach Anspruch 3, wobei
R³ für C₁-C₄-Alkyl, C₂-C₄-Alkenyl, C₂-C₄-Alkinyl, C₁-C₃-Alkoxy-C₁-C₃-alkyl, C₃-C₆-Cycloalkyl, C₃-C₆-Cycloalkyl-C₁-C₂-alkyl steht, wobei die Gruppen unsubstituiert oder halogeniert sind;
R⁶ für H, C₁-C₃-Alkyl oder C₁-C₃-Alkoxy steht.

5. Verbindungen der Formel (I.B) nach Anspruch 3 oder 4, wobei
R⁴ für H, C₁-C₃-Alkyl oder C₁-C₃-Alkoxy steht;
R⁵ für C₁-C₃-Alkyl oder C₁-C₃-Halogenalkyl steht.

6. Verbindungen der Formel (I) nach einem der Ansprüche 1 bis 5, wobei G für Pyridyl steht.

7. Verbindung der Formel (I) nach einem der Ansprüche 1 bis 6, wobei
R^{W} jeweils unabhängig für C₁-C₃-Alkyl oder C₁-C₃-Halogenalkyl steht und
m für 0 oder 2 steht.

8. Verbindung der Formel (I) nach einem der Ansprüche 1 bis 7, wobei der Index n für 1 oder 2 steht.

9. Pestizide Mischung, umfassend die Verbindung der Formel (I) gemäß einem der Ansprüche 1 bis 8, ein N-Oxid oder ein landwirtschaftlich unbedenkliches Salz davon und einen weiteren pestiziden Bestandteil.

10. Verwendung von Verbindungen der Formel (I) gemäß einem der Ansprüche 1 bis 8 als ein agrochemisches Pestizid.

11. Verbindung der Formel (I) gemäß einem der Ansprüche 1 bis 8 oder Zusammensetzung gemäß Anspruch 9 zur Verwendung bei einem Verfahren zum Bekämpfen oder Kontrollieren von wirbellosen Schädlingen, bei dem man den Schädling oder seine Nahrungsquelle, seinen Lebensraum oder seine Brutstätten mit einer pestizid wirksamen Menge mindestens einer Verbindung der Formel (I) nach einem der Ansprüche 1 bis 8 oder der Zusammensetzung nach Anspruch 9 in Berührung bringt.

12. Verfahren zum Schützen von wachsenden Pflanzen vor Angriffen oder Befall durch wirbellose Schädlinge, bei dem man eine Pflanze oder den Boden oder das Wasser, in dem die Pflanze wächst, mit einer pestizid wirksamen Menge mindestens einer Verbindung der Formel (I) nach einem der Ansprüche 1 bis 8 oder der Zusammensetzung nach Anspruch 9 in Berührung bringt.

13. Saatgut, umfassend eine Verbindung der Formel (I) gemäß einem der Ansprüche 1 bis 8 oder der Zusammensetzung gemäß Anspruch 9 in einer Menge von 0,1 g bis 10 kg pro 100 kg Saatgut.

14. Verwendung einer Verbindung der Formel (I) gemäß einem der Ansprüche 1 bis 8 oder der Zusammensetzungen gemäß Anspruch 9 zum Schützen von wachsenden Pflanzen vor Angriff oder Befall durch wirbellose Schädlinge.

15. Verbindung der Formel (I) gemäß einem der Ansprüche 1 bis 8 oder Zusammensetzung gemäß Anspruch 9 zur Verwendung bei einem Verfahren zum Behandeln oder Schützen eines Tiers vor Befall oder Infektion durch wirbellose Schädlinge, bei dem man das Tier mit einer pestizid wirksamen Menge einer Verbindung der Formel (I) gemäß einem der Ansprüche 1 bis 8 oder der Zusammensetzung gemäß Anspruch 9 in Berührung bringt.

## Revendications

1. Composé de formule (I) 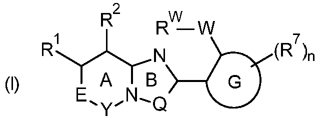 dans laquelle
les cycles A et B sont totalement insaturés ;
Y est C=X, X étant O ou S ;
E est N(R³) ou C(R⁴) ;
Q est N, N(R⁵) ou C(R⁶) ;
G est phényle ou un hétéroaryle de 5 ou 6 chaînons ;
W est S, S(O), S(O)₂ ou S(O)(NR^{W}) ;
R¹ est H, halogène, alkyle en C₁-C₆, alcényle en C₂-C₆, alcynyle en C₂-C₆, alcoxy en C₁-C₆, (alcoxy en C₁-C₆) - (alkyle en C₁-C₆), cycloalkyle en C₃-C₆, cycloalcoxy en C₃-C₆, alkylsulfényle en C₁-C₆, alkylsulfinyle en C₁-C₆ ou alkylsulfonyle en C₁-C₆, lesdits groupes étant non substitués ou halogénés ;
R³, R⁵ sont indépendamment alkyle en C₁-C₆, alcoxy en C₁-C₆, alcényle en C₂-C₆, alcynyle en C₂-C₆, (alcoxy en C₁-C₆) - (alkyle en C₁-C₄), (alcoxy en C₁-C₆) - (alcoxy en C₁-C₄) cycloalkyle en C₃-C₆, (cycloalkyle en C₃-C₆)-(alkyle en C₁-C₄) , ou (cycloalcoxy en C₃-C₆) - (alkyle en C₁-C₄) , qui sont non substitués ou halogénés ;
C(=O)OR^{A}, NR^{B}R^{C}, (alkylène en C₁-C₆) -NR^{B}R^{C}, O- (alkylène en C₁-C₆)-NR^{B}R^{C}, (alkylène en C₁-C₆)-CN, NH-(alkylène en C₁-C₆)-NR^{B}R^{C}, C(=O)NR^{B}R^{C}, C(=O)R^{D}, C(=S)R^{D}, SO₂NR^{B}R^{C}, S(=O)ₘR^{E} ;
phényle ou benzyle, le cycle phényle étant non substitué ou substitué par un ou plusieurs R^{F} ;
R², R⁴, R⁶ sont indépendamment H, halogène, N₃, CN, NO₂, SCN, SF₅, alkyle en C₁-C₆, alcoxy en C₁-C₆, alcényle en C₂-C₆, tri (alkyle en C₁-C₆) silyle, alcynyle en C₂-C₆, (alcoxy en C₁-C₆) - (alkyle en C₁-C₄), (alcoxy en C₁-C₆) - (alcoxy en C₁-C₄) , cycloalkyle en C₃-C₆, cycloalcoxy en C₃-C₆, (cycloalkyle en C₃-C₆) - (alkyle en C₁-C₄), (cycloalcoxy en C₃-C₆) - (alkyle en C₁-C₄), lesdits groupes étant non substitués ou substitués par halogène, C(=O)OR^{A}, NR^{B}R^{C}, NOR^{A}, ONR^{B}R^{C}, (alkylène en C₁-C₆) -NR^{B}R^{C}, O-(alkylène en C₁-C₆) -NR^{B}R^{C}, (alkylène en C₁-C₆) -CN, NH-(alkylène en C₁-C₆)-NR^{B}R^{C}, C(=O)NR^{B}R^{C}, C(=O)R^{D}, C(=S)R^{D}, SO₂NR^{B}R^{C}, S(=O)ₘR^{E} ;
phényle ou benzyle, le cycle phényle étant non substitué ou substitué par un ou plusieurs R^{F} ;
chaque R⁷ est indépendamment H, halogène, OH, CN, NC, NO₂, N₃, SCN, NCS, NCO, SF₅ ;
alkyle en C₁-C₆, cycloalkyle en C₃-C₆, alcényle en C₂-C₆, cycloalcényle en C₃-C₆, alcynyle en C₂-C₆, (cycloalkyle en C₃-C₆) - (alkyle en C₁-C₃), lesdits groupes étant non substitués ou substitués par un ou plusieurs R^{G} ;
un cycle ou système cyclique hétérocyclique saturé, partiellement insaturé ou totalement insaturé de 3 à 12 chaînons, ledit cycle ou système cyclique hétérocyclique comprenant un ou plusieurs hétéroatomes O, N ou S identiques ou différents , et étant non substitué ou substitué par un ou plusieurs R^{H} ;
phényle, qui est non substitué ou substitué par un ou plusieurs R^{J} ;
OR^{K}, SR^{K}, OC(=O)R^{K}, OC(=O)OR^{K}, OC(=O)NR^{L}R^{M}, OC(=O)SR^{K}, OC(=S)NR^{L}R^{M}, OC(=S)SR^{K}, OS(=O)ₘR^{K}, OS (=O)ₘNR^{L}R^{M}, ONR^{L}R^{M}, ON=CR^{N}R^{O}, NR^{L}R^{M}, NOR^{K}, ONR^{L}R^{M}, N=CR^{N}R^{C}, NNR^{L}, N(R^{L})C(=O)R^{K}, N(R^{L})C(=O)OR^{K}, S (=O)ₘR^{V}, SC(=O)SR^{K}, SC (=O) NR^{L}R^{M}, S(=O)ₘNR^{L}R^{M}, C(=O)R^{P}, C(=S)R^{P}, C(=O)NR ^{L} R ^{M}, C(=O)OR^{K}, C(=S)NR^{L}R^{M}, C (=S)OR^{K}, C (=S)SR^{K}, C(=NR^{L})R^{M}, C(=NR^{L})NR^{M}R^{R}, Si(R^{S})₂R^{T} ;
ou deux substituants R⁷ forment, conjointement avec les chaînons de cycle du cycle G auquel ils sont liés, un carbocycle ou hétérocycle saturé, partiellement insaturé ou totalement insaturé de 5 ou 6 chaînons, ledit carbocycle ou hétérocycle étant non substitué ou substitué par un ou plusieurs R^{J}, et ledit hétérocycle comprenant un ou plusieurs hétéroatomes O, N ou S identiques ou différents ; ou
-C(CN)R^{X}R^{Y}, -C(R^{O})=N-NR^{M}R^{N}, -C(R^{O})=N-OR^{L} ou cycloalkyle en C₃-C₆, qui est substitué par CN et qui ne comporte pas d'autres substituants ou est en outre substitué par un ou plusieurs R^{z} ;
dans laquelle au moins un R⁷ est un groupe de formule (S) 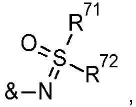
chaque R⁷¹, R⁷² est indépendamment choisi parmi alkyle en C₁-C₆, cycloalkyle en C₃-C₆, alcényle en C₂-C₆, cycloalcényle en C₃-C₆, alcynyle en C₂-C₆, (cycloalkyle en C₃-C₆) - (alkyle en C₁-C₃), lesdits groupes étant non substitués ou substitués par un ou plusieurs R^{G} ;
un cycle ou système cyclique hétérocyclique saturé, partiellement insaturé ou totalement insaturé de 3 à 12 chaînons, ledit cycle ou système cyclique hétérocyclique comprenant un ou plusieurs hétéroatomes O, N ou S identiques ou différents , et étant non substitué ou substitué par un ou plusieurs R^{H} ;
phényle, qui est non substitué ou substitué par un ou plusieurs R^{J} ;
ou deux substituants R⁷¹, R⁷² forment, conjointement avec l'atome de soufre auquel ils sont liés, un hétérocycle saturé, partiellement insaturé ou totalement insaturé de 4, 5 ou 6 chaînons, ledit hétérocycle étant non substitué ou substitué par un ou plusieurs R^{J}, et ledit hétérocycle comprenant aucun, un ou plusieurs hétéroatomes O, N ou S identiques ou différents en plus de l'atome de soufre auquel R⁷¹ et R⁷² sont liés ;
chaque R^{A} est indépendamment H, alkyle en C₁-C₆, alcényle en C₂-C₆, alcynyle en C₂-C₆, (alcoxy en C₁-C₆) - (alkyle en C₁-C₄), cycloalkyle en C₃-C₆, (cycloalkyle en C₃-C₆)-(alkyle en C₁-C₄), (cycloalcoxy en C₃-C₆)-(alkyle en C₁-C₄), lesdits groupes étant non substitués ou substitués par halogène ;
phényle ou benzyle, le cycle phényle étant non substitué ou substitué par un ou plusieurs R^{z} ;
chaque R^{B} est indépendamment H, alkyle en C₁-C₆, alcényle en C₂-C₆, alcynyle en C₂-C₆, (alcoxy en C₁-C₆) - (alkyle en C₁-C₄), cycloalkyle en C₃-C₆, (cycloalkyle en C₃-C₆)-(alkyle en C₁-C₄), (cycloalcoxy en C₃-C₆)-(alkyle en C₁-C₄), (alkyle en C₁-C₆) carbonyle, (alcoxy en C₁-C₆) carbonyle, lesdits groupes étant non substitués ou substitués par halogène ;
phényle et benzyle, lesdits groupes étant non substitués ou substitués par un ou plusieurs R^{F} ;
chaque R^{C} est indépendamment H, alkyle en C₁-C₆, alcényle en C₂-C₆, alcynyle en C₂-C₆, (alcoxy en C₁-C₆) - (alkyle en C₁-C₄), cycloalkyle en C₃-C₆, (cycloalkyle en C₃-C₆) - (alkyle en C₁-C₄), (cycloalcoxy en C₃-C₆)-(alkyle en C₁-C₄), (alkyle en C₁-C₆) carbonyle, (alcoxy en C₁-C₆) carbonyle, lesdits groupes étant non substitués ou substitués par halogène ;
phényle ou benzyle, le cycle phényle étant non substitué ou substitué par un ou plusieurs R^{F} ;
chaque fragment NR^{B}R^{C} pouvant également former un hétérocycle de 5 à 8 chaînons saturé, lié à N qui, en plus de l'atome d'azote, peut comporter 1 ou 2 hétéroatomes ou fragments hétéroatomiques supplémentaires choisis parmi O, S(=O)ₘ et N-R', R' étant H ou alkyle en C₁-C₆ et l'hétérocycle lié à N étant non substitué ou substitué par un ou plusieurs, substituants identiques ou différents choisis parmi halogène, alkyle en C₁-C₄, halogénoalkyle en C₁-C₄, alcoxy en C₁-C₄ et halogénoalcoxy en C₁-C₄ ;
chaque R^{D} est indépendamment H, CN, OH, alkyle en C₁-C₆, alcényle en C₂-C₆, alcynyle en C₂-C₆, (alcoxy en C₁-C₆) - (alkyle en C₁-C₄), cycloalkyle en C₃-C₆, (cycloalkyle en C₃-C₆) - (alkyle en C₁-C₄), (cycloalcoxy en C₃-C₆) - (alkyle en C₁-C₄), lesdits groupes étant non substitués ou substitués par halogène ;
phényle ou benzyle, le cycle phényle étant non substitué ou substitué par un ou plusieurs R^{F} ;
chaque R^{E} est indépendamment alkyle en C₁-C₆, cycloalkyle en C₃-C₆, (cycloalkyle en C₃-C₆) - (alkyle en C₁-C₄), qui sont non substitués ou substitués par halogène ; ou phényle ou benzyle, le cycle phényle étant non substitué ou substitué par un ou plusieurs R^{F} ;
chaque R^{F} est indépendamment halogène, N₃, OH, CN, NO₂, SCN, SF₅, alkyle en C₁-C₆, alcoxy en C₁-C₆, alcényle en C₂-C₆, alcynyle en C₂-C₆, (alcoxy en C₁-C₆) - (alkyle en C₁-C₄), (alcoxy en C₁-C₆) -(alcoxy en C₁-C₄), cycloalkyle en C₃-C₆, cycloalcoxy en C₃-C₆, (cycloalkyle en C₃-C₆) -(alkyle en C₁-C₄), (cycloalcoxy en C₃-C₆) -(alkyle en C₁-C₄), lesdits groupes étant non substitués ou substitués par halogène ;
chaque R^{G} est indépendamment halogène, OH, CN, NC, NO₂ ; alkyle en C₁-C₆, cycloalkyle en C₃-C₆, cycloalcényle en C₃-C₆, lesdits groupes étant non substitués ou substitués par un ou plusieurs, substituants identiques ou différents choisis parmi halogène, OH, CN, alcoxy en C₁-C₃, halogénoalcoxy en C₁-C₃ et (alkyle en C₁-C₃) carbonyle ; un cycle ou système cyclique hétérocyclique saturé, partiellement insaturé ou totalement insaturé de 3 à 12 chaînons, ledit cycle ou système cyclique hétérocyclique comprenant un ou plusieurs hétéroatomes O, N ou S identiques ou différents, et étant non substitué ou substitué par un ou plusieurs substituants identiques ou différents choisis parmi halogène, OH, CN, alcoxy en C₁-C₃, halogénoalcoxy en C₁-C₃ et (alkyle en C₁-C₃) carbonyle ;
phényle, qui est non substitué ou substitué par un ou plusieurs, substituants identiques ou différents choisis parmi halogène, OH, CN, NO₂, alkyle en C₁-C₃, halogénoalkyle en C₁-C₃, alcoxy en C₁-C₃, halogénoalcoxy en C₁-C₃ et (alkyle en C₁-C₃) carbonyle ;
OR^{K}, SR^{K}, OC(=O)R^{K}, OC (=O)OR^{K}, OC (=O)NR^{L}R^{M}, OC (=O)SR^{K}, OC(=S)NR^{L}R^{M}, OC(=S)SR^{K}, OS(=O)ₘR^{K}, OS(=O)ₘNR^{L}R^{M}, ONR^{L}R^{M}, ON=CR^{N}R^{O}, NR^{L}R^{M}, NOR^{K}, ONR^{L}R^{M}, N=CR^{N}R^{O}, NNR^{L}, N(R^{L})C(=O)R^{K}, N(R^{L})C(=O)OR^{K}, S(=O)ₘR^{V}, SC(=O)SR^{K}, SC(=O)NR^{L}R^{M}, S(=O)ₘNR^{L}R^{M}, C(=O)R^{P}, C(=S)R^{P}, C(=O)NR^{L}R^{M}, C(=O)OR^{K}, C(=S)NR^{L}R^{M}, C(=S)OR^{K}, C(=S)SR^{K}, C(=NR^{L})R^{M}, C(=NR^{L})NR^{M}R^{R} , Si(R^{S})₂R^{T};
chaque R^{H} est indépendamment halogène, CN, NC, NO₂, SCN, NCS, NCO ;
alkyle en C₁-C₆, cycloalkyle en C₃-C₆, cycloalcényle en C₃-C₆, lesdits groupes étant non substitués ou substitués par un ou plusieurs, substituants identiques ou différents choisis parmi halogène, OH, CN, alcoxy en C₁-C₃, halogénoalcoxy en C₁-C₃ et (alkyle en C₁-C₃) carbonyle ; phényle, qui est non substitué ou substitué par un ou plusieurs, substituants identiques ou différents choisis parmi halogène, OH, CN, NO₂, alkyle en C₁-C₃, halogénoalkyle en C₁-C₃, OR^{K}, SR^{K}, OC(=O)R^{K}, OC(=O)OR^{K}, OC(=O)NR^{L}R^{M}, OC(=O)SR^{K}, OC(=S)NR^{L}R^{M}, OC(=S)SR^{K}, OS(=O)ₘR^{K}, OS(=O)ₘNR^{L}R^{M}, ONR^{L}R^{M}, ON=CR^{N}R^{O}, NR^{L}R^{M}, NOR^{K}, ONR^{L}R^{M}, N=CR^{N}R^{O}, NNR^{L}, N(R^{L})C (=O) R^{K}, N(R^{L})C (=O)OR^{K}, S(=O)ₘR^{V}, SC(=O)SR^{K}, SC(=O)NR^{L}R^{M}, S(=O)ₘNR^{L}R^{M}, C(=O)R^{P}, C(=S)R^{P}, C(=O)NR^{L}R^{M}, C(=O)OR^{K}, C(=S)NR^{L}R^{M}, C(=S)OR^{K}, C(=S)SR^{K}, C(=NR^{L})R^{M}, C(=NR^{L})NR^{M}R^{R}, Si(R^{S})₂R^{T} ; ou
deux substituants R^{H} géminaux forment, conjointement avec l'atome auquel ils sont liés, un groupe =O, =S ou =NR^{L}. chaque R^{J} est indépendamment halogène, CN, NC, NO₂, SCN, NCS, NCO ;
alkyle en C₁-C₆, cycloalkyle en C₃-C₆, cycloalcényle en C₃-C₆, lesdits groupes étant non substitués ou substitués par un ou plusieurs, substituants identiques ou différents choisis parmi halogène, OH, CN, alcoxy en C₁-C₃, halogénoalcoxy en C₁-C₃ et (alkyle en C₁-C₃) carbonyle ; phényle, qui est non substitué ou substitué par un ou plusieurs, substituants identiques ou différents choisis parmi halogène, OH, CN, NO₂, alkyle en C₁-C₃, halogénoalkyle en C₁-C₃, OR^{K}, SR^{K}, OC (=O) R^{K}, OC (=O) OR^{K}, OC (=O) NR^{L}R^{M}, OC(=O)SR^{K}, OC(=S)NR^{L}R^{M}, OC(=S)SR^{K}, OS (=O)ₘR^{K}, OS(=O)ₘNR^{L}R^{M}, ONR^{L}R^{M}, ON=CR^{N}R^{O}, NR^{L}R^{M}, NOR^{K}, ONR^{L}R^{M}, N=CR^{N}R^{O}, NNR^{L}, N(R^{L})C (=O) R^{K}, N(R^{L})C (=O) OR^{K}, S(=O)ₘR^{V}, SC(=O)SR^{K}, SC(=O)NR^{L}R^{M}, S(=O)ₘNR^{L}R^{M}, C(=O)R^{P}, C(=S)R^{P}, C(=O)NR^{L}R^{M}, C(=O)OR^{K}, C(=S) NR^{L}R^{M}, C(=S)OR^{K}, C(=S)SR^{K}, C(=NR^{L})R^{M}, C(=NR^{L})NR^{M}R^{R}, Si(R^{S})₂R^{T} ;
chaque R^{K} est indépendamment H ;
alkyle en C₁-C₆, alcényle en C₂-C₆, alcynyle en C₂-C₆, (alcoxy en C₁-C₆) - (alkyle en C₁-C₄), cycloalkyle en C₃-C₆, (cycloalkyle en C₃-C₆) - (alkyle en C₁-C₄), (cycloalcoxy en C₃-C₆) - (alkyle en C₁-C₄), lesdits groupes étant non substitués ou substitués par un ou plusieurs substituants identiques ou différents choisis parmi halogène, CN, NR^{L}R^{M} ; C (=O) NR^{L}R^{M}, C(=O)R^{T} ; ou phényle ou benzyle, le cycle phényle étant non substitué ou substitué par un ou plusieurs R^{F} ;
chaque R^{L}, R^{R} est indépendamment H ;
alkyle en C₁-C₆, alcényle en C₂-C₆, alcynyle en C₂-C₆, (alcoxy en C₁-C₆) - (alkyle en C₁-C₄), cycloalkyle en C₃-C₆, (cycloalkyle en C₃-C₆) - (alkyle en C₁-C₄), (cycloalcoxy en C₃-C₆) - (alkyle en C₁-C₄), lesdits groupes étant non substitués ou substitués par halogène ;
phényle et benzyle, lesdits groupes étant non substitués ou substitués par un ou plusieurs R^{F} ;
chaque R^{M} est indépendamment H, alkyle en C₁-C₆, alcényle en C₂-C₆, alcynyle en C₂-C₆, (alcoxy en C₁-C₆) - (alkyle en C₁-C₄), cycloalkyle en C₃-C₆, (cycloalkyle en C₃-C₆) - (alkyle en C₁-C₄), (cycloalcoxy en C₃-C₆)-(alkyle en C₁-C₄), (alkyle en C₁-C₆) carbonyle, (alcoxy en C₁-C₆) carbonyle, lesdits groupes étant non substitués ou substitués par halogène ;
(alkylène en C₁-C₆)-CN ;
phényle et benzyle, lesdits groupes étant non substitués ou substitués par un ou plusieurs R^{F} ;
chaque R^{N} est indépendamment H, alkyle en C₁-C₆, alcényle en C₂-C₆, alcynyle en C₂-C₆, (alcoxy en C₁-C₆) -(alkyle en C₁-C₄), cycloalkyle en C₃-C₆, (cycloalkyle en C₃-C₆) - (alkyle en C₁-C₄), (cycloalcoxy en C₃-C₆)-(alkyle en C₁-C₄), (alkyle en C₁-C₆) carbonyle, (alcoxy en C₁-C₆)carbonyle lesdits groupes étant non substitués ou substitués par halogène ;
phényle ou benzyle, le cycle phényle étant non substitué ou substitué par un ou plusieurs R^{F} ;
chaque fragment NR^{M}R^{N}, R^{M}R^{R} et R^{L}R^{M} pouvant également former un hétérocycle de 5 à 8 chaînons saturé, lié à N qui, en plus de l'atome d'azote, peut comporter 1 ou 2 hétéroatomes ou fragments hétéroatomiques supplémentaires choisis parmi O, S(=O)ₘ et N-R", R" étant H ou alkyle en C₁-C₆ et hétérocycle lié à N étant non substitué ou substitué par un ou plusieurs substituants identiques ou différents choisis parmi halogène, alkyle en C₁-C₄, halogénoalkyle en C₁-C₄, alcoxy en C₁-C₄ et halogénoalcoxy en C₁-C₄ ;
chaque R^{O} est indépendamment H, CN, OH, alkyle en C₁-C₆, alcényle en C₂-C₆, alcynyle en C₂-C₆, (alcoxy en C₁-C₆)-(alkyle en C₁-C₄), cycloalkyle en C₃-C₆, (cycloalkyle en C₃-C₆) - (alkyle en C₁-C₄), (cycloalcoxy en C₃-C₆) - (alkyle en C₁-C₄), lesdits groupes étant non substitués ou substitués par halogène ;
phényle ou benzyle, le cycle phényle étant non substitué ou substitué par un ou plusieurs R^{F} ;
chaque R^{P} est indépendamment H ;
alkyle en C₁-C₆, alcényle en C₂-C₆, alcynyle en C₂-C₆, (alcoxy en C₁-C₆)-(alkyle en C₁-C₄), cycloalkyle en C₃-C₆, (cycloalkyle en C₃-C₆) -(alkyle en C₁-C₄), (cycloalcoxy en C₃-C₆) - (alkyle en C₁-C₄), lesdits groupes étant non substitués ou substitués par halogène ;
phényle ou benzyle, le cycle phényle étant non substitué ou substitué par un ou plusieurs R^{F} ;
chaque R^{S}, R^{T} est indépendamment H, alkyle en C₁-C₆, halogénoalkyle en C₁-C₆, alcoxy en C₁-C₆, (alcoxy en C₁-C₄) - (alkyle en C₁-C₄), cycloalkyle en C₃-C₆, halogénocycloalkyle en C₃-C₆, (halogénoalcoxy en C₁-C₄)-(alkyle en C₁-C₄) ou phényle ;
chaque R^{V} est indépendamment alkyle en C₁-C₆, cycloalkyle en C₃-C₆, (cycloalkyle en C₃-C₆) - (alkyle en C₁-C₄), qui sont non substitués ou substitués par halogène ; ou phényle ou benzyle, le cycle phényle étant non substitué ou substitué par R^{F} ;
chaque R^{W} est indépendamment H, alkyle en C₁-C₆, cycloalkyle en C₃-C₆, (cycloalkyle en C₃-C₆) -(alkyle en C₁-C₄), lesdits groupes étant halogénés ou non halogénés ; benzyle ou phényle, qui est non substitué ou substitué par R^{F} ;
chaque R^{X}, R^{Y} est indépendamment H, halogène, CN, alkyle en C₁-C₆, halogénoalkyle en C₁-C₆, cycloalkyle en C₃-C₆, alcoxy en C₁-C₄, (alcoxy en C₁-C₄)- (alkyle en C₁-C₄), (alkyle en C₁-C₄)sulfanyle, (alkylsulfanyle en C₁-C₄)-(alkyle en C₁-C₄), (alkylsulfinyle en C₁-C₄)- (alkyle en C₁-C₄), (alkylsulfonyle en C₁-C₄)-(alkyle en C₁-C₄)ou (alcoxy en C₁-C₄)carbonyle ;
chaque R^{Z} est halogène, CN, NH₂, C(=O)H, OH, cycloalkyle en C₃-C₆, C(=O)OH, C(=O)NH₂, halogénoalcoxy en C₁-C₄, alcoxy en C₁-C₄, halogénoalkylsulfanyle en C₁-C₄, halogénoalkylsulfinyle en C₁-C₄, halogénoalkylsulfonyle en C₁-C₄, (alcoxy en C₁-C₄)carbonyle, (halogénoalcoxy en C₁-C₄)carbonyle, (alkyle en C₁-C₄)carbonyle, halogénoalkyle en C₁-C₄)carbonyle, di(alkyle en C₁-C₄) aminocarbonyle, (alkyle en C₁-C₄)aminocarbonyle, (alkyle en C₁-C₄)carbonylamino, di(alkyle en C₁-C₄) carbonylamino, (alcoxy en C₁-C₄)carbonylamino ou un groupe -C(R^{Z1})=NOR^{Z2} ;
phényle, qui est non substitué ou substitué par un ou plusieurs substituants identiques ou différents choisis parmi halogène, CN, alkyle en C₁-C₄, halogénoalkyle en C₁-C₄, halogénoalcoxy en C₁-C₄, alcoxy en C₁-C₄, halogénoalkylsulfanyle en C₁-C₄, halogénoalkylsulfinyle en C₁-C₄, halogénoalkylsulfonyle en C₁-C₄ et C(=O) (halogénoalkyle en C₁-C₄);
alkyle en C₁-C₄ qui est non substitué ou substitué par un ou plusieurs R^{Z3} ;
R^{Z1} et R^{Z2} sont indépendamment H, alkyle en C₁-C₄ ou halogénoalkyle en C₁-C₄ ;
chaque R^{Z3} est halogène, CN, NH₂, C(=O)H, OH, cycloalkyle en C₃-C₆, hydroxycarbonyle, aminocarbonyle, halogénoalcoxy en C₁-C₄, alcoxy en C₁-C₄, halogénoalkylsulfanyle en C₁-C₄, halogénoalkylsulfinyle en C₁-C₄, halogénoalkylsulfonyle en C₁-C₄, (alcoxy en C₁-C₄) carbonyle, (halogénoalcoxy en C₁-C₄)carbonyle, (alkyle en C₁-C₄) carbonyle, halogénoalkyle en C₁-C₄) carbonyle, di(alkyle en C₁-C₄)aminocarbonyle, (alkyle en C₁-C₄) aminocarbonyle, (alkyle en C₁-C₄)carbonylamino, di(alkyle en C₁-C₄)carbonylamino, (alcoxy en C₁-C₄) carbonylamino ou un groupe -C(R^{Z1})=NOR^{Z2} ;
l'indice n est 1, 2, 3 ou 4 si G est phényle ou un hétéroaryle à 6 chaînons ;
ou 1, 2 ou 3 si X est un hétéroaryle à 5 chaînons ; et l'indice m est 0, 1 ou 2 ;
et les N-oxydes, stéréoisomères, tautomères et sels acceptables en agriculture ou en médecine vétérinaire de celui-ci.

2. Composé de formule (I) selon la revendication 1,
R¹ est H, alkyle en C₁-C₃ ou alcoxy en C₁-C₃, lesdits groupes étant non substitués ou halogénés ;
R² est H, halogène, alkyle en C₁-C₃ ou alcoxy en C₁-C₃, alcényle en C₂-C₃ ou alcynyle en C₂-C₃, lesdits groupes étant non substitués ou halogénés.

3. Composé de formule (I) selon la revendication 1 ou 2, le composé de formule (I) étant un composé de formule (I.A), (I.B) ou (I.C) ; 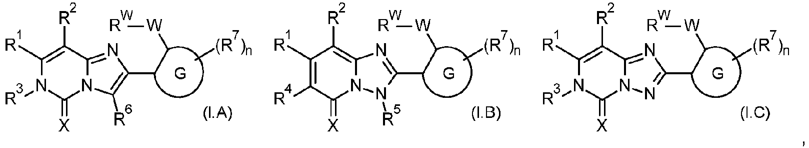 dans lesquelles toutes les variables ont une signification telle que définie pour la formule (I).

4. Composé de formule (I.A) selon la revendication 3, dans lequel
R³ est alkyle en C₁-C₄, alcényle en C₂-C₄, alcynyle en C₂-C₄, (alcoxy en C₁-C₃)- (alkyle en C₁-C₃) , cycloalkyle en C₃-C₆, (cycloalkyle en C₃-C₆) - (alkyle en C₁-C₂) , lesdits groupes étant non substitués ou halogénés ;
R⁶ est H, alkyle en C₁-C₃ ou halogénoalkyle en C₁-C₃.

5. Composé de formule (I.B) selon la revendication 3 ou 4, dans lequel
R⁴ est H ou alkyle en C₁-C₃ ou halogénoalkyle en C₁-C₃.
R⁵ est alkyle en C₁-C₃ ou halogénoalkyle en C₁-C₃.

6. Composé de formule (I) selon l'une quelconque des revendications 1 à 5, dans lequel G est pyridyle.

7. Composé de formule (I) selon l'une quelconque des revendications 1 à 6, dans lequel
chaque R^{W} est indépendamment alkyle en C₁-C₃ ou halogénoalkyle en C₁-C₃, et
m est 0 ou 2.

8. Composé de formule (I) selon l'une quelconque des revendications 1 à 7, dans lequel l'indice n est 1 ou 2.

9. Mélange pesticide comprenant le composé de formule (I), tel que défini dans l'une quelconque des revendications 1 à 8, un N-oxyde, ou un sel acceptable sur le plan agricole de celui-ci et un composant pesticide supplémentaire.

10. Utilisation de composés de formule (I) tels que définis dans l'une quelconque des revendications 1 à 8 en tant que pesticide agrochimique.

11. Composé de formule (I) tel que défini dans l'une quelconque des revendications 1 à 8, ou composition telle que définie dans la revendication 9, destiné à être utilisé dans un procédé de lutte par l'intermédiaire de contrôle contre des organismes nuisibles invertébrés, ledit procédé comprenant la mise en contact dudit organisme nuisible ou de son alimentation, son habitat ou ses lieux de reproduction avec une quantité efficace sur le plan pesticide d'au moins un composé de formule (I) selon l'une quelconque des revendications 1 à 8 ou de la composition selon la revendication 9.

12. Procédé de protection de végétaux en croissance contre une attaque ou une infestation par des organismes nuisibles invertébrés, ledit procédé comprenant la mise en contact d'un végétal, ou du sol ou de l'eau dans lequel/laquelle le végétal pousse, avec une quantité efficace sur le plan pesticide d'au moins un composé de la formule (I) selon l'une quelconque des revendications 1 à 8 ou de la composition selon la revendication 9.

13. Graine comprenant un composé de formule (I), tel que défini dans l'une quelconque des revendications 1 à 8, ou la composition telle que définie dans la revendication 9, à raison de 0,1 g à 10 kg pour 100 kg de graines.

14. Utilisation d'un composé de formule (I), tel que défini dans l'une quelconque des revendications 1 à 8, ou des compositions telles que définies dans la revendication 9, pour protéger des végétaux en croissance contre une attaque ou une infestation par des organismes nuisibles invertébrés.

15. Composé de formule (I) tel que défini dans l'une quelconque des revendications 1 à 8, ou composition telle que définie dans la revendication 9, destiné à être utilisé dans un procédé de traitement ou de protection d'un animal contre une infestation ou une infection par des organismes nuisibles invertébrés qui comprend la mise en contact de l'animal avec une quantité efficace sur le plan pesticide d'un composé de formule (I) tel que défini dans l'une quelconque des revendications 1 à 8, ou d'une composition telle que définie dans la revendication 9.
